# EUROPEAN PATENT APPLICATION

(11) **EP 4 506 344 A1**
(43) Date of publication of application: **12.02.2025**
(21) Application number: 23778186.9
(22) Date of filing: 28.03.2023
(51) Int. Cl.: C07D 401/14, C07D 401/04, C07D 231/38, A61K 31/454, A61P 35/00

(54) **1,4-DIHETEROCYCLIC SUBSTITUTED AROMATIC RING OR AROMATIC HETEROCYCLIC COMPOUND AND USE THEREOF**

(30) Priority: 31.03.2022 CN 202210344552
(71) Applicant: Bebetter Med Inc., Guangzhou, Guangdong 510660 (CN)
(72) Inventor: CAI, Xiong, Guangzhou, Guangdong 510660 (CN); QING, Yuanhui, Guangzhou, Guangdong 510660 (CN); HE, Qijie, Guangzhou, Guangdong 510660 (CN); LIU, Yiting, Guangzhou, Guangdong 510660 (CN); WU, Shaobin, Guangzhou, Guangdong 510660 (CN); HE, Yaonan, Guangzhou, Guangdong 510660 (CN); TAN, Huichen, Guangzhou, Guangdong 510660 (CN); WENG, Yunwo, Guangzhou, Guangdong 510660 (CN); LIU, Bin, Guangzhou, Guangdong 510660 (CN); LIN, Mingsheng, Guangzhou, Guangdong 510660 (CN); DENG, Xinlan, Guangzhou, Guangdong 510660 (CN); ZHU, Junling, Guangzhou, Guangdong 510660 (CN); FENG, Qiao, Guangzhou, Guangdong 510660 (CN); QIAN, Changgeng, Guangzhou, Guangdong 510660 (CN)
(74) Representative: Fuchs Patentanwälte Partnerschaft mbB
(86) International application number: PCT/CN2023/084352
(87) International publication number: WO 2023/185821

(57) **Abstract**

The present invention disclosed a 1,4-diheterocyclic substituted aromatic ring or aromatic heterocyclic compound represented by formula (I). The compound can effectively inhibit the generation of 20-HETE, and has high activity, high selectivity and good pharmacokinetic characteristics. By means of the inhibition on the generation of 20-HETE, the compound can be used for treating various diseases related to 20-HETE, and thus has a great application value.

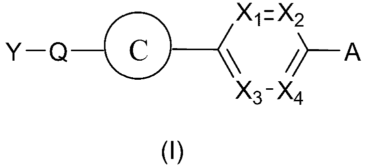

## Description

The present application is a national phase application of PCT/CN2023/084352 filed on March 28, 2023, which claims priority of Chinese patent application no. 2022103445529 filed on March 31, 2022, entitled "1,4-DIHETEROCYCLIC SUBSTITUTED AROMATIC RING OR AROMATIC HETEROCYCLIC COMPOUND AND USE THEREOF". The entirety of the above-mentioned patent application is hereby incorporated by reference herein and made a part of this specification.

### BACKGROUND

### Technical Field

The present disclosure relates to the field of chemical and pharmaceutical technology, in particular to a 1,4-diheterocyclic substituted aromatic ring or aromatic heterocyclic compound and the use thereof.

### Description of Related Art

20-hydroxyeicosatetraenoic acid (20-HETE) is a metabolite of arachidonic acid produced by cytochrome P450 (CYP) 4A and 4F family enzymes. In mice, the main enzyme that produces 20-HETE is Cyp4a12; while in rats, the enzymes that produce 20-HETE are Cyp4a1, Cyp4a2, and Cyp4a3. The main enzymes that produce 20-HETE in human body are CYP4A11 and CYP4F2 (PNAS 2017, 114-3181-3185; Onco Targets Ther. 2013; 6: 243-255; Hypertension. 2008; 51:1393-1398).

20-HETE can promote vascular smooth muscle contraction, endothelial dysfunction, inflammation, and cell proliferation (Curr Opin Nephrol Hypertens 26:74-82, 2017). Multiple studies have shown that the expression levels of 20-HETE were significantly increased in various diseases such as hypertension, stroke, coronary artery disease, myocardial infarction, acute renal failure, chronic kidney disease, polycystic kidney disease, tumor growth and metastasis, terminal organ injury and fibrosis (J Lipid Res 54: 786-793, 2013., Pharmacol Ther 125: 446-463, 2010.).

20-HETE also plays an important role in the occurrence and progression of obesity, insulin resistance, and metabolic syndrome. Preclinical animal models showed that 20-HETE level was associated with weight gain and metabolic syndrome (including hyperglycemia, diabetes, and diabetes related retinopathy and nephropathy). (Am J Physiol Endocrinol Metab 302(5):E500-9, 2012., Am J Physiol Regul Integr Comp Physiol 315(5): R934-r44, 2018.) Clinical studies have shown that 20-HETE levels in urine and plasma were associated with body mass index (BMI) and increased in obese, diabetes and metabolic syndrome patients (Other Lipid Mediat 100-101:15-21, 2013. Prostaglandins Other Lipid Mediat 123: 68-77, 2016. Free Radic Biol Med 46(2):263-70, 2009.).

In 2017, Garcia et al. discovered that GPR75 is a 20-HETE receptor and reported the proximal signaling mechanism by which 20-HETE induced hypertension and vascular dysfunction (Circ Res 2017; 120:1776-1788). By sequencing nearly 650000 people and identifying individuals with rare protective mutations, it was found that individuals with at least one inactive copy of the G protein coupled receptor 75 (GPR75) gene had a lower body mass index (BMI), tended to weigh about 12 pounds less than those without the mutation, reducing the risk of obesity by 54%, as well as showing improvement in diabetes parameters, including glucose reduction.

In a high-fat diet mice model, knocking out the GPR75 gene in mice resulted in resistance to weight gain and improved blood glucose control. Inhibiting GPR75 may provide a therapeutic strategy for obesity (Science 373, eabf8683, 2021.) .

Since the 20-HETE/GPR75 pathway plays an important role in the occurrence and development of the above diseases, 20-HETE ligand generation inhibitors or GPR75 receptor antagonists may provide options for the treatment of obesity, diabetes and other metabolic diseases. HET0016 is a highly active and selective inhibitor of 20-HETE, with an inhibitory activity of 8.9 nM, and it has no effect on the activity of other enzymes related to arachidonic acid metabolism, such as cyclooxygenase and lipoxygenase. However, due to its poor solubility and short half-life, it has not entered clinical development (Drug Metabolism & Disposition 36: 2324-2330, 2008). In recent years, 20-SOLA, which is more water-soluble, has been used as a 20-HETE antagonist as a tool drug in animal research for the treatment of related diseases. 20-SOLA can restore blood pressure to normal in Cyp4a14-/-hypertensive mice (J Pharmacol Exp Ther 363: 412-418, 2017). At the same time, 20-SOLA can reduce the weight gain induced by a high-fat diet, and improve hyperglycemia and hyperinsulinemia in Cyp4a14-/- mice (Am J Physiol Regul Integral Comp Physiol. 315: 934-944, 2018).

### Summary of the Disclosure

Since 20-HETE is related to the occurrence and development of various diseases, and there are no effective 20-HETE inhibitors in clinical trials yet, the present disclosure provides a class of 1,4-diheterocyclic substituted aromatic ring or aromatic heterocyclic compound, which can effectively inhibit the generation of 20-HETE with high activity, high selectivity, and good pharmacokinetic properties. By inhibiting the generation of 20-HETE, it can be used to treat various diseases related to 20-HETE.

The present disclosure includes the following technical solutions.

A 1,4-diheterocyclic substituted aromatic ring or aromatic heterocyclic compound with a structure shown in formula (I) or its pharmaceutically acceptable salts or stereoisomers 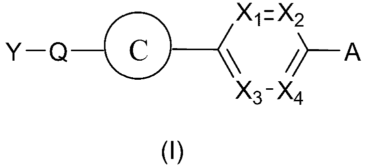
wherein, X₁, X₂, X₃, and X₄ are independently selected from: N, CH, and CR₁;
each R₁ is independently selected from: halogen, C₁-C₆ alkyl, C₂-C₆ alkenyl, C₂-C₆ alkynyl, C₃-C₈ cycloalkyl, C₃-C₈ cycloalkyl methyl, halogen-substituted C₁-C₆ alkyl, hydroxyl-substituted C₁-C₆ alkyl, C₁-C₆ alkoxy-substituted C₁-C₆ alkyl, amino-substituted C₁-C₆ alkyl, C₁-C₆ alkylamine-substituted C₁-C₆ alkyl, C₆-C₁₀ aryl, 5-10 membered heteroaryl, nitro, cyano, -OR, -N(R)₂, -SR, -C(O)OR, -C(O)N(R)₂, -C(O)R, -S(O)R, -S(O)₂R, -S(O)₂N(R)₂, -N(R)C(O)R;
Q is selected from: -(CR₂R₃)ₚ-, -C(O)-, -C(S)-, -S(O)-, -S(O)₂-, -C(O)O-, -N(R₄)C(O)-, -C(O)N(R₄)-, -C(O)N(R₄)(CR₂R₃)-, -N(R₄)C(O)(CR₂R₃)-, -C(O)(CR₂R₃)-, -O-, -N(R₄)-, -S-, wherein, P is selected from: 0, 1, or 2; R₂, R₃, and R₄ are independently selected from: H, C₁-C₆ alkyl ;
A is selected from: substituted or unsubstituted 5-10 membered heterocyclic, substituted or unsubstituted 5-10 membered heteroaryl;
Y is selected from: C₁-C₁₂ alkyl, C₂-C₈ alkenyl, C₂-C₈ alkynyl, C₃-C₈ cycloalkyl methyl, halogen-substituted C₁-C₁₂ alkyl, hydroxyl-substituted C₁-C₁₂ alkyl, C₁-C₆ alkoxy-substituted C₁-C₁₂ alkyl, amino substituted C₁-C₁₂ alkyl, C₁-C₆ alkylamine-substituted C₁-C₁₂ alkyl, C6-C10 aryl-substituted C₁-C₁₂ alkyl, 5-10 membered heteroaryl-substituted C₁-C₁₂ alkyl, acetylene-substituted C₁-C₁₂ alkyl, ethylene-substituted C₁-C₁₂ alkyl, 5-10-membered heteroaryl, - C(O)R, - C(O)OR; or Y is selected from: substituted or unsubstituted 4-12-membered single or double rings, wherein the single or double ring is saturated, partially unsaturated, or aromatic single ring, fused ring, bridged ring, or spiro ring, and the atoms on the ring of the single or double ring are one or more chemically acceptable combinations selected from C, O, N, and S;
each R is independently selected from: H, C₁-C₆ alkyl, C₂-C₆ alkenyl, C₂-C₆ alkynyl, C₃-C₈ cycloalkyl, C₃-C₈ cycloalkyl methyl, halogen-substituted C₁-C₆ alkyl, hydroxyl-substituted C₁-C₆ alkyl, C₁-C₆ alkoxy-substituted C₁-C₆ alkyl, amino-substituted C₁-C₆ alkyl, C₁-C₆ alkylamine-substituted C₁-C₆ alkyl, C₆-C₁₀ aryl;

Ring C is selected from: substituted or unsubstituted 4-10-membered heterocyclyl, substituted or unsubstituted 5-10-membered heteroaryl.

In some embodiments, Ring C is selected from: substituted or unsubstituted 4-10 membered heterocyclyl , and the heteroatoms in the heterocyclyl are 1-2 nitrogen atoms; the substituents in the heterocyclyl are selected from: H, halogen, C₁-C₆ alkyl, halogen-substituted C₁-C₆ alkyl, C₁-C₆ alkoxy-substituted C₁-C₆ alkyl, cyano, hydroxyl; or two substituents together with their linked C form substituted or unsubstituted 4-8-membered carbon rings or 4-8-membered heterocycles.

In some embodiments, Ring C is selected from: 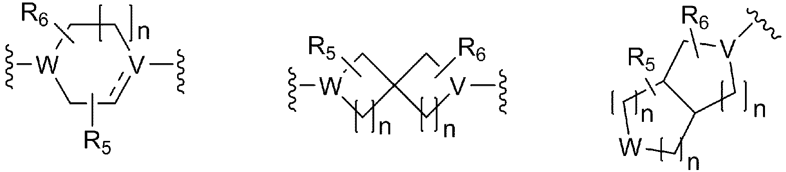 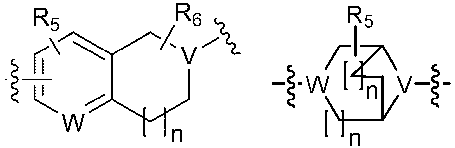 wherein,
the dashed line in the rings containing W and V indicates that it is a single bond or there is none;
each R₅ and R₆ are independently selected from: H, halogen, C₁-C₆ alkyl, halogen-substituted C₁-C₆ alkyl, C₁-C₆ alkoxy-substituted C₁-C₆ alkyl, cyano, hydroxyl; or R₅, R₆ together with their linked C atoms form substituted or unsubstituted 4-8-membered carbon rings or 4-8-membered heterocycles;
each n is independently selected from: 0, 1, 2;
V and W are independently selected from: CH, N, and furthermore V and W cannot be both CH.

In some embodiments, Ring C together with Q form the following groups:

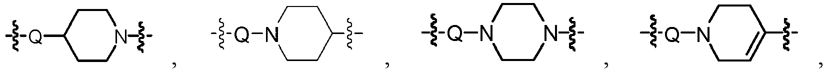

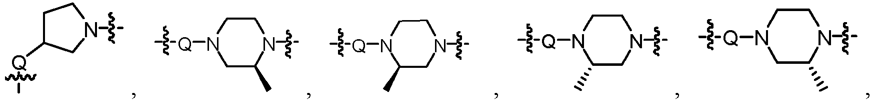

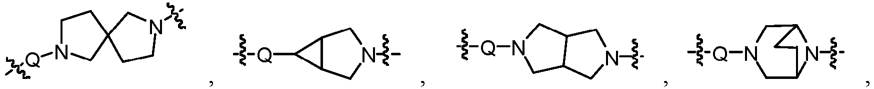

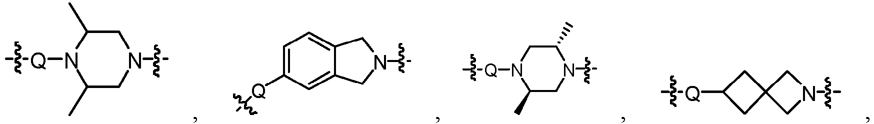

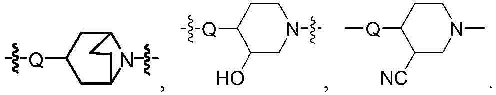

In some embodiments, the 1,4-diheterocyclic substituted aromatic ring or aromatic heterocyclic compound has a structure shown in formula (II)

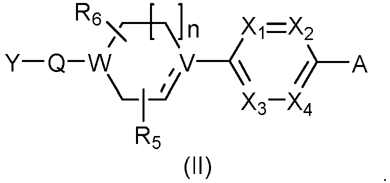

In some embodiments, A is selected from: 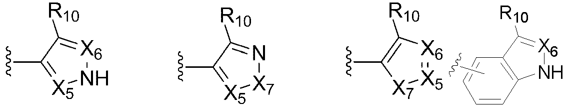 wherein,
X₅ and X₆ are independently selected from: CH and N;
X₇ is selected from: O, S;
R₁₀ is selected from: H, C₁-C₆ alkyl.

In some embodiments, A is selected from:

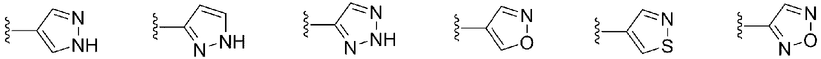

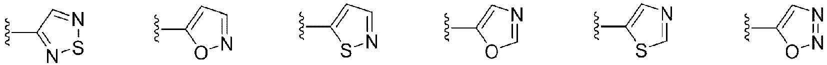

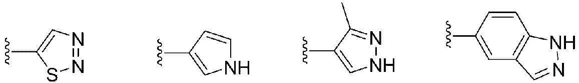

In some embodiments, A is selected from:

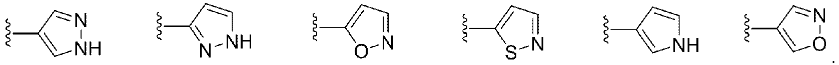

In some embodiments, Y is selected from: C₁-C₁₀ alkyl, halogen-substituted C₁-C₆ alkyl, hydroxyl substituted C₁-C₆ alkyl, C₁-C₃ alkoxy-substituted C₁-C₆ alkyl, phenyl substituted C₁-C₆ alkyl, acetylene-substituted C₁-C₆ alkyl, ethylene-substituted C₁-C₆ alkyl, C₃-C₆ cycloalkyl methyl, 5-6-membered heteroaryl; or Y is selected from: 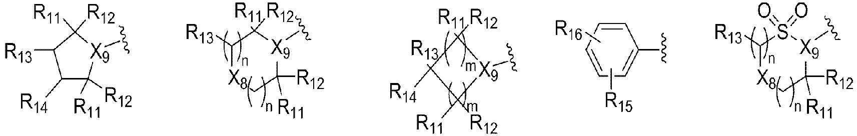
wherein, each X₈ is independently selected from: O, S, NR₁₀, CHR₁₄;
each X₉ is independently selected from: CR₁₈, N; R₁₈ is selected from: H, hydroxyl, C₁-C₆ alkyl, C₁-C₆ alkoxy, halogen;
each m is independently selected from: 1, 2, 3;
each n is independently selected from: 0, 1, 2;
each R₁₀ is independently selected from: H, C₁-C₆ alkyl, C₁-C₆ alkyl acyl;
each R₁₁ and R₁₂ are independently selected from: H, C₁-C₆ alkyl, hydroxyl, halogen; or each pair of R₁₁ and R₁₂ can independently and selectively together with their linked C atoms form C=O ;
each R₁₃ and R₁₄ are independently selected from: H, C₁-C₆ alkyl, C₁-C₆ alkoxy, C₃-C₈ cycloalkyl, 3-8-membered heterocyclyl, hydroxy-substituted C₁-C₆ alkyl, hydroxyl, halogen, amino, C₁-C₆ alkyl acyl; or R₁₃, R₁₄ on the same carbon atoms together with their linked C atoms form C=O; or R₁₃, R₁₄ together with their linked C atoms form one or more R₁₇ substituted or unsubstituted C₃-C₈ cycloalkyl, C₆-C₁₀ aryl, 3-8-membered heterocyclyl, or 5-6-membered heteroaryl; or R₁₁, R₁₃ together with their linked C atoms form one or more R₁₇ substituted or unsubstituted C₃-C₈ cycloalkyl or 3-8-membered heterocyclyl;
R₁₅ and R₁₆ are independently selected from: H, halogen, C₁-C₆ alkyl, C₂-C₆ alkenyl, C₂-C₆ alkynyl, C₃-C₈ cycloalkyl, C₃-C₈ cycloalkyl methyl, halogen substituted C₁-C₆ alkyl, hydroxyl substituted C₁-C₆ alkyl, C₁-C₆ alkoxy substituted C₁-C₆ alkyl, amino substituted C₁-C₆ alkyl, C₁-C₆ alkylamine substituted C₁-C₆ alkyl, C₆-C₁₀ aryl, 5-10-membered heteroaryl, nitro, cyano, - OR, - N(R)₂, -SR, - C(O)OR,-C(O)N(R)₂, - C(O)N(R)₂, - C(O)O)R, - S(O)R, - S(O)₂R, - S(O)₂N(R)₂, - N(R)C(O)R; or adjacent R₁₅, R₁₆ together with their linked C atoms form one or more R₁₇ substituted or unsubstituted C₃-C₈ cycloalkyl, C₆-C₁₀ aryls, 3-8-membered heterocyclyl, or 5-6-membered heteroaryl;
each R₁₇ is independently selected from: halogen, hydroxyl, cyano, C₁-C₆ alkyl, C₁-C₆ alkyl acyl, C₁-C₆ alkoxyl, or two R₁₇ atoms on the same carbon atoms together with their linked carbon atoms form C=O.

In some embodiments, Y is selected from: C₁-C₈ alkyl, hydroxy-substituted C₁-C₆ alkyl, phenyl-substituted C₁-C₆ alkyl, acetylene-substituted C₁-C₆ alkyl, vinyl-substituted C₁-C₆ alkyl, C₅-C₆ cycloalkyl methyl; or, Y is selected from: 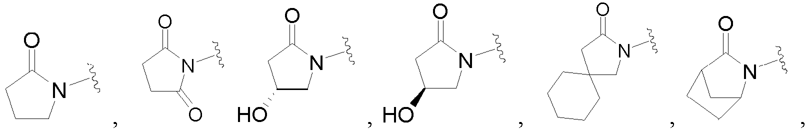 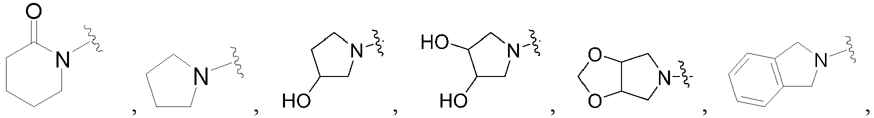 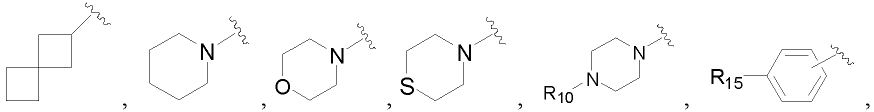 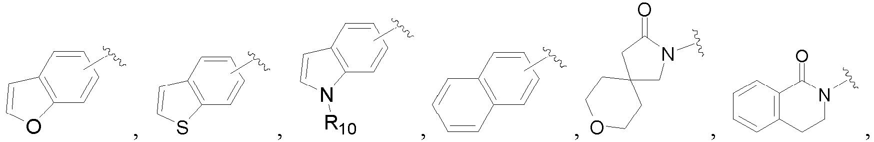 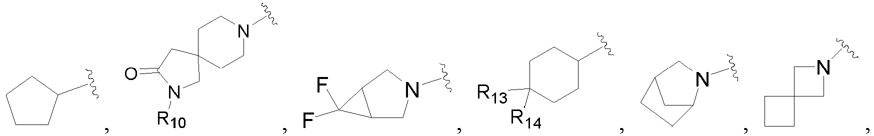 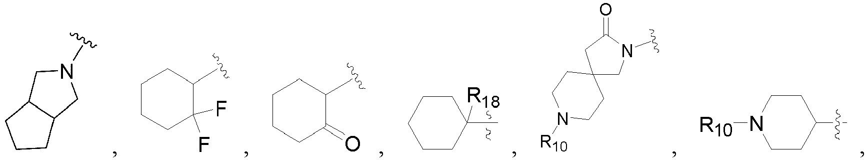 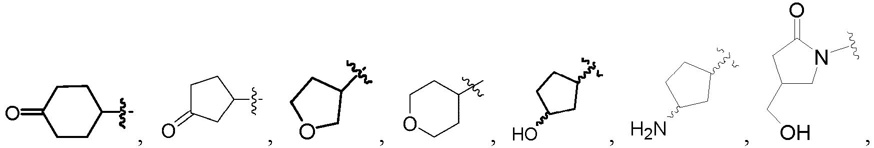 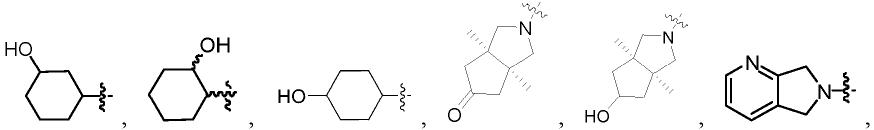 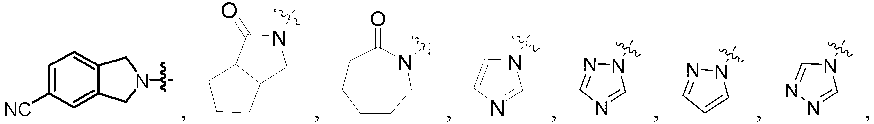 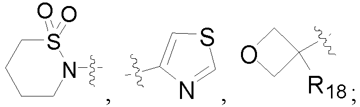
wherein, each R₁₀ is independently selected from: H, C₁-C₃ alkyl, C₁-C₃ alkyl acyl;
   each R₁₃ and R₁₄ are independently selected from: H, halogen, C₁-C₆ alkyl, C₁-C₆ alkoxy, hydroxyl;
R₁₅ is selected from: H, halogen, C₁-C₃ alkyl, C₁-C₃ alkoxy;
each R₁₈ is independently selected from: H, hydroxyl, halogen, C₁-C₃ alkoxy, C₁-C₃ alkyl.

In some embodiments, Y is selected from: C₄-C₇ alkyl, cyclopentyl, naphthyl, hydroxyl substituted C₄-C₆ alkyl, phenyl-substituted C₁-C₆ alkyl, acetylene-substituted C₄-C₆ alkyl, ethylene-substituted C₄-C₆ alkyl, C₅-C₆ cycloalkyl methyl; or, Y is selected from: 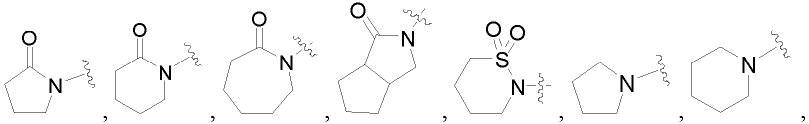 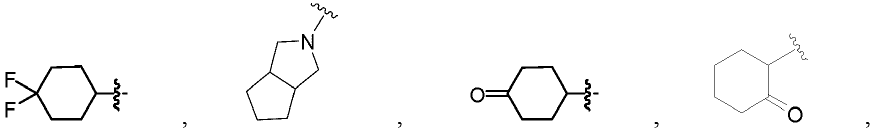 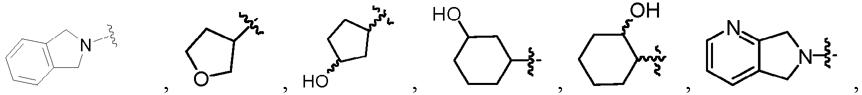 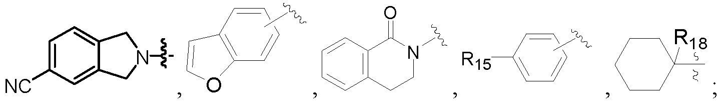 wherein R₁₅ is selected from: H or halogen; R₁₈ is selected from: H, hydroxyl, halogen, C₁-C₃ alkoxy.

In some embodiments, Q and Y form the following groups: -Y-S(O)₂-, -Y-(CR₂R₃)ₚ-, -Y-C(O)-, -Y-N(R₄)C(O)-, -Y-C(O)N(R₄)-, -Y-C(O)N(R₄)(CR₂R₃)-, or -Y-N(R₄)C(O)(CR₂R₃)-, wherein, p is selected from 0, 1 or 2; R₂, R₃, and R₄ are all H.

In some embodiments, Q and Y form -Y-CH₂-, wherein Y is selected from:

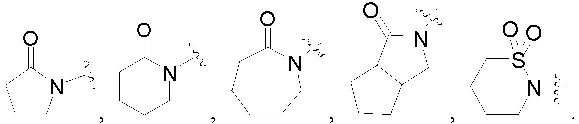

In some embodiments, Q and Y form -Y-C=O, wherein Y is selected from cyclopentyl, cyclohexyl, benzyl, hydroxy substituted butyl, cyclopentyl methyl,

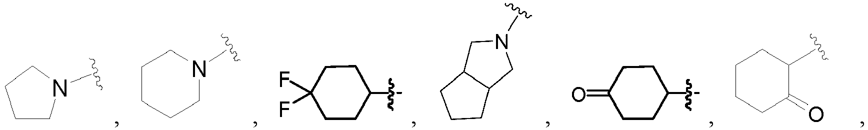

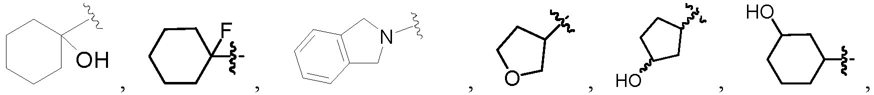

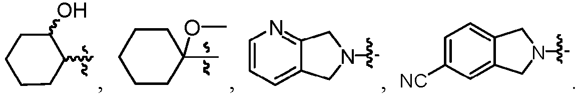

In some embodiments, Q and Y form Y-C(O)NHCH₂-, wherein Y is selected from butyl, pentyl, hexyl, cyclopentyl, phenyl, phenyl-substituted n-pentyl, acetylene-substituted n-pentyl, vinyl-substituted n-pentyl.

In some embodiments, Q and Y form -Y-NHC(O)-, wherein Y is selected from: cyclopentyl methyl, cyclohexyl, phenyl, 4-fluorophenyl, naphthyl, benzyl, hydroxy substituted butyl,

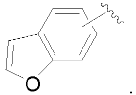

In some embodiments, wherein Q and Y form -Y-NHC(O)-, and Y is selected from cyclopentane, and the Ring C together with Q form the following groups:

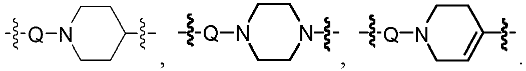

In some embodiments, Q and Y form -Y-S(O)₂-, wherein Y is selected from: cyclohexyl, 4-fluorophenyl.

In some embodiments, X₁, X₂, X₃, and X₄ are independently selected from CH and CR₁.

In some embodiments, X₁ and X₄ cannot be both CR₁, and X₂ and X₃ cannot be both CR₁.

In some embodiments, R₁ is selected from: halogen, C₁-C₆ alkyl, halogen-substituted C₁-C₆ alkyl, hydroxyl-substituted C₁-C₆ alkyl, C₁-C₃ alkoxy-substituted C₁-C₆ alkyl, amino-substituted C₁-C₆ alkyl, C₁-C₃ alkylamine-substituted C₁-C₆ alkyl, cyano, C₁-C₆ alkoxy, -N(C₁-C₃ alkyl)₂, -C(O)OC₁-C₃ alkyl, -C(O)NH₂, -C(O)N(C₁-C₃ alkyl)₂, -C(O)(C₁-C₃ alkyl)₂,- C(O)H.

In some embodiments, R₁ is selected from: halogen, C₁-C₃ alkyl, halogen-substituted C₁-C₃ alkyl, cyano, formaldehyde, hydroxyl-substituted C₁-C₃ alkyl.

In some embodiments, R₁ is selected from: fluorine, chlorine, monofluoromethyl, difluoromethyl, trifluoromethyl, methyl, cyano, methoxy, formaldehyde, aminoformyl, hydroxymethyl, methoxycarbonyl, dimethylamino.

In some embodiments, the 1,4-diheterocyclic substituted aromatic ring or aromatic heterocyclic compound, is selected from the following compounds:

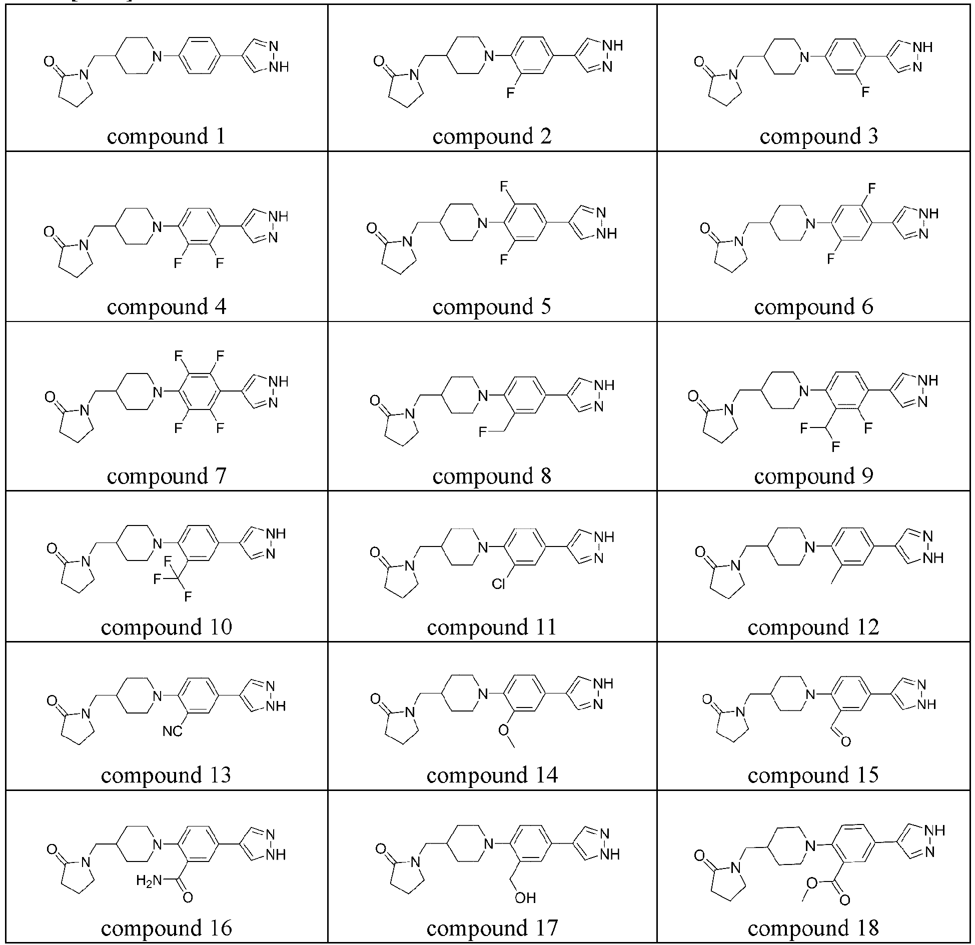

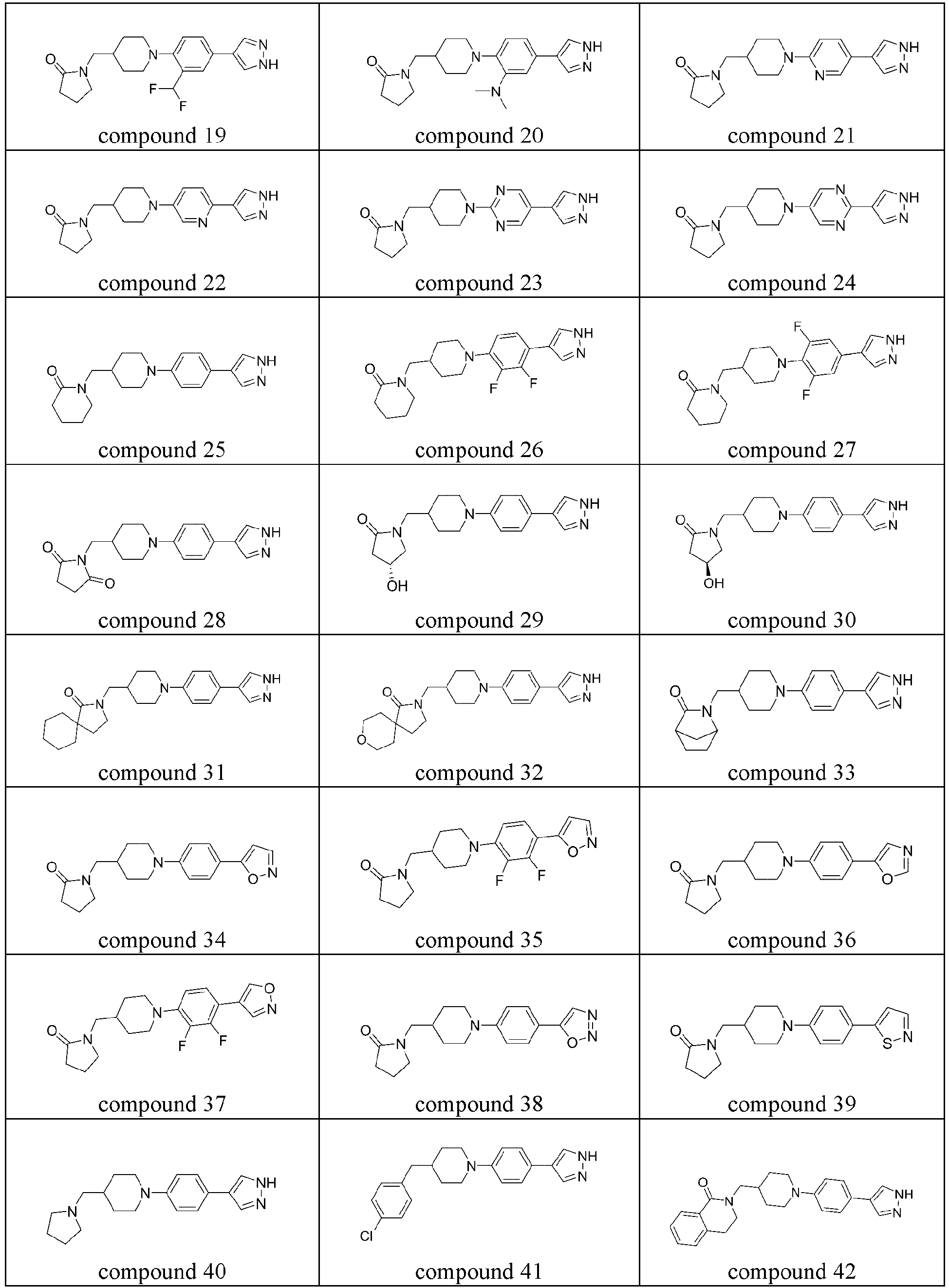

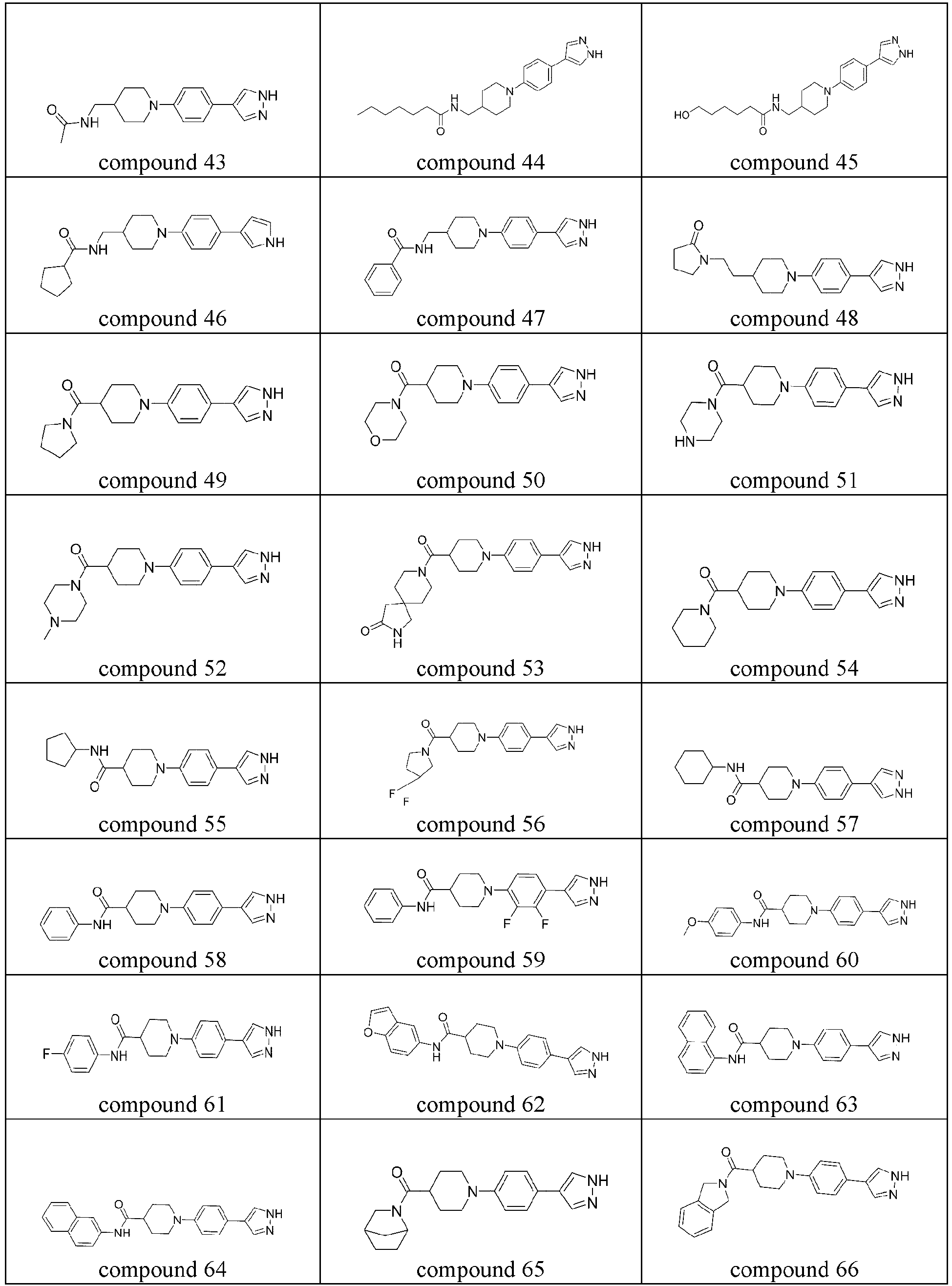

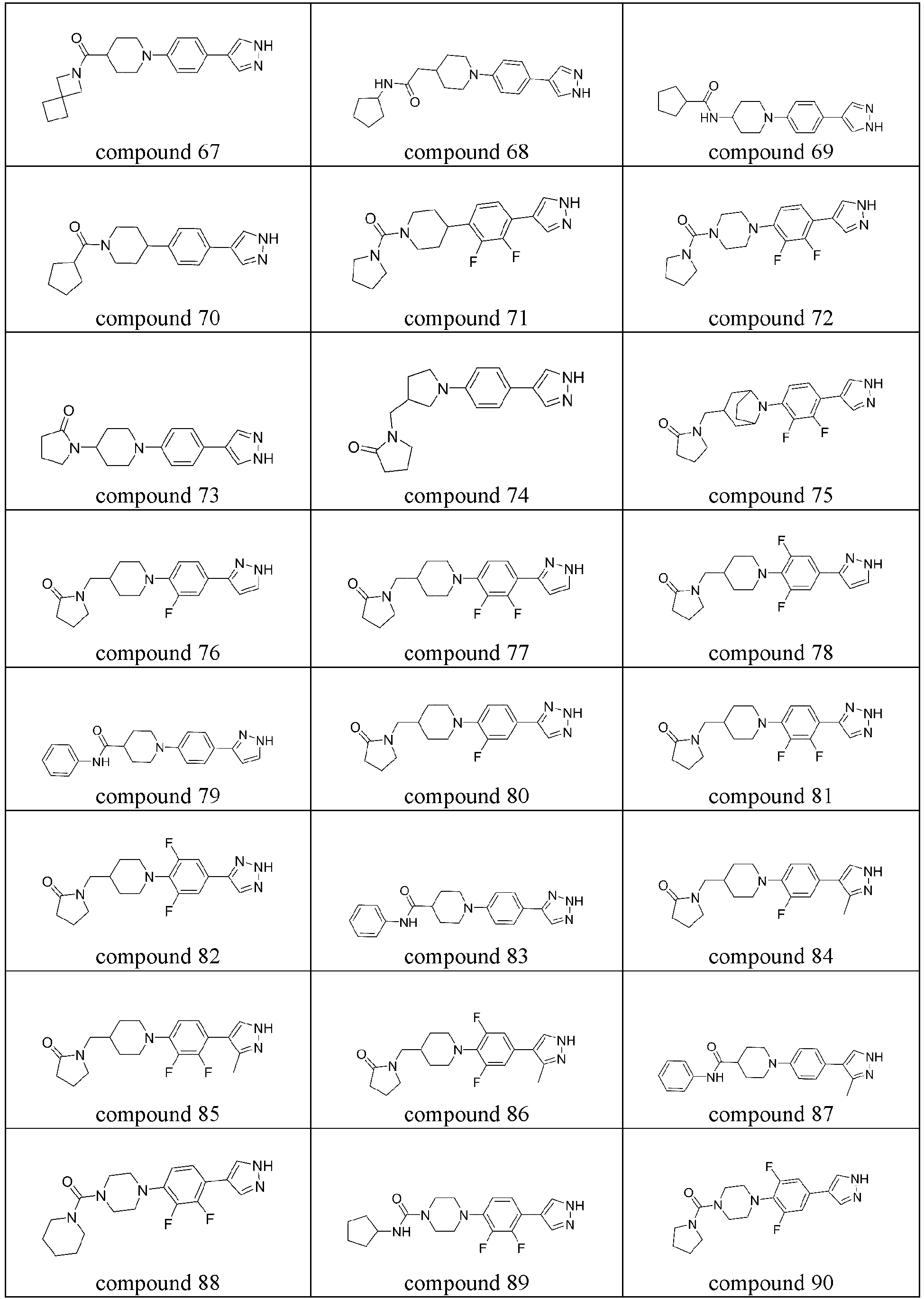

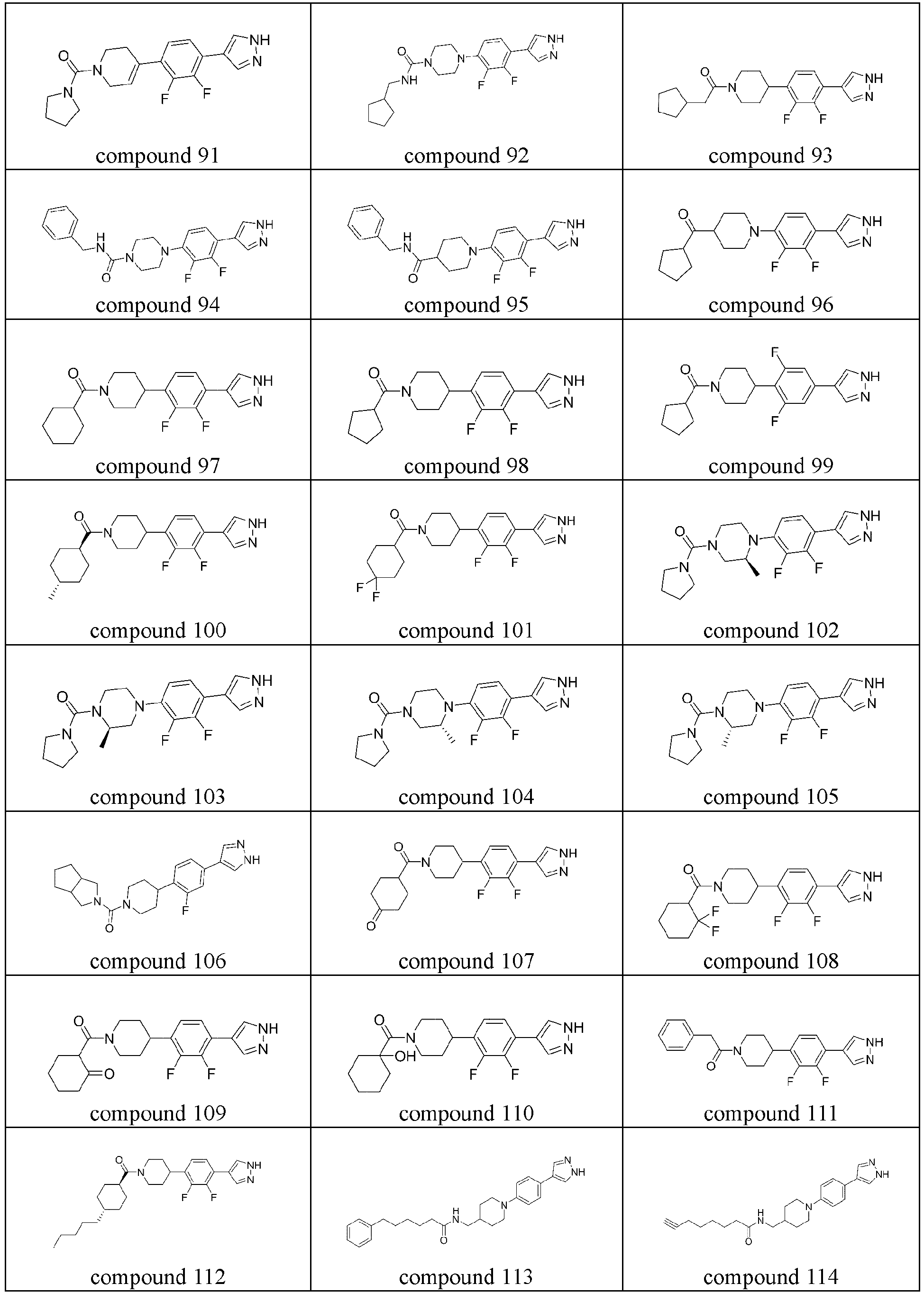

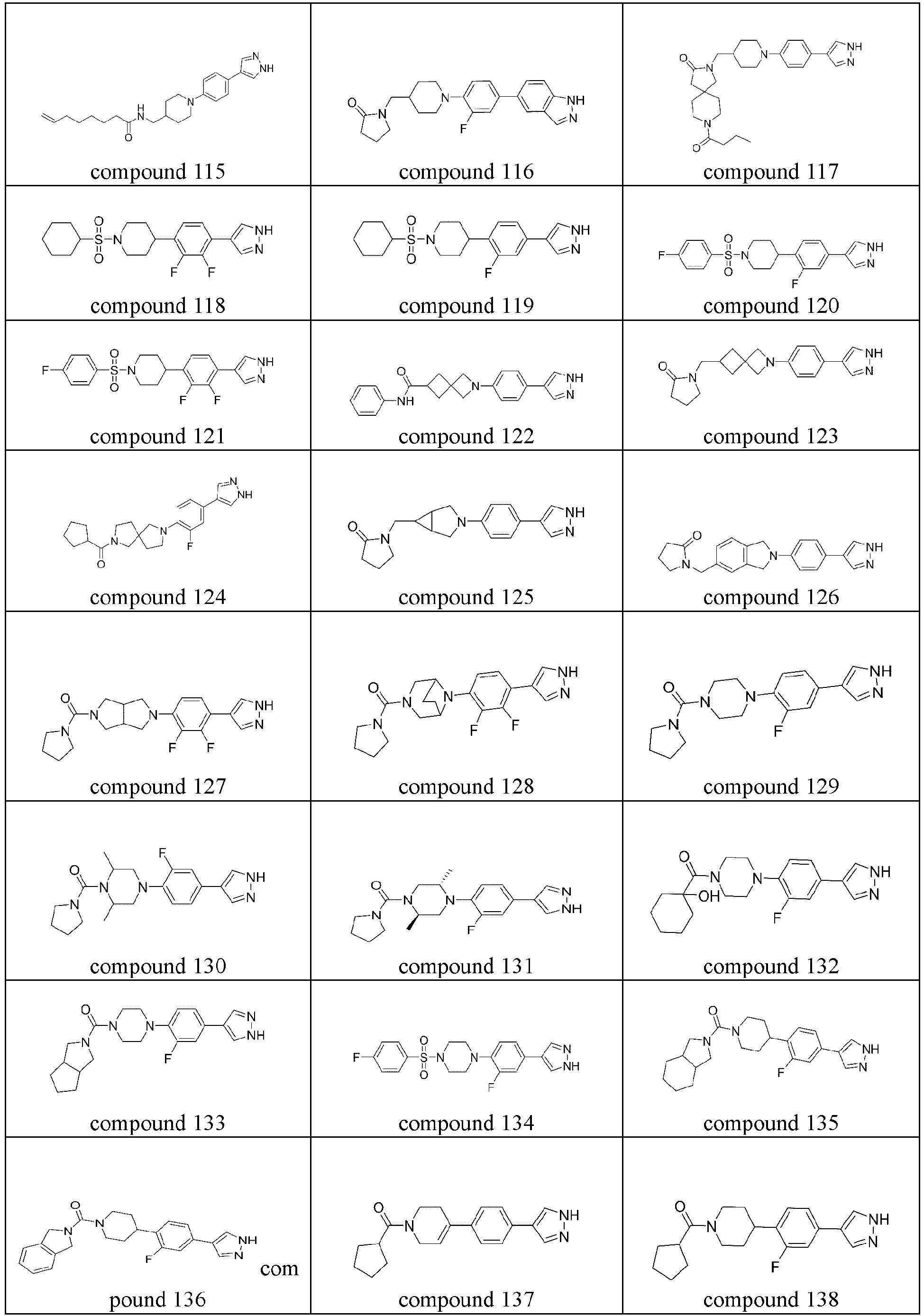

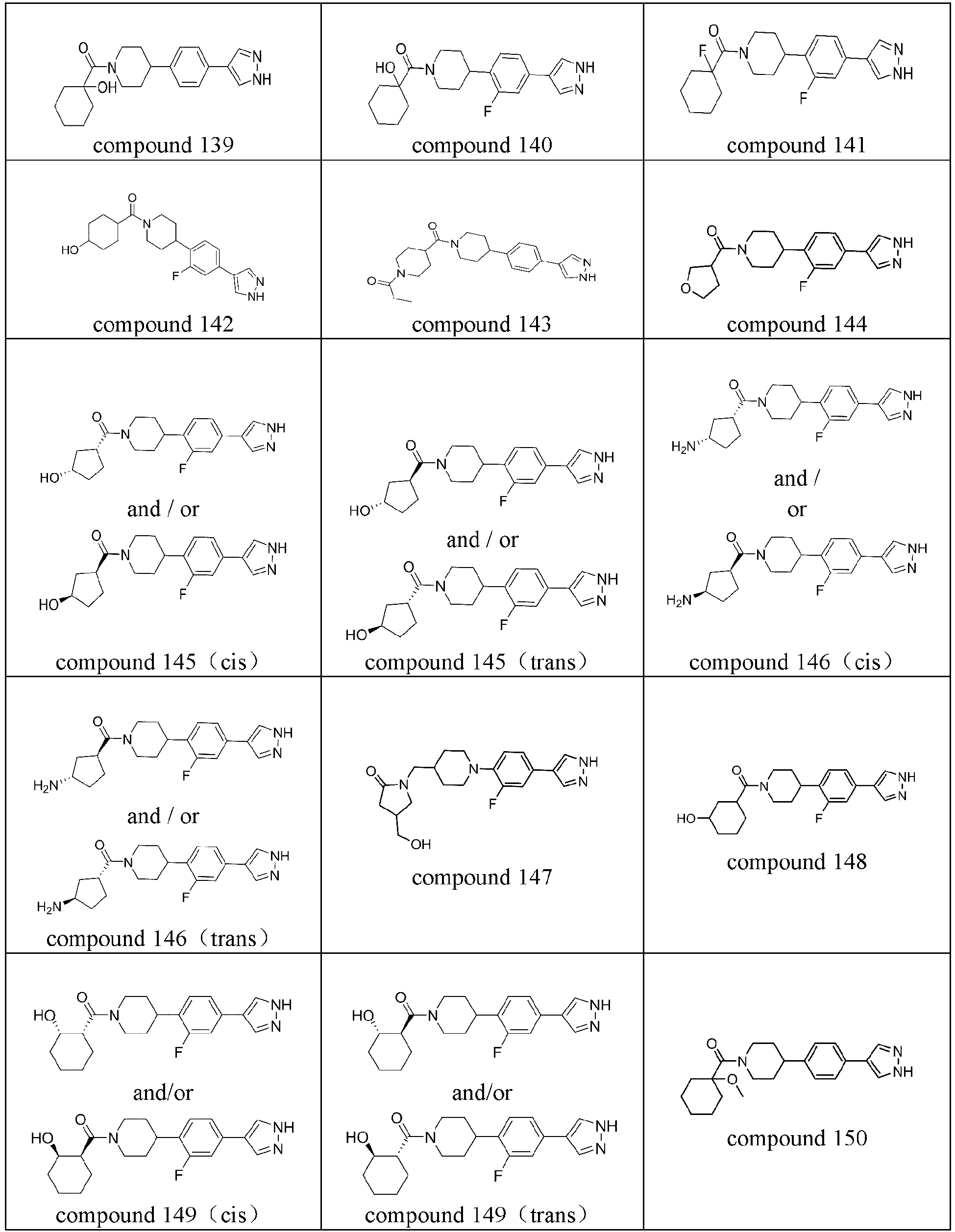

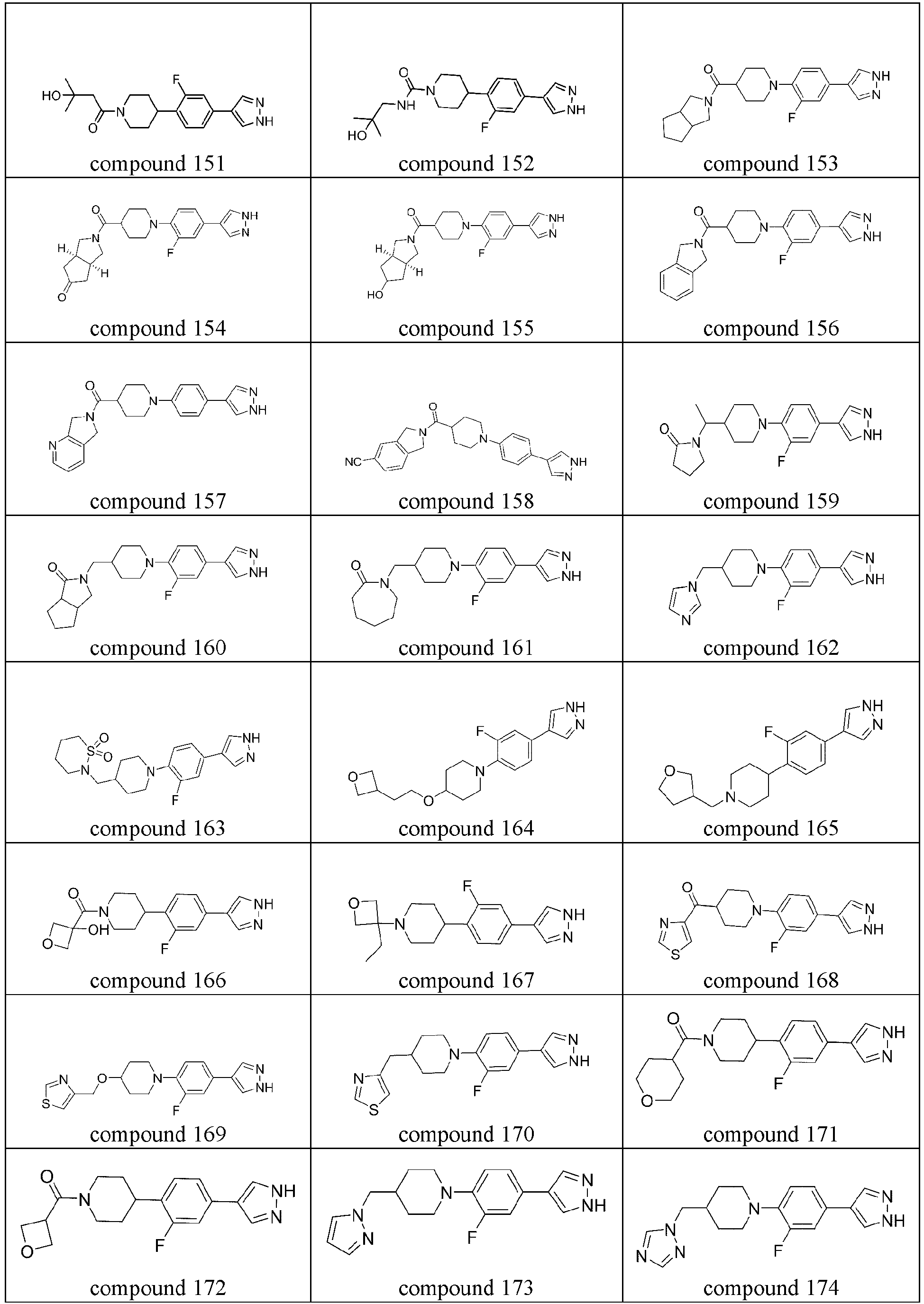

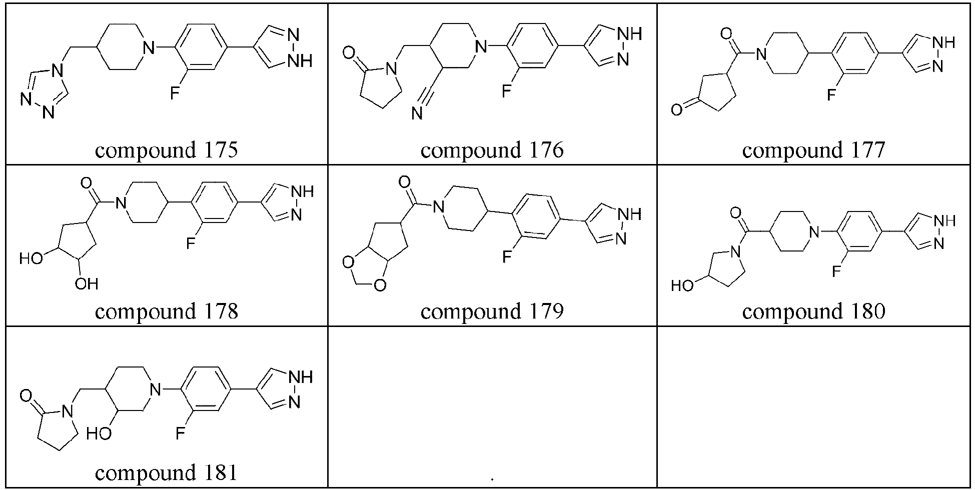

The present disclosure also provides applications of the 1,4-diheterocyclic substituted aromatic ring or aromatic heterocyclic compound or its pharmaceutically acceptable salts or stereoisomers, comprising the following technical solutions.

An application of the 1,4-diheterocyclic substituted aromatic ring or aromatic heterocyclic compound or its pharmaceutically acceptable salts or stereoisomers in the preparation of 20-HETE generation inhibitors.

An application of the 1,4-diheterocyclic substituted aromatic ring or aromatic heterocyclic compound or its pharmaceutically acceptable salts or stereoisomers in the prevention and/or treatment of diseases and/or symptoms related to the 20-HETE signaling pathway.

An application of GLP-1 receptor agonists in combination with the 1,4-diheterocyclic substituted aromatic ring or aromatic heterocyclic compound or its pharmaceutically acceptable salts or stereoisomers in the prevention and/or treatment of diseases and/or symptoms related to the 20-HETE signaling pathway.

In some embodiments, the GLP-1 receptor agonist is semaglutide.

In some embodiments, the diseases and/or symptoms related to the 20-HETE signaling pathway are selected from: obesity, metabolic syndrome, dyslipidemia, diabetes, diabetes retinopathy, diabetes cerebrovascular disease, diabetes neuropathy, insulin resistance, hyperglycemia, hyperlipidemia, diabetes nephropathy, hypertension, cataract, osteoporosis, hyperuricemia, multiple infections caused by diabetes, non-alcoholic fatty liver disease, non-alcoholic fatty liver disease, fibrosis, heart disease, stroke, cirrhosis, metabolic acidosis, ketosis, cardiovascular discomfort, epilepsy, atherosclerosis, Parkinson's disease, myocardial infarction, acute renal failure, chronic kidney disease, polycystic kidney disease, tumor, end organ damage, Alzheimer's disease.

In some embodiments, the diabetes is type 1 diabetes mellitus(T1DM), type 2 diabetes mellitus(T2DM), gestational diabetes mellitus, idiopathic T1D, early-onset T2DM, maturity-onset diabetes of the young, atypical diabetes in adolescents, malnutrition related diabetes, and latent autoimmune diabetes in adults.

The present disclosure also provides a method for preventing and/or treating diseases and/or symptoms related to the 20-HETE signaling pathway, comprising the following technical solutions.

A method for preventing and/or treating diseases and/or symptoms related to the 20-HETE signaling pathway, including administering an effective amount of the 1,4-diheterocyclic substituted aromatic ring or aromatic heterocyclic compound or its pharmaceutically acceptable salts or stereoisomers.

In some embodiments, the diseases and/or symptoms related to the 20-HETE signaling pathway are selected from: obesity, metabolic syndrome, dyslipidemia, diabetes, diabetes retinopathy, diabetes cerebrovascular disease, diabetes neuropathy, insulin resistance, hyperglycemia, hyperlipidemia, diabetes nephropathy, hypertension, cataract, osteoporosis, hyperuricemia, multiple infections caused by diabetes, non-alcoholic fatty liver disease, non-alcoholic fatty liver disease, fibrosis, heart disease, stroke, cirrhosis, metabolic acidosis, ketosis, cardiovascular discomfort, epilepsy, atherosclerosis, Parkinson's disease, myocardial infarction, acute renal failure, chronic kidney disease, polycystic kidney disease, tumor, end organ damage, Alzheimer's disease.

In some embodiments, the diabetes is T1DM, T2DM, gestational diabetes mellitus, idiopathic T1D, early-onset T2DM, maturity-onset diabetes of the young, atypical diabetes in adolescents, malnutrition related diabetes, and latent autoimmune diabetes in adults.

The present disclosure also provides a pharmaceutical composition for the prevention and/or treatment of diseases and/or symptoms associated with the 20-HETE signaling pathway, comprising the following technical solutions.

A pharmaceutical composition for the prevention and/or treatment of diseases and/or symptoms associated with the 20-HETE signaling pathway, comprising an active ingredient and pharmaceutically acceptable excipients and/or carriers, wherein the active ingredient comprises the 1,4-diheterocyclic substituted aromatic ring or aromatic heterocyclic compound or its pharmaceutically acceptable salts or stereoisomers.

The present disclosure also provides a combination drug for the prevention and/or treatment of diseases and/or symptoms related to the 20-HETE signaling pathway, comprising the following technical solutions.

A combination drug for the prevention and/or treatment of diseases and/or symptoms related to the 20-HETE signaling pathway, wherein its active ingredients include a GLP-1 receptor agonist, and the 1,4-diheterocyclic substituted aromatic ring or aromatic heterocyclic compound or its pharmaceutically acceptable salts or stereoisomers; wherein, the GLP-1 receptor agonist and the 1,4-diheterocyclic substituted aromatic ring or aromatic heterocyclic compound or its pharmaceutically acceptable salts or stereoisomers, are independent drug delivery units or jointly form a combined drug delivery unit.

In some embodiments, the GLP-1 receptor agonist is semaglutide.

The present disclosure has the following beneficial effects:
The 1,4-diheterocyclic substituted aromatic ring or aromatic heterocyclic compound provided by the present disclosure can effectively inhibit the generation of 20-HETE, with high activity, high selectivity, and good pharmacokinetic properties. By inhibiting the generation of 20-HETE, it can be used to treat various diseases related to 20-HETE and has great application value.

### Brief Description of Figures

Fig. 1 shows mean concentration-time profiles of compound 1, compound 2, compound 4, compound 5, compound 26, compound 66, compound 70, and control 83 in rats by intragastric administration of drugs (20 mg/kg).
Fig. 2 shows the mean concentration-time profiles of compound 71, compound 72, compound 91, compound 98, compound 113, compound 153, compound 163, and control 83 in rats by intragastric administration of drugs (20 mg/kg) .
Fig. 3 shows the weight growth curves in each group of animals with compound 1 in the DIO mouse model.
Fig. 4 shows the weight change curves in each group of animals with compound 1 in the DIO mouse model.
Fig. 5 shows the OGTT curves in each group of animals with compound 1 in the DIO mouse model.
Fig. 6 shows the weight growth curves of mice with compound 2 or compound 4 in the DIO mouse model.
Fig. 7 shows the weight change curves of mice with compound 2 or compound 4 in the DIO mouse model.
Fig. 8 shows the OGTT-blood glucose level curves of mice with compound 2 or compound 4 in the DIO mouse model.
Fig. 9 shows the OGTT-blood glucose ACU bar chart of mice with compound 2 or compound 4 in the DIO mouse model.
Fig. 10 shows the weight growth curves in each group of animals with compound 2 in the DIO mouse model.
Fig. 11 shows the weight change curves in each group of animals with compound 2 in the DIO mouse model.
Fig. 12 shows the food intake curves in each group of animals with compound 2 in the DIO mouse model.
Fig. 13 shows OGTT curves in each group of animals administered orally with compound 2 in the DIO mouse model.
Fig. 14 shows the weight growth curves of mice with compound 2 or semaglutide in the DIO mouse model.

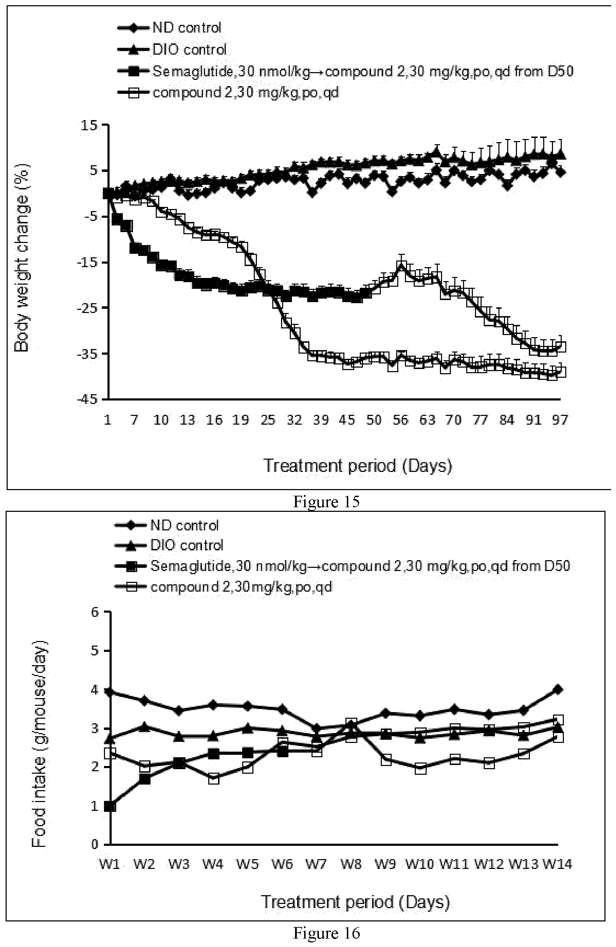

Fig. 15 shows the weight change curves of mice with compound 2 or semaglutide in the DIO mouse model.
Fig. 16 shows the food intake curves of mice with compound 2 or semaglutide in the DIO mouse model.
Fig. 17 shows the relative content of 20-HETE in various organs of mice with compound 2 in the DIO mouse model.
Fig. 18 shows the relative content of 20-HETE in various organs of rat with compound 2 in the DIO rat model.

### DETAILED DESCRIPTION OF THE DISCLOSURE

In the compound of the present disclosure, when any variable (e.g. R, etc.) occurs more than once in any component, its definition at each occurrence is independent from the definition at each other occurrence. Similarly, combinations of substituents and variables are permissible if only the compound with such combinations are stabilized. A line from a substituent to a ring system indicates that the indicated bond may be attached to any substitutable ring atom. If the ring system is polycyclic, it means that such bonds are only attached to any suitable carbon atoms adjacent to the ring. It is understood that an ordinary skilled in the art can select substituents and substitution patterns of the compound of the present disclosure to provide compounds that are chemically stable and can be readily synthesized from the available starting materials by the methods described below. If a substituent itself is substituted by more than one group, it should be understood that these groups may be on the same carbon atom or on different carbon atoms, so long as the structure is stabilized.

The term "alkyl" in the present disclosure is meant to include branched and straight chain saturated aliphatic hydrocarbon groups having the specified number of carbon atoms. For example, the definition of "C₁-C₆" in "C₁-C₆ alkyl" includes groups having 1, 2, 3, 4, 5 or 6 carbon atoms arranged in a straight or branched chain. For example, "C₁-C₆ alkyl" specifically includes methyl, ethyl, n-propyl, isopropyl, n-butyl, tert-butyl, isobutyl, pentyl, hexyl.

The term "cycloalkyl" refers to a monocyclic saturated fatty hydrocarbon group with a specific number of carbon atoms. For example, "cycloalkyl" includes cyclopropyl, cyclobutyl, cyclopentyl, or cyclohexyl groups.

The term "alkoxy" refers to a group with an -O- alkyl structure, such as -OCH₃, -OCH₂CH₃, -OCH₂CH₂CH₃, -O-CH₂CH(CH₃)₂, -OCH₂CH₂CH₂CH₃, -O-CH(CH₃)₂, etc.

The term "heterocycloalkyl" is a saturated or partially unsaturated monocyclic or polycyclic substituent spiral cyclic, thick cyclic, and bridge cyclic wherein one or more ring atoms are heteroatoms selected from N, O or S(O)ₘ (wherein m is an integer from 0 to 2 ), and the remaining ring atoms are carbon, bicyclic or polycyclic such as: morpholinyl, piperidinyl, tetrahydropyrrolyl, pyrrolidinyl, dihydroimidazolyl, dihydroisoxazolyl, dihydroisothiazolyl, dihydro oxadiazolyl, dihydrooxazolyl, dihydropyrazinyl, dihydropyrazolyl, dihydropyridyl, dihydropyrimidinyl, dihydropyrrolyl, dihydrotetrazolyl, dihydrothiadiazolyl, dihydrothiazolyl, dihydrothienyl, dihydrotriazolyl, dihydroazetidine, tetrahydrofuranyl, tetrahydrothienyl, etc., and their N-oxides. The connection of heterocyclic substituents can be achieved through carbon atoms or through heteroatoms.

The term "heteroaryl" as used herein refers to an aromatic ring containing one or more heteroatoms selected from O, N or S. The heteroaryl groups within the scope of the present disclosure include but are not limited to: quinoline group, pyrazole group, pyrrole group, thiophene group, furan group, pyridine group, pyrimidine group, pyrazine group, triazole group, imidazole group, oxazole group, isoxazole group, pyridazine group, benzofuran group, benzothiophene group, benzoxazole group, indole group, etc; "heteroaryl" is also understood as including any N-oxide derivative of heteroaryl with nitrogen.

The term "substituted" refers to replacing the hydrogen in a specific structure with a designated substituent group.

As understood by the skilled in the art, "halogen" or "halo" as used herein means chlorine, fluorine, bromine and iodine.

Unless otherwise defined, alkyl, cycloalkyl, aryl, heteroaryl and heterocycloalkyl substituents can be unsubstituted or substituted. For example, a C₁-C₆ alkyl group can be substituted by one, two, or three substituents selected from OH, halogen, alkoxy, dialkylamino, or heterocyclyl groups such as morpholinyl, piperidinyl, etc.

The present disclosure includes free forms of compounds of Formula I or Formula II, as well as pharmaceutically acceptable salts and stereoisomers thereof. The term "free form" refers to the amine compounds in non-salt form. Some specific exemplary compounds in the present disclosure are protonation salts of amine compounds. The pharmaceutically acceptable salts include not only exemplary salts of the particular compounds described herein, but also typical pharmaceutically acceptable salts of free form of all compounds of Formula I. The free forms of specific salts of the compounds can be isolated using techniques known in the art. For example, the free form can be regenerated by treating the salt with an appropriate dilute aqueous base, such as dilute aqueous NaOH, dilute aqueous potassium carbonate, dilute aqueous ammonia, and dilute aqueous sodium bicarbonate. The free forms differ somewhat from their respective salt forms in certain physical properties such as solubility in polar solvents, but for the purposes of the disclosure, such salts of acid or base are otherwise pharmaceutically equivalent to their respective free forms.

The pharmaceutically acceptable salts of the present disclosure can be synthesized from the compounds containing a basic or acidic moiety in the present disclosure by conventional chemical methods. Generally, salts of basic compounds can be prepared by ion exchanged chromatography or by reacting the free base with a stoichiometric or excess amount of inorganic or organic acid in the desired salt form in a suitable solvent or combination of solvents. Similarly, salts of acidic compounds can be formed by reaction with a suitable inorganic or organic base.

Accordingly, the pharmaceutically acceptable salts of the compounds in the present disclosure include conventional non-toxic salts of the compounds in the present disclosure formed by reacting a basic compound of the present disclosure with an inorganic or organic acid. For example, conventional non-toxic salts include those derived from inorganic acids such as hydrochloric acid, hydrobromic acid, sulfuric acid, sulfamic acid, phosphoric acid, nitric acid, etc. They also include those derived from organic acids such as acetic acid, propionic acid, succinic acid, glycolic acid, hard Fatty acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2 - acetoxy - benzoic acid, fumaric acid, toluenesulfonic acid, methanesulfonic acid, ethanedisulfonic acid, oxalic acid, isethionic acid, and trifluoroacetic acid, etc.

If the compounds of the present disclosure are acidic, the appropriate "pharmaceutically acceptable salts" refer to the salts prepared from pharmaceutically acceptable non-toxic bases including inorganic bases and organic bases. The salts derived from inorganic bases include aluminum, ammonium, calcium, copper, iron, ferrous, lithium, magnesium, manganese, manganous, potassium, sodium, zinc, etc. Particularly preferably, ammonium salts, calcium salts, magnesium salts, potassium salts, and sodium salts. The salts derived from pharmaceutically acceptable organic non-toxic bases include salts of primary, secondary and tertiary amines. Substituted amines include naturally occurring substituted amines, cyclic amines and basic ion exchange resins such as Amino acid, betaine, caffeine, choline, N, N'-dibenzylethylenediamine, diethylamine, 2-diethylaminoethanol, 2-dimethylaminoethanol, aminoethanol, ethanolamine, ethyl Diamine, N-ethylmorpholine, N-ethylpiperidine, Glucosamine, Glucosamine, Histidine, Hydroxocobalamin, Isopropylamine, Lysine, Methylglucamine, Morpholine, Piperazine, Piperidine, quack, polyamine resin, procaine, purine, theobromine, triethylamine, trimethylamine, tripropylamine, tromethamine, etc.

The preparation of the pharmaceutically acceptable salts described above and other typical pharmaceutically acceptable salts was described in more detail by Berg et al., in "Pharmaceutical Salts," J. Pharm. Sci. 1977:66: 1-19.

Since under physiological conditions, the deprotonated acidic moieties (such as carboxyl groups) in compounds can be anions, which carry electric charges and can be neutralized by internally cationic protonated or alkylated basic moieties (such as tetravalent nitrogen atoms), so it should be noted that the compounds of the present disclosure are potential inner salts or zwitterions.

Synthesis method: In addition to the standard methods known in literature or exemplified in experimental procedures, the compounds in the present disclosure can be prepared using the methods described in the following synthesis embodiments (Embodiments 1 to 13). By combining the following synthesis embodiments, a better understanding of the compound and synthesis method described in the present disclosure can be obtained. The described synthesis examples describe methods that can be used to prepare the compound described in the present disclosure. The methods are only an explanatory scheme description for the purpose of illustration, but do not constitute a limitation on the scope of the present disclosure.

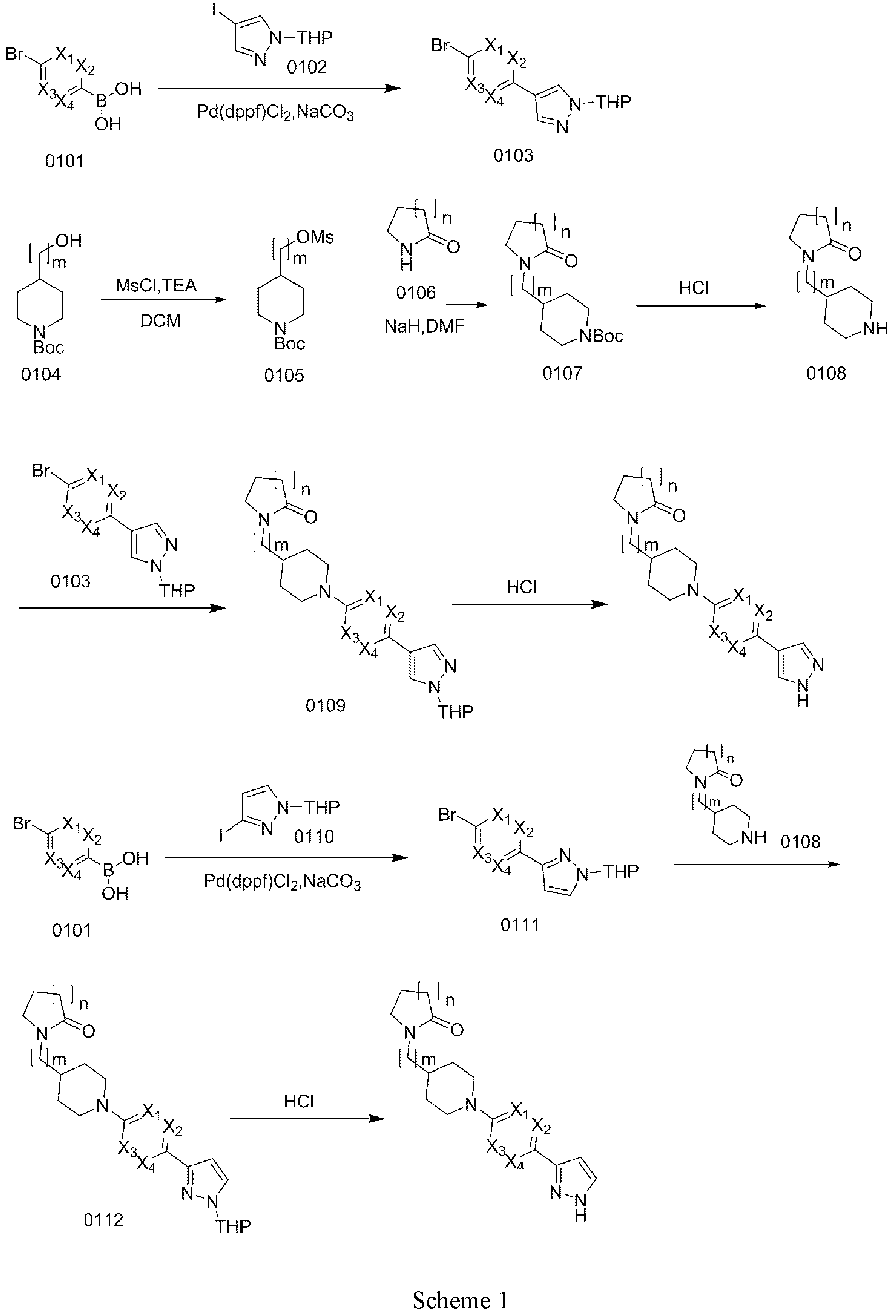

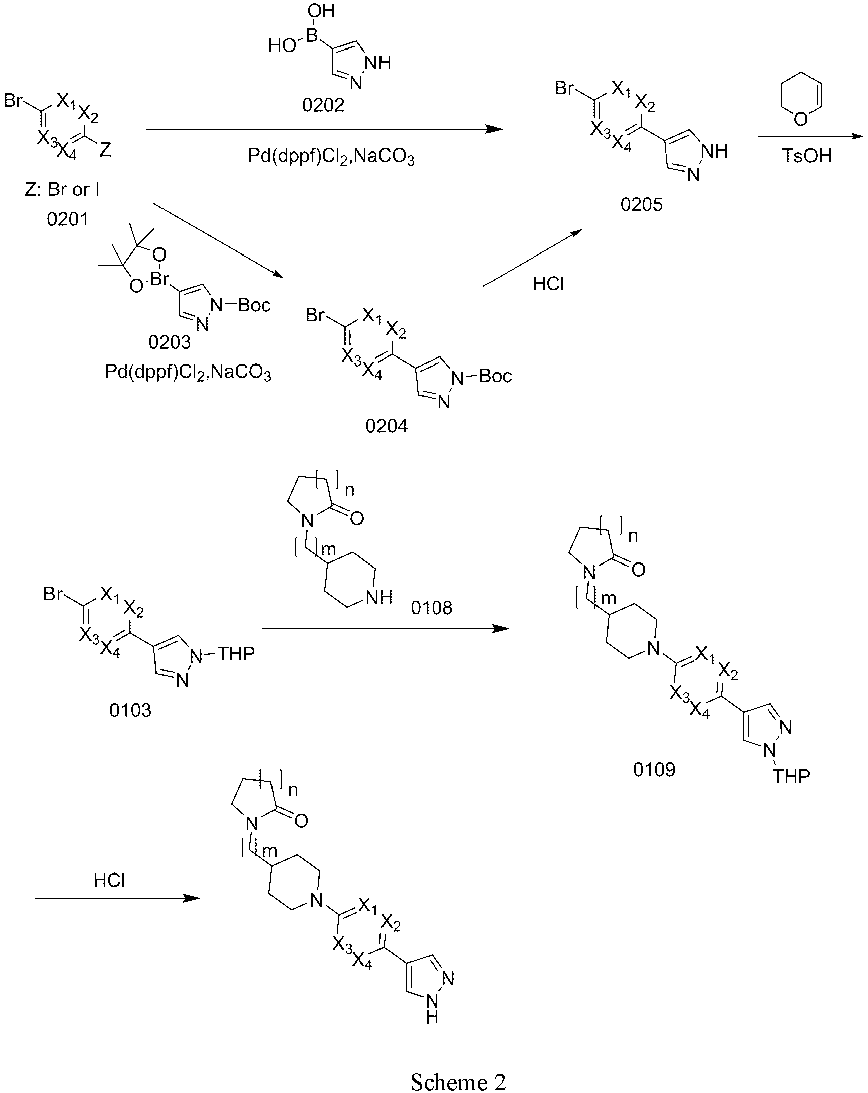

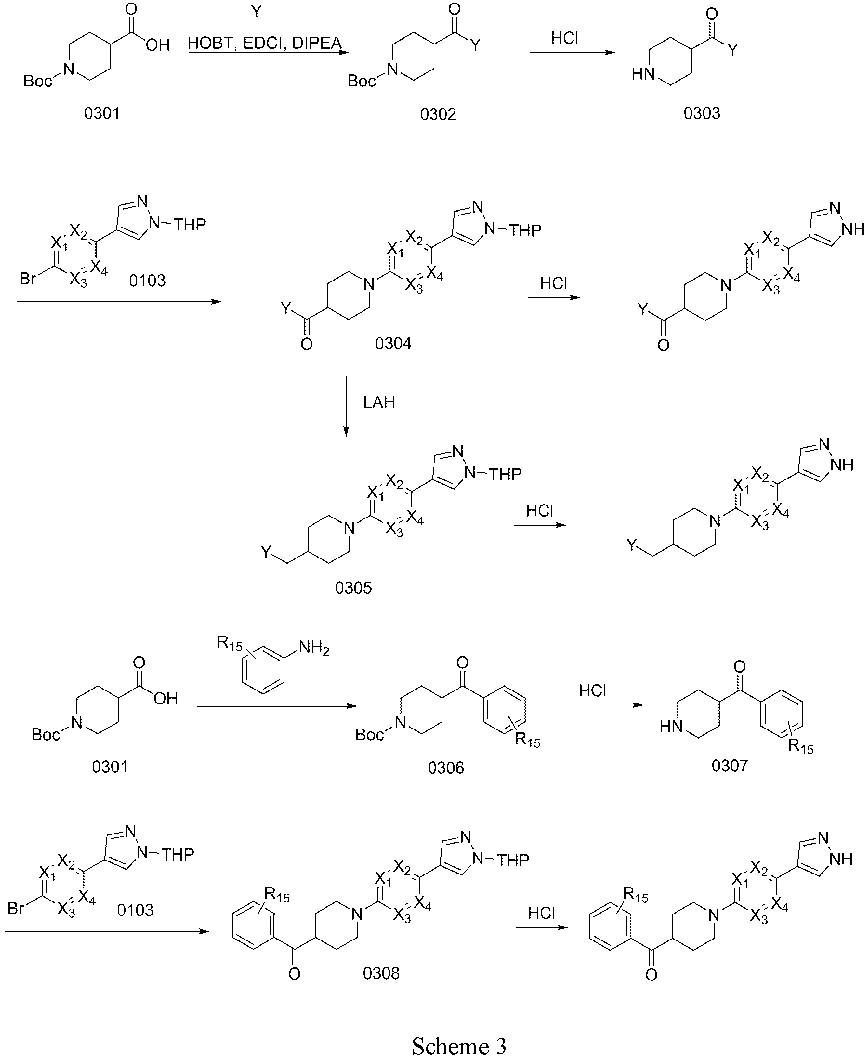

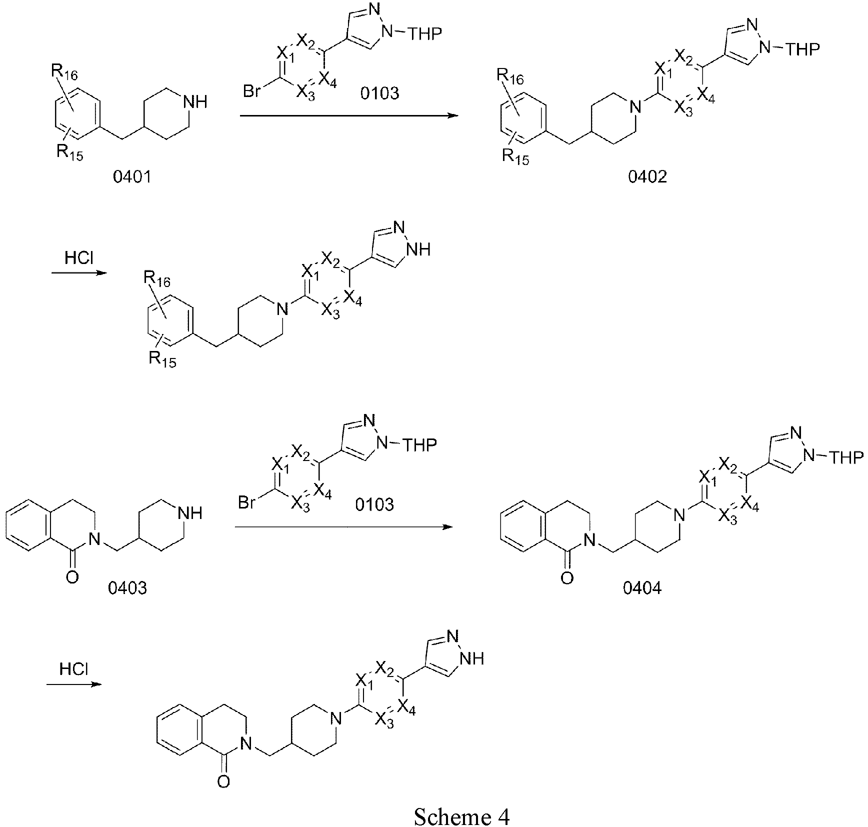

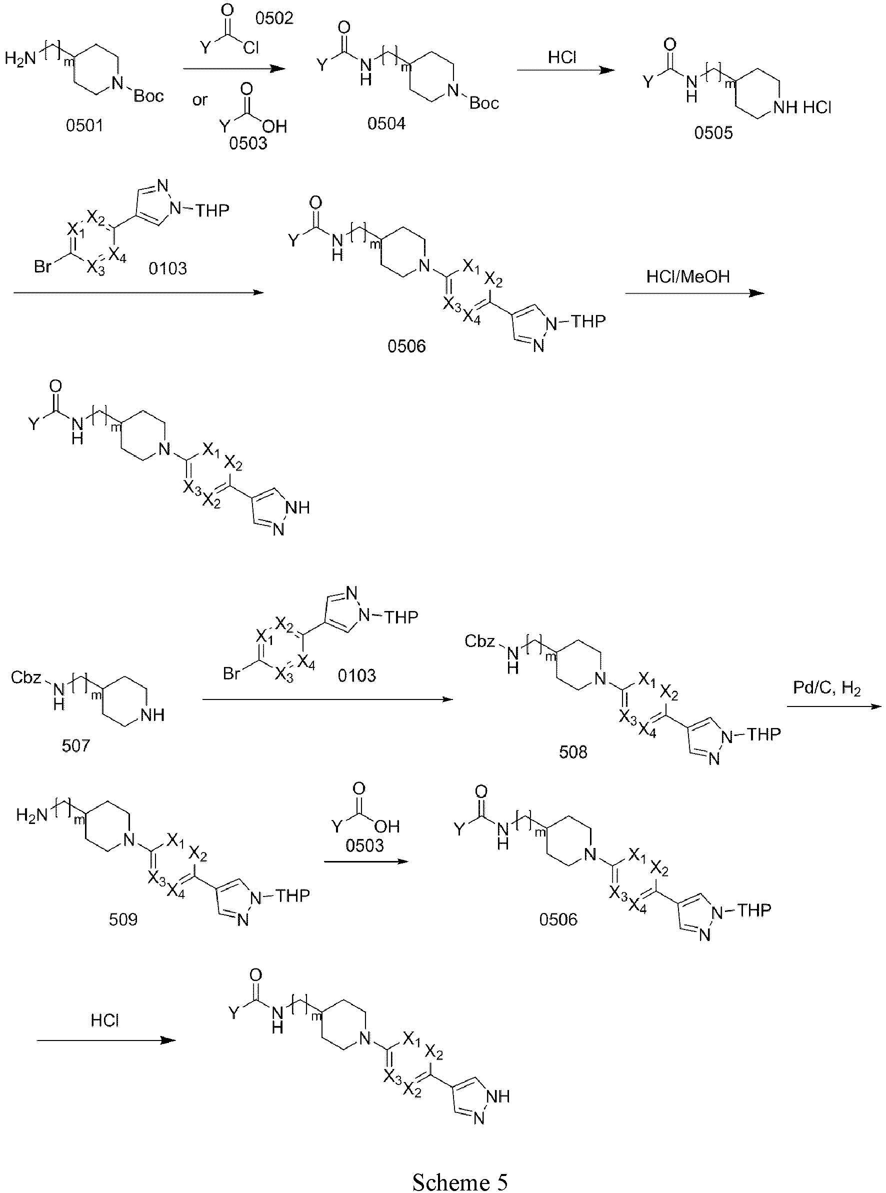

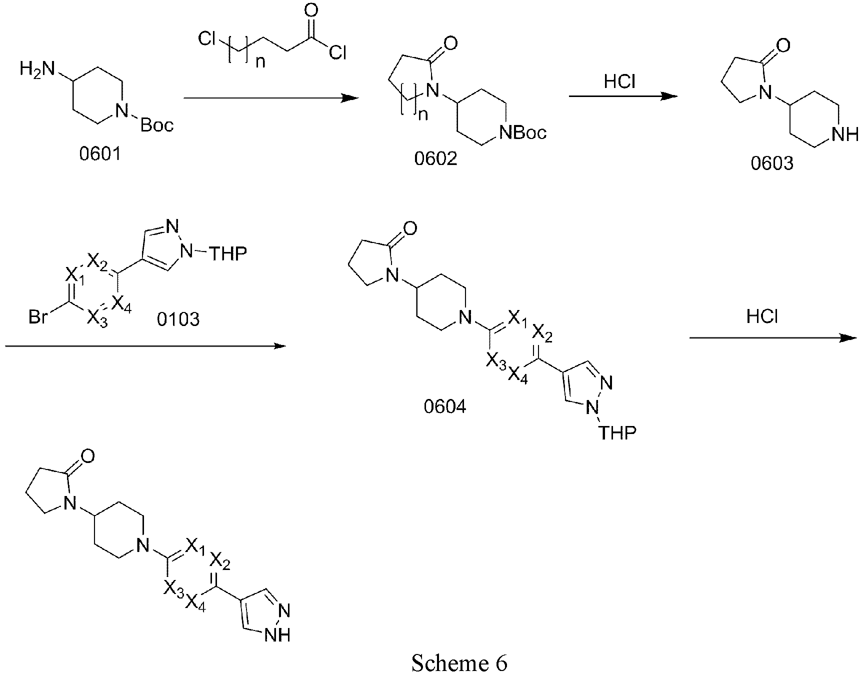

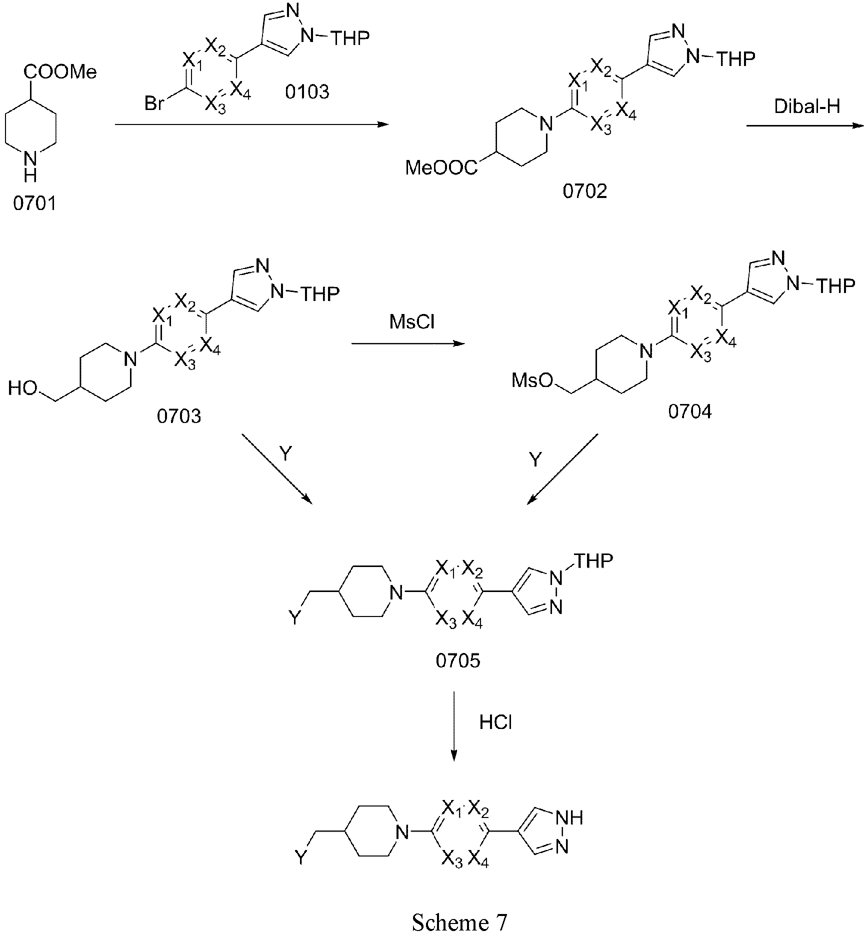

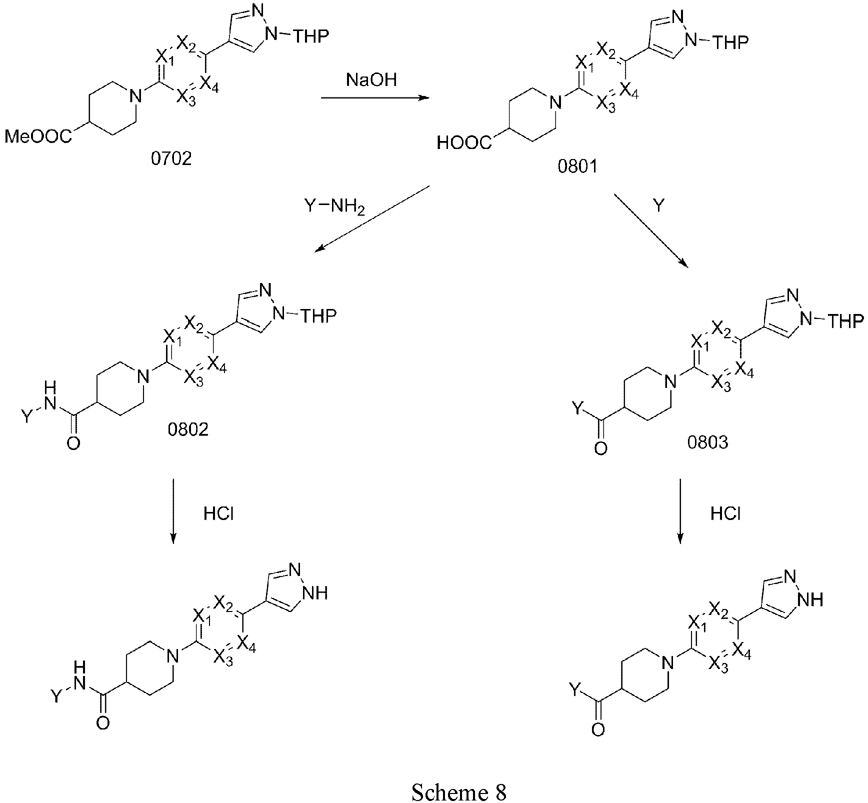

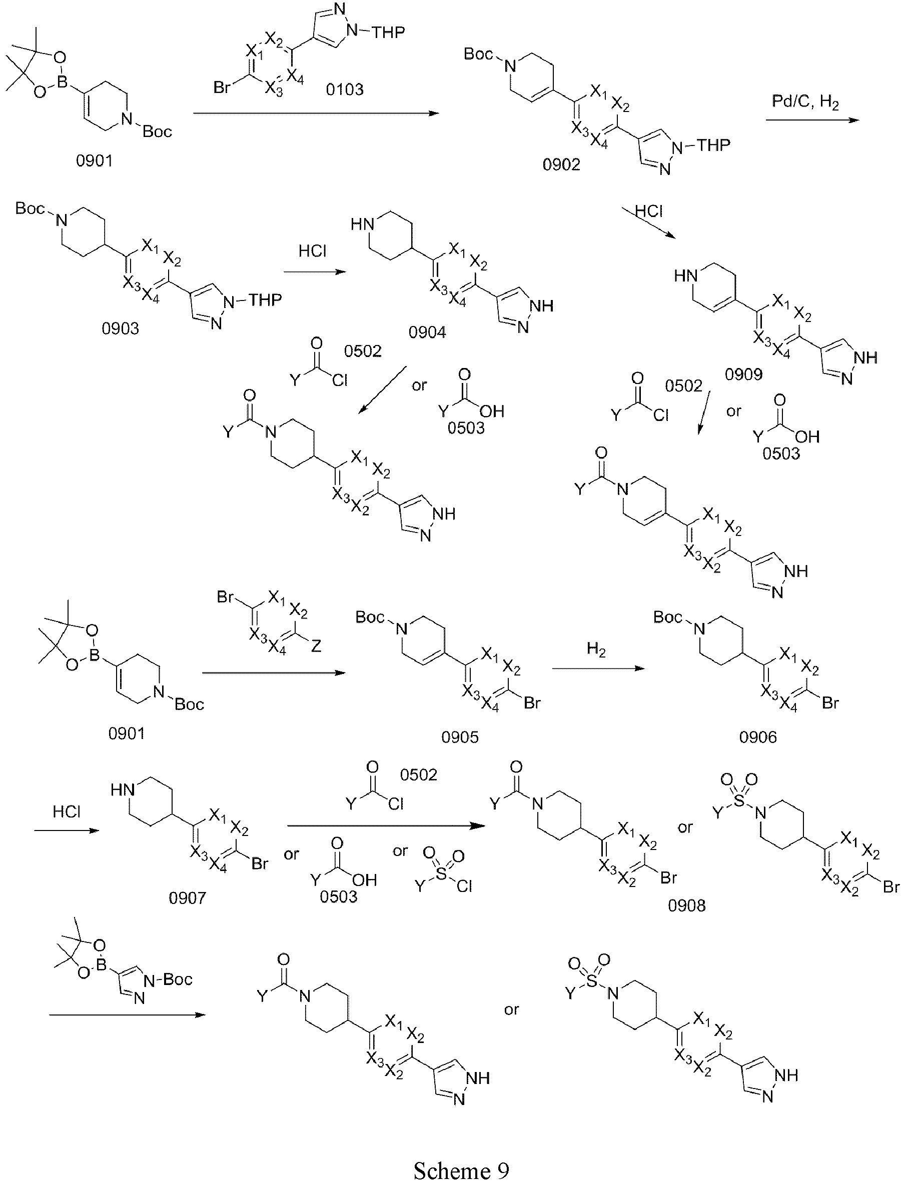

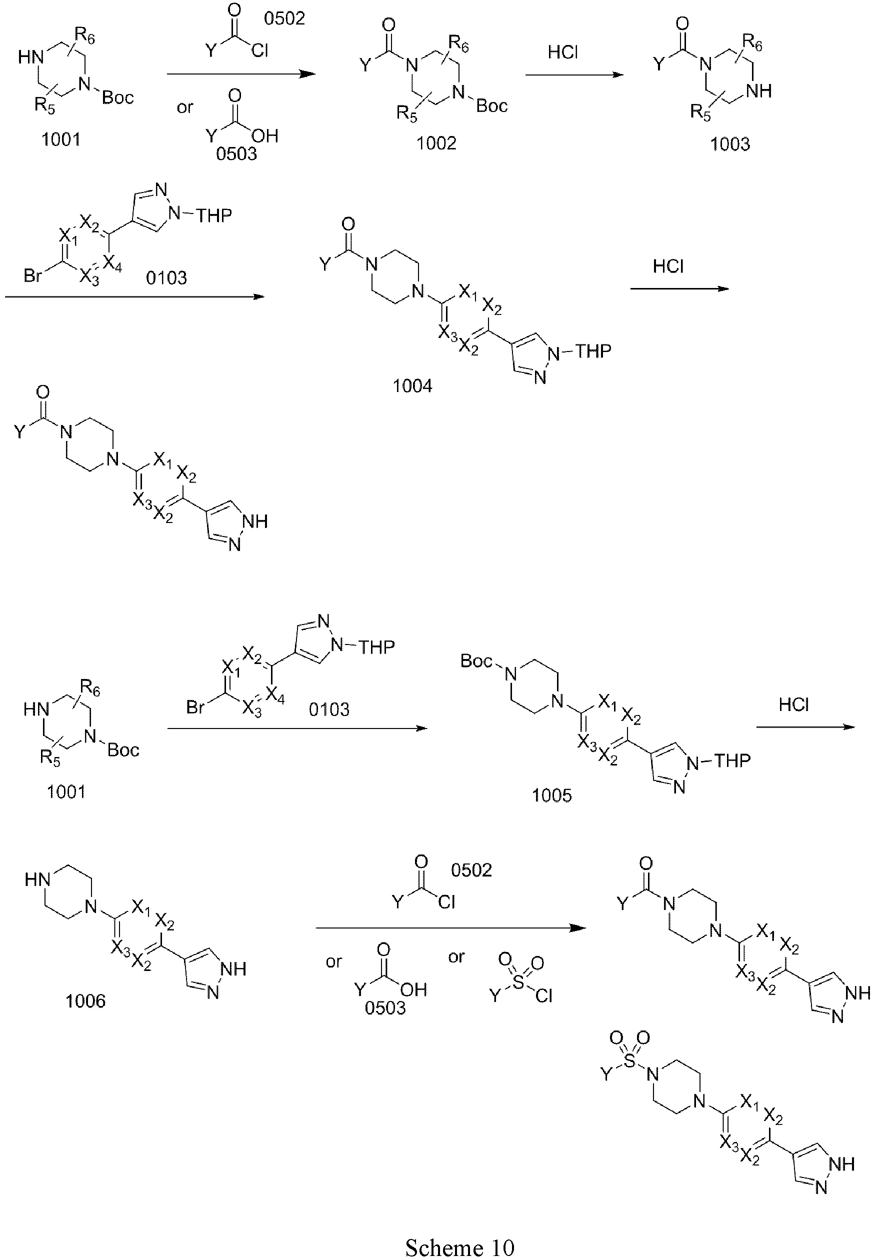

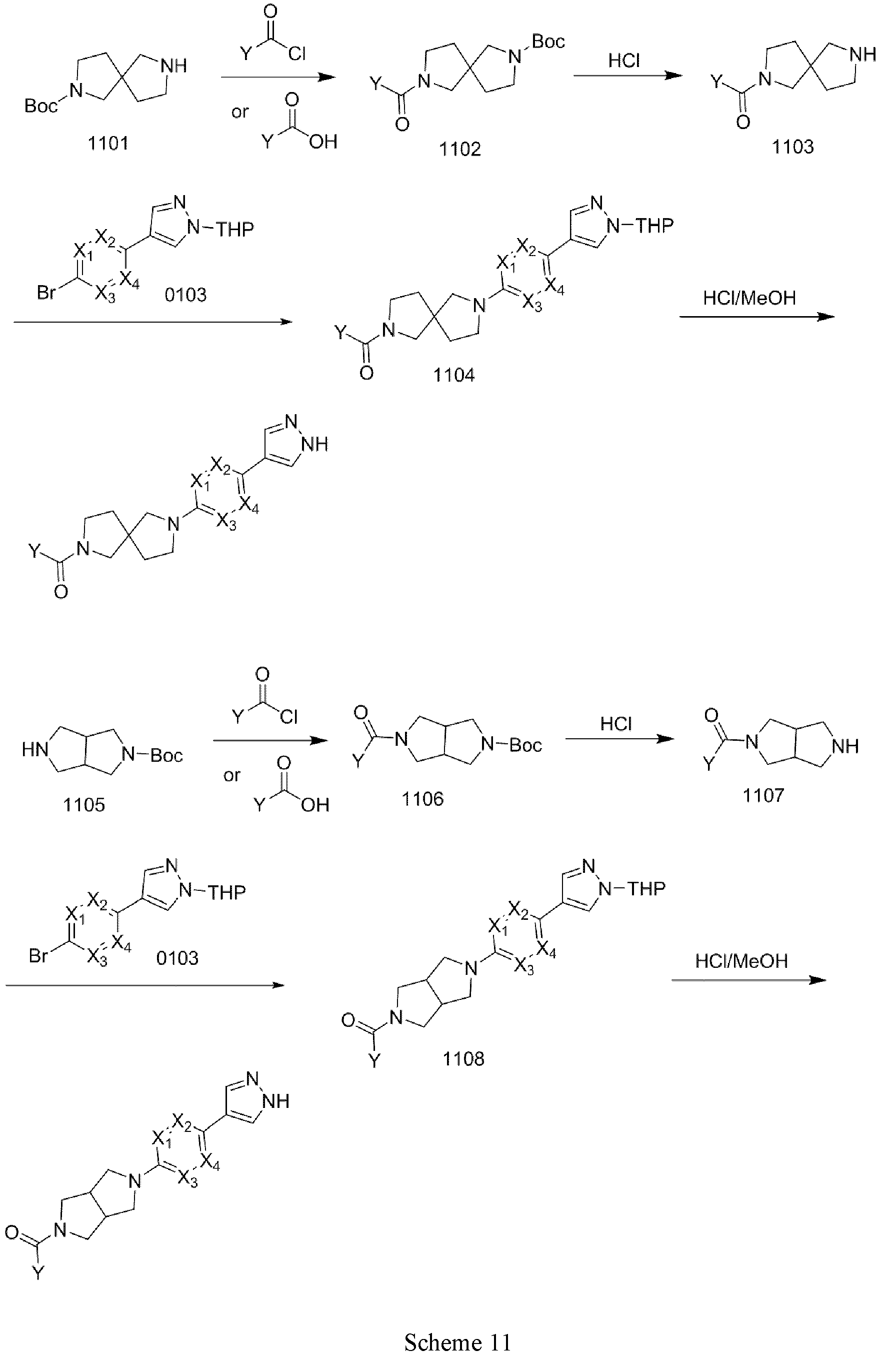

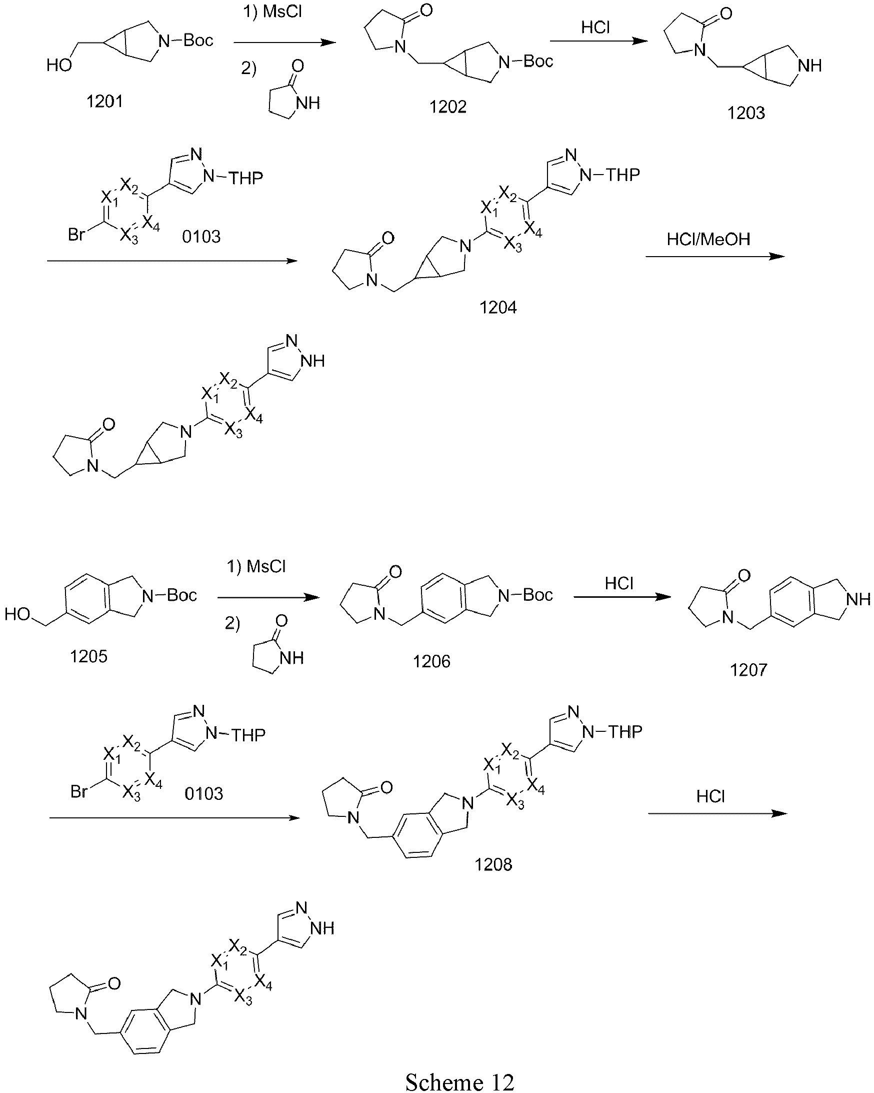

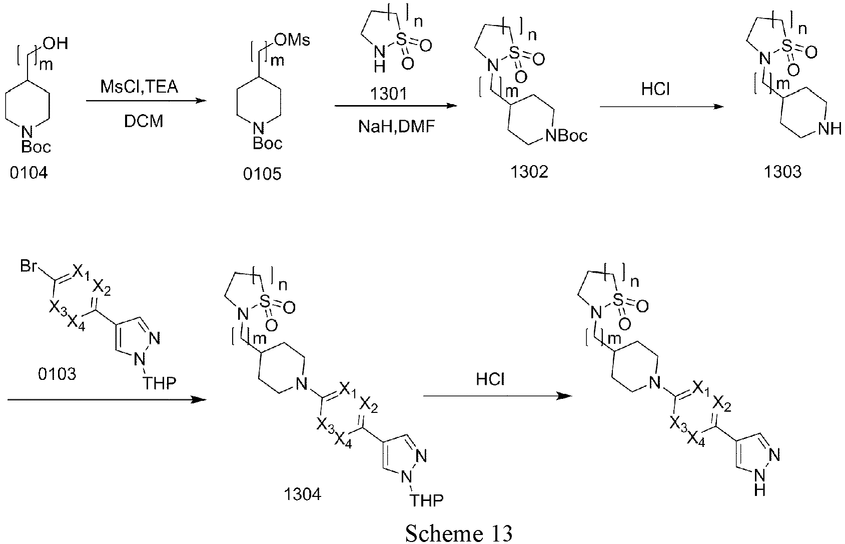

### Example 1: Preparation of 1-((1-(4-(1H-pyrazol-4-yl)phenyl)piperidin-4-yl)methyl)pyrrolidin-2-one (Compound 1) (Prepared according to Scheme 1)

Step 1a: Preparation of 4-iodo-1-(tetrahydro-2H-pyran-2-yl)-1H-pyrazole (Compound 0102-1): A mixture of 4-iodo-1H-pyrazole (15 g, 77.32 mmol, 1.0 eq.), 3,4-dihydro-2H-pyran (7.16 g, 85.11 mmol, 1.1 eq.), p-toluenesulfonic acid (1.46 g, 7.73 mmol, 0.1 eq.) in dichloromethane (100 ml) was stirred at room temperature for 3.0 hours. The reaction was concentrated under reduced pressure. The residue was purified by column chromatography on silica gel (petroleum ether/ethyl acetate = 10/1 to 5/1) to obtain a colorless oil product 4-iodo-1-(tetrahydro-2H-pyran-2-yl)-1H-pyrazole (20.8 g, yield: 96.7%). LCMS (ESI): m/z = 279 [M+1]⁺.

Step 1b: Preparation of 4-(4-bromophenyl)-1-(tetrahydro-2H-pyran-2-yl)-1H-pyrazole (Compound 0103-1): Under nitrogen protection, a mixture of (4-bromophenyl)boronic acid (0101-1) (2.4 g, 11.95 mmol, 1.0 eq.), 4-iodo-1-(tetrahydro-2H-pyran-2-yl)-1H-pyrazole (0102-1) (5.0 g, 17.9 mmol, 1.5 eq.), 1,1'-bis(diphenylphosphino)ferrocenepalladium(II) chloride (874 mg, 1.19 mmol, 0.1 eq.), sodium carbonate (3.8 g, 35.85 mmol, 3.0 eq.) in dioxane/water (30 ml/3 ml) was stirred at 90 °C for 1 hour. The reaction solution was then cooled to room temperature, diluted with water, and extracted with ethyl acetate. The organic layer was dried and concentrated under reduced pressure. The residue was purified by column chromatography on silica gel (petroleum ether/ethyl acetate = 10/1) to give a yellow solid product 4-(4-bromophenyl)-1-(tetrahydro-2H-pyran-2-yl)-1H-pyrazole (1.52 g, yield: 41.59%). LCMS (ESI): m/z = 308 [M+1]⁺..

Step 1c: Preparation of tert-butyl 4-(((methylsulfonyl)oxy)methyl)piperidine-1-carboxylate (Compound 0105-1): Under nitrogen protection, a mixture of tert-butyl 4-(hydroxymethyl)piperidine-1-carboxylate (0104-1) (10 g, 46.45 mmol, 1.0 eq.), methanesulfonyl chloride (13.3 g, 116.13 mmol, 2.5 eq.), triethylamine (18.76 g, 185.78 mmol, 4.0 eq.) in dichloromethane ( 120 ml) was stirred at room temperature for 4.0 hours. Sodium carbonate solution was added to quench the reaction, diluted with water, and extracted with dichloromethane (50 ml × 3). The organic layer was washed with saturated brine (100 ml), dried over anhydrous sodium sulfate, and concentrated under reduced pressure. The residue was purified by column chromatography on silica gel (petroleum ether/ethyl acetate = 4/1 to 1/1) to give a yellow solid product tert-butyl 4-(((methylsulfonyl)oxy)methyl)piperidine-1-carboxylate (13.1 g, yield: 96.3%). LCMS (ESI): m/z = 294 [M+1]⁺.

Step 1d: Preparation of tert-butyl 4-((2-oxopyrrolidin-1-yl)methyl)piperidine-1-carboxylate (Compound 0107-1): Under nitrogen protection, sodium hydride (60%, 2.84 g, 70.97 mmol, 1.6 eq.) was added to a mixture of 2-pyrrolidone (0106-1) (4.5 g, 53.24 mmol, 1.2 eq.) in N,N-dimethylformamide (15 ml) at 0 °C, and then stirred at room temperature for half an hour. To the reaction solution was added dropwise tert-butyl 4-(((methylsulfonyl)oxy)methyl)piperidine-1-carboxylate (13.1 g, 44.36 mmol, 1.0 eq.) in N,N-dimethylformamide (10 ml), and the mixture was reacted at 65 °C for 6 hours. The reaction was diluted with water (120 ml) and extracted with ethyl acetate (25 ml × 5). The combined organic phases was washed with saturated brine (80 ml × 3), dried over anhydrous sodium sulfate, and concentrated under reduced pressure. The residue was purified by column chromatography on silica gel (petroleum ether/ethyl acetate = 5/1 to 1/2) to obtain a light yellow oily product tert-butyl 4-((2-oxopyrrolidin-1-yl)methyl)piperidine-1-carboxylate (7.25 g, yield: 57.9%). LCMS (ESI): m/z = 283 [M+1]⁺.

Step 1e: Preparation of 1-(piperidin-4-ylmethyl)pyrrolidin-2-one (Compound 0108-1): A mixture of tert-butyl 4-((2-oxopyrrolidin-1-yl)methyl)piperidine-1-carboxylate (0107-1) (7.25 g, 25.71 mmol, 1.0 eq.) in hydrogen chloride-dioxane (4 M, 30 mL) was stirred at room temperature for 2.0 hours. The solvent was removed under reduced pressure, and the residue was diluted with water (50 mL), adjusted to pH = 9 with sodium carbonate solution, and extracted with dichloromethane (10 mL × 15). The organic phase was dried over anhydrous sodium sulfate and concentrated under reduced pressure to give 1-(piperidin-4-ylmethyl)pyrrolidin-2-one ( 3.18 g, yield: 67.9%) as a yellow solid. LCMS (ESI): m/z = 183 [M+1]⁺.

Step 1f: Preparation of 1-((1-(4-(1-(tetrahydro-2H-pyran-2-yl)-1H-pyrazol-4-yl)phenyl)piperidin-4-yl)methyl) pyrrolidin-2-one (Compound 0109-1): Under nitrogen protection, a mixture of 1-(piperidin-4-ylmethyl)pyrrolidin-2-one (0108-1) (2.03 g, 11.17 mmol, 1.2 eq.), sodium tert-butoxide (2.24 g, 23.3 mmol, 2.5 eq.), 4,5-bis(diphenylphosphino-9,9-dimethylxanthene (539 mg, 0.932 mmol, 0.1 eq.) and tris(dibenzylideneacetone)dipalladium(0) (427 mg, 0.466 mmol, 0.05 eq.) in toluene (30 ml) was heated to 120 °C. A mixture of 4-(4-bromophenyl)-1-(tetrahydro-2H-pyran-2-yl)-1H-pyrazole (0103-1) (2.86 g, 9.32 mmol, 1.0 eq.) in toluene (15 ml) was added dropwise, and the mixture was stirred at 120 °C for 10 hours. The reaction was diluted with water (100 ml) and extracted with ethyl acetate (30 ml × 3). The organic phase was washed with saturated brine (80 ml × 1), dried over anhydrous sodium sulfate, and concentrated under reduced pressure. The residue was purified by column chromatography on silica gel (ethyl acetate/methanol = 200/1 to 100/1) to obtain a yellow solid product 1-((1-(4-(1-(tetrahydro-2H-pyran-2-yl)-1H-pyrazol-4-yl)phenyl)piperidin-4-yl)methyl) pyrrolidin-2-one (2.09 g, yield: 54.8%). LCMS (ESI): m/z =409[M+1]⁺.

Step 1g: Preparation of 1-((1-(4-(1H-pyrazol-4-yl)phenyl)piperidin-4-yl)methyl)pyrrolidin-2-one (Compound 1): A mixture of 1-((1-(4-(1-(tetrahydro-2H-pyran-2-yl)-1H-pyrazol-4-yl)phenyl)piperidin-4-yl)methyl) pyrrolidin-2-one (0109-1) (2.09 g, 5.11 mmol, 1.0 eq.) in hydrogen chloride-dioxane (4 M, 20 mL) was stirred at room temperature for 4.0 hours. The solvent was removed under reduced pressure, and the residue was diluted with water (20 mL), adjusted to pH = 9 with sodium carbonate solution, and extracted with dichloromethane (8 mL × 5). The organic phase was dried over anhydrous sodium sulfate and concentrated under reduced pressure. The residue was purified by column chromatography on silica gel (dichloromethane/methanol = 20/1 to 15/1) to give 1-((1-(4-(1H-pyrazol-4-yl)phenyl)piperidin-4-yl)methyl)pyrrolidin-2-one (1.16 g, yield: 70.1%) as a white solid. LCMS(ESI): m/z =325[M+1]⁺. ¹ H NMR (500 MHz, DMSO) δ 12.75 (s, 1H), 7.89 (d, *J =* 105.8 Hz, 2H), 7.41 (d, *J =* 8.6 Hz, 2H), 6.91 (d, *J =* 8.6 Hz, 2H), 3.66 (d, *J =* 12.4 Hz, 2H), 3.40 - 3.32 (m, 4H), 3.08 (d, *J =* 7.3 Hz, 2H), 2.63 (t, *J =* 11.1 Hz, 2H), 2.23 (t, *J =* 8.0 Hz, 2H), 2.00 - 1.89 (m, 2H), 1.74 (ddd, *J =* 11.1, 7.5, 3.8 Hz, 1H), 1.65 (d, *J =* 12.3 Hz, 2H).

### Example 2: Preparation of 1-((1-(2-fluoro-4-(1H-pyrazol-4-yl)phenyl)piperidin-4-yl)methyl)pyrrolidin-2-one (Compound 2) (Prepared according to Scheme 1)

Step 2a: Preparation of 4-(4-bromo-3-fluorophenyl)-1-(tetrahydro-2H-pyran-2-yl)-1H-pyrazole (Compound 0103-2): Under nitrogen protection, a mixture of (4-bromo-3-fluorophenyl)boronic acid (0101-2) (500 mg, 2.28 mmol, 1.0 eq.), 4-iodo-1-(tetrahydro-2H-pyran-2-yl)-1H-pyrazole (0102-1) (635 mg, 2.28 mmol, 1.0 eq.), [1,1'-bis(diphenylphosphino)ferrocene]palladium dichloride (166.7 mg, 0.228 mmol, 0.1 eq.), potassium carbonate (787 mg, 5.70 mmol, 2.5 eq.) in dioxane/water (15 ml/3 ml) was stirred at 70 °C for 3 hours. After cooling to room temperature, the reaction was quenched with water and extracted with ethyl acetate. The organic phase was washed with saturated brine, dried over anhydrous sodium sulfate, and concentrated under reduced pressure. The residue was purified by column chromatography on silica gel (eluent: petroleum ether/ethyl acetate = 10/1 to 4/1) to obtain a yellow oily product 4-(4-bromo-3-fluorophenyl)-1-(tetrahydro-2H-pyran-2-yl)-1H-pyrazole (550 mg, yield: 74.22%). MS (ES⁺): m/z=327 [M+H]⁺.

Step 2b: Preparation of 1-((1-(2-fluoro-4-(1-(tetrahydro-2H-pyran-2-yl)-1H-pyrazol-4-yl)phenyl)piperidin-4-yl)methyl)pyrrolidin-2-one (Compound 0109-2): Under nitrogen protection, a mixture of 1-(piperidin-4-ylmethyl)pyrrolidin-2-one (0108-1) (84 mg, 0.46 mmol, 1.0 eq.), 4-(4-bromo-3-fluorophenyl)-1-(tetrahydro-2H-pyran-2-yl)-1H-pyrazole (0103-2) (150 mg, 0.46 mmol, 1.0 eq.), sodium tert-butoxide (111 mg, 1.15 mmol, 2.5 eq.), 4,5-bis(diphenylphosphino-9,9-dimethylxanthene) (27 mg, 0.05 mmol, 0.1 eq.) and tris(dibenzylideneacetone)dipalladium(0) (22 mg, 0.023 mmol, 0.05 eq.) in toluene (15 ml) was stirred at 120 °C overnight. After cooling to room temperature, the reaction was quenched with water and extracted with ethyl acetate. The organic phase was washed with saturated brine, dried over anhydrous sodium sulfate, and concentrated under reduced pressure. The residue was purified by column chromatography on silica gel (eluent: ethyl acetate/methanol = 150/1 to 80/1) to give 1-((1-(2-fluoro-4-(1-(tetrahydro-2H-pyran-2-yl)-1H-pyrazol-4-yl)phenyl)piperidin-4-yl)methyl)pyrrolidin-2-one (75 mg, yield: 38.27%) as an orange solid. MS (ES⁺): m/z = 427 [M+H]⁺.

Step 2c: Preparation of 1-((1-(2-fluoro-4-(1H-pyrazol-4-yl)phenyl)piperidin-4-yl)methyl)pyrrolidin-2-one ( Compound 2): A mixture of 1-((1-(2-fluoro-4-(1-(tetrahydro-2H-pyran-2-yl)-1H-pyrazol-4-yl)phenyl)piperidin-4-yl)methyl)pyrrolidin-2-one (0109-2) (70 mg, 0.16 mmol, 1.0 eq.) in hydrogen chloride-dioxane solution (4 M, 5 ml) was stirred at room temperature for 1 hour. The solvent was removed under reduced pressure. The residue was diluted with water, adjusted to pH = 9 with sodium carbonate solution, and extracted with dichloromethane. The organic phase was dried and concentrated under reduced pressure. The residue was purified by prep-TLC (silica gel, eluent: dichloromethane/methanol = 15/1) to give 1-((1-(2-fluoro-4-(1H-pyrazol-4-yl)phenyl)piperidin-4-yl)methyl)pyrrolidin-2-one (35 mg, yield: 62.42%) as a yellow solid. MS (ESES⁺): m/z = 343 [M+H]⁺. ¹ H NMR (500 MHz, DMSO) δ 12.86 (s, 1H), 8.12 (s, 1H), 7.86 (s, 1H), 7.34 (ddd, *J =* 11.2, 10.1, 1.8 Hz, 2H), 6.99 (t, *J =* 8.9 Hz, 1H), 3.35 (dd, *J =* 17.9, 10.8 Hz, 4H), 3.11 (d, *J =* 7.1 Hz, 2H), 2.62 (t, *J =* 10.9 Hz, 2H), 2.23 (t, *J =* 8.1 Hz, 2H), 2.02 - 1.90 (m, 2H), 1.77 - 1.58 (m, 3H), 1.40 - 1.26 (m, 2H).

### Example 3: Preparation of 1-((1-(3-fluoro-4-(1H-pyrazol-4-yl)phenyl)piperidin-4-yl)methyl)pyrrolidin-2-one (Compound 3) (Prepared according to Scheme 1)

Step 3a: Preparation of 4-(4-bromo-2-fluorophenyl)-1-(tetrahydro-2H-pyran-2-yl)-1H-pyrazole (Compound 0103-3): Under nitrogen protection, a mixture of (4-bromo-2-fluorophenyl)boronic acid (0101-3) (500 mg, 2.28 mmol, 1.0 eq.), 4-iodo-1-(tetrahydro-2H-pyran-2-yl)-1H-pyrazole (0102-1) (760 mg, 2.74 mmol, 1.2 eq.), [1,1'-bis(diphenylphosphino)ferrocene]dichloropalladium (166.7 mg, 0.228 mmol, 0.1 eq.), sodium carbonate (725 mg, 6.84 mmol, 3.0 eq.) in dioxane/water (10 ml/2 ml) was stirred at 70 °C for 2.0 hours. The reaction was diluted with water and extracted with ethyl acetate. The organic layer was dried and concentrated under reduced pressure. The residue was purified by column chromatography on silica gel (petroleum ether/ethyl acetate = 10/1 to 4/1) to give a yellow solid product 4-(4-bromo-2-fluorophenyl)-1-(tetrahydro-2H-pyran-2-yl)-1H-pyrazole (420 mg, yield: 56.6%).

Step 3b: Preparation of 1-((1-(3-fluoro-4-(1-(tetrahydro-2H-pyran-2-yl)-1H-pyrazol-4-yl)phenyl)piperidin-4-yl)methyl)pyrrolidin-2-one (Compound 0109-3): Under nitrogen protection, a mixture of 1-(piperidin-4-ylmethyl)pyrrolidin-2-one hydrochloride (0108-1) (80 mg, 0.44 mmol, 1.0 eq.), 4-(4-bromo-2-fluorophenyl)-1-(tetrahydro-2H-pyran-2-yl)-1H-pyrazole (0103-3) (171 mg, 0.527 mmol, 1.2 eq.), sodium tert-butoxide (105.7 mg, 1.10 mmol, 2.5 eq.), 4,5-bis(diphenylphosphino-9,9-dimethylxanthene) (25.5 mg, 0.044 mmol, 0.1 eq.) and tris(dibenzylideneacetone)dipalladium(0) (20 mg, 0.022 mmol, 0.05 eq.) in toluene (12 ml) was stirred at 120°C overnight. The reaction was diluted with water and extracted with ethyl acetate, and the organic phase was dried and concentrated under reduced pressure. The residue was purified by prep-TLC (silica gel, ethyl acetate/methanol = 150/1 to 80/1) to obtain a yellow solid product 1-((1-(3-fluoro-4-(1-(tetrahydro-2H-pyran-2-yl)-1H-pyrazol-4-yl)phenyl)piperidin-4-yl)methyl)pyrrolidin-2-one (97 mg, yield: 51.7%). LCMS (ESI): m/z = 427 [M+1]⁺.

Step 3c: Preparation of 1-((1-(3-fluoro-4-(1H-pyrazol-4-yl)phenyl)piperidin-4-yl)methyl)pyrrolidin-2-one (Compound 3): A mixture of 1-((1-(3-fluoro-4-(1-(tetrahydro-2H-pyran-2-yl)-1H-pyrazol-4-yl)phenyl)piperidin-4-yl) methyl)pyrrolidin-2-one (97 mg, 0.227 mmol, 1.0 eq.) in hydrochloric acid-dioxane (4 M, 5 mL) was stirred at room temperature for 4.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product 1-((1-(3-fluoro-4-(1H-pyrazol-4-yl)phenyl)piperidin-4-yl)methyl)pyrrolidin-2-one (60 mg, yield: 77.3%). LCMS (ESI): m/z = 343 [M+1]⁺. ¹ H NMR (500 MHz, DMSO) δ 12.88 (s, 1H), 7.97 (s, 1H), 7.80 (s, 1H), 7.49 (t, *J =* 9.1 Hz, 1H), 6.75 (dd, *J =* 7.9, 6.4 Hz, 2H), 3.72 (d, *J =* 12.6 Hz, 2H), 3.35 (t, *J =* 7.0 Hz, 2H), 3.08 (d, *J =* 7.3 Hz, 2H), 2.67 (dd, *J =* 22.9, 12.4 Hz, 2H), 2.23 (t, *J =* 8.0 Hz, 2H), 2.00 - 1.89 (m, 2H), 1.77 (ddd, *J =* 11.1, 7.4, 3.8 Hz, 1H), 1.63 (d, *J =* 12.0 Hz, 2H), 1.27 - 1.06 (m, 2H).

### Example 4: Preparation of 1-((1-(2,3-difluoro-4-(1H-pyrazol-4-yl)phenyl)piperidin-4-yl)methyl)pyrrolidin-2-one (Compound 4) (Prepared according to Scheme 2)

Step 4a: Preparation of 4-(4-bromo-2,3-difluorophenyl)-1H-pyrazole (Compound 0205-4): Under nitrogen protection, a mixture of 1,4-dibromo-2,3-difluorobenzene (0201-4) (800 mg, 2.94 mmol, 1.0 eq.), (1H-pyrazol-4-yl)boronic acid (0202-4) (346 mg, 3.08 mmol, 1.05 eq.), [1,1'-bis(diphenylphosphino)ferrocene]dichloropalladium (103.2 mg, 0.147 mmol, 0.05 eq.) and sodium carbonate (623 mg, 5.88 mmol, 2.0 eq.) in dioxane/water (volume ratio of 5/1, 33 ml) was stirred at 80 °C for 8.0 hours. The reaction was diluted with water and extracted with ethyl acetate. The organic layer was dried and concentrated under reduced pressure. The residue was purified by column chromatography on silica gel (petroleum ether/ethyl acetate = 20/1 to 5/1) to give a yellow solid product 4-(4-bromo-2,3-difluorophenyl)-1H-pyrazole (345 mg, yield: 45.5%). MS (ES⁺): m/z=259 (M+H)⁺.

Step 4b: Preparation of 4-(4-bromo-2,3-difluorophenyl)-1-(tetrahydro-2H-pyran-2-yl)-1H-pyrazole (Compound 0103-4): 3,4-dihydro-2H-pyran (120 mg, 1.41 mmol, 1.2 eq.) was added dropwise to a mixture of 4-(4-bromo-2,3-difluorophenyl)-1H-pyrazole (0205-4) (305 mg, 1.18 mmol, 1.0 eq.), p-toluenesulfonic acid (33 mg, 0.177 mmol, 0.15 eq.) in dichloromethane (12 ml), and the reaction was stirred at room temperature for 8 hours. Water and dichloromethane were added for extraction, and the organic layer was washed with brine and concentrated under reduced pressure to give a yellow solid product 4-(4-bromo-2,3-difluorophenyl)-1-(tetrahydro-2H-pyran-2-yl)-1H-pyrazole (220 mg, yield: 54.4%). MS (ES⁺): m/z=343(M+H)⁺.

Step 4c: Preparation of 1-((1-(2,3-difluoro-4-(1-(tetrahydro-2H-pyran-2-yl)-1H-pyrazol-4-yl)phenyl)piperidin -4-yl)methyl)pyrrolidone (Compound 0109-4): Under nitrogen protection, a mixture of 1-(piperidin-4-ylmethyl)pyrrolidin-2-one (0108-1) (140 mg, 0.76 mmol, 1.3 eq.), 4-(4-bromo-2,3-difluorophenyl)-1-(tetrahydro-2H-pyran-2-yl)-1H-pyrazole (0103-4) (200 mg, 0.588 mmol, 1.0 eq.), sodium tert-butoxide (157 mg, 1.764 mmol, 3.0 eq.), 4,5-bis(diphenylphosphino)-9,9-dimethylxanthene (33.8 mg, 0.058 mmol, 0.1 eq.) and tris(dibenzylideneacetone)dipalladium(0) (53.8 mg, 0.058 mmol, 0.1 eq.) in toluene (20 ml) was stirred at 120°C overnight. The reaction was diluted with water and extracted with ethyl acetate, and the organic phase was dried and concentrated under reduced pressure. The residue was purified by prep-TLC (silica gel, dichloromethane/methanol = 150/1 to 50/1) to obtain a yellow solid product 1-((1-(2,3-difluoro-4-(1-(tetrahydro-2H-pyran-2-yl)-1H-pyrazol-4-yl)phenyl)piperidin -4-yl)methyl)pyrrolidone (90 mg, yield: 34.4%). LCMS (ESI): m/z = 445 [M+1]⁺.

Step 4d: Preparation of 1-((1-(2,3-difluoro-4-(1H-pyrazol-4-yl)phenyl)piperidin-4-yl)methyl)pyrrolidin-2-one (Compound 4): A mixture of 1-((1-(2,3-difluoro-4-(1-(tetrahydro-2H-pyran-2-yl)-1H-pyrazol-4-yl)phenyl)piperidin -4-yl)methyl)pyrrolidin-2-one (0109-4) (90 mg, 0.202 mmol, 1.0 eq.) in hydrogen chloride-dioxane (4 M, 5 ml) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product 1-((1-(2,3-difluoro-4-(1H-pyrazol-4-yl)phenyl)piperidin-4-yl)methyl)pyrrolidin-2-one (46 mg, yield: 63.1%). LCMS (ESI): m/z = 361 [M+1]⁺. Melting point : 175~184 °C. ¹ H NMR (500 MHz, DMSO) δ 13.04 (s, 1H), 8.08 (s, 1H), 7.87 (s, 1H), 7.38 (td, *J =* 8.5, 1.8 Hz, 1H), 6.84 (t, *J =* 8.4 Hz, 1H), 3.42 - 3.32 (m, 4H), 3.14 - 3.05 (m, 2H), 2.68 (dt, *J =* 22.6, 11.3 Hz, 2H), 2.22 (q, *J =* 7.8 Hz, 2H), 2.01 - 1.89 (m, 2H), 1.80-1.62 (m, 3H), 1.34 - 1.17 (m, 2H).

### Example 5: 1-((1-(2,6-difluoro-4-(1H-pyrazol-4-yl)phenyl)piperidin-4-yl)methyl)pyrrolidin-2-one (Compound 5) (Prepared according to Scheme 2)

Step 5a: Preparation of 4-(4-bromo-3,5-difluorophenyl)-1H-pyrazole (Compound 0205-5): Under nitrogen protection, a mixture of tert-butyl 4-(4,4,5,5-tetramethyl-1,3,2-dioxaborolan-2-yl)-1H-pyrazole-1-carboxylate (0203-5) (507 mg, 1.72 mmol, 1.1 eq.), 2-bromo-1,3-difluoro-5-iodobenzene (0201-5) (500 mg, 1.57 mmol, 1.0 eq.), [1,1'-bis(diphenylphosphino)ferrocene]palladium dichloride (115 mg, 0.157 mmol, 0.15 eq.), sodium carbonate (499 mg, 4.71 mmol, 3.0 eq.) in dioxane/water (10 ml/2 ml) was stirred at 90 ° C for 2.0 hours. The reaction was diluted with water (30 ml) and extracted with ethyl acetate (8 ml × 4). The combined organic layer was washed with saturated brine (30 ml × 1), dried over anhydrous sodium sulfate, and concentrated under reduced pressure. The residue was purified by column chromatography on silica gel (petroleum ether/ethyl acetate = 5/1 to 1/1) to obtain a yellow solid product 4-(4-bromo-3,5-difluorophenyl)-1H-pyrazole (268 mg, yield: 66.1%). LCMS (ESI): m/z = 259 [M+1]⁺.

Step 5b: Preparation of 4-(4-bromo-3,5-difluorophenyl)-1-(tetrahydro-2H-pyran-2-yl)-1H-pyrazole (Compound 0103-5): A mixture of 4-(4-bromo-3,5-difluorophenyl)-1H-pyrazole (0205-5) (268 mg, 1.04 mmol, 1.0 eq.), 3,4-dihydro-2H-pyran (96.2 mg, 1.14 mmol, 1.1 eq.), p-toluenesulfonic acid (20 mg, 0.104 mmol, 0.1 eq.) in dichloromethane (8 ml) was stirred at room temperature overnight. The reaction was concentrated under reduced pressure. The residue was purified by column chromatography on silica gel (petroleum ether/ethyl acetate = 5/1 to 2/1) to obtain a yellow solid product 4-(4-bromo-3,5-difluorophenyl)-1-(tetrahydro-2H-pyran-2-yl)-1H-pyrazole (259 mg, yield: 72.6%). LCMS (ESI): m/z = 343 [M+1]⁺.

Step 5c: Preparation of 1-((1-(2,6-difluoro-4-(1-(tetrahydro-2H-pyran-2-yl)-1H-pyrazol-4-yl)phenyl)piperidin -4-yl)methyl)pyrrolidin-2-one (Compound 0109-5): Under nitrogen protection, a mixture of 4-(4-bromo-3,5-difluorophenyl)-1-(tetrahydro-2H-pyran-2-yl)-1H-pyrazole (0103-5) (209 mg, 0.609 mmol, 1.0 eq.), 1- (piperidine-4-ylmethyl) pyrrolidin-2-one (0108-1) (122 mg, 0.67 mmol, 1.1 eq.), sodium tert-butoxide (146 mg, 1.52 mmol, 2.5 eq.), 4,5-bis(diphenylphosphino-9,9-dimethylxanthene) (35 mg, 0.0609 mmol, 0.1 eq.) and tris(dibenzylideneacetone)dipalladium(0) (28 mg, 0.0305 mmol, 0.05 eq.) in toluene (6 ml) was stirred at 120 °C for 10 hours. The reaction was diluted with water (20 ml) and extracted with ethyl acetate (8 ml × 3). The combined organic phase was washed with saturated brine (20 ml × 1), dried over anhydrous sodium sulfate and concentrated under reduced pressure. The residue was purified by column chromatography on silica gel (ethyl acetate/methanol = 200/1 to 100/1) to give yellow solid product 1-((1-(2,6-difluoro-4-(1-(tetrahydro-2H-pyran-2-yl)-1H-pyrazol-4-yl)phenyl)piperidin -4-yl)methyl)pyrrolidin-2-one (69 mg, yield: 25.6%). LCMS (ESI): m/z = 445 [M+1]⁺.

Step 5d: Preparation of 1-((1-(2,6-difluoro-4-(1H-pyrazol-4-yl)phenyl)piperidin-4-yl)methyl)pyrrolidin-2-one (Compound 5): A mixture of 1-((1-(2,6-difluoro-4-(1-(tetrahydro-2H-pyran-2-yl)-1H-pyrazol-4-yl)phenyl)piperidin -4-yl)methyl)pyrrolidin-2-one (0109-5) (54 mg, 0.122 mmol, 1.0 eq.) in hydrogen chloride-dioxane (4 M, 2 mL) was stirred at room temperature for 1.0 h. The solvent was removed under reduced pressure, and the residue was diluted with water (8 mL), adjusted to pH = 9 with sodium carbonate solution, and extracted with dichloromethane (3 mL × 5). The combined organic phase was dried over anhydrous sodium sulfate and concentrated under reduced pressure. The residue was purified by prep-TLC (silica gel, dichloromethane/methanol = 12/1) to give a white solid product 1-((1-(2,6-difluoro-4-(1H-pyrazol-4-yl)phenyl)piperidin-4-yl)methyl)pyrrolidin-2-one (37.1 mg, yield: 86.3%). LCMS(ESI): m/z =361[M+1]⁺. ¹ H NMR (500 MHz, DMSO) δ 12.95 (s, 1H), 8.22 (s, 1H), 7.94 (s, 1H), 7.30 (t, *J =* 8.0 Hz, 2H), 3.35 (t, *J =* 7.0 Hz, 2H), 3.19 - 3.05 (m, 4H), 2.98 (t, *J =* 11.3 Hz, 2H), 2.23 (t, *J =* 8.1 Hz, 2H), 1.94 (dd, *J =* 14.9, 7.4 Hz, 2H), 1.76 - 1.67 (m, 1H), 1.69 (d, *J =* 12.4 Hz, 2H), 1.31 - 1.23 (m, 2H).

### Example 6: Preparation of 1-((1-(2,5-difluoro-4-(1H-pyrazol-4-yl)phenyl)piperidin-4-yl)methyl)pyrrolidin-2-one (Compound 6) (Prepared according to Scheme 2)

Step 6a: Preparation of 4-(4-bromo-2,5-difluorophenyl)-1H - pyrazole (compound 0205-6) : Under nitrogen protection, 1,4-dibromo-2,5-difluorobenzene (0201-6) (653 mg, 2.4 mmol, 1.2 eq.), 4-(1H - pyrazol-4-yl)boronic acid (224 mg, 2.0 mmol, 1.0 eq.), sodium carbonate (424 mg, 4.0 mmol, 2.0 eq.) and [1,1'-bis(diphenylphosphino)ferrocene]dichloropalladium dichloromethane complex (81 mg, 0.1 mmol, 0.05 eq.) were stirred in a mixed solvent of 20 mL dioxane and 2 mL water at 90 °C overnight. After cooling to room temperature, the reaction was extracted with ethyl acetate, the organic phase was washed with water and saturated brine, then the organic phase was distilled under reduced pressure. The residue was purified by column chromatography on silica gel (eluent: dichloromethane/methanol = 200/1 to 30/1) to obtain a yellow solid product 4-(4-bromo-2,5-difluorophenyl)-1H-pyrazole (119 mg, yield: 22.8%). MS (ES⁺): m/z =259 (M+H)⁺.

Step 6b: Preparation of 4-(4-bromo-2,5-difluorophenyl)-1-(tetrahydro- 2H -pyran-2-yl)-1H - pyrazole (Compound 0103-6): 4-(4-bromo-2,5-difluorophenyl) -1H -pyrazole (0205-6) (119 mg, 0.46 mmol, 1.0 eq.) was dissolved in 20 mL dichloromethane, and 3,4-dihydro-2H-pyran (78 mg, 092 mmol, 2.0 eq.) and p-toluenesulfonic acid monohydrate (18 mg, 0.092 mmol, 0.2 eq.) were added. The mixture was stirred at room temperature for 2 hours. The reaction was quenched with aqueous sodium carbonate solution and dichloromethane was added for extraction, and the mixture was and washed with water and saturated brine. The organic phase was concentrated under reduced pressure, and the residue was purified by column chromatography on silica gel (eluent: petroleum ether/ethyl acetate = 10/1 to 4/1) to give a yellow oily product 4-(4-bromo-2,5-difluorophenyl)-1-(tetrahydro- 2H -pyran-2-yl)-1H - pyrazole (169 mg, crude product). MS (ES⁺): m/z =343(M+H) ⁺.

Step 6c: Preparation of 1-((1-(2,5-difluoro-4-(1-(tetrahydro- 2H -pyran-2-yl)-1H - pyrazol-4-yl)phenyl)piperidin-4-yl)methyl)pyrrolidin-2-one (Compound 0109-6): Under nitrogen protection, a mixture of 4- (4-bromo-2,5-difluorophenyl) -1-(tetrahydro-2H-pyran-2-yl) -1H-pyrazole (0103-6) (134 mg, 0.39 mmol, 1.0 eq.), 1-(piperidin-4-ylmethyl)pyrrolidin-2-one ((0108-1) 86 mg, 0.47 mmol, 1.2 eq.), sodium tert-butoxide (154 mg, 1.6 mmol, 4.0 eq.), 4,5-bis(diphenylphosphino)-9,9-dimethylxanthene (47 mg, 0.08 mmol, 0.2 eq.) and tris(dibenzylideneacetone)dipalladium(0) (37 mg, 0.04 mmol, 0.1 eq.) in toluene (15 ml) was stirred at 120 °C overnight. After cooling to room temperature, the reaction was filtered through celite and the filter cake was washed with ethyl acetate. The filtrate was concentrated under reduced pressure, and the residue was purified by prep-TLC (silica gel, eluent: dichloromethane/methanol = 20/1) to give yellow solid product 1-((1-(2,5-difluoro-4-(1-(tetrahydro-2H- pyran -2-yl)-1H-pyrazol-4-yl)phenyl)piperidin-4-yl)methyl)pyrrolidin-2-one (33 mg, yield: 18.9%). MS (ES⁺): m/z =445 (M+H)⁺.

Step 6d: Preparation of 1-((1-(2,5-difluoro-4-(1H-pyrazol-4-yl)phenyl)piperidin-4-yl)methyl)pyrrolidin-2-one (Compound 6): 1-((1-(2,5-difluoro-4-(1-(tetrahydro-2H - pyran-2-yl)-1H-pyrazol-4-yl)phenyl)piperidin-4-yl)methyl)pyrrolidin-2-one (0109-6) (33 mg, 0.074 mmol, 1.0 eq.) was added to 5 mL 4M hydrogen chloride-methanol solution and the reaction was stirred at room temperature for 2 hours. The mixture was concentrated to dryness under reduced pressure. Water was added and the pH was adjusted to 10 with saturated aqueous sodium carbonate solution. Ethyl acetate was added for extraction, washed with water, the organic phase was dried over anhydrous sodium sulfate, concentrated under reduced pressure, and the residue was purified by prep-TLC (silica gel, eluent: dichloromethane/methanol = 12/1) to obtain a yellow solid product 1-((1-(2,5-difluoro-4-(1H-pyrazol-4-yl)phenyl)piperidin-4-yl)methyl)pyrrolidin-2-one (21 mg, yield: 78.6%). MS (ES⁺): m/z =361 (M+H)⁺. Melting point: 220 ° C to brown. ¹ H NMR (500 MHz, DMSO) δ 13.00 (s, 1H), 8.09 (s, 1H), 7.89 (s, 1H), 7.51 (dd, *J =* 13.7, 7.3 Hz, 1H), 6.87 (dd, *J =* 12.8, 7.6 Hz, 1H), 3.44 - 3.34 (m, 4H), 3.10 (d, *J =* 7.2 Hz, 2H), 2.64 (t, *J =* 11.1 Hz, 2H), 2.23 (t, *J =* 8.0 Hz, 2H), 2.01 - 1.88 (m, 2H), 1.80 - 1.71 (m, 1H), 1.67 (d, *J =* 13.2 Hz, 2H), 1.26 (dd, *J =* 12.5, 5.2 Hz, 2H).

### Example 7: Preparation of 1-((1-(4-(1H-pyrazol-4-yl)-2-(trifluoromethyl)phenyl)piperidin-4-yl)methyl)pyrro lidin-2-one (Compound 10) (Prepared according to Scheme 2)

Step 7a: Preparation of 4-(4-bromo-3-(trifluoromethyl)phenyl)-1H-pyrazole (Compound 0205-10): To a mixture of 1-bromo-4-iodo-2-(trifluoromethyl)benzene (0201-10) (0.5 g, 1.43 mmol, 1.0 eq.), [1,1'-bis(diphenylphosphino)ferrocene]dichloropalladium (83 mg, 0.11 mmol, 0.08 eq.) and sodium carbonate (453 mg, 4.27 mmol, 3.0 eq.) in dioxane (10 ml) and water (1 ml) was added tert-butyl 4-(4,4,5,5-tetramethyl-1,3,2-dioxaborolan-2-yl)-1H-pyrazole-1-carboxylate (524 mg, 1.78 mmol, 1.25 eq.). The mixture was heated to 85°C under nitrogen atmosphere for 5 hours. The solvent was removed under reduced pressure. The residue was separated by column chromatography on silica gel (dichloromethane: methanol 30: 1) to give 4-(4-bromo-3-(trifluoromethyl)phenyl)-1H-pyrazole (335 mg, yield: 81%) as a pale yellow oil. LCMS (ESI): m/z 291 [M+1]⁺; TLC: Rf 0.3 (dichloromethane: methanol = 30: 1).

Step 7b: Preparation of 4-(4-bromo-3-(trifluoromethyl)phenyl)-1-(tetrahydro-2H-pyran-2-yl)-1H-pyrazole (Compound 0103-10): To a mixture of 4-(4-bromo-3-(trifluoromethyl)phenyl)-1H-pyrazole (0205-10) (335 mg, 1.15 mmol, 1.0 eq.) and p-toluenesulfonic acid monohydrate (23 mg, 0.12 mmol, 0.1 eq.) in dichloromethane (5 ml) was added 3,4-dihydro-2H-pyran (193 mg, 2.30 mmol, 2.0 eq.). The mixture was stirred at room temperature overnight. The solvent was removed under reduced pressure. The residue was separated by column chromatography on silica gel (petroleum ether: ethyl acetate = 6: 1) to give a pale yellow oily product 4-(4-bromo-3-(trifluoromethyl)phenyl)-1-(tetrahydro-2H-pyran-2-yl)-1H-pyrazole (430 mg, yield: 100%). LCMS (ESI): m/z 375 [M+1]⁺; TLC: Rf 0.3 (petroleum ether: ethyl acetate = 6: 1).

Step 7c: Preparation of 1-((1-(4-(1-(tetrahydro-2H-pyran-2-yl)-1H-pyrazol-4-yl)-2-(trifluoromethyl)phenyl)pi peridin-4-yl)methyl)pyrrolidin-2-one (Compound 0109-10): 1-(Piperidin-4-ylmethyl)pyrrolidin-2-one (0108-1) (79 mg, 0.43 mmol, 1.2 eq.) was added to a mixture of 4-(4-bromo-3-(trifluoromethyl)phenyl)-1-(tetrahydro-2H-pyran-2-yl)-1H-pyrazole (0103-10) (135 mg, 0.36 mmol, 1.0 eq.), tris(dibenzylideneacetone)dipalladium(0) (16.5 mg, 0.018 mmol, 0.05 eq.), 2-dicyclohexylphosphino-2',6'-diisopropoxy-1,1'-biphenyl (33.6 mg, 0.072 mmol, 0.2 eq.) and sodium tert-butoxide (104 mg, 1.08 mmol, 3.0 eq.) in dioxane (6 ml). The mixture was heated to 110°C under nitrogen atmosphere for 7 hours. The mixture was diluted with water (20 ml). The aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, dichloromethane: methanol = 25: 1) to give a pale yellow solid product 1-((1-(4-(1-(tetrahydro-2H-pyran-2-yl)-1H-pyrazol-4-yl)-2-(trifluoromethyl)phenyl)pi peridin-4-yl)methyl)pyrrolidin-2-one (20 mg, yield: 12%). LCMS (ESI): m/z 477 [M+1]⁺; TLC: Rf 0.5 (dichloromethane: methanol = 25: 1).

Step 7d: Preparation of 1-((1-(4-(1H-pyrazol-4-yl)-2-(trifluoromethyl)phenyl)piperidin-4-yl)methyl)pyrrolidi n-2-one (Compound 10): A mixture of 1-((1-(4-(1-(tetrahydro-2H-pyran-2-yl)-1H-pyrazol-4-yl)-2-(trifluoromethyl)phenyl)pi peridin-4-yl)methyl)pyrrolidin-2-one (0109-10) (20 mg, 0.042 mmol, 1.0 eq.) in hydrogen chloride-methanol solution (4M, 1.5 ml) was stirred at room temperature for 2 hours. The mixture was diluted with water (15 ml). Solid sodium carbonate was added to adjust pH = 10, and then the aqueous layer was extracted with ethyl acetate (15 ml × 3). The combined organic layer was washed with saturated brine (15 ml × 1), dried over anhydrous sodium sulfate and concentrated. The residue was purified by Pre-TLC (silica gel, ethyl acetate: methanol = 10: 1) to give 1-((1-(4-(1H-pyrazol-4-yl)-2-(trifluoromethyl)phenyl) piperidin -4-yl)methyl)pyrrolidin-2-one (13 mg, yield: 79%) as a white solid. LCMS (ESI): m/z 393 [M+1]⁺; TLC: Rf 0.5 (dichloromethane: methanol = 10: 1). ¹ H NMR (500 MHz, MeOD) δ 7.97 (s, 2H), 7.78 (s, 2H), 7.46 (s, 1H), 3.51 (s, 2H), 3.24 (s, 2H), 3.04 (s, 2H), 2.76 (s, 2H), 2.40 (s, 2H), 2.07 (s, 2H), 1.82 (s, 1H), 1.70 (s, 2H), 1.44 (s, 2H).

### Example 8: Preparation of 1-((1-(2-chloro-4-(1H-pyrazol-4-yl)phenyl)piperidin-4-yl)methyl)pyrrolidin-2-one (Compound 11) (Prepared according to Scheme 2)

Step 8a: Preparation of tert-butyl 4-(4-bromo-3-chlorophenyl)-1H-pyrazole-1-carboxylate (Compound 0204-11): Under nitrogen protection, a mixture of 1-bromo-2-chloro-4-iodobenzene (0201-11) (318 mg, 1.0 mmol, 1.0 eq.), tert-butyl 4-(4,4,5,5-tetramethyl-1,3,2-dioxaborolan-2-yl)-1H-pyrazole-1-carboxylate (353 mg, 1.2 mmol, 1.2 eq.), potassium carbonate (277 mg, 2.0 mmol, 2.0 eq.) and [1,1'-bis(diphenylphosphino)ferrocene]dichloropalladium(II) (74 mg, 0.1 mmol, 0.1 eq.) in 30 mL dioxane and 3 mL water was stirred at 80 °C overnight. After cooling to room temperature, the reaction was extracted with ethyl acetate. The organic phase was washed with water and saturated brine, and then the organic phase was distilled under reduced pressure. The residue was purified by column chromatography on silica gel (eluent: petroleum ether/ethyl acetate = 20/1 to 5/1) to obtain a yellow solid product tert-butyl 4-(4-bromo-3-chlorophenyl)-1H - pyrazole-1-carboxylate (217 mg, yield: 60.6%). MS (ES⁺): m/z =357 (M+H)⁺.

Step 8b: Preparation of 4-(4-bromo-3-chlorophenyl)-1H - pyrazole (Compound 0205-11): tert-butyl 4-(4-bromo-3-chlorophenyl)-1H - pyrazole-1-carboxylate (0204-11) (200 mg, 0.56 mmol, 1.0 eq.) was added to 5 mL 4M hydrogen chloride-dioxane solution, and the mixture was stirred at room temperature for 2 hours. The mixture was concentrated to dryness under reduced pressure. Water was added and the pH was adjusted to 8 with saturated sodium carbonate aqueous solution. Ethyl acetate was added for extraction, washed with water, and the organic phase was dried over anhydrous sodium sulfate and concentrated under reduced pressure to give a yellow solid product 4-(4-bromo-3-chlorophenyl)-1H - pyrazole (113 mg, yield: 78.5%). MS (ES⁺): m/z =257 (M+H)⁺.

Step 8c: Preparation of 4-(4-bromo-3-chlorophenyl)-1-(tetrahydro- 2H -pyran-2-yl)-1H - pyrazole (Compound 0103-11): 4-(4-bromo-3-chlorophenyl) -1H -pyrazole (0205-11) (113 mg, 0.44 mmol, 1.0 eq.) was dissolved in 10 mL dichloromethane, and 3,4-dihydro-2H - pyran (45 mg, 0.53 mmol, 1.2 eq.) and p-toluenesulfonic acid monohydrate (8 mg, 0.044 mmol, 0.1 eq.) were added. The mixture was stirred at room temperature for 2 hours. The reaction was quenched with aqueous sodium carbonate solution. Dichloromethane was added for extraction, and the mixture was washed with water and saturated brine. The organic phase was concentrated under reduced pressure, and the residue was purified by column chromatography on silica gel chromatography (eluent: petroleum ether/ethyl acetate = 20/1 to 5/1) to obtain a yellow oily product 4-(4-bromo-3-chlorophenyl)-1-(tetrahydro- 2H -pyran-2-yl)-1H - pyrazole (125 mg, yield: 83.1%). MS (ES⁺): m/z =341(M+H)⁺.

Step 8d : Preparation of 1-((1-(2-chloro-4-(1-(tetrahydro- 2H -pyran-2-yl)-1H - pyrazol-4-yl)phenyl)piperidin-4-yl)methyl)pyrrolidin-2- one (Compound 0109-11): Under nitrogen protection, a mixture of 4- (4-bromo-3-chlorophenyl) -1-(tetrahydro-2H-pyran-2-yl) -1H-pyrazole (0103-11), 1-(piperidin-4-ylmethyl)pyrrolidin-2-one (0108-1) (64 mg, 0.35 mmol, 1.1 eq.), sodium tert-butoxide (123 mg, 1.28 mmol, 4.0 eq.), 4,5-bis(diphenylphosphino)-9,9-dimethylxanthene (37 mg, 0.064 mmol, 0.2 eq.) and tris(dibenzylideneacetone)dipalladium( 0) (29 mg, 0.032 mmol, 0.1 eq.) in toluene (40 ml) was stirred at 120 °C overnight. After cooling to room temperature, the reaction was filtered through celite and the filter cake was washed with ethyl acetate. The filtrate was concentrated under reduced pressure. The residue was purified by prep-TLC (silica gel, eluent: dichloromethane/methanol = 33/1) to give yellow solid product 1-((1-(2-chloro-4-(1-(tetrahydro- 2H -pyran-2-yl)-1H - pyrazol-4-yl)phenyl)piperidin-4-yl)methyl)pyrrolidin-2-one (51 mg, yield: 35.9%). MS (ES⁺): m/z = 443 (M+H)⁺.

Step 8e: Preparation of 1-((1-(2-chloro-4-(1H - pyrazol-4-yl)phenyl)piperidin-4-yl)methyl)pyrrolidin-2-one (Compound 11): 1-((1-(2-chloro-4-(1-(tetrahydro-2H - pyran-2-yl)-1H - pyrazol-4-yl)phenyl)piperidin-4-yl)methyl)pyrrolidin-2-one (0109-11) (51 mg, 0.12 mmol, 1.0 eq.) was added to 2 mL 4M hydrogen chloride- methanol solution and stirred at room temperature for 2 hours. The mixture was concentrated to dryness under reduced pressure. Water was added and the pH was adjusted to 10 with saturated aqueous sodium carbonate solution. Ethyl acetate was added for extraction, washed with water, the organic phase was dried over anhydrous sodium sulfate, concentrated under reduced pressure, and the residue was purified by prep-TLC (silica gel, eluent: dichloromethane/methanol = 12/1) to obtain a yellow solid 1-((1-(2-chloro-4-(1H - pyrazol-4-yl)phenyl)piperidin-4-yl)methyl)pyrrolidin-2-one (30 mg, yield: 72.0%). MS (ES⁺): m/z =359 (M+H)⁺, Melting point: 188~190 ° C. ¹ H NMR (500 MHz, DMSO) δ 12.89 (s, 1H), 8.16 (s, 1H), 7.89 (s, 1H), 7.64 (d, *J =* 2.0 Hz, 1H), 7.49 (dd, *J =* 8.3, 2.1 Hz, 1H), 7.10 (d, *J =* 8.4 Hz, 1H), 3.36 (t, *J =* 7.0 Hz, 2H), 3.25 (d, *J =* 11.7 Hz, 2H), 3.12 (d, *J =* 7.2 Hz, 2H), 2.60 (dd, *J =* 11.6, 9.9 Hz, 2H), 2.23 (t, *J* = 8.1 Hz, 2H), 1.98 - 1.87 (m, 2H), 1.77 - 1.59 (m, 3H), 1.37 - 1.27 (m, 2H).

### Example 9: Preparation of 1-((1-(2-methyl-4-(1H-pyrazol-4-yl)phenyl)piperidin-4-yl)methyl)pyrrolidin-2-on e (Compound 12) (Prepared according to Scheme 1)

Step 9a: Preparation of 4-(4-bromo-3-methylphenyl)-1-(tetrahydro-2H-pyran-2-yl)-1H-pyrazole (Compound 0103-12): Under nitrogen protection, a mixture of 4-bromo-3-methylphenylboronic acid (0101-12) (350 mg, 1.63 mmol, 1 eq.), 4-iodo-1-(tetrahydro-2H-pyran-2-yl)-1H-pyrazole (0102-1) (453 mg, 1.63 mmol, 1 eq.), potassium carbonate (674 mg, 4.88 mmol, 3 eq.), 1,1'-bis(diphenylphosphino)ferrocenepalladium(II) chloride (60 mg, 0.082 mmol, 0.05 eq.) in 1,4-dioxane (8 ml) and water (0.8 ml) was stirred at 90 °C for 30 min. The reaction was diluted with water and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 10/1) to obtain a yellow oily product 4-(4-bromo-3-methylphenyl)-1-(tetrahydro-2H-pyran-2-yl)-1H-pyrazole (246 mg, yield: 47.16%). LCMS (ESI) [M+1]⁺ : m/z = 321.

Step 9b: Preparation of 1-((1-(2-methyl-4-(1-(tetrahydro-2H-pyran-2-yl)-1H-pyrazol-4-yl)phenyl)piperidin-4-yl)methyl)pyrrolidin-2-one (Compound 0109-12): Under nitrogen protection, , 4-(4-bromo-3-methylphenyl)-1-(tetrahydro-2H-pyran-2-yl)-1H-pyrazole (0103-12) (128 mg, 0.40 mmol, 1 eq.), 1-(piperidin-4-ylmethyl)pyrrolidin-2-one (0108-1) (80 mg, 0.44 mmol, 1.1 eq.), sodium tert-butoxide (106 mg, 4.88 mmol, 3 eq.), 4,5-bis(diphenylphosphino-9,9-dimethylxanthene (23 mg, 0.040 mmol, 0.1 eq.), tris(dibenzylideneacetone)dipalladium (18 mg, 0.020 mmol, 0.05 eq.), toluene (5 ml) were mixed and stirred at 120 °C for 8 hours. The reaction was diluted with water and extracted with ethyl acetate. The organic layer was washed with saturated brine, dried over anhydrous sodium sulfate, and concentrated under reduced pressure. The residue was purified by column chromatography on silica gel (dichloromethane/methanol = 40/1) to obtain a yellow solid product 1-((1-(2-methyl-4-(1-(tetrahydro-2H-pyran-2-yl)-1H-pyrazol-4-yl)phenyl)piperidin-4-yl)methyl)pyrrolidin-2-one (60 mg, yield: 35.55%). LCMS (ESI) [M+1]⁺ : m/z = 422.

Step 9c: Preparation of 1-((1-(2-methyl-4-(1H-pyrazol-4-yl)phenyl)piperidin-4-yl)methyl)pyrrolidin-2-one (Compound 12): A mixture of 1-((1-(2-methyl-4-(1-(tetrahydro-2H-pyran-2-yl)-1H-pyrazol-4-yl)phenyl)piperidin-4-yl)methyl)pyrrolidin-2-one (0109-12) (60 mg, 0.14 mmol, 1.0 eq.) in hydrogen chloride-methanol solution (4 M, 2 ml) was stirred at room temperature for 2 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a white solid product 1-((1-(2-methyl-4-(1H-pyrazol-4-yl)phenyl)piperidin-4-yl)methyl)pyrrolidin-2-one (32 mg, yield: 67.62%). LCMS (ESI) [M+1]⁺ : m/z = 339. ¹ H NMR (500 MHz, DMSO) δ 12.81 (s, 1H), 7.93 (d, *J =* 107.0 Hz, 2H), 7.51 - 7.23 (m, 2H), 6.98 (d, *J =* 7.5 Hz, 1H), 3.36 (s, 2H), 3.13 (d, *J =* 5.8 Hz, 2H), 3.04 (d, *J =* 9.6 Hz, 2H), 2.57 (d, *J* = 10.7 Hz, 2H), 2.25 (s, 5H), 1.95 (d, *J =* 6.0 Hz, 2H), 1.67 (d, *J =* 11.9 Hz, 3H), 1.30 (d, *J =* 10.1 Hz, 2H).

### Example 10: Preparation of 2-(4-((2-oxopyrrolidin-1-yl)methyl)piperidin-1-yl)-5-(1H-pyrazol-4-yl)benzonitril e (Compound 13) (Prepared according to Scheme 1)

Step 10a: Preparation of 2-fluoro-5-(1-(tetrahydro-2H-pyran-2-yl)-1H-pyrazol-4-yl)benzaldehyde (Compound 0103-13): Under nitrogen protection, a mixture of (4-fluoro-3-formylphenyl)boronic acid (0101-13) (1 g, 5.99 mmol, 1.0 eq.), 4-iodo-1-(tetrahydro-2H-pyran-2-yl)-1H-pyrazole (0102-1) (1.7 g, 5.99 mmol, 1.0 eq.), 1,1'-bis(diphenylphosphino)ferrocenepalladium(II) chloride (657 mg, 0.899 mmol, 0.15 eq.), potassium carbonate (2.5 g, 17.97 mmol, 3.0 eq.) in dioxane/water (20 ml/4 ml) was stirred at 70 °C for 2.0 hours. The reaction was diluted with water (50 ml) and extracted with ethyl acetate (10 ml × 4). The organic layer was washed with saturated brine (40 ml × 1), dried over anhydrous sodium sulfate, and concentrated under reduced pressure. The residue was purified by column chromatography on silica gel (petroleum ether/ethyl acetate = 10/1 to 5/1) to obtain a yellow oily product 2-fluoro-5-(1-(tetrahydro-2H-pyran-2-yl)-1H-pyrazol-4-yl)benzaldehyde (884 mg, yield: 53.9%). LCMS (ESI): m/z = 275 [M+1]⁺.

Step 10b: Preparation of 2-(4-((2-oxopyrrolidin-1-yl)methyl)piperidin-1-yl)-5-(1-(tetrahydro-2H-pyran-2-yl)-1 H-pyrazol-4-yl)benzaldehyde (Compound 0109-13): Under nitrogen protection, a mixture of 1-(piperidin-4-yl)pyrrolidin-2-one (0108-1) (364 mg, 2.0 mmol, 1.1 eq.), 2-fluoro-5-(1-(tetrahydro-2H-pyran-2-yl)-1H-pyrazol-4-yl)benzaldehyde (0103-13) (500 mg, 1.82 mmol, 1.0 eq.), potassium carbonate (753 mg, 5.46 mmol, 3.0 eq.) in N-methylpyrrolidone (8 ml) was stirred at 130 °C overnight. The reaction was diluted with water (40 ml) and extracted with ethyl acetate ( 8 ml × 5). The organic phase was washed with saturated brine (30 ml × 3), dried over anhydrous sodium sulfate and concentrated under reduced pressure. The residue was purified by column chromatography on silica gel (ethyl acetate/methanol = 250/1 to 100/1) to obtain a yellow solid product 2-(4-((2-oxopyrrolidin-1-yl)methyl)piperidin-1-yl)-5-(1-(tetrahydro-2H-pyran-2-yl)-1 H-pyrazol-4-yl)benzaldehyde (409 mg, yield: 51.6%). LCMS (ESI): m/z = 437 [M+1]⁺.

Step 10c: Preparation of 2-(4-((2-oxopyrrolidin-1-yl)methyl)piperidin-1-yl)-5-(1H-pyrazol-4-yl)benzonitrile (Compound 13): Under nitrogen protection, a mixture of 2-(4-((2-oxopyrrolidin-1-yl)methyl)piperidin-1-yl)-5-(1-(tetrahydro-2H-pyran-2-yl)-1 H-pyrazol-4-yl)benzaldehyde (0109-13) (210 mg, 0.482 mmol, 1.0 eq.), hydroxylamine hydrochloride (46.9 mg, 0.675 mmol, 1.4 eq.), sodium ethoxide (79.1 mg, 0.964 mmol, 2.0 eq.) in acetonitrile/water (5.1 ml/1.5 ml) was stirred at room temperature for 4.0 hours. The reaction was diluted with water (20 ml) and extracted with ethyl acetate (8 ml × 4). The combined organic layer was washed with saturated brine (30 ml × 1), dried over anhydrous sodium sulfate, and concentrated under reduced pressure. The residue was purified by column chromatography on silica gel (dichloromethane/methanol = 60/1 to 40/1) to obtain a yellow solid product (Z)-2-(4-((2-oxopyrrolidin-1-yl)methyl)piperidin-1-yl)-5-(1-(tetrahydro-2H-pyran-2-y 1)-1H-pyrazol-4-yl)benzaldehyde oxime (131 mg, yield: 60.4%). LCMS (ESI): m/z 452 [M+1]⁺.

Under nitrogen protection, a mixture of (Z)-2-(4-((2-oxopyrrolidin-1-yl)methyl)piperidin-1-yl)-5-(1-(tetrahydro-2H-pyran-2-y 1)-1H-pyrazol-4-yl)benzaldehyde oxime (131 mg, 0.288 mmol, 1.0 eq.) prepared above, potassium carbonate (79.6 mg, 0.576 mmol, 2.0 eq.), acetic anhydride (59 mg, 0.576 mmol, 2.0 eq.) in dimethyl sulfoxide (5 ml) was stirred at 50 °C for 3.0 hours. The reaction was diluted with water (20 ml) and extracted with ethyl acetate (8 ml × 4). The combined organic phases was washed with saturated brine (30 ml × 3), dried over anhydrous sodium sulfate and concentrated under reduced pressure. The residue was purified by column chromatography on silica gel (dichloromethane/methanol = 60/1 to 40/1) to give 2-(4-((2-oxopyrrolidin-1-yl)methyl)piperidin-1-yl)-5-(1-(tetrahydro-2H-pyran-2-yl)-1 H-pyrazol-4-yl)benzonitrile (105 mg, yield: 84.7%) as a yellow oil. LCMS (ESI): m/z = 434 [M+1]⁺.

A mixture of 2-(4-((2-oxopyrrolidin-1-yl)methyl)piperidin-1-yl)-5-(1-(tetrahydro-2H-pyran-2-yl)-1 H-pyrazol-4-yl)benzonitrile ( 105 mg, 0.242 mmol, 1.0 eq.) obtained above in hydrogen chloride-dioxane (4 M, 5 ml) was stirred at room temperature for 1.0 hour. The solvent was removed under reduced pressure, and the residue was diluted with water (8 ml), adjusted to pH = 9 with sodium carbonate solution, and extracted with dichloromethane (4 ml × 5). The combined organic phase was dried over anhydrous sodium sulfate and concentrated under reduced pressure. The residue was purified by prep-TLC (silica gel, dichloromethane/methanol = 12/1) to give a light yellow solid product 2-(4-((2-oxopyrrolidin-1-yl)methyl)piperidin-1-yl)-5-(1H-pyrazol-4-yl)benzonitrile (72 mg, yield: 85.7%). LCMS (ESI): m/z = 350 [M+1]⁺. ¹ H NMR (500 MHz, DMSO) δ 12.92 (s, 1H), 8.21 (s, 1H), 7.94 (d, *J =* 2.1 Hz, 2H), 7.80 (dd, *J =* 8.6, 2.2 Hz, 1H), 7.13 (d, *J =* 8.6 Hz, 1H), 3.48 (t, *J =* 13.9 Hz, 2H), 3.36 (t, *J =* 7.0 Hz, 2H), 3.12 (d, *J* = 7.1 Hz, 2H), 2.76 (t, *J =* 11.0 Hz, 2H), 2.24 (t, *J =* 8.0 Hz, 2H), 1.99 - 1.87 (m, 2H), 1.75 (ddd, *J =* 24.9, 14.4, 8.3 Hz, 3H), 1.32 (qd, *J =* 12.1, 3.4 Hz, 2H).

### Example 11: Preparation of 1-((1-(2-methoxy-4-(1H-pyrazol-4-yl)phenyl)piperidin-4-yl)methyl)pyrrolidin-2-o ne (Compound 14) (Prepared according to Scheme 2):

Step 11a: Preparation of tert-butyl 4-(4-bromo-3-methoxyphenyl)-1H-pyrazole-1-carboxylate (Compound 0204-14): Under nitrogen protection, a mixture of tert-butyl 4-(4,4,5,5-tetramethyl-1,3,2-dioxaborolan-2-yl)-1H-pyrazole-1-carboxylate (1.0 g, 3.4 mmol, 1.0 eq.), 1-bromo-4-iodo-2-methoxybenzene (0201-14) (1.6 g, 5.1 mmol, 1.5 eq.), [1,1'-bis(diphenylphosphino)ferrocene]dichloropalladium (249 mg, 0.34 mmol, 0.1 eq.), sodium carbonate (1.08 g, 10.2 mmol, 3.0 eq.) in dioxane/water (30 ml/3 ml) was stirred at 85 °C for 1.5 hours. Then the mixture was cooled to room temperature, diluted with water and extracted with ethyl acetate. The organic layer was dried and concentrated under reduced pressure. The residue was purified by column chromatography on silica gel (petroleum ether/ethyl acetate = 5/1) to obtain a white solid product tert-butyl 4-(4-bromo-3-methoxyphenyl)-1H-pyrazole-1-carboxylate (564 mg, yield: 47.11%). LCMS (ESI): m/z = 353 [M+1]⁺.

Step 11b: Preparation of 4-(4-bromo-3-methoxyphenyl)-1H-pyrazole (Compound 0205-14): A mixture of tert-butyl 4-(4-bromo-3-methoxyphenyl)-1H-pyrazole-1-carboxylate (0204-14) (564 mg, 1.6 mmol, 1.0 eq.) in hydrogen chloride-dioxane (4M, 5 ml) was stirred at room temperature for 1 hour. The solvent was removed under reduced pressure, the residue was diluted with water, adjusted to pH = 9 with sodium carbonate solution, and extracted with dichloromethane. The organic phase was dried and concentrated under reduced pressure, and the residue was used directly in the next step without further purification. LCMS (ESI): m/z = 253 [M+1]⁺.

Step 11c: Preparation of 4-(4-bromo-3-methoxyphenyl)-1-(tetrahydro-2H-pyran-2-yl)-1H-pyrazole (Compound 0103-14): A mixture of 4-(4-bromo-3-methoxyphenyl)-1H-pyrazole (0205-14) (291 mg, 1.15 mmol, 1.0 eq.), 3,4-dihydro-2H-pyran (106.2 mg, 1.3 mmol, 1.1 eq.) and p-toluenesulfonic acid monohydrate (21.8 mg, 0.1 mmol, 0.1 eq.) in dichloromethane (10 ml) was stirred overnight at room temperature. The reaction was diluted with water and extracted with dichloromethane. The organic layer was dried and concentrated under reduced pressure. The residue was purified by column chromatography on silica gel (petroleum ether/ethyl acetate = 3/1) to give a white solid product 4-(4-bromo-3-methoxyphenyl)-1-(tetrahydro-2H-pyran-2-yl)-1H-pyrazole (200 mg, yield: 51.59%). LCMS (ESI): m/z = 338 [M+1]⁺.

Step 11d: Preparation of 1-((1-(2-methoxy-4-(1-(tetrahydro-2H-pyran-2-yl)-1H-pyrazol-4-yl)phenyl)piperidin-4-yl)methyl)pyrrolidin-2-one (Compound 0109-14): Under nitrogen protection, a mixture of 1-(piperidin-4-ylmethyl)pyrrolidin-2-one (0108-1) (200 mg, 1.1 mmol, 1.0 eq.), 4- (4-bromo-3-methoxyphenyl) -1- (tetrahydro-2H-pyran-2-yl) -1H-pyrazole (0103-14) (407 mg, 1.2 mmol, 1.1 eq.), sodium tert-butoxide (211 mg, 2.2 mmol, 2.0 eq.), 4,5-bis(diphenylphosphino)-9,9-dimethylxanthene (63.19 mg, 0.11 mmol, 0.1 eq.) and tris(dibenzylideneacetone)dipalladium(0) (50.3 mg, 0.055 mmol, 0.05 eq.) in toluene (10 ml) was stirred at 120 °C overnight. The reaction was diluted with water and extracted with ethyl acetate, and the organic phase was dried and concentrated under reduced pressure. The residue was purified by prep-TLC (silica gel, dichloromethane/methanol = 10/1) to give a yellow solid product 1-((1-(2-methoxy-4-(1-(tetrahydro-2H-pyran-2-yl)-1H-pyrazol-4-yl)phenyl)piperidin-4-yl)methyl)pyrrolidin-2-one (150 mg, yield : 31.18%). LCMS (ESI): m/z = 439 [M+1]⁺.

Step 11e: Preparation of 1-((1-(2-methoxy-4-(1H-pyrazol-4-yl)phenyl)piperidin-4-yl)methyl)pyrrolidin-2-one (Compound 14): A mixture of 1-((1-(2-methoxy-4-(1-(tetrahydro-2H-pyran-2-yl)-1H-pyrazol-4-yl)phenyl)piperidin-4-yl)methyl)pyrrolidin-2-one (0109-14) (96 mg, 0.22 mmol, 1.0 eq.) in hydrogen chloride-methanol (4 M, 5 ml) was stirred at room temperature for 1.5 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 15/1) to give a yellow solid product 1-((1-(2-methoxy-4-(1H-pyrazol-4-yl)phenyl)piperidin-4-yl)methyl)pyrrolidin-2-one (91 mg, yield: 75.2%). LCMS (ESI): m/z = 355 [M+1]⁺. ¹ H NMR (500 MHz, DMSO) δ 12.80 (s, 1H), 8.08 (s, 1H), 7.84 (s, 1H), 7.14 - 7.04 (m, 2H), 6.84 (d, *J =* 8.1 Hz, 1H), 3.83 (s, 3H), 3.34 (dd, *J =* 15.8, 8.9 Hz, 4H), 3.10 (d, *J =* 7.1 Hz, 2H), 2.48 (d, *J* = 13.3 Hz, 2H), 2.23 (t, *J =* 8.0 Hz, 2H), 2.03 - 1.88 (m, 2H), 1.88 - 1.56 (m, 3H), 1.28 (ddd, *J =* 15.5, 8.9 Hz, 4H). 12.4, 3.7 Hz, 2H).

### Example 12: Preparation of 2-(4-((2-oxopyrrolidin-1-yl)methyl)piperidin-1-yl)-5-(1H-pyrazol-4-yl)benzaldehy de (Compound 15) (Prepared according to Scheme 1)

A mixture of 2-(4-((2-oxopyrrolidin-1 -yl)methyl)piperidin-1 -yl)-5-(1 -(tetrahydro-2H-pyran-2-yl)-1 H-pyrazol-4-yl)benzaldehyde (0109-13) ( 139 mg, 0.32 mmol, 1.0 eq.) in hydrogen chloride-dioxane (4 M, 5 mL) was stirred at room temperature for 1.0 h. The solvent was removed under reduced pressure, and the residue was diluted with water (8 mL), adjusted to pH = 9 with sodium carbonate solution, and extracted with dichloromethane (4 mL × 5). The organic phase was dried over anhydrous sodium sulfate and concentrated under reduced pressure. The residue was purified by prep-TLC (silica gel, dichloromethane/methanol = 12/1) to give 2-(4-((2-oxopyrrolidin-1-yl)methyl)piperidin-1-yl)-5-(1H-pyrazol-4-yl)benzaldehyde (45.8 g, yield: 40.8%) as a yellow solid. LCMS(ESI): m/z =353[M+1]⁺. ¹ H NMR (500 MHz, DMSO) δ 12.91 (s, 1H), 10.20 (s, 1H), 8.18 (s, 1H), 8.03 - 7.78 (m, 3H), 7.20 (d, *J =* 8.5 Hz, 1H), 3.37 (t, *J =* 7.0 Hz, 2H), 3.22 (d, *J =* 12.1 Hz, 2H), 3.14 (d, *J* = 7.2 Hz, 2H), 2.83 (dd, *J =* 11.7, 10.3 Hz, 2H), 2.24 (t, *J =* 8.1 Hz, 2H), 2.02 - 1.88 (m, 2H), 1.88 - 1.62 (m, 3H), 1.40 (td, *J=* 12.0, 3.2 Hz, 2H).

### Example 13: Preparation of 2-(4-((2-oxopyrrolidin-1-yl)methyl)piperidin-1-yl)-5-(1H-pyrazol-4-yl)benzamide (Compound 16):

A mixture of 2-(4-((2-oxopyrrolidin-1-yl)methyl)piperidin-1-yl)-5-(1H-pyrazol-4-yl)benzonitrile (Compound 13) (30 mg, 0.086 mmol, 1.0 eq.), sodium hydroxide (10.3 mg, 0.258 mmol, 3.0 eq.) in dimethyl sulfoxide/hydrogen peroxide (2 ml/0.5 ml) was stirred at 30 °C for 2.0 hours. The reaction was diluted with water (10 ml) and extracted with ethyl acetate (5 ml × 4). The combined organic layer was washed with saturated brine (20 ml × 1), dried over anhydrous sodium sulfate, and concentrated under reduced pressure. The residue was purified by prep-TLC (silica gel, dichloromethane/methanol = 10/1) to give a white solid product 2-(4- ((2 -oxopyrrolidin-1-yl)methyl)piperidin-1-yl)-5-(1H-pyrazol-4-yl)benzamide (25.5 mg, yield: 80.7%). LCMS (ESI): m/z 368 [M+1]⁺. ¹ H NMR (500 MHz, DMSO) δ 12.89 (s, 1H), 8.72 (s, 1H), 8.14 (s, 1H), 7.95 (d, *J =* 2.0 Hz, 1H), 7.85 (s, 1H), 7.64 (dd, *J =* 8.3, 2.0 Hz, 1H), 7.50 (s, 1H), 7.19 (d, *J =* 8.4 Hz, 1H), 3.35 (t, *J =* 7.0 Hz, 2H), 3.12 (d, *J* = 7.2 Hz, 4H), 2.68 (t, *J=* 11.1 Hz, 2H), 2.23 (t, *J =* 8.0 Hz, 2H), 1.99 - 1.87 (m, 2H), 1.78 - 1.62 (m, 3H), 1.40 - 1.27 (m, 2H).

### Example 14: Preparation of 1-((1-(2-(hydroxymethyl)-4-(1H-pyrazol-4-yl)phenyl)piperidin-4-yl)methyl)pyrro lidin-2-one (Compound 17)

Under nitrogen protection, 2-(4-((2-oxopyrrolidin-1-yl)methyl)piperidin-1-yl)-5-(1-(tetrahydro-2H-pyran-2-yl)-1 H-pyrazol-4-yl)benzaldehyde (0109-13) (120 mg, 0.275 mmol, 1.0 eq.) and sodium borohydride (41.5 mg, 1.1 mmol, 4.0 eq.) were stirred at room temperature for 1.5 hours. The reaction was diluted with water (20 ml) and extracted with ethyl acetate (8 ml × 5). The combined organic layer was washed with saturated brine (30 ml × 1), dried over anhydrous sodium sulfate, and concentrated under reduced pressure to give a white solid product 1-((1-(2-(hydroxymethyl)-4-(1-(tetrahydro-2H-pyran-2-yl)-1H-pyrazol-4-yl)phenyl)pi peridin-4-yl)methyl)pyrrolidin-2-one (118 mg, yield: 98.3%). LCMS (ESI): m/z 439 [M+1]⁺.

A mixture of 1-((1-(2-(hydroxymethyl)-4-(1-(tetrahydro-2H-pyran-2-yl)-1H-pyrazol-4-yl)phenyl)pi peridin-4-yl)methyl)pyrrolidin-2-one (118 mg, 0.269 mmol, 1.0 eq.) prepared above in hydrogen chloride-dioxane (4 M, 5 ml) was stirred at room temperature for 1.0 hour. The solvent was removed under reduced pressure, and the residue was diluted with water (8 ml), adjusted to pH = 9 with sodium carbonate solution, and extracted with dichloromethane (4 ml × 5). The combined organic phase was dried over anhydrous sodium sulfate and concentrated under reduced pressure. The residue was purified by prep-TLC (silica gel, dichloromethane/methanol = 10/1) to give a white solid product 1-((1-(2-(hydroxymethyl)-4-(1H-pyrazol-4-yl)phenyl)piperidin-4-yl)methyl)pyrrolidi n-2-one (72 mg, yield: 98.3%). LCMS (ESI): m/z = 355 [M+1]⁺. ¹ H NMR (500 MHz, DMSO) δ 12.82 (s, 1H), 7.92 (d, *J =* 94.0 Hz, 2H), 7.62 (d, *J* = 1.9 Hz, 1H), 7.40 (dd, *J =* 8.2, 2.1 Hz, 1H), 7.01 (d, *J =* 8.3 Hz, 1H), 5.04 (t, *J =* 5.4 Hz, 1H), 4.54 (d, *J =* 5.1 Hz, 2H), 3.36 (t, *J =* 7.0 Hz, 2H), 3.12 (d, *J =* 7.2 Hz, 2H), 3.02 (d, *J =* 11.8 Hz, 2H), 2.58 (t, *J =* 10.9 Hz, 2H), 2.23 (t, *J =* 8.0 Hz, 2H), 1.99 - 1.86 (m, 2H), 1.69 (ddd, *J =* 25.8, 14.9, 8.1 Hz, 3H), 1.35 - 1.24 (m, 2H).

### Example 15: Preparation of methyl 2-(4-((2-oxopyrrolidin-1-yl)methyl)piperidin-1-yl)-5-(1H-pyrazol-4-yl)benzoate (Compound 18)

Under nitrogen protection, a mixture of 2-(4-((2-oxopyrrolidin-1-yl)methyl)piperidin-1-yl)-5-(1-(tetrahydro-2H-pyran-2-yl)-1 H-pyrazol-4-yl)benzaldehyde (0109-13) (80 mg, 0.183 mmol, 1.0 eq.), a methanol solution of sodium methoxide (2 ml), and a mixture of methanol (2 ml) and hydrogen peroxide (2 ml) was stirred at 45 °C for 3.0 hours. The reaction was concentrated under reduced pressure. The residue was purified by column chromatography on silica gel (petroleum ether/ethyl acetate = 3/1 to 1/1) to obtain a yellow solid product 2-(4-((2-oxopyrrolidin-1-yl)methyl)piperidin-1-yl)-5-(1-(tetrahydro-2H-pyran-2-yl)-1 H-pyrazol-4-yl)benzoic acid (45 mg, yield: 54.8%). LCMS (ESI): m/z =453[M+1]⁺.

A mixture of 2-(4-((2-oxopyrrolidin-1-yl)methyl)piperidin-1-yl)-5-(1-(tetrahydro-2H-pyran-2-yl)-1 H-pyrazol-4-yl)benzoic acid (45 mg, 0.099 mmol, 1.0 eq.) in hydrogen chloride-methanol (4 M, 3 ml) was stirred at room temperature for 1.0 hour. The solvent was removed under reduced pressure and the residue was diluted with water. The pH was adjusted to 7 with sodium carbonate solution, and extracted with dichloromethane. The organic phase was dried and concentrated under reduced pressure to give a yellow solid product 2-(4-((2-oxopyrrolidin-1-yl)methyl)piperidin-1-yl)-5-(1H-pyrazol-4-yl)benzoic acid (25 mg, yield: 69.4%). LCMS (ESI): m/z = 369 [M+1]⁺.

A mixture of 2-(4-((2-oxopyrrolidin-1-yl)methyl)piperidin-1-yl)-5-(1H-pyrazol-4-yl)benzoic acid (25 mg, 0.068 mmol, 1.0 eq.) and thionyl chloride (12 mg, 0.101 mmol, 1.5 eq.) in methanol (2 ml) was stirred at 65 °C for 8.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to obtain a yellow solid product methyl 2-(4-((2-oxopyrrolidin-1-yl)methyl)piperidin-1-yl)-5-(1H-pyrazol-4-yl)benzoate (15 mg, yield: 57.6%). LCMS (ESI): m/z = 383 [M + 1] ⁺. Melting point : 165 ~ 174 ° C. ¹ H NMR (500 MHz, DMSO) δ 12.86 (s, 1H), 7.98 (d, *J =* 100.0 Hz, 2H), 7.79 - 7.59 (m, 2H), 7.07 (d, *J =* 7.3 Hz, 1H), 3.81 (s, 3H), 3.35 (s, 2H), 3.19 (d, *J =* 9.8 Hz, 2H), 3.10 (d, *J =* 4.7 Hz, 2H), 2.66 (t, *J =* 10.7 Hz, 2H), 2.23 (s, 2H), 1.93 (s, 2H), 1.76-1.51 (m, 3H), 1.25 (d, *J =* 9.9 Hz, 2H).

### Example 16: Preparation of 1-((1-(2-(difluoromethyl)-4-(1H-pyrazol-4-yl)phenyl)piperidin-4-yl)methyl)pyrrol idin-2-one (Compound 19)

Under nitrogen protection, to a mixture of 2-(4-((2-oxopyrrolidin-1-yl)methyl)piperidin-1-yl)-5-(1-(tetrahydro-2H-pyran-2-yl)-1 H-pyrazol-4-yl)benzaldehyde (0109-13) (120 mg, 0.275 mmol, 1.0 eq.) in dichloromethane (5 ml) was added dropwise (diethylamino)sulfur trifluoride (132 mg, 0.825 mmol, 3.0 eq.) at 0 °C. The mixture was stirred at room temperature for 5.0 hours. The reaction was diluted with water (20 ml) and extracted with dichloromethane (8 ml × 3). The organic layer was washed with saturated brine (30 ml × 1), dried over anhydrous sodium sulfate, and concentrated under reduced pressure. The residue was purified by prep-TLC (silica gel, dichloromethane/methanol = 15/1) to give yellow solid product 1-((1-(2-(difluoromethyl)-4-(1-(tetrahydro-2H-pyran-2-yl)-1H-pyrazol-4-yl)phenyl)pi peridin-4-yl)methyl)pyrrolidin-2-one (79 mg, yield: 62.7%). LCMS (ESI): m/z 459 [M+1]⁺.

A mixture of 1-((1-(2-(difluoromethyl)-4-(1-(tetrahydro-2H-pyran-2-yl)-1H-pyrazol-4-yl)phenyl)pi peridin-4-yl)methyl)pyrrolidin-2-one (79 mg, 0.172 mmol, 1.0 eq.) obtained above in hydrogen chloride-dioxane (4 M, 4 ml) was stirred at room temperature for 1.0 hour. The solvent was removed under reduced pressure, and the residue was diluted with water (8 ml), adjusted to pH = 9 with sodium carbonate solution, and extracted with dichloromethane (4 ml × 5). The combined organic phase was dried over anhydrous sodium sulfate and concentrated under reduced pressure. The residue was purified by prep-TLC (silica gel, dichloromethane/methanol = 12/1) to obtain a yellow solid product 1-((1-(2-(difluoromethyl)-4-(1H-pyrazol-4-yl)phenyl)piperidin-4-yl)methyl)pyrrolidin -2-one (34.4 mg, yield: 53.3%). LCMS(ESI): m/z =375[M+1]⁺. ¹ H NMR (500 MHz, DMSO) δ 12.93 (s, 1H), 8.21 (s, 1H), 7.92 (s, 1H), 7.81 - 7.59 (m, 2H), 7.28 (d, *J =* 8.3 Hz, 1H), 7.12 (t, *J =* 55.4 Hz, 1H), 3.36 (t, *J =* 7.0 Hz, 2H), 3.12 (d, *J =* 7.2 Hz, 2H), 2.99 (d, *J =* 11.7 Hz, 2H), 2.76 (dd, *J =* 11.6, 9.9 Hz, 2H), 2.23 (t, *J =* 8.1 Hz, 2H), 2.00 - 1.88 (m, 2H), 1.80 - 1.60 (m, 3H), 1.35 (qd, *J =* 12.1, 3.6 Hz, 2H).

### Example 17: Preparation of 1-((1-(2-(dimethylamino)-4-(1H-pyrazol-4-yl)phenyl)piperidin-4-yl)methyl)pyrro lidin-2-one (Compound 20)

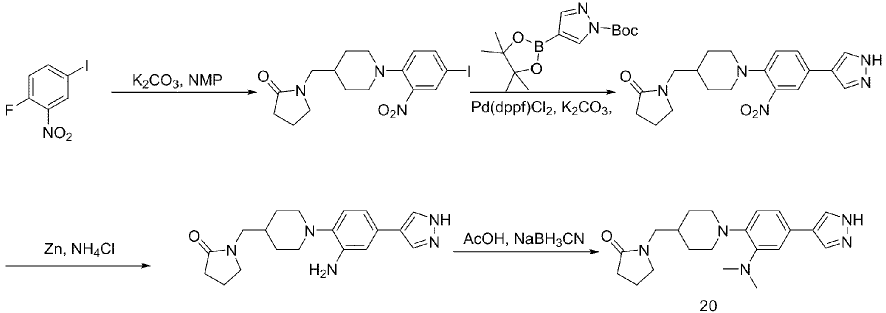

Step 17a: To a mixture of 1-fluoro-4-iodo-2-nitrobenzene (0.13 g, 0.49 mmol, 1.0 eq.) and potassium carbonate (0.10 g, 0.73 mmol, 1.5 eq.) in N-methylpyrrolidone (1.5 ml) was added 1-(piperidin-4-ylmethyl)pyrrolidin-2-one (97 mg, 0.54 mmol, 1.1 eq.). The mixture was heated to 105 °C under nitrogen atmosphere for 6 hours. The mixture was diluted with water (20 ml) and then extracted with ethyl acetate (15 ml × 3). The combined organic layer was washed with saturated brine (20 ml × 1), dried over anhydrous sodium sulfate and concentrated to give 1-((1-(4-iodo-2-nitrophenyl)piperidin-4-yl)methyl)pyrrolidin-2-one (0.21 g, crude) as a brown oil. LCMS (ESI): m/z 430 [M+1]⁺; TLC: Rf 0.5 (dichloromethane:methanol=30:1).

Step 17b: To a mixture of 1-((1-(4-iodo-2-nitrophenyl)piperidin-4-yl)methyl)pyrrolidin-2-one (0.21 g, 0.49 mmol, 1.0 eq.) prepared above, [1,1'-bis(diphenylphosphino)ferrocene]palladium dichloride (36 mg, 0.05 mmol, 0.1 eq.) and potassium carbonate (202 mg, 1.46 mmol, 3.0 eq.) in dioxane (5 ml) and water (1 ml) was added tert-butyl 4-(4,4,5,5-tetramethyl-1,3,2- dioxaborolan -2-yl)-1H-pyrazole-1-carboxylate (172 mg, 0.58 mmol, 1.2 eq.). The mixture was heated to 90 °C overnight under nitrogen atmosphere. The solvent was removed under reduced pressure. The residue was subjected to column chromatography on silica gel (dichloromethane: methanol 25: 1) to give 1-((1-(2-nitro-4-(1H-pyrazol-4-yl)phenyl)piperidin-4-yl)methyl)pyrrolidin-2-one (150 mg, yield: 83%) as a brown oil. LCMS (ESI): m/z 370 [M+1]⁺; TLC: Rf 0.5 (dichloromethane: methanol = 10: 1).

Step 17c: Zinc powder (264 mg, 4.1 mmol, 10.0 eq.) was added to a mixture of 1-((1-(2-nitro-4-(1H-pyrazol-4-yl)phenyl)piperidin-4-yl)methyl)pyrrolidin-2-one (150 mg, 0.41 mmol, 1.0 eq.) prepared above and ammonium chloride (219 mg, 4.1 mmol, 10.0 eq.) in methanol ( 5 ml). The mixture was heated to 50 °C under nitrogen atmosphere for 1.5 hours. The mixture was diluted with water (20 ml) and extracted with ethyl acetate (15 ml × 3). The combined organic layer was washed with saturated brine (20 ml × 1), dried over anhydrous sodium sulfate and concentrated to give 1-((1-(2-amino-4-(1H-pyrazol-4-yl)phenyl)piperidin-4-yl)methyl)pyrrolidin-2-one (75 mg, yield: 54%) as a pale yellow solid. LCMS (ESI): m/z 340 [M+1]⁺; TLC: Rf 0.4 (dichloromethane:methanol=10:1).

Step 17d: To a mixture of 1-((1-(2-amino-4-(1H-pyrazol-4-yl)phenyl)piperidin-4-yl)methyl)pyrrolidin-2-one (75 mg, 0.22 mmol, 1.0 eq.) obtained above, paraformaldehyde (159 mg, 1.77 mmol, 8.0 eq.) and acetic acid (20 mg, 0.33 mmol, 1.5 eq.) in methanol (4 ml) was added sodium cyanoborohydride (84 mg, 1.33 mmol, 6.0 eq.). The mixture was stirred at room temperature overnight. The mixture was diluted with water (20 ml). Solid sodium carbonate was added to adjust pH = 10, and then the aqueous layer was extracted with ethyl acetate (15 ml × 3). The combined organic layer was washed with saturated brine (20 ml × 1), dried over anhydrous sodium sulfate and concentrated. The residue was purified by Pre-TLC (ethyl acetate: methanol = 10: 1) to give 1-((1-(2-(dimethylamino)-4-(1H-pyrazol-4-yl)phenyl)piperidin-4-yl)methyl)pyrrolidin -2-one (15 mg, yield: 19%) as a white solid. LCMS (ESI): m/z 368 [M+1]⁺; TLC: Rf 0.5 (ethyl acetate: methanol = 10: 1). ¹ H NMR (500 MHz, MeOD) δ 7.83 (s, 2H), 7.22 - 7.02 (m, 2H), 6.91 (d, *J =* 8.0 Hz, 1H), 3.67 - 3.57 (m, 2H), 3.50 (t, *J =* 7.0 Hz, 2H), 3.23 (d, *J =* 7.2 Hz, 2H), 2.86 (s, 6H), 2.51 (t, *J =* 10.9 Hz, 2H), 2.41 (t, *J =* 8.1 Hz, 2H), 2.10 - 1.99 (m, 2H), 1.76 (dd, *J =* 23.7, 8.3 Hz, 3H), 1.44 (dd, *J =* 11.9, 3.1 Hz, 2H).

### Example 18: Preparation of 1-((1-(5-(1H-pyrazol-4-yl)pyridin-2-yl)piperidin-4-yl)methyl)pyrrolidin-2-one (Compound 21) (Prepared according to Scheme 1)

Step 18a: Preparation of 2-bromo-5-(1-(tetrahydro-2H-pyran-2-yl)-1H-pyrazol-4-yl)pyridine (Compound 0103-21): Under nitrogen protection, a mixture of (6-bromopyridin-3-yl)boronic acid (0101-21) (500 mg, 2.47 mmol, 1.1 eq.), 4-iodo-1-(tetrahydro-2H-pyran-2-yl)-1H-pyrazole (0102-1) (625 mg, 2.25 mmol, 1.0 eq.), [1,1'-bis(diphenylphosphino)ferrocene]palladium dichloride (82.4 mg, 0.113 mmol, 0.05 eq.), potassium carbonate (931 mg, 6.75 mmol, 3.0 eq.) in dioxane/water (20 ml/2 ml) was stirred at 105 ° C for 3.0 hours. The reaction was diluted with water and extracted with ethyl acetate. The organic layer was dried and concentrated under reduced pressure. The residue was purified by column chromatography on silica gel (petroleum ether/ethyl acetate = 10/1 to 3/1) to obtain a yellow solid product 2-bromo-5-(1-(tetrahydro-2H-pyran-2-yl)-1H-pyrazol-4-yl)pyridine (346 mg, yield: 50.1%). LCMS (ESI): m/z = 308 [M+1]⁺.

Step 18b: Preparation of 1-((1-(5-(1-(tetrahydro-2H-pyran-2-yl)-1H-pyrazol-4-yl)pyridin-2-yl)piperidin-4-yl)m ethyl)pyrrolidin-2-one (Compound 0109-21): Under nitrogen protection, a mixture of 1-(piperidin-4-ylmethyl)pyrrolidin-2-one (0108-1) (84 mg, 0.458 mmol, 1.0 eq.), 2-bromo-5-(1-(tetrahydro-2H-pyran-2-yl)-1H-pyrazol-4-yl)pyridine (0103-21) (155 mg, 0.504 mmol, 1.1 eq.), sodium tert-butoxide (122.5 mg, 1.377 mmol, 3.0 eq.), di-tert-butyl N,N-diethylphosphoramidite (11.4 mg, 0.045 mmol, 0.1 eq.) and tris(dibenzylideneacetone)dipalladium(0) (20 mg, 0.023 mmol, 0.05 eq.) in toluene (10 ml) was stirred at 120°C overnight. The reaction was diluted with water and extracted with ethyl acetate, and the organic phase was dried and concentrated under reduced pressure. The residue was purified by prep-TLC (silica gel, ethyl acetate/methanol = 100/1 to 50/1) to give a yellow solid product 1-((1-(5-(1-(tetrahydro-2H-pyran-2-yl)-1H-pyrazol-4-yl)pyridin-2-yl)piperidin-4-yl)m ethyl)pyrrolidin-2-one (68 mg, yield: 36.3%). LCMS (ESI): m/z = 410 [M+1]⁺.

Step 18c: Preparation of 1-((1-(5-(1H-pyrazol-4-yl)pyridin-2-yl)piperidin-4-yl)methyl)pyrrolidin-2-one (Compound 21): A mixture of 1-((1-(5-(1-(tetrahydro-2H-pyran-2-yl)-1H-pyrazol-4-yl)pyridin-2-yl)piperidin-4-yl)m ethyl)pyrrolidin-2-one (0109-21) (68 mg, 0.166 mmol, 1.0 eq.) in hydrogen chloride-dioxane (4 M, 5 mL) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product 1-((1-(5-(1H-pyrazol-4-yl)pyridin-2-yl)piperidin-4-yl)methyl)pyrrolidin-2-one (35 mg, yield: 64.8%). LCMS (ESI): m/z = 326 [M+1]⁺. Melting point : 125-134 ° C. ¹ H NMR (500 MHz, DMSO) δ 12.78 (s, 1H), 8.36 (s, 1H), 7.93 (s, 2H), 7.71 (d, *J =* 8.7 Hz, 1H), 6.83 (d, *J =* 8.6 Hz, 1H), 4.24 (d, *J =* 12.8 Hz, 2H), 3.35 (d, *J =* 5.8 Hz, 2H), 3.06 (d, *J =* 6.9 Hz, 2H), 2.78 (t, *J =* 12.4 Hz, 2H), 2.22 (t, *J =* 7.5 Hz, 2H), 1.94 (dd, *J* = 14.1, 6.9 Hz, 2H), 1.84 (s, 1H), 1.62 (d, *J =* 12.9 Hz, 2H), 1.11 (q, *J* = 12.2 Hz, 2H).

### Example 19: Preparation of 1-((1-(6-(1H-pyrazol-4-yl)pyridin-3-yl)piperidin-4-yl)methyl)pyrrolidin-2-one (Compound 22)

### (Prepared according to Scheme 2)

Step 19a: Preparation of 5-bromo-2-(1H - pyrazol-4-yl)pyridine (Compound 0205-22): Under nitrogen protection, 2,5-dibromopyridine (0201-22) (593 mg, 2.5 mmol, 1.0 eq.), 4-(1H- pyrazol -4-yl)boronic acid (280 mg, 2.5 mmol, 1.0 eq.), sodium carbonate (530 mg, 5.0 mmol, 2.0 eq.) and [1,1'-bis(diphenylphosphino)ferrocene]dichloropalladium (II) (118 mg, 0.16 mmol, 0.065 eq.) were stirred in a mixture of 60 mL dioxane and 6 mL water at 90 °C overnight. After cooling to room temperature, the reaction was extracted with ethyl acetate, the organic phase was washed with water and saturated brine. The organic phase was distilled under reduced pressure, and the residue was purified by column chromatography on silica gel (eluent: dichloromethane/methanol = 100/1 to 20/1) to obtain a yellow solid product 5-bromo-2-(1H - pyrazol-4-yl)pyridine (190 mg, yield: 33.9%). MS (ES⁺): m/z = 224 (M+H)⁺.

Step 19b: Preparation of 5- bromo-2-(1-(tetrahydro- 2H -pyran-2-yl)-1H-pyrazol-4-yl)pyridine (Compound 0103-22): 5-bromo-2-( 1H -pyrazol-4-yl)pyridine (0205-22) (190 mg, 0.85 mmol, 1.0 eq.) was dissolved in 20 mL dichloromethane, and 3,4-dihydro-2H - pyran (143 mg, 1.70 mmol, 2.0 eq.) and p-toluenesulfonic acid monohydrate (16 mg, 0.085 mmol, 0.1 eq.) were added. The mixture was stirred at room temperature for 2 hours. The reaction was quenched with aqueous sodium carbonate solution. Dichloromethane was added for extraction, and the mixture was washed with water and saturated brine. The organic phase was concentrated under reduced pressure, and the residue was purified by column chromatography on silica gel (eluent: petroleum ether /ethyl acetate = 10/1 to 4/1) to obtain a yellow oily product 5-bromo-2-(1-(tetrahydro- 2H -pyran-2-yl)-1H - pyrazol-4-yl)pyridine (181 mg, yield: 69.0%). MS (ES⁺): m/z =308(M+H)⁺.

Step 19c: Preparation of 1-((1-(6-(1-(tetrahydro- 2H -pyran-2-yl)-1H-pyrazol-4-yl)pyridin-3-yl)piperidin-4-yl)methyl)pyrrolidin-2- one (Compound 0109-22): Under nitrogen protection, a mixture of 5- bromo-2-(1-(tetrahydro- 2H -pyran-2-yl)-1H - pyrazol-4-yl)pyridine (0103-22) (150 mg, 0.49 mmol, 1.0 eq.), 1-(piperidin-4-ylmethyl)pyrrolidin-2-one (0108-1) (107 mg, 0.58 mmol, 1.2 eq.), sodium tert-butoxide (187 mg, 1.95 mmol, 4.0 eq.), 4,5-bis(diphenylphosphino)-9,9-dimethylxanthene (57 mg, 0.098 mmol, 0.2 eq.) and tris(dibenzylideneacetone)dipalladium(0) (45 mg, 0.049 mmol, 0.1 eq.) in toluene (50 ml) was stirred at 120 °C overnight. After cooling to room temperature, the reaction was filtered through celite and the filter cake was washed with ethyl acetate. The filtrate was concentrated under reduced pressure, and the residue was purified by prep-TLC (silica gel, eluent: dichloromethane/methanol = 30/1) to give a brown solid product 1-((1-(6-(1-(tetrahydro-2H - pyran-2-yl)-1H - pyrazol-4-yl)pyridin-3-yl)piperidin-4-yl)methyl)pyrrolidin-2-one (23 mg, yield: 11.5%). MS (ES⁺): m/z =410 (M+H)⁺.

Step 19d: Preparation of 1-((1-(6-(1H-pyrazol-4-yl)pyridin-3-yl)piperidin-4-yl)methyl)pyrrolidin-2-one (Compound 22): 1-((1-(6-(1-(tetrahydro-2H - pyran-2-yl)-1H - pyrazol-4-yl)pyridin-3-yl)piperidin-4-yl)methyl)pyrrolidin-2-one (0109-22) (23 mg, 0.056 mmol, 1.0 eq.) was added to 2 mL 4M hydrogen chloride- methanol solution and stirred at room temperature for 2 hours. The mixture was concentrated to dryness under reduced pressure. Water was added and the pH was adjusted to 10 with saturated aqueous sodium carbonate solution. Ethyl acetate was added for extraction, and the mixture was washed with water. The organic phase was dried over anhydrous sodium sulfate and concentrated under reduced pressure. The residue was purified by prep-TLC (silica gel, eluent: dichloromethane/methanol = 12/1) to give a yellow solid 1-((1-(6-(1H - pyrazol-4-yl)pyridin-3-yl)piperidin-4-yl)methyl)pyrrolidin-2-one (13 mg, yield: 71.4%). MS (ES⁺): m/z =326 (M+H)⁺. Melting point: 187-189 ° C. ¹ H NMR (500 MHz, DMSO) δ 12.84 (s, 1H), 8.21 (d, J = 2.9 Hz, 1H), 8.18 - 7.82 (m, 2H), 7.49 (d, J = 8.7 Hz, 1H), 7.31 (dd, J = 8.8, 3.0 Hz, 1H), 3.71 (d, J = 12.5 Hz, 2H), 3.36 (d, J = 7.0 Hz, 2H), 3.09 (d, J = 7.3 Hz, 2H), 2.68 (td, J = 12.2, 2.3 Hz, 2H), 2.23 (t, J = 8.1 Hz, 2H), 2.00 - 1.87 (m, 2H), 1.77 (dtd, J = 14.9, 7.4, 3.7 Hz, 1H), 1.66 (d, J = 12.7 Hz, 2H), 1.24 (dd, J = 9.2, 4.4 Hz, 2H).

### Example 20: Preparation of 1-((1-(5-(1H-pyrazol-4-yl)pyrimidin-2-yl) piperidin -4- yl) methyl) pyrrolidine -2- one ( Compound 23) ( Prepared according to Scheme 1)

Step 20a: Preparation of 2-chloro-5-(1-(tetrahydro-2H-pyran-2-yl)-1H-pyrazol-4-yl)pyrimidine (Compound 0103-23): Under nitrogen protection, a mixture of (2-chloropyrimidin-5-yl)boronic acid (0101-23) (300 mg, 1.9 mmol, 1.0 eq.), 4-iodo-1-(tetrahydro-2H-pyran-2-yl)-1H-pyrazole (0102-1) (580 mg, 2.09 mmol, 1.1 eq.), [1,1'-bis(diphenylphosphino)ferrocene]palladium dichloride (139 mg, 0.19 mmol, 0.1 eq.), potassium carbonate (650 mg, 4.75 mmol, 2.5 eq.) in dioxane/water (5 ml/1 ml) was stirred at 105 ° C for 3.0 hours. The reaction was diluted with water and extracted with ethyl acetate. The organic layer was dried and concentrated under reduced pressure. The residue was purified by column chromatography on silica gel (petroleum ether/ethyl acetate = 10/1 to 3/1) to obtain a brown solid product 2-chloro-5-(1-(tetrahydro-2H-pyran-2-yl)-1H-pyrazol-4-yl)pyrimidine (220 mg, yield: 43.7%). LCMS (ESI): m/z = 265 [M+1]⁺.

Step 20b: Preparation of 1-((1-(5-(1-(tetrahydro-2H-pyran-2-yl)-1H-pyrazol-4-yl)pyrimidin-2-yl)piperidin-4-yl) methyl)pyrrolidin-2-one (Compound 0109-23): Under nitrogen protection, a mixture of 1-(piperidin-4-ylmethyl)pyrrolidin-2-one (0108-1) (82.3 mg, 0.46 mmol, 1.1 eq.), 2-chloro-5-(1-(tetrahydro-2H-pyran-2-yl)-1H-pyrazol-4-yl)pyrimidine (0103-23) (110 mg, 0.42 mmol, 1.0 eq.) and potassium carbonate (116 mg, 0.84 mmol, 2.0 eq.) in dimethylformamide (5 ml) was stirred at 80 °C for 1.5 hours. The reaction was diluted with water and extracted with ethyl acetate, and the organic phase was dried and concentrated under reduced pressure. The residue was purified by prep-TLC (silica gel, ethyl acetate/methanol = 100/1 to 30/1) to obtain a yellow solid product 1-((1-(5-(1-(tetrahydro-2H-pyran-2-yl)-1H-pyrazol-4-yl)pyrimidin-2-yl)piperidin-4-yl) methyl)pyrrolidin-2-one (150 mg, yield: 87.1%). LCMS (ESI): m/z = 411 [M+1]⁺.

Step 20c: Preparation of 1-((1-(5-(1H-pyrazol-4-yl)pyrimidin-2-yl)piperidin-4-yl)methyl)pyrrolidin-2-one (Compound 23): A mixture of 1-((1-(5-(1-(tetrahydro-2H-pyran-2-yl)-1H-pyrazol-4-yl)pyrimidin-2-yl)piperidin-4-yl) methyl)pyrrolidin-2-one (0109-23) (150 mg, 0.365 mmol, 1.0 eq.) in hydrogen chloride-methanol (4 M, 5 mL) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product 1-((1-(5-(1H-pyrazol-4-yl)pyrimidin-2-yl)piperidin-4-yl)methyl)pyrrolidin-2-one (50 mg, yield: 42.0%). LCMS (ESI): m/z = 327 [M+1]⁺. ¹ H NMR (500 MHz, DMSO) δ 12.92 (s, 1H), 8.60 (s, 2H), 8.10 (s, 1H), 7.85 (s, 1H), 4.61 (d, *J =* 13.2 Hz, 2H), 3.35 (t, *J =* 7.0 Hz, 2H), 3.06 (d, *J =* 7.4 Hz, 2H), 2.88 (td, *J =* 13.1, 2.4 Hz, 2H), 2.22 (t, *J* = 8.1 Hz, 2H), 2.00 - 1.82 (m, 3H), 1.68 - 1.59 (m, 2H), 1.06 (qd, *J =* 12.4, 4.1 Hz, 2H).

### Example 21: Preparation of 1-((1-(4-(1H-pyrazol-4-yl)phenyl)piperidin-4-yl)methyl)piperidin-2-one (Compound 25) (Prepared according to Scheme 1)

Step 21a: Preparation of tert-butyl 4-((2-oxopiperidin-1-yl)methyl)piperidine-1-carboxylate (Compound 0107-25): Under nitrogen protection, sodium hydride (136.5 mg, 3.4 mmol, 2.0 eq.) was added to a mixture of piperidin-2-one (0106-25) (338 mg, 3.4 mmol, 2.0 eq.) in N,N-dimethylformamide (20 ml) in an ice bath, and the mixture was stirred at 80 °C for 30 minutes. tert-Butyl 4-(((methylsulfonyl)oxy)methyl)piperidine-1-carboxylate (0105-1) (500 mg, 1.7 mmol, 1.0 eq.) was added to the mixture and stirred at 85 °C overnight. After cooling to room temperature, the reaction was diluted with ammonium chloride and extracted with ethyl acetate. The organic layer was washed with brine, dried over anhydrous sodium sulfate, and concentrated under reduced pressure. The residue was purified by column chromatography on silica gel (petroleum ether/ethyl acetate = 5/1) to give yellow solid product tert-butyl 4-((2-oxopiperidin-1-yl)methyl)piperidine-1-carboxylate (250 mg, yield: 49.5%). LCMS (ESI): m/z = 241 [M+1]⁺.

Step 21b: Preparation of 1-(piperidin-4-ylmethyl)piperidin-2-one hydrochloride (Compound 0108-25): A mixture of tert-butyl 4-((2-oxopiperidin-1-yl)methyl)piperidine-1-carboxylate (0107-25) (250 mg, 0.84 mmol, 1.0 eq.) in hydrogen chloride-dioxane (4M, 5 mL) was stirred at room temperature for one hour. The solvent was removed under reduced pressure and the residue was used directly in the next step without further purification. LCMS (ESI): m/z = 197 [M+1]⁺.

Step 21c: Preparation of 1-((1-(4-(1-(tetrahydro-2H-pyran-2-yl)-1H-pyrazol-4-yl)phenyl)piperidin-4-yl)methyl) piperidin-2-one (Compound 0109-25): Under nitrogen protection, a mixture of 1-(piperidin-4-ylmethyl)piperidin-2-one hydrochloride (0108-25) (200 mg, 1.02 mmol, 1.0 eq.) and sodium tert-butoxide (587.7 mg, 6.12 mmol, 6.0 eq.) in toluene (10 ml) was stirred at 120 °C for 30 minutes. Then, 4-(4-bromophenyl)-1-(tetrahydro-2H-pyran-2-yl)-1H-pyrazole (0103-1) (343.5 mg, 1.12 mmol, 1.1 eq.), 4,5-bis(diphenylphosphino)-9,9-dimethylxanthene (58.7 mg, 0.10 mmol, 0.1 eq.) and tris(dibenzylideneacetone)dipalladium(0) (46.7 mg, 0.051 mmol, 0.05 eq.) was added to the mixture and stirred at 120 °C overnight. The reaction was diluted with water and extracted with ethyl acetate, and the organic phase was dried and concentrated under reduced pressure. The residue was purified by prep-TLC (silica gel, dichloromethane/methanol = 20/1) to give yellow solid product 1-((1-(4-(1-(tetrahydro-2H-pyran-2-yl)-1H-pyrazol-4-yl)phenyl)piperidin-4-yl)methyl) piperidin-2-one (170 mg, yield: 39.5%). LCMS(ESI): m/z = 423[M+1]⁺.

Step 21d: Preparation of 1-((1-(4-(1H-pyrazol-4-yl)phenyl)piperidin-4-yl)methyl)piperidin-2-one (Compound 25): A mixture of 1-((1-(4-(1-(tetrahydro-2H-pyran-2-yl)-1H-pyrazol-4-yl)phenyl)piperidin-4-yl)methyl) piperidin-2-one (0109-25) (170 mg, 0.4 mmol, 1.0 eq.) in hydrogen chloride-methanol (4 M, 5 mL) was stirred at room temperature for 1.5 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 15/1) to give a yellow solid product 1-((1-(4-1-((1-(4-(1H-pyrazol-4-yl)phenyl)piperidin-4-yl)methyl)piperidin-2-one (70 mg, yield: 52.94%). LCMS (ESI): m/z = 339 [M+1]⁺. ¹ H NMR (500 MHz, DMSO) δ 12.75 (s, 1H), 7.99 (s, 1H), 7.78 (s, 1H), 7.41 (d, *J =* 8.6 Hz, 2H), 6.90 (d, *J =* 8.7 Hz, 2H), 3.66 (d, *J =* 12.4 Hz, 2H), 3.26 (t, *J =* 5.5 Hz, 2H), 3.20 (d, *J =* 7.4 Hz, 2H), 2.62 (dd, *J =* 12.0, 10.2 Hz, 2H), 2.22 (t, *J =* 6.2 Hz, 2H), 1.87 - 1.59 (m, 7H), 1.34 - 1.20 (m, 2H).

### Example 22: Preparation of 1-((1-(4-(1H-pyrazol-4-yl)phenyl)piperidin-4-yl)methyl)pyrrolidine-2,5-dione (Compound 28) (Prepared according to Scheme 7)

Step 22a: Preparation of methyl 1-(4-(1-(tetrahydro-2H-pyran-2-yl)-1H-pyrazol-4-yl)phenyl)piperidine-4-carboxyl ate (Compound 0702-28): To a mixture of 4-(4-bromophenyl)-1-(tetrahydro-2H-pyran-2-yl)-1H-pyrazole (0103-1) (200 mg, 0.65 mmol, 1.0 eq.), tris(dibenzylideneacetone)dipalladium(0) (30 mg, 0.033 mmol, 0.05 eq.), 2-dicyclohexylphosphino-2',6'-diisopropoxy-1,1'-biphenyl (61 mg, 0.13 mmol, 0.2 eq.) and cesium carbonate (530 mg, 1.63 mmol, 2.5 eq.) in dioxane (8 ml) was added methyl piperidine-4-carboxylate ( 0701-28) (117 mg, 0.81 mmol, 1.25 eq.). The mixture was heated to 100 °C under a nitrogen atmosphere and stirred for 10 hours. The solvent was removed under reduced pressure. The residue was purified by column chromatography on silica gel (petroleum ether: ethyl acetate = 3: 1) to give a pale yellow solid product methyl 1-(4-(1-(tetrahydro-2H-pyran-2-yl)-1H-pyrazol-4-yl)phenyl)piperidine-4-carboxyl ate (186 mg, yield: 78%). LCMS (ESI): m/z 370 [M+1]⁺; TLC: Rf 0.5 (petroleum ether: ethyl acetate = 1: 1).

Step 22b: Preparation of (1-(4-(1-(tetrahydro-2H-pyran-2-yl)-1H-pyrazol-4-yl)phenyl)piperidin-4-yl)methanol (Compound 0703-28): To a mixture of methyl 1-(4-(1-(tetrahydro-2H-pyran-2-yl)-1H-pyrazol-4-yl)phenyl)piperidine-4-carboxyl ate (0702-28) (130 mg, 0.35 mmol, 1.0 eq.) in dichloromethane (6 ml) was added diisobutylaluminum hydride (1.5 M toluene solution, 0.59 ml, 0.88 mmol, 2.5 eq.) dropwise at 0°C. The mixture was stirred at 0°C for 1 hour. Saturated ammonium chloride solution (20 ml) was added to quench the reaction. The aqueous layer was extracted with dichloromethane (20 ml × 3). The combined organic layer was washed with saturated brine (20 ml × 1), dried over anhydrous sodium sulfate and concentrated to give a pale yellow solid product (1-(4-(1-(tetrahydro-2H-pyran-2-yl)-1H-pyrazol-4-yl)phenyl)piperidin-4-yl)methanol (118 mg, yield: 99%). LCMS (ESI): m/z 342 [M+1]⁺; TLC: Rf 0.3 (petroleum ether: ethyl acetate = 1:1).

Step 22c: Preparation of 1-((1-(4-(1-(tetrahydro-2H-pyran-2-yl)-1H-pyrazol-4-yl)phenyl)piperidin-4-yl)methyl) pyrrolidine-2,5-dione (Compound 0705-28): To a mixture of (1-(4-(1-(tetrahydro-2H-pyran-2-yl)-1H-pyrazol-4-yl)phenyl)piperidin-4-yl)methanol (0703-28) (119 mg, 0.35 mmol, 1.0 eq.), succinimide (69 mg, 0.70 mmol, 2.0 eq.) and triphenylphosphine (138 mg, 0.53 mmol, 1.5 eq.) in tetrahydrofuran (6 ml) was added diisopropyl azodicarboxylate (107 mg, 0.53 mmol, 1.5 eq.). The mixture was heated under nitrogen atmosphere at 30 °C. and stirred overnight. The solvent was removed under reduced pressure. The residue was purified by column chromatography on silica gel (ethyl acetate: methanol = 60: 1) to give a pale yellow solid product 1-((1-(4-(1-(tetrahydro-2H-pyran-2-yl)-1H-pyrazol-4-yl)phenyl)piperidin-4-yl)methyl) pyrrolidine-2,5-dione (66 mg, yield: 45%). LCMS (ESI): m/z 423 [M+1]⁺; TLC: Rf 0.5 (dichloromethane: methanol = 20: 1).

Step 22d: Preparation of 1-((1-(4-(1H-pyrazol-4-yl)phenyl)piperidin-4-yl)methyl)pyrrolidine-2,5-dione (Compound 28): A mixture of 1-((1-(4-(1-(tetrahydro-2H-pyran-2-yl)-1H-pyrazol-4-yl)phenyl)piperidin-4-yl)methyl) pyrrolidine-2,5-dione (0705-28) (66 mg, 0.16 mmol, 1.0 eq.) in hydrogen chloride-methanol solution (4M, 3 ml) was stirred at room temperature for 2 hours. The mixture was diluted with water (20 ml). Solid sodium carbonate was added to adjust pH = 10, and then the aqueous layer was extracted with ethyl acetate (20 ml × 3). The combined organic layer was washed with saturated brine (20 ml × 1), dried over anhydrous sodium sulfate and concentrated. The residue was purified by Pre-TLC (ethyl acetate: dichloromethane: methanol = 10: 10: 1) to give 1-((1-(4-(1H-pyrazol-4-yl)phenyl)piperidin-4-yl)methyl)pyrrolidine-2,5-dione (25 mg, yield: 47%) as a white solid. LCMS (ESI): m/z 339 [M+1]⁺; TLC: Rf 0.5 (dichloromethane: methanol = 10: 1). ¹ H NMR (500 MHz, DMSO) δ 12.75 (s, 1H), 7.89 (s, 2H), 7.41 (s, 2H), 6.91 (s, 2H), 3.66 (s, 2H), 3.33 (s, 4H), 2.61 (d, J = 31.7 Hz, 4H), 1.70 (d, J = 38.6 Hz, 2H), 1.24 (s, 3H).

### Example 23: Preparation of 2-((1-(4-(1H-pyrazol-4-yl)phenyl)piperidin-4-yl)methyl)-2-azaspiro[4.5]decan-1-o ne (Compound 31) (Prepared according to Scheme 7)

Step 23a: Preparation of (1-(4-(1-(tetrahydro-2H-pyran-2-yl)-1H-pyrazol-4-yl)phenyl)piperidin-4-yl)methyl methanesulfonate (Compound 0704-31): To a mixture of (1-(4-(1-(tetrahydro-2H-pyran-2-yl)-1H-pyrazol-4-yl)phenyl)piperidin-4-yl)methanol (0703-28) (294 mg, 0.86 mmol, 1.0 eq.) and N,N-diisopropylethylamine (222 mg, 1.72 mmol, 2.0 eq.) in dichloromethane (6 mL) was added methanesulfonyl chloride (148 mg, 1.29 mmol, 1.5 eq.) at 0 °C. The mixture was warmed to room temperature and then stirred for 0.5 h. The solvent was removed under reduced pressure. The residue was purified by column chromatography on silica gel (petroleum ether: ethyl acetate = 1: 1) to give (1-(4-(1-(tetrahydro-2H-pyran-2-yl)-1H-pyrazol-4-yl)phenyl)piperidin-4-yl)methyl methanesulfonate (288 mg, yield: 80%) as a white solid. LCMS (ESI): m/z 420 [M+1]⁺; TLC: Rf 0.5 (dichloromethane: methanol = 20:1).

Step 23b: Preparation of 2-((1-(4-(1-(tetrahydro-2H-pyran-2-yl)-1H-pyrazol-4-yl)phenyl)piperidin-4-yl)methyl) -2-azaspiro[4.5]decan-1-one (Compound 0705-31): To a mixture of sodium hydride (60% dispersion in mineral oil, 17 mg, 0.43 mmol, 2.0 eq.) in N,N-dimethylformamide (3 ml) was added 2-azaspiro[4.5]decan-1-one (0704-31) (66 mg, 0.43 mmol, 2.0 eq.). The mixture was stirred at room temperature for 10 minutes, and (1-(4-(1-(tetrahydro-2H-pyran-2-yl)-1H-pyrazol-4-yl)phenyl)piperidin-4-yl)methyl methanesulfonate (90 mg, 0.22 mmol, 1.0 eq.) was added. The mixture was then heated to 65 °C for 3 hours under a nitrogen atmosphere. The mixture was diluted with water (20 ml), and the aqueous layer was extracted with ethyl acetate (15 ml × 3). The combined organic layer was washed with saturated brine (15 ml × 1), dried over anhydrous sodium sulfate and concentrated. The residue was purified by Pre-TLC (silica gel, ethyl acetate: dichloromethane: methanol = 15: 15: 1) to give 2-((1-(4-(1-(tetrahydro-2H-pyran-2-yl)-1H-pyrazol-4-yl)phenyl)piperidin-4-yl)methyl) -2-azaspiro[4.5]decan-1-one (52 mg, yield: 51%) as a pale yellow oil. LCMS (ESI): m/z 477 [M+1]⁺; TLC: Rf 0.5 (dichloromethane: methanol = 20:1).

Step 23c: Preparation of 2-((1-(4-(1H-pyrazol-4-yl)phenyl)piperidin-4-yl)methyl)-2-azaspiro[4.5]decan-1-one (Compound 31): A mixture of 2-((1-(4-(1-(tetrahydro-2H-pyran-2-yl)-1H-pyrazol-4-yl)phenyl)piperidin-4-yl)methyl) -2-azaspiro[4.5]decan-1-one (0705-31 (52 mg, 0.11 mmol, 1.0 eq.) in hydrogen chloride-methanol solution (4 M, 3 ml) was stirred at room temperature. 2 hours. The mixture was diluted with water (30 ml). Solid sodium carbonate was added to adjust the pH to 10, and then the aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, dichloromethane: methanol = 10: 1) to give a white solid 2-((1-(4-(1H-pyrazol-4-yl)phenyl)piperidin-4-yl)methyl)-2-azaspiro[4.5]decane-1-one (30 mg, yield: 70%). LCMS (ESI): m/z 393[M+1]⁺; TLC: Rf 0.5 (dichloromethane:methanol=10:1). ¹ H NMR (500 MHz, MeOD) δ 7.83 (s, 2H), 7.43 (d, *J =* 8.6 Hz, 2H), 6.98 (d, *J =* 8.6 Hz, 2H), 3.66 (t, *J =* 10.6 Hz, 2H), 3.40 (t, *J =* 7.0 Hz, 2H), 3.21 (d, *J =* 7.3 Hz, 2H), 2.69 (t, *J =* 11.3 Hz, 2H), 2.01 (t, *J =* 6.9 Hz, 2H), 1.93 - 1.78 (m, 1H), 1.77 - 1.55 (m, 6H), 1.39 (dt, *J =* 19.9, 11.5 Hz, 6H), 1.29 (d, *J =* 3.6 Hz, 2H).

### Example 24: Preparation of 2-((1-(4-(1H-pyrazol-4-yl)phenyl)piperidin-4-yl)methyl)-2-azabicyclo[2.2.1]hepta n-3-one (Compound 33) (Prepared according to Scheme 7)

Step 24a: Preparation of 2-((1-(4-(1-(tetrahydro-2H-pyran-2-yl)-1H-pyrazol-4-yl)phenyl)piperidin-4-yl)methyl) -2-azabicyclo[2.2.1]heptan-3-one (Compound 0705-33): To a mixture of sodium hydride (60% dispersion in mineral oil, 15 mg, 0.38 mmol, 2.0 eq.) in N,N-dimethylformamide (2.5 mL) was added 2-azabicyclo[2.2.1]heptan-3-one (0704-31) (42 mg, 0.38 mmol, 2.0 eq.). The mixture was stirred at room temperature for 10 minutes. (1-(4-(1-(tetrahydro-2H-pyran-2-yl)-1H-pyrazol-4-yl)phenyl)piperidin-4-yl)methyl methanesulfonate (80 mg, 0.19 mmol, 1.0 eq.) was added, and the mixture was heated to 65 °C under a nitrogen atmosphere and reacted for 3 hours. The mixture was diluted with water (20 ml), and the aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, ethyl acetate: dichloromethane: methanol = 15: 15: 1) to give a pale yellow oily product 2-((1-(4-(1-(tetrahydro-2H-pyran-2-yl)-1H-pyrazol-4-yl)phenyl)piperidin-4-yl)methyl) -2-azabicyclo[2.2.1]heptan-3-one (40 mg, yield: 48%). LCMS (ESI): m/z 435 [M+1]⁺; TLC: Rf 0.5 (dichloromethane: methanol = 20:1).

Step 24b: Preparation of 2-((1-(4-(1H-pyrazol-4-yl)phenyl)piperidin-4-yl)methyl)-2-azabicyclo[2.2.1]heptan-3 -one (Compound 33): A mixture of 2-((1-(4-(1-(tetrahydro-2H-pyran-2-yl)-1H-pyrazol-4-yl)phenyl)piperidin-4-yl)methyl) -2-azabicyclo[2.2.1]heptan-3-one (0705-33) (40 mg, 0.092 mmol, 1.0 eq.) in a solution of hydrogen chloride-methanol (4M, 2.5 ml) was stirred at room temperature for 2 hours. The mixture was diluted with water (15 ml). Solid sodium carbonate was added to adjust pH = 10, and then the aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, dichloromethane: methanol: aqueous ammonia = 10: 1: 0.1) to give 2-((1-(4-(1H-pyrazol-4-yl)phenyl)piperidin-4-yl)methyl)-2-azabicyclo[2.2.1]heptan-3 -one as a white solid (17 mg, yield: 53%). LCMS (ESI): m/z 351 [M+1]⁺; TLC: Rf 0.5 (dichloromethane: methanol = 10: 1). ¹ H NMR (500 MHz, MeOD) δ 7.83 (s, 2H), 7.44 (d, *J =* 8.5 Hz, 2H), 6.99 (d, *J =* 8.6 Hz, 2H), 3.95 (s, 1H), 3.68 (d, *J =* 11.6 Hz, 2H), 3.28 (d, *J=* 7.6 Hz, 1H), 2.92 - 2.64 (m, 4H), 2.00 - 1.64 (m, 7H), 1.44 (ddd, *J=* 28.4, 23.3, 13.1 Hz, 4H).

### Example 25: Preparation of 1-((1-(4-(isoxazol-5-yl)phenyl)piperidin-4-yl)methyl)pyrrolidin-2-one (Compound 34)

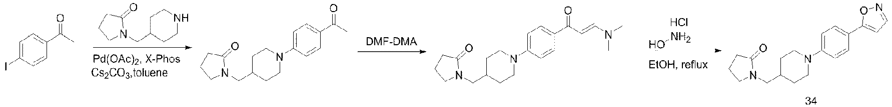

1-(Piperidin-4-ylmethyl)pyrrolidin-2-one (120 mg, 0.66 mmol, 1.0 eq.) was added to a mixture of 4-iodoacetophenone (162 mg, 0.66 mmol, 1.0 eq.), palladium acetate (14.8 mg, 0.066 mmol, 0.1 eq.), 2-dicyclohexylphosphino-2',4',6'-triisopropylbiphenyl (62 mg, 0.132 mmol, 0.2 eq.) and cesium carbonate (430 mg, 1.32 mmol, 2.0 eq.) in toluene (15 ml). The mixture was heated to 100 °C under nitrogen atmosphere for 5 hours. The solvent was removed under reduced pressure. The residue was purified by column chromatography on silica gel (dichloromethane: methanol = 30: 1) to give 1-((1-(4-acetylphenyl)piperidin-4-yl)methyl)pyrrolidin-2-one (68 mg, yield: 34%) as a pale yellow solid. LCMS (ESI): m/z 301 [M+1]⁺; TLC: Rf 0.5 (dichloromethane: methanol = 10: 1).

To a mixture of 1-((1-(4-acetylphenyl)piperidin-4-yl)methyl)pyrrolidin-2-one (68 mg, 0.23 mmol, 1.0 eq.) obtained above in N,N-dimethylformamide (1 ml) was added 1,1-dimethoxy-N,N-dimethylmethanamine (0.5 ml). The mixture was heated to 125 °C under a nitrogen atmosphere and reacted overnight. The solvent was removed under reduced pressure and then concentrated in vacuo to obtain a pale yellow solid product (E)-1-((1-(4-(3-(dimethylamino)acryloyl)phenyl)piperidin-4-yl)methyl)pyrrolidin-2-o ne (85 mg, crude). LCMS (ESI): m/z 356 [M+1]⁺; TLC: Rf 0.3 (dichloromethane:methanol = 20:1).

To a mixture of (E)-1-((1-(4-(3-(dimethylamino)acryloyl)phenyl)piperidin-4-yl)methyl)pyrrolidin-2-o ne (81 mg, 0.23 mmol, 1.0 eq.) obtained above in ethanol (3 ml) was added hydroxylamine hydrochloride (20.5 mg, 0.30 mmol, 1.3 eq.). The mixture was heated to 90 °C under a nitrogen atmosphere for 1.5 hours. The mixture was diluted with water (15 ml). Solid sodium carbonate was added to adjust the pH to 10, and then the aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, ethyl acetate: dichloromethane: methanol = 15: 15: 2) to give a pale yellow solid product (1-((1-(4-(isoxazol-5-yl)phenyl)piperidin-4-yl)methyl)pyrrolidin-2-one (28 mg, yield: 38%). LCMS (ESI): m/z 326 [M+1]⁺; TLC: Rf 0.5 (dichloromethane: methanol = 15: 1). ¹ H NMR (500 MHz, MeOD) δ 8.33 (d, *J =* 1.9 Hz, 1H), 7.67 (t, *J =* 5.8 Hz, 2H), 7.02 (d, *J =* 8.9 Hz, 2H), 6.54 (d, *J =* 1.9 Hz, 1H), 3.86 (d, *J =* 12.8 Hz, 2H), 3.50 (t, *J* = 7.1 Hz, 2H), 3.20 (d, *J =* 7.4 Hz, 2H), 2.81 (td, *J =* 12.5, 2.5 Hz, 2H), 2.40 (t, *J =* 8.1 Hz, 2H), 2.15 - 1.97 (m, 2H), 1.88 (ddd, *J =* 11.3, 7.5, 3.8 Hz, 1H), 1.75 (d, *J =* 12.5 Hz, 2H), 1.36 (ddd, *J =* 17.9, 12.1, 6.2 Hz, 2H).

### Example 26: Preparation of 1-(4-(1H-pyrazol-4-yl)phenyl)-4-(pyrrolidin-1-ylmethyl)piperidine (Compound 40) (Prepared according to Scheme 3)

Step 26a: Preparation of tert-butyl 4-(pyrrolidine-1-carbonyl)piperidine-1-carboxylate (Compound 0302-40): To a mixture of 1-(tert- butoxycarbonyl)piperidine-4-carboxylic acid (0301-40) (350 mg, 1.53 mmol, 1.0 eq.), 1-ethyl-(3-dimethylaminopropyl)carbodiimide hydrochloride (380 mg, 1.98 mmol, 1.3 eq.), 1-hydroxybenzotriazole (267 mg, 1.98 mmol, 1.3 eq.) and N,N-diisopropylethylamine (592 mg, 4.59 mmol, 3.0 eq.) in dichloromethane (10 ml) was added tetrahydropyrrole (141 mg, 1.98 mmol, 1.3 eq.). The mixture was stirred at room temperature overnight. The solvent was removed under reduced pressure. The residue was separated by column chromatography on silica gel (dichloromethane: methanol 150: 1) to give tert-butyl 4-(pyrrolidine-1-carbonyl)piperidine-1-carboxylate (420 mg, yield: 97%) as a pale yellow oil. LCMS (ESI): m/z 283 [M+1]⁺; TLC: Rf 0.5 (dichloromethane: methanol = 60: 1).

Step 26b: Preparation of piperidin-4-yl(pyrrolidin-1-yl)methanone (Compound 0303-40): A mixture of tert-butyl 4-(pyrrolidine-1-carbonyl)piperidine-1-carboxylate (0302-40) (300 mg, 1.06 mmol, 1.0 eq.) in hydrogen chloride- dioxane solution (4 M, 3 ml) was stirred at room temperature for 1.5 hours. The mixture was diluted with water (20 ml). Solid sodium carbonate was added to adjust the pH to 10, and then the aqueous layer was extracted with dichloromethane (10 ml × 8). The combined organic layer was washed with saturated brine (20 ml × 1), dried over anhydrous sodium sulfate and concentrated to give piperidin-4-yl(pyrrolidin-1-yl)methanone (120 mg, yield: 62%) as a pale yellow oil. LCMS (ESI): m/z 183 [M+1]⁺; TLC: Rf 0.3 (dichloromethane:methanol=5:1).

Step 26c: Preparation of pyrrolidin-1-yl(1-(4-(1-(tetrahydro-2H-pyran-2-yl)-1H-pyrazol-4-yl)phenyl)piperidin-4-yl)methanone (Compound 0304-40): To a mixture of 4-(4-bromophenyl)-1-(tetrahydro-2H-pyran-2-yl)-1H-pyrazole (0103-1) (50 mg, 0.16 mmol, 1.0 eq.), tris(dibenzylideneacetone)dipalladium(0) (7.5 mg, 0.008 mmol, 0.05 eq.), 4,5-bis(diphenylphosphino-9,9-dimethylxanthene) (9.3 mg, 0.016 mmol, 0.1 eq.) and sodium tert-butoxide (39 mg, 0.41 mmol, 2.5 eq.) in toluene (5 ml) was added piperidin-4-yl(pyrrolidin-1-yl)methanone (0303-40) (36 mg, 0.20 mmol, 1.2 eq.). The mixture was heated to 120 °C under nitrogen atmosphere and reacted overnight. The solvent was removed under reduced pressure. The residue was purified by Pre-TLC (silica gel, ethyl acetate: methanol = 30: 1) to give a colorless oily product pyrrolidin-1-yl(1-(4-(1-(tetrahydro-2H-pyran-2-yl)-1H-pyrazol-4-yl)phenyl)piperidin-4-yl)methanone (25 mg, yield: 38%). LCMS (ESI): m/z 409 [M+1]⁺; TLC: Rf 0.5 (ethyl acetate:methanol = 30:1).

Step 26d: Preparation of 4-(Pyrrolidin-1-ylmethyl)-1-(4-(1-(tetrahydro-2H-pyran-2-yl)-1H-pyrazol-4-yl)phenyl) piperidine (Compound 0305-40): Under nitrogen protection, lithium aluminum hydride (0.88 ml, 1 mol /L tetrahydrofuran solution, 3.0 eq.) was added to a tetrahydrofuran solution (12 ml) of pyrrolidin-1-yl (1-(4-(1-(tetrahydro-2H-pyran-2-yl)-1H-pyrazol-4-yl)phenyl)piperidin-4-yl)methanon e (0304-40) (120 mg, 0.294 mmol, 1.0 eq.) at 0 °C^{,} and the mixture was stirred at 0 °C for 40 minutes. The reaction was quenched with water, and then 15% sodium hydroxide solution was added. After filtration, the filtrate was extracted with ethyl acetate. The organic layer was dried and concentrated under reduced pressure. The residue was purified by prep-TLC (silica gel, dichloromethane/methanol = 12/1) to give a white solid product 4-(pyrrolidin-1-ylmethyl)-1-(4-(1-(tetrahydro-2H- pyran -2-yl)-1H-pyrazol-4-yl)phenyl)piperidine (84 mg, yield: 73.0%). LCMS (ESI): m/z = 395 [M+1]⁺.

Step 26e:. Preparation of 1-(4-(1H-pyrazol-4-yl)phenyl)-4-(pyrrolidin-1-ylmethyl)piperidine (Compound 40): A mixture of 4-(pyrrolidin-1-ylmethyl)-1-(4-(1-(tetrahydro-2H-pyran-2-yl)-1H-pyrazol-4-yl)phenyl) piperidine (0305-40) (50 mg, 0.118 mmol, 1.0 eq.) in hydrogen chloride-dioxane (4 M, 5 ml) was stirred at room temperature for 1.5 hours. The solvent was removed under reduced pressure, the residue was diluted with water, adjusted to pH = 9 with sodium carbonate solution, extracted with dichloromethane, and the organic phase was dried and concentrated under reduced pressure. The residue was purified by prep-TLC (silica gel, dichloromethane/methanol = 12/1) to give a white solid product 1-(4-(1H-pyrazol-4-yl)phenyl)-4-(pyrrolidin-1-ylmethyl)piperidine (40 mg, yield: 60.6%). LCMS (ESI): m/z = 311 [M+1]⁺. ¹ H NMR (500 MHz, DMSO) δ 12.76 (s, 1H), 7.99 (s, 1H), 7.78 (s, 1H), 7.41 (d, *J =* 8.7 Hz, 2H), 6.91 (d, *J =* 8.8 Hz, 2H), 3.60 (t, *J =* 38.4 Hz, 2H), 2.63 (dd, *J =* 12.0, 10.1 Hz, 3H), 2.43 (d, *J =* 65.9 Hz, 5H), 1.75 (dd, *J =* 70.4, 29.6 Hz, 7H), 1.26 - 1.17 (m, 2H).

### Example 27: Preparation of 1-(4-(1H-pyrazol-4-yl)phenyl)-4-(4-chlorobenzyl)piperidine (Compound 41) (Prepared according to Scheme 4)

### Step 27a: Preparation of 4-(4-chlorobenzyl)piperidine (Compound 0401-41):

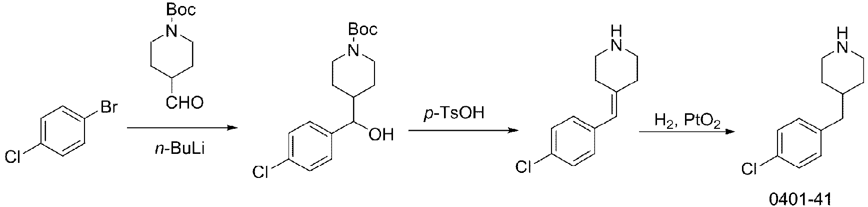

Under nitrogen protection, n-butyl lithium (2.5 mmol/L dissolved in tetrahydrofuran) (6.8 ml, 17.01 mmol, 1.3 eq.) was added dropwise to a solution of 4-bromochlorobenzene (2.5 g, 13.09 mmol, 1 eq.) in tetrahydrofuran (25 ml) at -70 °C. The mixture was stirred for 30 minutes. tert-butyl 4-formylpiperidine-1-carboxylate was slowly added dropwise to the mixture, and the reaction was stirred for 6 hours. The reaction was quenched with saturated ammonium chloride solution and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 6/1) to obtain a yellow oily tert-butyl 4-((4-chlorophenyl)(hydroxy)methyl)piperidine-1-carbonate (2.01 g, yield: 47.21%). LCMS (ESI) [M+1]⁺: m/z =326.

Tert-butyl 4-((4-chlorophenyl)(hydroxy)methyl)piperidine-1-carbonate (2.03 g, 6.25 mmol, 1 eq.) obtained above, p-toluenesulfonic acid monohydrate (2.38 g, 12.50 mmol, 2 eq.), and toluene (65 ml) were mixed and stirred at 120°C for 24 hours. The reaction was diluted with water and extracted with ethyl acetate. The organic layer was washed with saturated brine, dried over anhydrous sodium sulfate, and concentrated under reduced pressure. The residue was purified by column chromatography on basic alumina (dichloromethane/methanol = 20/1) to obtain a yellow oily product 4-(4-chlorobenzylidene)piperidine (810 mg, yield: 62.61%). LCMS (ESI) [M+1]⁺ : m/z = 208.

Under a hydrogen atmosphere, 4-(4-chlorobenzylidene)piperidine (443 mg, 2.14 mmol, 1.0 eq.) obtained above, platinum dioxide (48 mg, 0.21 mmol, 0.1 eq.), ethanol (2 ml) and acetic acid (2 ml) were stirred at room temperature for 2 hours. The mixture was filtered and the filtrate was concentrated under reduced pressure. The residue was adjusted to pH = 9 with sodium carbonate solution and extracted with dichloromethane. The organic phase was dried and concentrated under reduced pressure to obtain a pale yellow solid crude product 4-(4-chlorobenzyl)piperidine (423 mg, yield: 94.58%). LCMS (ESI) [M+1]⁺ : m/z = 210.

Step 27b: Preparation of 4-(4-chlorobenzyl)-1-(4-(1-(tetrahydro- 2H -pyran-2-yl)-1H - pyrazol-4-yl)phenyl)piperidine (Compound 0402-41): Under nitrogen protection, 4-(4-chlorobenzyl)piperidine (0401-41) (100 mg, 0.48 mmol, 1 eq.), 4-(4-bromophenyl)-1-(tetrahydro- 2H -pyran-2-yl)-1H - pyrazole (0103-1) (146 mg, 0.48 mmol, 1 eq.), sodium tert-butoxide (115 mg, 1.2 mmol, 2.5 eq.), 4,5-bis(diphenylphosphino-9,9-dimethylxanthene (28 mg, 0.048 mmol, 0.1 eq.), tris(dibenzylideneacetone)dipalladium (22 mg, 0.024 mmol, 0.05 eq.), toluene (4 ml) were mixed and stirred at 120 °C overnight. The reaction was diluted with water and extracted with ethyl acetate. The organic layer was washed with saturated brine, dried over anhydrous sodium sulfate, and concentrated under reduced pressure. The residue was purified by thick silica gel plate (dichloromethane/methanol = 30/1) to give a white solid product 4-(4-chlorobenzyl)-1-(4-(1-(tetrahydro- 2H -pyran-2-yl)-1H - pyrazol-4-yl)phenyl)piperidine (47 mg, yield: 22.56%). LCMS (ESI) [M+1]⁺ : m/z = 436.

Step 27c: Preparation of 1-(4-(1H-pyrazol-4-yl)phenyl)-4-(4-chlorobenzyl)piperidine (Compound 41): A mixture of 4-(4-chlorobenzyl)-1-(4-(1-(tetrahydro-2H-pyran-2-yl)-1H-pyrazol-4-yl)phenyl)piperi dine (0402-41) (47 mg, 0.11 mmol, 1.0 eq.) in hydrogen chloride-methanol solution (4M, 2 ml) was stirred at room temperature for 2 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by thick prep-TLC (silica gel, dichloromethane/methanol = 10/1) to give 1-(4-(1H-pyrazol-4-yl)phenyl)-4-(4-chlorobenzyl)piperidine (34 mg, yield: 90.26%) as a white solid. LCMS (ESI) [M+1]⁺ : m/z = 352. ¹ H NMR (500 MHz, DMSO) δ 12.74 (s, 1H), 7.88 (d, *J =* 87.4 Hz, 2H), 7.37 (dd, *J =* 31.1, 8.2 Hz, 4H), 7.22 (d, *J =* 8.0 Hz, 2H), 6.92 (t, *J =* 27.0 Hz, 2H), 3.64 (d, *J =* 12.2 Hz, 2H), 2.57 (dd, *J =* 24.9, 9.1 Hz, 4H), 1.63 (d, *J* = 11.5 Hz, 3H), 1.31 (dd, *J* = 20.8, 10.7 Hz, 2H).

### Example 28: Preparation of 2-((1-(4-(1H-pyrazol-4-yl)phenyl)piperidin-4-yl)methyl)-3,4-dihydroisoquinolin-1 (2H)-one (Compound 42) (Prepared according to Scheme 4)

Step 28a: Preparation of 2-(piperidin-4-ylmethyl)-3,4-dihydroisoquinolin-1(2H)-one hydrochloride (Compound 0403-42): Sodium hydride (262.1 mg, 5.46 mmol, 2.0 eq.) was added to a mixture of 3,4-dihydroisoquinolin-1(2H)-one (803.6 mg, 5.46 mmol, 2.0 eq.) in N,N-dimethylformamide (20 ml) under nitrogen protection and ice-bath, and the mixture was stirred at 65 °C for 30 minutes. tert-Butyl 4-(((methylsulfonyl)oxy)methyl)piperidine-1-carboxylate (0105-1) (800 mg, 2.73 mmol, 1.0 eq.) was added to the mixture, and stirred at 65 °C overnight. Then the reaction was cooled to room temperature, diluted with ammonium chloride and extracted with ethyl acetate. The organic layer was washed with brine, dried over anhydrous sodium sulfate, and concentrated under reduced pressure. The residue was purified by column chromatography on silica gel (petroleum ether/ethyl acetate = 5/1) to obtain a yellow solid product tert-butyl 4-((1-oxo-3,4-dihydroisoquinolin-2(1H)-yl)methyl)piperidine-1-carboxylate (784 mg, yield: 83.4%). LCMS (ESI): m/z = 289 [M+1]⁺.

A mixture of tert-butyl 4-((1-oxo-3,4-dihydroisoquinolin-2(1H)-yl)methyl)piperidine-1-carboxylate (784 mg, 2.72 mmol, 1.0 eq.) obtained above in hydrogen chloride-dioxane solution (4M, 5 ml) was stirred at room temperature for one hour. The solvent was removed under reduced pressure and the residue was used directly in the next step without further purification. LCMS (ESI): m/z = 233 [M+1]⁺.

Step 28b: Preparation of 2-((1-(4-(1-(tetrahydro-2H-pyran-2-yl)-1H-pyrazol-4-yl)phenyl)piperidin-4-yl)methyl) -3,4-dihydroisoquinolin-1(2H)-one (Compound 0404-42): Under nitrogen protection, a mixture of 2-(piperidin-4-ylmethyl)-3,4-dihydroisoquinolin-1(2H)-one hydrochloride (0403-42) (200 mg, 0.5 mmol, 1.0 eq.) and sodium tert-butoxide (334.8 mg, 3.49 mmol, 6.0 eq.) in toluene (10 ml) was stirred at 120 °C for 30 minutes. 4-(4-bromophenyl)-1-(tetrahydro-2H-pyran-2-yl)-1H-pyrazole (0103-1) (195.7 mg, 0.64 mmol, 1.1 eq.), 4,5-bis(diphenylphosphino)-9,9-dimethylxanthene (33.4 mg, 0.058 mmol, 0.1 eq.) and tris(dibenzylideneacetone)dipalladium(0) (26.6 mg, 0.029 mmol, 0.05 eq.) were then added to the mixture, and the mixture was stirred at 120 °C overnight. The reaction was diluted with water and extracted with ethyl acetate, and the organic phase was dried and concentrated under reduced pressure. The residue was purified by prep-TLC (silica gel, dichloromethane/methanol = 20/1) to give a yellow solid product 2-((1-(4-(1-(tetrahydro-2H-pyran-2-yl)-1H-pyrazol-4-yl)phenyl)piperidin-4-yl)methyl) -3,4-dihydroisoquinolin-1(2H)-one (70.3 mg, yield: 25.75 %). LCMS (ESI): m/z = 471 [M+1]⁺.

Step 28c: Preparation of 2-((1-(4-(1H-pyrazol-4-yl)phenyl)piperidin-4-yl)methyl)-3,4-dihydroisoquinolin-1(2H) -one (Compound 42): A mixture of 2-((1-(4-(1-(tetrahydro-2H-pyran-2-yl)-1H-pyrazol-4-yl)phenyl)piperidin-4-yl)methyl) -3,4-dihydroisoquinolin-1(2H)-one (0404-42) (70.3 mg, 0.15 mmol, 1.0 eq.) in hydrogen chloride-methanol (4 M, 5 mL) was stirred at room temperature for 1.5 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product 2-((1-(4-(1H-pyrazol-4-yl)phenyl)piperidin-4-yl)methyl)-3,4-dihydroisoquinolin-1(2H) -one (35 mg, yield: 60.64%). LCMS (ESI): m/z = 387 [M+1]⁺. ¹ H NMR (500 MHz, DMSO) δ 12.75 (s, 1H), 7.99 (s, 1H), 7.87 (d, *J =* 7.6 Hz, 1H), 7.78 (s, 1H), 7.49-7.37 (m, 3H), 7.39 - 7.22 (m, 2H), 6.91 (d, *J =* 8.4 Hz, 2H), 3.68 (d, *J =* 12.1 Hz, 2H), 3.57 (t, *J =* 6.3 Hz, 2H), 3.41 (d, *J =* 7.1 Hz, 2H), 2.98 (t, *J =* 6.0 Hz, 2H), 2.64 (t, *J =* 11.4 Hz, 2H), 1.87 (s, 1H), 1.71 (d, *J =* 12.1 Hz, 2H), 1.33 (dd, *J* = 20.8, 11.3 Hz, 2H).

### Example 29: Preparation of N-((1-(4-(1H-pyrazol-4-yl)phenyl)piperidin-4-yl)methyl)acetamide (Compound 43) (Prepared according to Scheme 5)

Step 29a: Preparation of tert-butyl 4-(acetamidomethyl)piperidine-1 -carboxylate (Compound 0504-43): Acetyl chloride ( 322 mg, 5.13 mmol, 1.1 eq.) was added to a mixture of tert-butyl 4-(aminomethyl)piperidine-1-carboxylate (0501-43) (800 mg, 4.67 mmol, 1.0 eq.) and diisopropylethylamine (1.4 g, 14.0 mmol, 3.0 eq.) in tetrahydrofuran under ice bath, and the reaction was stirred at room temperature for three hours. The reaction was diluted with water and extracted with ethyl acetate. The organic layer was dried and concentrated under reduced pressure. The residue was purified by column chromatography on silica gel (dichloromethane/methanol = 10/1) to give a yellow solid product tert-butyl 4-(acetamidomethyl)piperidine-1-carboxylate (554.6 mg, yield: 58.03%). LCMS (ESI): m/z = 308 [M+1]⁺.

Step 29b: Preparation of N-(piperidin-4-ylmethyl)acetamide (Compound 0505-43): A mixture of tert-butyl 4-(acetamidomethyl)piperidine-1-carboxylate (0504-43) (554.6 mg, 1.8 mmol, 1.0 eq.) in hydrogen chloride-dioxane (4M, 5 ml) was stirred at room temperature for one hour. The solvent was removed under reduced pressure, the pH was adjusted to alkaline with a small amount of saturated sodium bicarbonate solution and extracted with dichloromethane and concentrated under reduced pressure. The residue was used directly in the next step without further purification. LCMS (ESI): m/z = 157 [M+1]⁺.

Step 29c: Preparation of N-((1-(4-(1-(tetrahydro-2H-pyran-2-yl)-1H-pyrazol-4-yl)phenyl)piperidin-4-yl)methy l)acetamide (Compound 0506-43): Under nitrogen protection, a mixture of N-(piperidin-4-ylmethyl)acetamide (0505-43) (200 mg, 0.78 mmol, 1.0 eq.), 4-(4-bromophenyl)-1-(tetrahydro-2H-pyran-2-yl)-1H-pyrazole (0103-1) (263 mg, 0.86 mmol, 1.1 eq.), sodium tert-butoxide (450 mg, 4.68 mmol, 6.0 eq.), 4,5-bis(diphenylphosphino)-9,9-dimethylxanthene (45 mg, 0.039 mmol, 0.1 eq.) and tris(dibenzylideneacetone)dipalladium(0) (35 mg, 0.039 mmol, 0.05 eq.) in toluene (10 ml) was stirred at 120°C overnight. The reaction was diluted with water and extracted with ethyl acetate, and the organic phase was dried and concentrated under reduced pressure. The residue was purified by prep-TLC (silica gel, dichloromethane/methanol = 20/1) to give a yellow solid product N-((1-(4-(1-(tetrahydro-2H-pyran-2-yl)-1H-pyrazol-4-yl)phenyl)piperidin-4-yl)methy l)acetamide (91 mg, yield: 36.46%). LCMS (ESI): m/z = 383 [M+1]⁺.

Step 29d: Preparation of N-((1-(4-(1H-pyrazol-4-yl)phenyl)piperidin-4-yl)methyl)acetamide (Compound 43): A mixture of N-((1-(4-(1-(tetrahydro-2H-pyran-2-yl)-1H-pyrazol-4-yl)phenyl)piperidin-4-yl)methy l)acetamide (0506-43) (91 mg, 0.238 mmol, 1.0 eq.) in hydrogen chloride-methanol (4 M, 5 mL) was stirred at room temperature for 1.5 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product N-((1-(4-(1H-pyrazol-4-yl)phenyl)piperidin-4-yl)methyl)acetamide (36.52 mg, yield: 51.49%). LCMS (ESI): m/z = 299 [M+1]⁺. ¹H NMR (500 MHz, DMSO) δ 12.74 (s, 1H), 7.87 (dd, *J* = 44.2, 38.6 Hz, 3H), 7.41 (d, *J* = 8.6 Hz, 2H), 6.91 (d, *J* = 8.7 Hz, 2H), 3.66 (d, *J* = 12.3 Hz, 2H), 2.97 (t, *J* = 6.3 Hz, 2H), 2.74 - 2.52 (m, 3H), 1.81 (s, 3H), 1.71 (d, *J* = 11.7 Hz, 2H), 1.53 (ddt, *J* = 14.5, 7.4, 3.7 Hz, 1H), 1.27 - 1.23 (m, 2H).

### Example 30: Preparation of N-((1-(4-(1H-pyrazol-4-yl)phenyl)piperidin-4-yl)methyl)-6-hydroxyhexanamide (Compound 45) (Prepared according to Scheme 5)

Step 30a: Preparation of benzyl ((1-(4-(1-(tetrahydro-2H-pyran-2-yl)-1H-pyrazol-4-yl)phenyl)piperidin-4-yl)methyl)c arbamate (Compound 0508-45)): Under nitrogen protection, a mixture of benzyl (piperidin-4-ylmethyl)carbamate (0507-45) (400 mg, 1.61 mmol, 1.0 eq.), 4-(4-bromophenyl)-1-(tetrahydro-2H-pyran-2-yl)-1H-pyrazole (0103-1) (737 mg, 2.41 mmol, 1.5 eq.), sodium tert-butoxide (459 mg, 4.83 mmol, 3.0 eq.), 2-dicyclohexylphosphino-2',6'-diisopropoxy-1,1'-biphenyl (37.5 mg, 0.08 mmol, 0.05 eq.) and tris(dibenzylideneacetone)dipalladium(0) (73.7 mg, 0.08 mmol, 0.05 eq.) in toluene (15 ml) was stirred at 120°C overnight. The reaction was diluted with water and extracted with ethyl acetate, and the organic phase was dried and concentrated under reduced pressure. The residue was purified by prep-TLC (silica gel, dichloromethane/methanol = 150/1 to 50/1) to give a yellow solid product benzyl ((1-(4-(1-(tetrahydro-2H-pyran-2-yl)-1H-pyrazol-4-yl)phenyl)piperidin-4-yl)methyl)c arbamate (55 mg, yield: 7.2%). LCMS (ESI): m/z = 475 [M+1]⁺.

Step 30b: Preparation of (1-(4-(1-(tetrahydro-2H-pyran-2-yl)-1H-pyrazol-4-yl)phenyl)piperidin-4-yl)methana mine (Compound 0509-45): Under nitrogen protection, a mixture of benzyl ((1-(4-(1-(tetrahydro-2H-pyran-2-yl)-1H-pyrazol-4-yl)phenyl)piperidin-4-yl)methyl)c arbamate (0508-45) (55 mg, 0.116 mmol, 1.0 eq.), wet palladium on carbon (6 mg, 10% by weight) in methanol (20 ml) was stirred at room temperature for 5.0 hours. The mixture was filtered and the filtrate was concentrated under reduced pressure to give a yellow oily product (1-(4-(1-(tetrahydro-2H-pyran-2-yl)-1H-pyrazol-4-yl)phenyl)piperidin-4-yl)methana mine (32 mg, yield: 82.2%). MS (ES⁺): m/z=341 (M+H)⁺.

Step 30c: Preparation of 6-hydroxy-N-((1-(4-(1-(tetrahydro-2H-pyran-2-yl)-1H-pyrazol-4-yl)phenyl)piperidin-4-yl)methyl)hexanamide (Compound 0506-45): A mixture of (1-(4-(1-(tetrahydro-2H-pyran-2-yl)-1H-pyrazol-4-yl)phenyl)piperidin-4-yl)methana mine (0509-45) (32 mg, 0.094 mmol, 1.0 eq.), 6-hydroxyhexanoic acid (0503-45) (18.6 mg, 0.14 mmol, 1.5 eq.), triethylamine (18.9 mg, 0.188 mmol, 2.0 eq.), HATU (46.4 mg, 0.122 mmol, 1.3 eq.) in N,N-dimethylformamide (5 ml) was stirred at room temperature for 6.0 hours. The reaction was diluted with water and extracted with ethyl acetate. The organic layer was washed with brine, dried over anhydrous sodium sulfate, and concentrated under reduced pressure. The residue was purified by column chromatography on silica gel (petroleum ether/ethyl acetate = 50/1 to 3/1) to give a yellow oily product 6-hydroxy-N-((1-(4-(1-(tetrahydro-2H-pyran-2-yl)-1H-pyrazol-4-yl)phenyl)piperidin-4-yl)methyl)hexanamide (15 mg, yield: 35.2%). MS (ES⁺): m/z=455 (M+H)⁺.

Step 30d: Preparation of N-((1-(4-(1H-pyrazol-4-yl)phenyl)piperidin-4-yl)methyl)-6-hydroxyhexanamide (Compound 45): A mixture of 6-hydroxy-N-((1-(4-(1-(tetrahydro-2H-pyran-2-yl)-1H-pyrazol-4-yl)phenyl)piperidin-4-yl)methyl)hexanamide (0506-45) (15 mg, 0.032 mmol, 1.0 eq.) in hydrogen chloride -methanol (4 M, 3 mL) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product N-((1-(4-(1H-pyrazol-4-yl)phenyl)piperidin-4-yl)methyl)-6-hydroxyhexanamide (7 mg, yield: 58.3%). LCMS (ESI): m/z = 371 [M+1]⁺. Melting point : 103-114 °C. ¹ H NMR (500 MHz, DMSO) δ 12.74 (s, 1H), 7.99 (s, 1H), 7.77 (s, 2H), 7.41 (d, *J* = 8.5 Hz, 2H), 6.91 (d, *J* = 8.6 Hz, 2H), 4.30 (t, *J =* 4.8 Hz, 1H), 3.66 (d, *J =* 12.3 Hz, 2H), 3.37 (dd, *J* = 11.7, 6.3 Hz, 2H), 2.97 (t, *J* = 6.1 Hz, 2H), 2.60 (t, *J =* 11.6 Hz, 2H), 2.07 (t, *J* = 7.4 Hz, 2H), 1.71 (d, *J =* 11.8 Hz, 2H), 1.48 (dt, *J =* 28.4, 10.4 Hz, 3H), 1.44 - 1.34 (m, 2H), 1.26 (dd, *J* = 15.6, 8.7 Hz, 4H).

### Example 31: Preparation of N -((1-(4-(1H - pyrazol-4-yl)phenyl)piperidin-4-yl)methyl)cyclopentanecarboxamide (Compound 46) (Prepared according to Scheme 5)

Step 31a: Preparation of tert-butyl 4-(cyclopentanecarboxamidomethyl)piperidine-1-carboxylate (Compound 0504-46): To a mixture of cyclopentanecarboxylic acid (0503-46) (548 mg, 4.8 mmol, 1.2 eq.), HATU (1.98 g, 5.2 mmol, 1.3 eq.) and triethylamine (1.01 g, 10.0 mmol, 2.5 eq.) in 10 mL N,N -dimethylformamide was added tert-butyl 4-(aminomethyl)piperidine-1-carboxylate (0501-43) (856 mg, 4.0 mmol, 1.0 eq.). The mixture was stirred at room temperature for two hours. The reaction was diluted with ethyl acetate, and the organic phase was washed with water and saturated brine. Then, the organic phase was distilled under reduced pressure, and the residue was purified by column chromatography on silica gel chromatography ( eluent: dichloromethane/methanol = 200/1 to 30/1) to obtain a yellow oily product tert-butyl 4-(cyclopentanecarboxamidomethyl)piperidine-1-carboxylate (1.63 g, crude product). MS (ES⁺): m/z =311 (M+H)⁺.

Step 31b: Preparation of N- (piperidin-4-ylmethyl)cyclopentanecarboxamide hydrochloride (Compound 0505-46): tert-Butyl 4-(cyclopentanecarboxamidomethyl)piperidine-1-carboxylate (0504-46) (500 mg, 1.61 mmol, 1.0 eq.) was added to 5 mL 4M methanol solution of hydrogen chloride. The mixture was stirred at room temperature for 2 hours. The mixture was concentrated to dryness under reduced pressure and the residue was used directly in the next step without further purification (300 mg, crude). MS (ES⁺): m/z =211 (M+H)⁺.

Step 31c: Preparation of N -((1-(4-(1-(tetrahydro- 2H -pyran-2-yl)-1H - pyrazol-4-yl)phenyl)piperidin-4-yl)methyl)cyclopentanecarboxamide (Compound 0506-46): Under nitrogen protection, a mixture of 4-(4-bromophenyl)-1-(tetrahydro-2H-pyran-2-yl)-1H-pyrazole (0103-1) (150 mg, 0.49 mmol, 1.0 eq.), N-(piperidin-4-ylmethyl)cyclopentanecarboxamide hydrochloride (0505-46) (242 mg, 0.98 mmol, 2.0 eq.), sodium tert-butoxide (283 mg, 2.94 mmol, 6.0 eq.), 4,5-bis(diphenylphosphino)-9,9-dimethylxanthene (58 mg, 0.1 mmol, 0.2 eq.) and tris(dibenzylideneacetone)dipalladium(0) (46 mg, 0.05 mmol, 0.1 eq.) in toluene (40 ml) was stirred at 120°C overnight. After cooling to room temperature, the reaction was filtered through celite and the filter cake was washed with ethyl acetate. The filtrate was concentrated under reduced pressure, and the residue was purified by prep-TLC (silica gel, eluent: dichloromethane/methanol = 15/1) to give a yellow solid product N-((1-(4-(1-(tetrahydro-2H-pyran-2-yl)-1H-pyrazol-4-yl)phenyl)piperidin-4-yl)methy l)cyclopentanecarboxamide (51 mg, yield: 23.8%). MS (ES⁺): m/z = 437 (M+H)⁺.

Step 31d: Preparation of N-((1-(4-(1H-pyrazol-4-yl)phenyl)piperidin-4-yl)methyl)cyclopentanecarboxamide (Compound 46): N -((1-(4-(1-(tetrahydro- 2H -pyran-2-yl)-1H - pyrazol-4-yl)phenyl)piperidin-4-yl)methyl)cyclopentanecarboxamide (0506-46) (51 mg, 0.117 mmol, 1.0 eq.) was added to 10 mL 4M hydrogen chloride - methanol solution and stirred at room temperature for 2 hours. The mixture was concentrated to dryness under reduced pressure. Water was added and the pH was adjusted to 10 with saturated sodium carbonate aqueous solution. Ethyl acetate was added for extraction and washed with water. The organic phase was dried over anhydrous sodium sulfate and concentrated under reduced pressure. The residue was purified by prep-TLC (silica gel, eluent: dichloromethane /methanol = 12/1) to obtain a yellow solid N-((1-(4-(1H-pyrazol-4-yl)phenyl)piperidin-4-yl)methyl)cyclopentanecarboxamide (23 mg, yield: 54.7%). MS (ES⁺): m/z =353 (M+H)⁺. Melting point: 215-217 ° C. ¹ H NMR (500 MHz, DMSO) δ 12.73 (s, 1H), 7.86 (d, *J* = 7.5 Hz, 2H), 7.74 (t, *J* = 5.5 Hz, 1H), 7.41 (d, *J* = 8.4 Hz, 2H), 6.91 (d, *J* = 8.3 Hz, 2H), 3.66 (d, *J* = 12.2 Hz, 2H), 2.98 (t, *J =* 6.2 Hz, 2H), 2.68 - 2.52 (m, 3H), 1.71 (dd, *J =* 16.3, 6.9 Hz, 4H), 1.65 - 1.41 (m, 8H), 1.25 (s, 1H).

### Example 32: Preparation of N-((1-(4-(1H-pyrazol-4-yl)phenyl)piperidin-4-yl)methyl)benzamide (Compound 47) (Prepared according to Scheme 5)

Step 32a: Preparation of tert-butyl 4-(benzamidomethyl)piperidine-1 -carboxylate (Compound 0504-47): Under nitrogen protection, a mixture of benzoic acid (300 mg, 2.46 mmol, 1.0 eq.), 1-(3-dimethylaminopropyl)-3-ethylcarbodiimide hydrochloride (0501-43) (707 mg, 3.69 mmol, 1.5 eq.), 1-hydroxybenzotriazole (498 mg, 3.69 mmol, 1.5 eq.), N,N-diisopropylethylamine (952 mg, 7.38 mmol, 3.0 eq.) in N,N-dimethylformamide (8 ml) was stirred at room temperature for 15 minutes. Then, a solution of tert-butyl 4-(aminomethyl)piperidine-1-carboxylate (631 mg, 2.95 mmol, 1.2 eq.) in N,N-dimethylformamide (8 ml) was added to the reaction solution, and the mixture was stirred at room temperature for 3.0 hours. The reaction was diluted with water and extracted with ethyl acetate. The organic layer was dried and concentrated under reduced pressure. The residue was purified by column chromatography on silica gel (ethyl acetate) to obtain a yellow solid product tert-butyl 4-(benzamidomethyl)piperidine-1-carboxylate (690 mg, yield: 81.4%).

Step 32b: Preparation of N-(piperidin-4-ylmethyl)benzamide (Compound 0505-47): A mixture of tert-butyl 4-(benzamidomethyl)piperidine-1-carboxylate (0504-47) (510 mg, 0.675 mmol, 1.0 eq.) in hydrochloric acid-dioxane (4 M, 6 mL) was stirred at room temperature for 1.0 h. The solvent was removed under reduced pressure, and the residue was diluted with water, adjusted to pH = 9 with sodium carbonate solution, and extracted with dichloromethane. The organic phase was dried and concentrated under reduced pressure. The residue was used directly in the next step without further purification. LCMS (ESI): m/z = 219 [M+1]⁺.

Step 32c: Preparation of N-((1-(4-(1-(tetrahydro-2H-pyran-2-yl)-1H-pyrazol-4-yl)phenyl)piperidin-4-yl)methy l)benzamide (Compound 0506-47): Under nitrogen protection, a mixture of N-(piperidin-4-ylmethyl)benzamide (0505-47) (250 mg, 1.15 mmol, 1.0 eq.), 4-(4-bromophenyl)-1-(tetrahydro-2H-pyran-2-yl)-1H-pyrazole (0103-1) (352 mg, 1.15 mmol, 1.0 eq.), sodium tert-butoxide (275 mg, 2.86 mmol, 2.5 eq.), 4,5-bis(diphenylphosphino-9,9-dimethylxanthene) (66.5 mg, 0.115 mmol, 0.1 eq.) and tris(dibenzylideneacetone)dipalladium(0) were reacted. (52 mg, 0.057 mmol, 0.05 eq.) in toluene (15 ml) was stirred at 120°C overnight. The reaction was diluted with water and extracted with ethyl acetate, and the organic phase was dried and concentrated under reduced pressure. The residue was purified by prep-TLC (silica gel, petroleum ether/ethyl acetate = 4/1 to 1/2) to obtain a yellow solid product N-((1-(4-(1-(tetrahydro-2H-pyran-2-yl)-1H-pyrazol-4-yl)phenyl)piperidin-4-yl)methy l)benzamide (120 mg, yield: 23.57%). LCMS (ESI): m/z = 445 [M+1]⁺.

Step 32d: Preparation of N-((1-(4-(1H-pyrazol-4-yl)phenyl)piperidin-4-yl)methyl)benzamide (Compound 47): A mixture of N-((1-(4-(1-(tetrahydro-2H-pyran-2-yl)-1H-pyrazol-4-yl)phenyl)piperidin-4-yl)methy l)benzamide (0506-47) (120 mg, 0.270 mmol, 1.0 eq.) in hydrogen chloride-dioxane (4M, 5 ml) was stirred at room temperature for 1.5 hours. The solvent was removed under reduced pressure, the residue was diluted with water, adjusted to pH = 9 with sodium carbonate solution, extracted with dichloromethane, and the organic phase was dried and concentrated under reduced pressure. The residue was purified by prep-TLC (silica gel, dichloromethane/methanol = 12/1) to give a pink solid product N-((1-(4-(1H-pyrazol-4-yl)phenyl)piperidin-4-yl)methyl)benzamide (40 mg, yield: 33.61%). LCMS (ESI): m/z = 361 [M+1]⁺.

### Example 33: Preparation of 1-(2-(1-(4-(1H-pyrazol-4-yl)phenyl)piperidin-4-yl)ethyl)pyrrolidin-2-one (Compound 48) (Prepared according to Scheme 1)

Step 33 a: Preparation of tert-butyl 4-(2-((methylsulfonyl)oxy)ethyl)piperidine-1-carboxylate (Compound 0105-48): To a mixture of tert- butyl 4-(2-hydroxyethyl)piperidine-1-carboxylate (0104-48) (300 mg, 1.31 mmol, 1.0 eq.), triethylamine (595 mg, 5.89 mmol, 4.5 eq.) in ethdichloromethane (8 ml) was added a solution of methanesulfonyl chloride (275 mg, 3.27 mmol, 2.5 eq.) in dichloromethane (2 ml) at 0 °C under nitrogen. The mixture was stirred at room temperature for 3.0 hours. The reaction was diluted with water and extracted with dichloromethane. The organic layer was washed with brine and concentrated under reduced pressure. The residue was purified by column chromatography on silica gel (petroleum ether/ethyl acetate = 1/1) to give a white solid product tert-butyl 4-(2-((methylsulfonyl)oxy)ethyl)piperidine-1-carboxylate (400 mg, yield: 99.26%). LCMS (ESI): m/z = 308 [M+1]⁺.

Step 33b: Preparation of tert -butyl 4-(2-(2-oxopyrrolidin-1-yl)ethyl)piperidine-1-carboxylate (Compound 0107-48): To a mixture of pyrrolidone (0106-1) (123 mg, 1.44 mmol, 1.2 eq.) in N,N-dimethylformamide (8 mL) was added sodium hydride (77 mg, 1.92 mmol, 1.6 eq.) at 0 °C under nitrogen. The mixture was stirred at room temperature for 30 minutes. Then, a solution of tert-butyl 4-(2-((methylsulfonyl)oxy)ethyl)piperidine-1-carboxylate (0105-48) (370 mg, 1.20 mmol, 1.0 eq.) in N,N-dimethylformamide (2 mL) was added to the mixture. The mixture was stirred at 65 °C for 3 hours. The reaction was diluted with water and extracted with ethyl acetate. The organic layer was washed with brine and concentrated under reduced pressure. The residue was purified by column chromatography on silica gel (dichloromethane/methanol = 10/1) to give tert-butyl 4-(2-(2-oxopyrrolidin-1-yl)ethyl)piperidine-1-carboxylate (320 mg, yield: 90.14%) as a white oil. LCMS (ESI): m/z = 297 [M+1]⁺.

Step 33c: Preparation of 1-(2-(piperidin-4-yl)ethyl)pyrrolidin-2-one hydrochloride (Compound 0108-48): A mixture of tert-butyl 4-(2-(2-oxopyrrolidin-1-yl)ethyl)piperidine-1-carboxylate (0107-48) (320 mg, 1.08 mmol, 1.0 eq.) in hydrogen chloride -methanol solution (4M, 4 mL) was stirred at room temperature for 2.0 hours. The solvent was removed under reduced pressure to give 1-(2-(piperidin-4-yl)ethyl)pyrrolidin-2-one hydrochloride (270 mg, crude) as a white solid product. LCMS (ESI): m/z = 197 [M+1]⁺.

Step 33d: Preparation of 1-(2-(1-(4-(1-(tetrahydro-2H-pyran-2-yl)-1H-pyrazol-4-yl)phenyl)piperidin-4-yl)ethyl) pyrrolidin-2-one (Compound 0109-48): Under nitrogen protection, a mixture of 1-(2-(piperidin-4-yl)ethyl)pyrrolidin-2-one hydrochloride (0108-48) (100 mg, 0.43 mmol, 1.0 eq.), 4-(4-bromophenyl)-1-(tetrahydro-2H-pyran-2-yl)-1H-pyrazole (133 mg, 0.43 mmol, 1.0 eq.), sodium tert-butoxide (166 mg, 1.72 mmol, 4.0 eq.), 4,5-bis(diphenylphosphino)-9,9-dimethylxanthene (25 mg, 0.043 mmol, 0.1 eq.) and tris(dibenzylideneacetone)dipalladium(0) (20 mg, 0.022 mmol, 0.05 eq.) in toluene (8 ml) was stirred at 120°C overnight. The reaction was diluted with water and extracted with ethyl acetate, and the organic phase was dried and concentrated under reduced pressure. The residue was purified by prep-TLC (silica gel, dichloromethane/methanol = 10/1) to give a yellow solid product 1-(2-(1-(4-(1-(tetrahydro-2H-pyran-2-yl)-1H-pyrazol-4-yl)phenyl)piperidin-4-yl)ethyl) pyrrolidin-2-one (45 mg, yield: 24.86%). LCMS (ESI): m/z = 423 [M+1]⁺.

Step 33e: Preparation of 1-(2-(1-(4-(1H-pyrazol-4-yl)phenyl)piperidin-4-yl)ethyl)pyrrolidin-2-one (Compound 48): A mixture of 1-( 2-(1-(4-(1-(tetrahydro-2H-pyran-2-yl)-1H-pyrazol-4-yl)phenyl)piperidin-4-yl)ethy l)pyrrolidin-2-one (0109-48) (45 mg, 0.107 mmol, 1.0 eq.) in hydrogen chloride-methanol solution (4 M, 2 mL) was stirred at room temperature for 3.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product 1-(2-(1-(4-(1H-pyrazol-4-yl)phenyl)piperidin-4-yl)ethyl)pyrrolidin-2-one (25 mg, yield: 69.44%). LCMS(ESI): m/z =339[M+1]⁺. ¹H NMR (500 MHz, DMSO) δ 12.75 (s, 1H), 7.89 (d, *J =* 94.1 Hz, 2H), 7.41 (d, *J =* 8.7 Hz, 2H), 6.90 (d, *J =* 8.8 Hz, 2H), 3.64 (d, *J =* 12.4 Hz, 2H), 3.32 (t, *J =* 7.0 Hz, 2H), 3.23 (t, *J =* 7.2 Hz, 2H), 2.66 - 2.53 (m, 2H), 2.20 (t, *J =* 8.1 Hz, 2H), 1.97 - 1.82 (m, 2H), 1.77 (d, *J =* 11.2 Hz, 2H), 1.48 - 1.37 (m, 2H), 1.37 - 1.29 (m, 1H), 1.29 - 1.23 (m, 2H).

### Example 34: Preparation of (1-(4-(1H-pyrazol-4-yl)phenyl)piperidin-4-yl)(pyrrolidin-1-yl)methanone (Compound 49):

A mixture of pyrrolidin-1-yl(1-(4-(1-(tetrahydro-2H-pyran-2-yl)-1H-pyrazol-4-yl)phenyl)piperidin-4-yl)methanone (0304-40) (25 mg, 0.061 mmol, 1.0 eq.) in a hydrogen chloride-methanol solution (4M, 2 ml) was stirred at room temperature for 1.5 hours. The mixture was diluted with water (20 ml). Solid sodium carbonate was added to adjust pH = 10, and then the aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, ethyl acetate: dichloromethane: methanol = 10: 10: 2) to give a white solid product (1-(4-(1H-pyrazol-4-yl)phenyl)piperidin-4-yl)(pyrrolidin-1-yl)methanone (15 mg, yield: 76%). LCMS (ESI): m/z 325 [M+1]⁺; TLC: Rf 0.5 (dichloromethane:methanol=10:1). ¹ H NMR (500 MHz, MeOD) δ 7.83 (s, 2H), 7.44 (d, *J =* 8.7 Hz, 2H), 6.99 (d, *J =* 8.7 Hz, 2H), 3.74 (d, *J =* 12.4 Hz, 2H), 3.60 (t, *J =* 6.8 Hz, 2H), 3.42 (t, *J =* 6.9 Hz, 2H), 2.76 (td, *J =* 12.1, 3.2 Hz, 2H), 2.70 - 2.61 (m, 1H), 2.00 (p, *J* = 6.7 Hz, 2H), 1.94 - 1.78 (m, 6H).

### Example 35: Preparation of (1-(4-(1H-pyrazol-4-yl)phenyl)piperidin-4-yl)(morpholinyl)methanone (Compound 50) (Prepared according to Scheme 3)

Step 35a: Preparation of tert-butyl 4-(morpholine-4-carbonyl)piperidine-1-carboxylate (Compound 0302-50): A mixture of morpholine (228 mg, 2.62 mmol, 1.2 eq.), 1-(tert-butoxycarbonyl)piperidine-4-carboxylic acid (0301-40) (500 mg, 2.18 mmol, 1.0 eq.), triethylamine (550 mg, 5.45 mmol, 2.5 eq.), 1-hydroxybenzotriazole (412 mg, 3.05 mmol, 1.4 eq.) and 1-(3-dimethylaminopropyl)-3-ethylcarbodiimide hydrochloride (586 mg, 3.05 mmol, 1.4 eq.) in N,N-dimethylformamide (15 ml) was stirred at room temperature for 5 hours. Water and ethyl acetate were added for extraction, and the organic layer was washed with brine and concentrated under reduced pressure to obtain a transparent oily product tert-butyl 4-(morpholine-4-carbonyl)piperidine-1-carboxylate (620 mg, yield: 95.3%). MS (ES⁺): m/z=299 (M+H)⁺.

Step 35b: Preparation of morpholine (piperidin-4-yl) ketone (Compound 0303-50): A mixture of tert-butyl 4-(morpholine-4-carbonyl) piperidine-1-carboxylate (600 mg, 2.01 mmol, 1.0 eq.) in hydrogen chloride-methanol (4M, 10 ml) was stirred at room temperature for 3.0 hours. The solvent was removed under reduced pressure, the residue was diluted with water, the pH was adjusted to 9 with sodium carbonate solution, and extracted with dichloromethane, the organic phase was dried and concentrated under reduced pressure. The residue was purified by prep-TLC (silica gel, dichloromethane/methanol = 40/1) to give a yellow solid product morpholine (piperidin-4-yl) ketone (395 mg, yield: 99.2%). LCMS (ESI): m/z =199[M+1]⁺.

Step 35c: Preparation of morpholine (1- (4- (1- (tetrahydro-2H-pyran-2-yl) -1H-pyrazol-4-yl) phenyl) piperidin-4-yl)methanone (Compound 0304-50): Under nitrogen protection, a mixture of morpholine (piperidin-4-yl)methanone (0303-50) (200 mg, 1.01 mmol, 1.0 eq.), 4-(4-bromophenyl)-1-(tetrahydro-2H-pyran-2-yl)-1H-pyrazole (0103-1) (401 mg, 1.31 mmol, 1.3 eq.), sodium tert-butoxide (270 mg, 3.03 mmol, 3.0 eq.), 4,5-bis(diphenylphosphino)-9,9-dimethylxanthene (58.1 mg, 0.1 mmol, 0.1 eq.) and tris(dibenzylideneacetone)dipalladium(0) (91.6 mg, 0.1 mmol, 0.1 eq.) in toluene (15 ml) was stirred at 120°C overnight. The reaction was diluted with water and extracted with ethyl acetate, and the organic phase was dried and concentrated under reduced pressure. The residue was purified by prep-TLC (silica gel, dichloromethane/methanol = 120/1 to 40/1) to give a yellow solid product morpholine (1-(4-(1-(tetrahydro-2H-pyran-2-yl)-1H-pyrazol-4-yl)phenyl)piperidin-4-yl)methanon e (65 mg, yield: 15.1%). LCMS (ESI): m/z = 425 [M+1]⁺.

Step 35d: Preparation of (1-(4-(1H-pyrazol-4-yl)phenyl)piperidin-4-yl)(morpholinyl)methanone (Compound 50): A mixture of morpholinyl (1-(4-(1-(tetrahydro-2H-pyran-2-yl)-1H-pyrazol-4-yl)phenyl)piperidin-4-yl)methanon e (50 mg, 0.117 mmol, 1.0 eq.) in hydrogen chloride-dioxane (4 M, 5 ml) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product (1-(4-(1H-pyrazol-4-yl)phenyl)piperidin-4-yl)(morpholinyl)methanone (15 mg, yield: 37.5%). LCMS (ESI): m/z = 341 [M+1]⁺. Melting point: 185-192 ° C. ¹ H NMR (500 MHz, DMSO) δ 12.74 (s, 1H), 7.89 (d, *J =* 105.6 Hz, 2H), 7.55 - 7.28 (m, 2H), 7.03 - 6.78 (m, 2H), 3.82 - 3.42 (m, 10H), 2.83 - 2.64 (m, 3H), 1.78 - 1.58 (m, 4H).

### Example 36: Preparation of (1-(4-(1H-pyrazol-4-yl)phenyl)piperidin-4-yl)(piperazin-1-yl)methanone (Compound 51) (Prepared according to Scheme 3)

Step 36a: Preparation of benzyl 4-(1-(tert-butoxycarbonyl)piperidine-4-carbonyl)piperazine-1-carboxylate (Compound 0302-51): To a mixture of 1-(tert-butoxycarbonyl)piperidine-4-carboxylic acid (0301-40) (350 mg, 1.53 mmol, 1.0 eq.), 1-ethyl-(3-dimethylaminopropyl)carbodiimide hydrochloride (380 mg, 1.98 mmol, 1.3 eq.), 1-hydroxybenzotriazole (267 mg, 1.98 mmol, 1.3 eq.) and N,N-diisopropylethylamine (592 mg, 4.59 mmol, 3.0 eq.) in dichloromethane (10 ml) was added benzyl piperazine-1-carboxylate (403 mg, 1.83 mmol, 1.2 eq.). The mixture was stirred at room temperature overnight. The solvent was removed under reduced pressure. The residue was separated by column chromatography on silica gel (petroleum ether: ethyl acetate 8: 1) to give benzyl 4-(1-(tert-butyloxycarbonyl)piperidine-4-carbonyl)piperazine-1-carboxylate (615 mg, yield: 93%) as a pale yellow oil. LCMS (ESI): m/z 432 [M+1]⁺; TLC: Rf 0.5 (petroleum ether: ethyl acetate = 5: 1).

Step 36b: Preparation of benzyl 4-(piperidine-4-carbonyl)piperazine-1-carboxylate (Compound 0303-51): A mixture of benzyl 4-(1-(tert-butyloxycarbonyl)piperidine-4-carbonyl)piperazine-1-carboxylate (0302-51) (615 mg, 1.43 mmol, 1.0 eq.) in a solution of hydrogen chloride - dioxane (4M, 5 ml) was stirred at room temperature for 1.5 hours. The mixture was diluted with water (20 ml). Solid sodium carbonate was added to adjust pH = 10, and then the aqueous layer was extracted with dichloromethane (20 ml × 6). The combined organic layer was washed with saturated brine (20 ml × 1), dried over anhydrous sodium sulfate and concentrated to give benzyl 4-(piperidine-4-carbonyl)piperazine-1-carboxylate (0.47 g, yield: 100%) as a white solid. LCMS (ESI): m/z 332 [M+1]⁺; TLC: Rf 0.3 (dichloromethane:methanol=10:1).

Step 36c: Preparation of benzyl 4-(1-(4-(1-(tetrahydro-2H-pyran-2-yl)-1H-pyrazol-4-yl)phenyl)piperidine-4-carbonyl) piperazine-1-carboxylate (Compound 0304-51): Benzyl 4-(piperidine-4-carbonyl)piperazine-1-carboxylate (0303-51) (129 mg, 0.39 mmol, 1.2 eq.) was added to a mixture of 4-(4-bromophenyl)-1-(tetrahydro-2H-pyran-2-yl)-1H-pyrazole (0103-1) (100 mg, 0.33 mmol, 1.0 eq.), tris(dibenzylideneacetone)dipalladium(0) (14.7 mg, 0.016 mmol, 0.05 eq.), 4,5-bis(diphenylphosphino)-9,9-dimethylxanthene (18.8 mg, 0.033 mmol, 0.1 eq.) and cesium carbonate (266 mg, 0.82 mmol, 2.5 eq.) in toluene (8 ml). The mixture was heated to 120°C for 9 hours under nitrogen atmosphere. The solvent was removed under reduced pressure. The residue was purified by Pre-TLC (ethyl acetate:methanol=60:1) to give benzyl 4-(1-(4-(1-(tetrahydro-2H-pyran-2-yl)-1H-pyrazol-4-yl)phenyl)piperidine-4-carbonyl) piperazine-1-carboxylate (58 mg, yield: 32%) as a pale yellow solid. LCMS (ESI): m/z 558 [M+1]⁺; TLC: Rf 0.5 (ethyl acetate: methanol = 60: 1).

Step 36d: Preparation of (1-(4-(1H-pyrazol-4-yl)phenyl)piperidin-4-yl)(piperazin-1-yl)methanone (Compound 51): A mixture of benzyl 4-(1-(4-(1-(tetrahydro-2H-pyran-2-yl)-1H-pyrazol-4-yl)phenyl)piperidin-4-carbonyl)p iperazine-1- carboxylate ( 0304-51) (56 mg, 0.10 mmol, 1.0 eq.) in hydrogen bromide acetic acid solution (33% solution, 2 ml) was stirred at room temperature for 1 hour. The mixture was diluted with water (15 ml), solid sodium carbonate was added to adjust pH = 10, and then the aqueous layer was extracted with dichloromethane (15 ml × 4). The combined organic layer was washed with saturated brine (20 ml × 1), dried over anhydrous sodium sulfate and concentrated. The residue was purified by Pre-TLC (silica gel, dichloromethane: methanol: aqueous ammonia = 60: 10: 1) to give (1-(4-(1H-pyrazol-4-yl)phenyl)piperidin-4-yl)(piperazin-1-yl)methanone (18 mg, yield: 53%) as a white solid. LCMS (ESI): m/z 340 [M+1]⁺; TLC: Rf 0.3 (dichloromethane: methanol = 10: 1). ¹ H NMR (500 MHz, MeOD) δ 7.84 (s, 2H), 7.45 (d, *J =* 8.6 Hz, 2H), 7.01 (d, *J =* 8.6 Hz, 2H), 3.88 (d, *J =* 23.5 Hz, 4H), 3.73 (d, *J* = 12.4 Hz, 2H), 3.29 - 3.18 (m, 4H), 2.82 (ddd, *J =* 23.5, 11.1, 3.7 Hz, 3H), 1.94 - 1.78 (m, 4H).

### Example 37: Preparation of (1-(4-(1H-pyrazol-4-yl)phenyl)piperidin-4-yl)(piperidin-1-yl)methanone (Compound 54) (Prepared according to Scheme 3)

Step 37a: Preparation of tert-butyl 4-(piperidine-1-carbonyl)piperidine-1-carboxylate (Compound 0302-54): Under nitrogen protection, a mixture of 1-(tert-butyloxycarbonyl)piperidine-4-carboxylic acid (0301-40) (200 mg, 0.87 mmol, 1.0 eq.), piperidine hydrochloride (127 mg, 1.05 mmol, 1.2 eq.), 1-(3-dimethylaminopropyl)-3-ethylcarbodiimide hydrochloride (250 mg, 1.30 mmol, 1.5 eq.), 1-hydroxybenzotriazole (176 mg, 1.30 mmol, 1.5 eq.), N,N-diisopropylethylamine (562 mg, 4.36 mmol, 5.0 eq.) in N,N-dimethylformamide (10 ml) was stirred at room temperature for 4.0 hours. The reaction was diluted with water and extracted with ethyl acetate. The organic layer was dried and concentrated under reduced pressure. The residue was purified by column chromatography on silica gel (ethyl acetate) to give tert-butyl 4-(piperidine-1-carbonyl)piperidine-1-carboxylate (210 mg, yield: 81.4%) as a white solid.

Step 37b: Preparation of piperidin-1-yl(piperidin-4-yl)methanone hydrochloride (Compound 0303-54): A mixture of tert-butyl 4-(piperidine-1-carbonyl)piperidine-1-carboxylate (0302-54) (200 mg, 0.228 mmol, 1.0 eq.) in hydrogen chloride-dioxane (4 M, 8 mL) was stirred at room temperature for 1.0 h. The solvent was removed under reduced pressure and the residue was used directly in the next step without further purification. LCMS (ESI): m/z = 197 [M+1]⁺.

Step 37c: Preparation of piperidin-1-yl (1- (4- (1- (tetrahydro-2H-pyran-2-yl) -1H-pyrazol-4-yl) phenyl) piperidin-4-yl)methanone (Compound 0304-54): Under nitrogen protection, a mixture of piperidin-1-yl(piperidin-4-yl)methanone hydrochloride (0303-54) (150 mg, 0.44 mmol, 1.0 eq.), 4-(4-bromophenyl)-1-(tetrahydro-2H-pyran-2-yl)-1H-pyrazole (178 mg, 0.580 mmol, 0.9 eq.), sodium tert-butoxide (248 mg, 2.58 mmol, 4.0 eq.), 4,5-bis(diphenylphosphino)-9,9-dimethylxanthene (37 mg, 0.064 mmol, 0.1 eq.) and tris(dibenzylideneacetone)dipalladium(0) (29.5 mg, 0.032 mmol, 0.05 eq.) in toluene (12 ml) was stirred at 120°C overnight. The reaction was diluted with water and extracted with ethyl acetate, and the organic phase was dried and concentrated under reduced pressure. The residue was purified by prep-TLC (silica gel, petroleum ether/ethyl acetate = 4/1 to 1/2) to give a yellow solid product piperidin-1-yl(1-(4-(1-(tetrahydro-2H-pyran-2-yl)-1H-pyrazol-4-yl)phenyl)piperidin-4 -yl)methanone (70 mg, yield: 25.73%). LCMS (ESI): m/z =423[M+1]⁺.

Step 37d: Preparation of (1-(4-(1H-pyrazol-4-yl)phenyl)piperidin-4-yl)(piperidin-1-yl)methanone (Compound 54): A mixture of piperidin-1-yl(1-(4-(1-(tetrahydro-2H-pyran-2-yl)-1H-pyrazol-4-yl)phenyl)piperidin-4 -yl)methanone (0304-54) (50 mg, 0.118 mmol, 1.0 eq.) in hydrogen chloride-dioxane (4M, 5 ml) was stirred at room temperature for 1.5 hours. The solvent was removed under reduced pressure, the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 12/1) to give a yellow solid product (1-(4-(1H-pyrazol-4-yl)phenyl)piperidin-4-yl)(piperidin-1-yl)methanone (30 mg, yield: 75.22%). LCMS (ESI): m/z = 339 [M+1]⁺. ¹H NMR (500 MHz, DMSO) δ 12.75 (s, 1H), 7.89 (d, *J* = 109.1 Hz, 2H), 7.42 (d, *J =* 8.7 Hz, 2H), 6.91 (d, *J =* 8.8 Hz, 2H), 3.69 (d, *J =* 12.3 Hz, 2H), 3.47 (t, *J =* 20.6 Hz, 4H), 2.75 (ddt, *J =* 15.1, 12.3, 7.7 Hz, 3H), 1.72 - 1.63 (m, 4H), 1.59 (dt, *J =* 11.2, 5.6 Hz, 2H), 1.51 (s, 2H), 1.42 (s, 2H).

### Example 38: Preparation of 1-(4-(1H-pyrazol-4-yl)phenyl)-N-cyclopentylpiperidine-4-carboxamide (Compound 55) (Prepared according to Scheme 8)

Step 38a: Preparation of 1-(4-(1-(tetrahydro-2H-pyran-2-yl)-1H-pyrazol-4-yl)phenyl)piperidine-4-carboxylic acid (Compound 0801-55): To a mixture of methyl 1-(4-(1-(tetrahydro-2H-pyran-2-yl)-1H-pyrazol-4-yl)phenyl)piperidine-4-carboxylate (0702-28) (100 mg, 0.27 mmol, 1.0 eq.) in tetrahydrofuran (5 ml) and water (2 ml) was added sodium hydroxide (33 mg, 0.81 mmol, 3.0 eq.). The mixture was stirred at room temperature for 3 hours. The mixture was diluted with water (15 ml). 1N hydrochloric acid was added to adjust pH=4, and then the aqueous layer was extracted with dichloromethane (15 ml×4). The combined organic layer was washed with saturated brine (15 ml×1), dried over anhydrous sodium sulfate and concentrated to give 1-(4-(1-(tetrahydro-2H-pyran-2-yl)-1H-pyrazol-4-yl)phenyl)piperidine-4-carboxylic acid (90 mg, yield: 94%) as a white solid. LCMS (ESI): m/z 356 [M+1]⁺; TLC: Rf 0.4 (dichloromethane: ethyl methanol = 10:1).

Step 38b: Preparation of N-cyclopentyl-1-(4-(1-(tetrahydro-2H-pyran-2-yl)-1H-pyrazol-4-yl)phenyl)piperidine -4-carboxamide (Compound 0802-55): To a mixture of 1-(4-(1-(tetrahydro-2H-pyran-2-yl)-1H-pyrazol-4-yl)phenyl)piperidine-4-carboxylic acid (0801-55) (45 mg, 0.13 mmol, 1.0 eq.), 1-ethyl-(3-dimethylaminopropyl)carbodiimide hydrochloride (32 mg, 0.17 mmol, 1.3 eq.), 1-hydroxybenzotriazole (22 mg, 0.17 mmol, 1.3 eq.) and N,N-diisopropylethylamine (33 mg, 0.25 mmol, 2.0 eq.) in dichloromethane (5 ml) was added cyclopentylamine (14 mg, 0.17 mmol, 1.3 eq.). The mixture was stirred at room temperature overnight. The solvent was removed under reduced pressure. The residue was purified by Pre-TLC (ethyl acetate: dichloromethane: methanol = 15: 15: 1) to give white solid product N-cyclopentyl-1-(4-(1-(tetrahydro-2H-pyran-2-yl)-1H-pyrazol -4-yl)phenyl)piperidine -4-carboxamide (37 mg, yield: 69%). LCMS (ESI): m/z 423 [M+1]⁺; TLC: Rf 0.5 (dichloromethane: methanol = 20:1).

Step 38c: Preparation of 1-(4-(1H-pyrazol-4-yl)phenyl)-N-cyclopentylpiperidine-4-carboxamide (Compound 55): A mixture of N-cyclopentyl-1-(4-(1-(tetrahydro-2H-pyran-2-yl)-1H-pyrazol -4-yl)phenyl)piperidine -4-carboxamide (0802-55) (37 mg, 0.088 mmol, 1.0 eq.) in a solution of hydrogen chloride-methanol (4M, 3 mL) was stirred at room temperature for 2 hours. The mixture was diluted with water (20 mL) and solid sodium carbonate was added to adjust the pH to 10. The mixture was filtered. The solid was washed with water and then dried under vacuum to give 1-(4-(1H-pyrazol-4-yl)phenyl)-N-cyclopentylpiperidine-4-carboxamide (20 mg, yield: 67%) as a white solid. LCMS (ESI): m/z 339 [M+1]⁺; TLC: Rf 0.5 (dichloromethane:methanol=10:1). ¹ H NMR (500 MHz, DMSO) δ 12.72 (s, 1H), 7.89 (s, 2H), 7.70 (d, *J =* 5.9 Hz, 1H), 7.41 (d, *J =* 7.8 Hz, 2H), 6.91 (d, *J =* 7.7 Hz, 2H), 3.98 (d, *J =* 6.4 Hz, 1H), 3.69 (d, *J =* 11.2 Hz, 2H), 2.63 (t, *J* = 10.9 Hz, 2H), 2.23 (s, 1H), 1.70 (dd, *J=* 40.1, 24.5 Hz, 8H), 1.49 (s, 2H), 1.35 (d, *J* = 6.1 Hz, 2H).

### Example 39: Preparation of 1-(4-(1H-pyrazol-4-yl)phenyl)-N-phenylpiperidine-4-carboxamide (Compound 58) (Prepared according to Scheme 3)

Step 39a: Preparation of tert-butyl 4-(phenylcarbamoyl)piperidine-1-carboxylate (Compound 0306-58): Under nitrogen protection, a mixture of 1-(tert-butyloxycarbonyl)piperidine-4-carboxylic acid (0301-40) (500 mg, 2.18 mmol, 1.0 eq.), 1-(3-dimethylaminopropyl)-3-ethylcarbodiimide hydrochloride (628 mg, 3.28 mmol, 1.5 eq.), 1-hydroxybenzotriazole (442 mg, 3.28 mmol, 1.5 eq.), N,N-diisopropylethylamine (845 mg, 6.55 mmol, 3.0 eq.) in N,N-dimethylformamide (15 ml) was stirred at room temperature for 15 minutes. Aniline (203 mg, 2.18 mmol, 1.0 eq.) was then added to the mixture. The mixture was stirred at room temperature for 2 hours. The reaction was diluted with water and extracted with ethyl acetate. The organic layer was washed with brine and concentrated under reduced pressure. The residue was purified by column chromatography on silica gel (petroleum ether/ethyl acetate = 1/1) to obtain a yellow solid product tert-butyl 4-(phenylcarbamoyl)piperidine-1-carboxylate r (395 mg, yield: 59.31%). LCMS (ESI): m/z = 305 [M+1]⁺.

Step 39b: Preparation of N-phenylpiperidine-4-carboxamide hydrochloride (Compound 0307-58): A mixture of tert-butyl 4-(phenylcarbamoyl)piperidine-1-carboxylate (0306-58) (370 mg, 1.21 mmol, 1.0 eq.) in hydrogen chloride-methanol solution (4M, 8 ml) was stirred at room temperature for 1.0 h. The solvent was removed under reduced pressure and the residue was used directly in the next step without further purification. LCMS (ESI): m/z = 205 [M+1]⁺.

Step 39c: Preparation of N-phenyl-1-(4-(1-(tetrahydro-2H-pyran-2-yl)-1H-pyrazol-4-yl)phenyl)piperidine-4-ca rboxamide (Compound 0307-58): Under nitrogen protection, a mixture of N-phenylpiperidine-4-carboxamide hydrochloride (0306-58) (200 mg, 0.98 mmol, 1.0 eq.), 4-(4-bromophenyl)-1-(tetrahydro-2H-pyran-2-yl)-1H-pyrazole (0103-1) (300 mg, 0.98 mmol, 1.0 eq.), sodium tert-butoxide (282 mg, 2.93 mmol, 3.0 eq.), 4,5-bis(diphenylphosphino)-9,9-dimethylxanthene (57 mg, 0.098 mmol, 0.1 eq.) and tris(dibenzylideneacetone)dipalladium(0) (45 mg, 0.049 mmol, 0.05 eq.) in toluene (10 ml) was stirred at 120°C overnight. The reaction was diluted with water and extracted with ethyl acetate, and the organic phase was dried and concentrated under reduced pressure. The residue was purified by column chromatography on silica gel (petroleum ether/ethyl acetate = 4/1 to 1/1) to give a yellow solid product N-phenyl-1-(4-(1-(tetrahydro-2H-pyran-2-yl)-1H-pyrazol-4-yl)phenyl)piperidine-4-ca rboxamide (45 mg, yield: 11.46%). LCMS (ESI): m/z =431[M+1]⁺.

Step 39d: Preparation of 1-(4-(1H-pyrazol-4-yl)phenyl)-N-phenylpiperidine-4-carboxamide (Compound 58): A mixture of N-phenyl-1-(4-(1-(tetrahydro-2H-pyran-2-yl)-1H-pyrazol-4-yl)phenyl)piperidine-4-ca rboxamide (0308-58) (45 mg, 0.102 mmol, 1.0 eq.) in hydrogen chloride-dioxane solution (4M, 5 mL) was stirred at room temperature for 1.5 hours. The solvent was removed under reduced pressure and the residue was diluted with water. The pH was adjusted to 9 with sodium carbonate solution and the mixture was extracted with dichloromethane. The organic phase was dried and concentrated under reduced pressure. The residue was purified by prep-TLC (silica gel, dichloromethane/methanol = 12/1) to give a yellow solid product 1-(4-(1H-pyrazol-4-yl)phenyl)-N-phenylpiperidine-4-carboxamide (20 mg, yield: 55.56%). LCMS (ESI): m/z = 347 [M+1]⁺. ¹ H NMR (500 MHz, DMSO) δ 12.77 (s, 1H), 9.92 (s, 1H), 7.90 (s, 2H), 7.62 (d, *J =* 8.2 Hz, 2H), 7.43 (d, *J =* 8.4 Hz, 2H), 7.28 (t, *J =* 7.7 Hz, 2H), 7.02 (t, *J =* 7.3 Hz, 1H), 6.95 (d, *J =* 8.5 Hz, 2H), 3.76 (d, *J =* 12.3 Hz, 2H), 2.71 (t, *J =* 11.3 Hz, 2H), 2.52 (d, *J =* 15.0 Hz, 1H), 1.88 (d, *J =* 11.5 Hz, 2H), 1.82 - 1.71 (m, 2H).

### Example 40: Preparation of (1-(4-(1H-pyrazol-4-yl)phenyl)piperidin-4-yl)(2-azabicyclo [2.2.1] heptane-2-yl)me thanone (Compound 65) (Prepared according to Scheme 8)

Step 40a: Preparation of (2-azabicyclo[2.2.1]heptane-2-yl)(1-(4-(1-(tetrahydro-2H-pyran-2-yl)-1H-pyrazol-4-y l)phenyl)piperidin-4-yl)methanone (Compound 0803-65): To a mixture of 1-(4-(1-(tetrahydro-2H-pyran-2-yl)-1H-pyrazol-4-yl)phenyl)piperidine-4-carboxylic acid (0801-55) (58 mg, 0.16 mmol, 1.0 eq.), HATU (92 mg, 0.25 mmol, 1.5 eq.) and N,N-diisopropylethylamine (42 mg, 0.32 mmol, 2.0 eq.) in dichloromethane (5 mL) was added 2-azabicyclo[2.2.1]heptane (20 mg, 0.21 mmol, 1.3 eq.). The mixture was stirred at room temperature overnight. The solvent was removed under reduced pressure. The residue was purified by Pre-TLC (silica gel, dichloromethane: methanol = 20: 1) to give a pale yellow oil product (2-azabicyclo [2.2.1] heptane-2-yl) (1- (4-(1- (tetrahydro-2H-pyran-2-yl) -1H-pyrazol-4-yl) phenyl) piperidin-4-yl) ketone (50 mg, yield: 70%). LCMS (ESI): m/z = 435 [M + 1]⁺; TLC: Rf 0.5 (dichloromethane: methanol = 20: 1).

Step 40b: Preparation of (1-(4-(1H-pyrazol-4-yl)phenyl)piperidin-4-yl)(2-azabicyclo[2.2.1]heptane-2-yl)metha none (Compound 65): A mixture of (2-azabicyclo[2.2.1]heptane-2-yl)(1-(4-(1-(tetrahydro-2H-pyran-2-yl)-1H-pyrazol-4-y l)phenyl)piperidin-4-yl)methanone (15 mg, 0.035 mmol, 1.0 eq.) in hydrogen chloride-methanol solution (4M, 2 ml) was stirred at room temperature for 2 hours. The mixture was diluted with water (15 ml). Solid sodium carbonate was added to adjust pH = 10, and then the aqueous layer was extracted with ethyl acetate (10 ml × 3). The combined organic layer was washed with saturated brine (15 ml × 1), dried over anhydrous sodium sulfate and concentrated. The residue was purified by Pre-TLC (ethyl acetate: methanol = 10: 1) to give a white solid product (1-(4-(1H-pyrazol-4-yl)phenyl)piperidin-4-yl)(2-azabicyclo[2.2.1]heptane- 2-yl)methanone (10 mg, yield: 83%). LCMS (ESI): m/z 351 [M+1]⁺; TLC: Rf 0.5 (ethyl acetate: methanol = 10: 1). ¹ H NMR (500 MHz, DMSO) δ 12.74 (s, 1H), 8.12 - 7.67 (m, 2H), 7.42 (d, *J =* 8.2 Hz, 2H), 7.01 - 6.78 (m, 2H), 4.39 (s, 1H), 3.68 (t, *J =* 21.0 Hz, 2H), 3.43 (d, *J =* 8.7 Hz, 1H), 3.16 (d, *J =* 8.4 Hz, 1H), 2.75 - 2.55 (m, 3H), 1.79 - 1.27 (m, 11H).

### Example 41: Preparation of (1-(4-(1H-pyrazol-4-yl)phenyl)piperidin-4-yl)( isoindolin -2-yl)methanone (Compound 66) (Prepared according to Scheme 3)

Step 41a: Preparation of tert-butyl 4-(isoindoline-2-carbonyl)piperidine-1-carboxylate (Compound 0302-66): Under nitrogen protection, isoindoline hydrochloride (220 mg, 1.40 mmol, 1.0 eq.), 1-(tert-butyloxycarbonyl)piperidine-4-carboxylic acid (356 mg, 1.55 mmol, 1.1 eq.), N,N-diisopropylethylamine (547 mg, 4.24 mmol, 3.0 eq.), HATU (591 mg, 1.55 mmol, 1.1 eq.) were stirred in N,N-dimethylformamide (10 ml) for 3 hours. The reaction was diluted with water and extracted with ethyl acetate. The organic phase was washed with brine, dried over anhydrous sodium sulfate and concentrated under reduced pressure. The residue was purified by column chromatography on silica gel (eluent: ethyl acetate) to obtain a yellow solid product tert-butyl 4-(isoindoline-2-carbonyl)piperidine-1-carboxylate (408 mg, yield: 88.04%). LCMS (ESI): m/z = 331 [M+H]⁺.

Step 41b: Preparation of isoindoline -2-yl(piperidin-4-yl)methanone (Compound 0303-66): A mixture of 4-(isoindoline-2-carbonyl)piperidine-1-carboxylate (0302-66) (408 mg, 1.24 mmol, 1.0 eq.) in hydrogen chloride-dioxane (4M, 6 ml) was stirred at room temperature for 1.0 h. The solvent was removed under reduced pressure, and the residue was diluted with water, adjusted to pH = 9 with sodium carbonate solution, and extracted with dichloromethane. The organic phase was dried over anhydrous sodium sulfate and concentrated under reduced pressure. The residue was used directly in the next step without further purification. LCMS (ESI): m/z = 231 [M+H]⁺.

Step 41c: Preparation of isoindolin-2-yl(1-(4-(1-(tetrahydro-2H-pyran-2-yl)-1H-pyrazol-4-yl)phenyl)piperidin-4-yl)methanone (Compound 0304-66): Under nitrogen protection, a mixture of isoindolin-2-yl(piperidin-4-yl)methanone (0303-66) (260 mg, 0.98 mmol, 1.0 eq.), 4-(4-bromophenyl)-1-(tetrahydro-2H-pyran-2-yl)-1H-pyrazole (0103-1) (360 mg, 1.18 mmol, 1.2 eq.), sodium tert-butoxide (282 mg, 2.94 mmol, 2.5 eq.), 2-dicyclohexylphosphino-2',6'-di-i-propoxy-1,1'-bipheny (45 mg, 0.10 mmol, 0.1 eq.) and tris(dibenzylideneacetone)dipalladium(0) (46 mg, 0.05 mmol, 0.05 eq.) in toluene (15 ml) was stirred at 120°C overnight. The reaction was diluted with water and extracted with ethyl acetate. The organic phase was washed with brine, dried over anhydrous sodium sulfate and concentrated under reduced pressure. The residue was purified by column chromatography on silica gel (dichloromethane/methanol = 50/1 to 20/1) to obtain a pink solid product isoindolin-2-yl(1-(4-(1-(tetrahydro-2H-pyran-2-yl)-1H-pyrazol-4-yl)phenyl)piperidin-4-yl)methanone (290 mg, yield: 64.89%). LCMS (ESI): m/z = 457 [M+H]⁺.

Step 41d: Preparation of (1-(4-(1H-pyrazol-4-yl)phenyl)piperidin-4-yl)( isoindolin -2-yl)methanone (Compound 66): A mixture of isoindolin-2-yl(1-(4-(1-(tetrahydro-2H-pyran-2-yl)-1H-pyrazol-4-yl)phenyl)piperidin-4-yl)methanone (0304-66) (60 mg, 0.132 mmol, 1.0 eq.) in hydrogen chloride-dioxane solution (4M, 5 ml) was stirred at room temperature for 2 hours. The solvent was removed under reduced pressure. The residue was diluted with water, adjusted to pH = 9 with sodium carbonate solution, and extracted with dichloromethane. The organic phase was dried over anhydrous sodium sulfate and concentrated under reduced pressure. The residue was purified by prep-TLC (silica gel, dichloromethane/methanol = 15/1) to give a yellow solid product **(1-(4-(1H-pyrazol-4-yl)phenyl)piperidin-4-yl)(** isoindolin -2-yl)methanone (37 mg, yield: 75.43%). LCMS (ESI): m/z = 373 [M+H]⁺. ¹ H NMR (500 MHz, DMSO) δ 12.76 (s, 1H), 8.01 (s, 1H), 7.79 (s, 1H), 7.43 (d, *J =* 8.6 Hz, 2H), 7.33 (dd, *J =* 21.8, 8.1 Hz, 4H), 6.95 (d, *J =* 8.6 Hz, 2H), 4.96 (s, 2H), 4.65 (s, 2H), 3.76 (d, *J =* 12.2 Hz, 2H), 2.74 (dd, *J =* 26.1, 13.9 Hz, 3H), 1.84 (d, *J =* 12.1 Hz, 2H), 1.81 - 1.66 (m, 2H).

### Example 42: Preparation of (1-(4-(1H-pyrazol-4-yl)phenyl)piperidin-4-yl)(2-azaspiro[3.3]heptane-2-yl)metha none (Compound 67) (Prepared according to Scheme 3)

Step 42a: Preparation of tert-butyl 4-(2-azaspiro[3.3]heptane-2-carbonyl)piperidine-1-carboxylate (Compound 0302-67): 2-azaspiro[3.3]heptane hydrochloride (100 mg, 0.75 mmol, 1.0 eq.), 1-(tert-butyloxycarbonyl)piperidine-4-carboxylic acid (0301-40) (188.7 mg, 0.8232 mmol, 1.1 eq.), N,N-diisopropylethylamine (289.6 g, 2.2 mmol, 3.0 eq.) and HATU (312.8 mg, 0.8232 mmol, 1.1 eq.) were dissolved in 10 mL N,N-dimethylformamide. The mixture was stirred at room temperature for two hours. The reaction was diluted with ethyl acetate and the organic phase was washed with water and saturated brine. Then, the organic phase was purified by column chromatography on silica gel ( eluent: petroleum ether /ethyl acetate = 1/1 to 1/2) to obtain a yellow oily product tert-butyl 4-(2-azaspiro[3.3]heptane-2-carbonyl)piperidine-1-carboxylate (295.4 mg, crude product). MS (ES⁺): m/z=309(M+H)⁺.

Step 42b: Preparation of piperidin-4-yl(2-azaspiro[3.3]heptane-2-yl)methanone hydrochloride (Compound 0303-67): tert-Butyl 4-(2-azaspiro[3.3]heptane-2-carbonyl)piperidine-1-carboxylate (0302-67) (295.4 mg, 0.95 mmol, 1.0 eq.) was added to 5 mL 4M hydrogen chloride-dioxane solution. The mixture was stirred at room temperature for 2 hours. The mixture was concentrated to dryness under reduced pressure and the residue was used directly in the next step without further purification (289.5 mg, crude). MS (ES⁺): m/z=244 (M+H)⁺.

Step 42c: Preparation of (2-azaspiro[3.3]heptan-2-yl)(1-(4-(1-(tetrahydro-2H-pyran-2-yl)-1H-pyrazol-4-yl)phe nyl)piperidin-4-yl)methanone (Compound 0304-67): Under nitrogen protection, piperidin-4-yl (2-azaspiro [3.3] heptane-2-yl) ketone hydrochloride (0303-67) (236.4 mg, 0.96 mmol, 2.0 eq.), 4- (4-bromophenyl) -1- (tetrahydro-2H-pyran-2-yl) -1H-pyrazole (0103-1) (147.3 mg, 0.48 mmol, 1.0 eq.), sodium tert-butoxide (556 mg, 5.76 mmol, 12.0 eq.), 4,5-bis (diphenylphosphino) -9,9-dimethylxanthene (55.8 mg, 0.096 mmol, 0.2 eq.) and tris (dibenzylideneacetone) dipalladium (0) (44 mg, 0.048 mmol, 0.1 eq.) in toluene (16 The mixture of 40% by volume and 20% by volume (ml) was stirred at 120°C overnight. After cooling to room temperature, the reaction was filtered through celite and the filter cake was washed with ethyl acetate. The filtrate was concentrated under reduced pressure, and the residue was purified by prep-TLC (silica gel, eluent: dichloromethane/methanol = 10/1) to give a yellow solid product (2-azaspiro[3.3]heptan-2-yl)(1-(4-(1-(tetrahydro-2H-pyran-2-yl)-1H-pyrazol-4-yl)phe nyl)piperidin-4-yl)methanone (47.9 mg, yield: 23.0%). MS (ES⁺): m/z=435(M+H)⁺.

Step 42d: Preparation of (1-(4-(1H-pyrazol-4-yl)phenyl)piperidin-4-yl)(2-azaspiro[3.3]heptane-2-yl)methanone (Compound 67): (2-azaspiro[3.3]heptane-2-yl)(1-(4-(1-(tetrahydro-2H-pyran-2-yl)-1H-pyrazol-4-yl)ph enyl)piperidin-4-yl)methanone (0304-67) (47.9 mg, 0.110 mmol, 1.0 eq.) was added to 10 mL 4M hydrogen chloride - methanol solution and stirred at room temperature for 2 hours. The mixture was concentrated to dryness under reduced pressure. Water was added and the pH was adjusted to 10 with saturated aqueous sodium carbonate. Ethyl acetate was added for extraction, washed with water, dried over anhydrous sodium sulfate, concentrated under reduced pressure, and the residue was purified by prep-TLC (silica gel, eluent: dichloromethane/methanol = 20/1) to obtain a white solid product (1-(4-(1H-pyrazol-4-yl)phenyl)piperidin-4-yl)(2-azaspiro[3.3]heptane-2-yl)methanone (10.5 mg, yield: 27.0%). MS (ES⁺): m/z=351(M+H)⁺. Melting point: 229-231 ° C. ¹H NMR (500 MHz, DMSO) δ 12.76 (s, 1H), 7.89 (d, *J =* 103.5 Hz, 2H), 7.41 (d, *J =* 8.6 Hz, 2H), 6.91 (d, *J =* 8.7 Hz, 2H), 4.14 (s, 2H), 3.80 (s, 2H), 3.68 (d, *J =* 12.4 Hz, 2H), 2.68 (dd, *J =* 11.9, 10.0 Hz, 2H), 2.39 - 2.27 (m, 1H), 2.19 - 2.05 (m, 4H), 1.77 (p, *J* = 7.2 Hz, 2H), 1.71 - 1.54 (m, 4H).

### Example 43: Preparation of 1-(1-(4-(1H- pyrazol -4- yl) phenyl) piperidin -4-yl) pyrrolidin -2- one ( Compound 73) ( Prepared according to Scheme 6)

Step 43a: Preparation of tert-butyl 4-(2 -oxopyrrolidin -1- yl) piperidine -1 -carboxylate ( Compound 0603-73): Under nitrogen protection, a mixture of tert-butyl 4- aminopiperidine -1- carboxylate (0601-73) (2 g, 9.99 mmol, 1.0 eq.), 4-chlorobutyryl chloride (1.55 g, 10.99 mmol, 1.1 eq.), N,N -diisopropylethylamine (1.94 g, 14.99 mmol, 1.5 eq.) in dichloromethane (20 ml) was stirred at room temperature for 2.0 hours. Sodium carbonate solution was added to quench the reaction. The reaction was diluted with water and extracted with dichloromethane (10 ml × 4). The combined organic layer was washed with saturated brine (40 ml × 1), dried over anhydrous sodium sulfate, and concentrated under reduced pressure. The residue was dissolved in tetrahydrofuran (20 ml) and cooled to 0 ° C. NaH (60%, 799 mg, 19.98 mmol, 2.0 eq.) was added under nitrogen protection and the reaction was stirred at room temperature for 0.5 h. The mixture was then stirred at 85 °C for 6 hours. Saturated ammonium chloride solution (10 ml) was added to quench the reaction. The mixture was diluted with water (50 ml) and extracted with ethyl acetate (20 ml × 3). The combined organic layer was washed with brine (50 ml × 1), dried over anhydrous sodium sulfate and concentrated in vacuo. The residue was purified by column chromatography on silica gel (eluent: petroleum ether/ethyl acetate = 3/1 to 1/4) to give a white solid product tert-butyl 4-(2 -oxopyrrolidin -1- yl) piperidine -1 -carboxylate (1.46 g, yield: 54.2%).

Step 43b: Preparation of 1-( piperidin -4- yl) pyrrolidin -2- one ( Compound 0603-73) : A mixture of tert-butyl 4-(2- oxopyrrolidin -1- yl) piperidine -1-carboxylate (0602-73) (1.46 g, 5.45 mmol, 1.0 eq.) in hydrogen chloride - dioxane (4 M, 15 mL) was stirred at room temperature for 1.5 hours. The solvent was removed under reduced pressure, and the residue was diluted with water (15 mL), adjusted to pH = 9 with sodium carbonate solution, and extracted with dichloromethane (10 mL × 15). The combined organic phase was dried over anhydrous sodium sulfate and concentrated under reduced pressure to give a yellow solid product 1-(piperidin-4-yl)pyrrolidin-2-one ( 830 mg, yield: 90.7%). LCMS (ESI): m/z = 169 [M+1]⁺.

Step 43c: Preparation of 1-(1-(4-(1-(tetrahydro-2H-pyran-2-yl)-1H-pyrazol-4-yl)phenyl)piperidin-4-yl)pyrrolid in-2-one (Compound 0604-73): Under nitrogen protection, a mixture of 1-(piperidin-4-yl)pyrrolidin-2-one (0603-73) (98.6 mg, 0.587 mmol, 1.2 eq.), 4-(4-bromophenyl)-1-(tetrahydro-2H-pyran-2-yl)-1H-pyrazole (0103-1) (150 g, 0.489 mmol, 1.0 eq.), sodium tert-butoxide (117.5 mg, 1.22 mmol, 2.5 eq.), 4,5-bis(diphenylphosphino)-9,9-dimethylxanthene (28.3 mg, 0.0489 mmol, 0.1 eq.) and tris(dibenzylideneacetone)dipalladium(0) (22.4 mg, 0.025 mmol, 0.05 eq.) in toluene (5 ml) was stirred at 120°C for 10.0 hours. The reaction was diluted with water ( 25 ml) and extracted with ethyl acetate (10 ml × 3). The organic phase was washed with saturated brine (30 ml × 1), dried over anhydrous sodium sulfate and concentrated under reduced pressure. The residue was purified by column chromatography on silica gel (dichloromethane/methanol = 60/1 to 30/1) to give yellow solid product 1-(1-(4-(1-(tetrahydro-2H-pyran-2-yl)-1H-pyrazol-4-yl)phenyl)piperidin-4-yl)pyrrolid in-2-one (138 mg, yield: 71.8%). LCMS (ESI): m/z = 395 [M+1]⁺.

Step 43d: Preparation of 1-(1-(4-(1H-pyrazol-4-yl)phenyl)piperidin-4-yl)pyrrolidin-2-one (Compound 73): A mixture of 1-(1-(4-(1-(tetrahydro-2H-pyran-2-yl)-1H-pyrazol-4-yl)phenyl)piperidin-4-yl)pyrrolid in-2-one (0604-73) (138 mg, 0.35 mmol, 1.0 eq.) in hydrogen chloride-dioxane (4 M, 5 mL) was stirred at room temperature for 1.0 hour. The solvent was removed under reduced pressure, the residue was diluted with water (8 mL), adjusted to pH = 9 with sodium carbonate solution, extracted with dichloromethane (4 mL × 5), and the organic phase was dried over anhydrous sodium sulfate and concentrated under reduced pressure. The residue was purified by prep-TLC (silica gel, dichloromethane/methanol = 12/1) to give a white solid product 1-(1-(4-(1H-pyrazol-4-yl)phenyl)piperidin-4-yl)pyrrolidin-2-one (45.8 g, yield: 42.4%). LCMS(ESI): m/z =311[M+1]⁺. ¹H NMR (500 MHz, DMSO) δ 12.76 (s, 1H), 8.00 (s, 1H), 7.79 (s, 1H), 7.42 (d, *J =* 8.7 Hz, 2H), 6.93 (d, *J =* 8.8 Hz, 2H), 3.90 (tt, *J* = 12.0, 4.0 Hz, 1H), 3.75 (d, *J =* 12.6 Hz, 2H), 3.32 (d, *J =* 6.9 Hz, 2H), 2.82 - 2.63 (m, 2H), 2.23 (dd, *J =* 10.4, 5.8 Hz, 2H), 1.91 (dd, *J* = 15.0, 7.4 Hz, 2H), 1.89 - 1.72 (m, 2H), 1.62 (d, *J =* 9.9 Hz, 2H).

### Example 44: Preparation of 1-((1-(4-(1H-pyrazol-4-yl)phenyl)pyrrolidin-3-yl)methyl)pyrrolidin-2-one (Compound 74) ( Prepared according to Scheme 1)

Step 44a: Preparation of tert-butyl 3-((( methylsulfonyl) oxy) methyl) pyrrolidine -1 - carboxylate ( Compound 0105-74) : To a mixture of tert- butyl 3-( hydroxymethyl) pyrrolidine -1- carboxylate ( 0105-74) (1.0 g, 4.97 mmol, 1.0 eq.) and triethylamine (1.0 g, 9.94 mmol, 2.0 eq.) in dichloromethane (20 ml) was added dropwise methylsulfonyl chloride (740 mg, 6.46 mmol, 1.3 eq.) under nitrogen protection at 0 °C. The mixture was stirred at room temperature for 4.0 hours. The reaction was diluted with water and extracted with dichloromethane. The organic layer was washed with brine, dried over anhydrous sodium sulfate, and concentrated under reduced pressure. The residue was purified by column chromatography on silica gel (petroleum ether/ethyl acetate = 50/1 to 10/1) to give a yellow oily product tert-butyl 3-(((methylsulfonyl)oxy)methyl)pyrrolidine-1-carboxylate (1.32 g, yield: 95.2%). MS (ES⁺): m/z = 280 (M+H)⁺.

Step 44b: Preparation of tert - butyl 3-((2- oxopyrrolidin -1- yl) methyl) pyrrolidine -1- carboxylate ( Compound 0107-74): To a mixture of 2- pyrrolidone (0106-1) (201 mg, 2.36 mmol, 1.1 eq.) in N,N- dimethylformamide (15 mL) was added sodium hydride (130 mg, 3.23 mmol, 1.5 eq.) under nitrogen. The mixture was stirred at 0 °C for 1 hour. Then, tert-butyl 3-(((methylsulfonyl)oxy)methyl)pyrrolidine-1-carboxylate (0105-74) (600 mg, 2.15 mmol, 1.0 eq.) was added dropwise at 0 °C, and the reaction was stirred at 65 °C for 8 hours. Water and ethyl acetate were added for extraction, and the organic layer was washed with brine and concentrated under reduced pressure to obtain a yellow oily product tert-butyl 3-((2-oxopyrrolidin-1-yl)methyl)pyrrolidine-1-carboxylate (395 mg, yield: 68.5%). MS (ES⁺): m/z=269(M+H)⁺.

Step 44c: Preparation of 1-( pyrrolidin -3- ylmethyl) pyrrolidin -2- one ( Compound 0108-74) : A mixture of tert-butyl 3-((2 -oxopyrrolidin -1- yl) methyl) pyrrolidine -1- carboxylate (0107-74) (390 mg, 1.45 mmol, 1.0 eq.) in hydrogen chloride - methanol (4M, 8 ml) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure to give 1-(pyrrolidin-3-ylmethyl)pyrrolidin-2-one (240 mg, yield: 98.3%) as a yellow oil. LCMS (ESI): m/z = 169 [M+1]⁺.

Step 44d: Preparation of 1-((1-(4-(1-(tetrahydro-2H-pyran-2-yl)-1H-pyrazol-4-yl)phenyl)pyrrolidin-3-yl)methy l)pyrrolidin-2-one (Compound 0109-74): Under nitrogen protection, a mixture of 1-(pyrrolidin-3-ylmethyl)pyrrolidin-2-one (0108-74) (174 mg, 0.849 mmol, 1.3 eq.), 4-(4-bromophenyl)-1-(tetrahydro-2H-pyran-2-yl)-1H-pyrazole (0103-1) (200 mg, 0.653 mmol, 1.0 eq.), sodium tert-butoxide (186 mg, 1.959 mmol, 3.0 eq.), 2-dicyclohexylphosphino-2',6'-diisopropoxy-1,1'-biphenyl (14.9 mg, 0.032 mmol, 0.05 eq.) and tris(dibenzylideneacetone)dipalladium(0) (29.3 mg, 0.032 mmol, 0.05 eq.) in toluene (10 ml) was stirred at 120°C overnight. The reaction was diluted with water and extracted with ethyl acetate, and the organic phase was dried and concentrated under reduced pressure. The residue was purified by column chromatography on silica gel (dichloromethane/methanol = 150/1 to 50/1) to give a yellow solid product 1-((1-(4-(1-(tetrahydro-2H-pyran-2-yl)-1H-pyrazol-4-yl)phenyl)pyrrolidin-3-yl)methy l)pyrrolidin-2-one (105 mg, yield: 40.8%). LCMS (ESI): m/z = 395 [M+1]⁺.

Step 44e: Preparation of 1-((1-(4-(1H-pyrazol-4-yl)phenyl)pyrrolidin-3-yl)methyl)pyrrolidin-2-one (Compound 74): A mixture of 1-((1-(4-(1-(tetrahydro-2H-pyran-2-yl)-1H-pyrazol-4-yl)phenyl)pyrrolidin-3-yl)methy l)pyrrolidin-2-one (0109-74) (105 mg, 0.266 mmol, 1.0 eq.) in hydrogen chloride-methanol (4M, 5 ml) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product 1-((1-(4-(1H-pyrazol-4-yl)phenyl)pyrrolidin-3-yl)methyl)pyrrolidin-2-one (36 mg, yield: 43.9%). LCMS (ESI): m/z = 311 [M+1]⁺. Melting point : 95-104 °C. ¹ H NMR (500 MHz, DMSO) δ 12.70 (s, 1H), 7.84 (d, *J =* 82.1 Hz, 2H), 7.38 (d, *J =* 8.5 Hz, 2H), 6.51 (d, *J =* 8.5 Hz, 2H), 3.40 (t, *J =* 6.9 Hz, 2H), 3.35 (d, *J =* 9.1 Hz, 1H), 3.28 - 3.16 (m, 4H), 2.94 (dd, *J =* 9.1, 6.6 Hz, 1H), 2.59 (dt, *J =* 14.2, 7.1 Hz, 1H), 2.24 (t, *J* = 8.0 Hz, 2H), 2.0 Hz, 1H), 2.00 - 1.85 (m, 2H), 1.76 - 1.61 (m, 1H).

### Example 45 : Preparation of 1-((1-(2,3-difluoro-4-(1H-pyrazol-4-yl)phenyl)piperidin-4-yl)methyl)piperidin-2-o ne (Compound 26) (Prepared according to Scheme 1)

Step 45a: Preparation of 1-((1-(2,3-difluoro-4-(1-(tetrahydro-2H-pyran-2-yl)-1H-pyrazol-4-yl)phenyl)piperidin -4-yl)methyl)piperidin-2-one ( Compound 0109-26) : Under nitrogen protection, a mixture of 4-(4-bromo-2,3-difluorophenyl)-1-(tetrahydro-2H-pyran-2-yl)-1H-pyrazole (0103-4) (170 mg, 0.50 mmol, 1.0 eq.), 1-(piperidin-4-ylmethyl)piperidin-2-one (0108-26) (147 mg, 0.75 mmol, 1.5 eq.), sodium tert-butoxide (209 mg, 2.25 mmol, 4.5 eq.), 2-dicyclohexylphosphino-2',6'-di-i-propoxy-1,1'-bipheny(24 mg, 0.05 mmol, 0.1 eq.) and tris(dibenzylideneacetone)dipalladium (23 mg, 0.025 mmol, 0.05 eq.) in toluene (10 ml) was stirred at 120°C overnight. After cooling to room temperature, the reaction was quenched with water and extracted with ethyl acetate. The organic phase was washed with saturated brine, dried over anhydrous sodium sulfate, and concentrated under reduced pressure. The residue was purified by column chromatography on silica gel (eluent: dichloromethane/methanol = 50/1 to 20/1) to give yellow solid product 1-((1-(2,3-difluoro-4-(1-(tetrahydro-2H-pyran-2-yl)-1H-pyrazol-4-yl)phenyl)piperidin -4-yl)methyl)piperidin-2-one (198 mg, yield: 86.46%). MS (ES⁺): m/z = 459 [M+H]⁺.

Step 45b: Preparation of 1-((1-(2,3-difluoro-4-(1H-pyrazol-4-yl)phenyl)piperidin-4-yl)methyl)piperidin-2-one (Compound 26): A mixture of 1-((1-(2,3-difluoro-4-(1-(tetrahydro-2H-pyran))-2-yl)-1H-pyrazol-4-yl)phenyl)piperid in-4-yl)methyl)piperidin-2-one (0109-26) (50 mg, 0.11 mmol, 1.0 eq.) in hydrogen chloride-methanol (4 M, 3 mL) was stirred at room temperature for 2 hours. The solvent was removed under reduced pressure, and the pH of the residue was adjusted to 9 with sodium carbonate solution and extracted with dichloromethane. The organic phase was dried and concentrated under reduced pressure. The residue was purified by prep-TLC (silica gel, eluent: dichloromethane/methanol = 15/1) to give 1-((1-(2,3-difluoro-4-(1H-pyrazol-4-yl)phenyl)piperidin-4-yl)methyl)piperidin-2-one (27 mg, yield: 65.55%) as a yellow solid. MS (ES⁺): m/z = 375 [M+H]⁺. 1H NMR (500 MHz, DMSO) δ 13.04 (s, 1H), 8.08 (s, 1H), 7.86 (s, 1H), 7.38 (t, *J =* 7.8 Hz, 1H), 6.84 (t, *J =* 8.2 Hz, 1H), 3.39 (s, 2H), 3.28 - 3.25 (m, 2H), 3.22 (d, *J =* 7.4 Hz, 2H), 2.67 (t, *J =* 11.4 Hz, 2H), 2.22 (t, *J =* 6.0 Hz, 2H), 1.81 (s, 1H), 1.69 (dd, *J =* 21.3, 9.0 Hz, 6H), 1.32 (dd, *J =* 21.1, 11.4 Hz, 2H).

### Example 46 : Preparation of 1-((1-(2,6-difluoro-4-(1H-pyrazol-4-yl)phenyl)piperidin-4-yl)methyl)piperidin-2-o ne (Compound 27) (Prepared according to Scheme 1)

Step 46a: Preparation of 1-((1-(2,6 -difluoro -4-(1-( tetrahydro -2H- pyran -2-yl)-1H- pyrazol -4- yl) phenyl) piperidin -4- yl) methyl) piperidin -2- one ( Compound 0109-27) : Under nitrogen protection, a mixture of 4-(4- bromo -3,5 -difluorophenyl)-1-( tetrahydro -2H - pyran -2- yl)-1H- pyrazole (0103-5) (173 mg, 0.88 mmol, 1.0 eq.), 1-( piperidin -4 -ylmethyl) piperidin -2- one (0108-26) (173 mg, 0.88 mmol, 1.5 eq.), sodium tert-butoxide (198 mg, 2.07 mmol, 3.5 eq.), 4,5-bis(diphenylphosphino)-9,9-dimethylxanthene (35 mg, 0.06 mmol, 0.1 eq.) and tris ( dibenzylideneacetone) dipalladium (0) (27 mg, 0.03 mmol, 0.05 eq.) in toluene (10 ml) was stirred at 120 °C overnight. After cooling to room temperature, the reaction was quenched with water and extracted with ethyl acetate. The organic phase was washed with saturated brine, dried over anhydrous sodium sulfate, and concentrated under reduced pressure. The residue was purified by column chromatography on silica gel (eluent: dichloromethane/methanol = 50/1 to 20/1) to give a yellow solid product 1-((1-(2,6 -difluoro -4-(1-( tetrahydro -2H- pyran -2- yl)-1H- pyrazol -4- yl) phenyl) piperidin -4- yl) methyl) piperidin -2- one (35 mg, yield: 12.92%). MS (ES⁺): m/z = 459 [M+H]⁺.

Step 46b: Preparation of 1-((1-(2,6-difluoro-4-(1H-pyrazol-4-yl)phenyl)piperidin-4-yl)methyl)piperidin-2-one (Compound 27): A mixture of 1-((1-(2,6 -difluoro -4-(1-( tetrahydro -2H- pyran -2-yl)-1H- pyrazol -4- yl) phenyl) piperidin -4- yl) methyl) piperidin -2- one (0109-27) (35 mg, 0.076 mmol, 1.0 eq.) in hydrogen chloride-methanol (4 M, 3 mL) was stirred at room temperature for 2 hours. The solvent was removed under reduced pressure, and the pH of the residue was adjusted to 9 with sodium carbonate solution and extracted with dichloromethane. The organic phase was dried and concentrated under reduced pressure. The residue was purified by prep-TLC (silica gel, eluent: dichloromethane/methanol = 15/1) to give 1-((1-(2,6-difluoro-4-(1H-pyrazol-4-yl)phenyl)piperidin-4-yl)methyl)piperidin-2-one (22 mg, yield: 77.40%) as a yellow solid. MS (ES⁺): m/z = 375 [M+H]⁺. ¹ H NMR (500 MHz, DMSO) δ 12.95 (s, 1H), 8.21 (s, 1H), 7.94 (s, 1H), 7.29 (d, *J =* 10.4 Hz, 2H), 3.28 - 3.25 (m, 2H), 3.21 (d, *J =* 7.4 Hz, 2H), 3.14 (d, *J =* 11.6 Hz, 2H), 2.97 (t, *J* = 11.3 Hz, 2H), 2.22 (t, *J =* 6.0 Hz, 2H), 1.74 (dd, *J =* 30.6, 7.6 Hz, 5H), 1.61 (d, *J* = 11.9 Hz, 2H), 1.27 - 1.17 (m, 2H).

### Example 47 : Preparation of 1-((1-(2,3-difluoro-4-(isoxazol-4-yl)phenyl)piperidin-4-yl)methyl)pyrrolidin-2-one (Compound 37) :

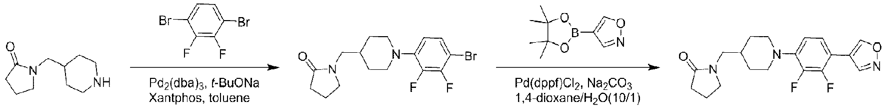

Step 47a: Preparation of 1-((1-(4-bromo-2,3-difluorophenyl)piperidin-4-yl)methyl)pyrrolidin-2-one: Under nitrogen protection, 1- (piperidin-4-ylmethyl)pyrrolidin-2-one (0108-1) (500 mg, 2.75 mmol, 1 eq.), 1,4-dibromo-2,3-difluorobenzene (0201-4) (822 mg, 3.02 mmol, 1.1 eq.), 4,5-bis(diphenylphosphino-9,9-dimethylxanthene) (162 mg, 0.28 mmol, 0.1 eq.), tris(dibenzylideneacetone)dipalladium (44 mg, 0.12 mmol, 0.05 eq.), sodium tert-butoxide (660 mg, 6.87 mmol, 2.5 eq.) in toluene (20 ml) were mixed and stirred at 120 °C for 2 hours. The reaction was filtered and the filtrate was concentrated under reduced pressure. The residue was purified by column chromatography on silica gel (dichloromethane/methanol = 30/1) to obtain a yellow solid product 1-((1-(4-bromo-2,3-difluorophenyl)piperidin-4-yl)methyl)pyrrolidin-2-one (689 mg, yield: 67.4%). LCMS (ESI) [M+1]⁺ : m/z = 373.

Step 47b: Preparation of 1-((1-(2,3-difluoro-4-(isoxazol-4-yl)phenyl)piperidin-4-yl)methyl)pyrrolidin-2-one (Compound 37): Under nitrogen protection, 1-(( 1-(4-bromo-2,3-difluorophenyl)piperidin-4-yl)methyl)pyrrolidin-2-one (120 mg, 0.32 mmol, 1 eq.), 4-isoxazoleboronic acid pinacol ester (125 mg, 0.64 mmol, 2 eq.), sodium carbonate (68 mg, 0.64 mmol, 2 eq.), 1,1'-bis(diphenylphosphino)ferrocenepalladium(II) chloride (24 mg, 0.032 mmol, 0.1 eq.) in 1,4-dioxane (5 ml), water (0.5 ml) were mixed and stirred at 90 °C for 6.5 hours. The reaction was diluted with water and extracted with ethyl acetate. The organic layer was washed with saturated brine, dried over anhydrous sodium sulfate, and concentrated under reduced pressure. The residue was purified by preparative plate (dichloromethane / methanol = 30/1) to give a yellow solid crude 1-((1-(2,3-difluoro-4-(isoxazol-4-yl)phenyl)piperidin-4-yl)methyl)pyrrolidin-2-one (38 mg, purity: 88%). The crude product was purified by HPLC to give a white solid product 1-((1-(2,3-difluoro-4-(isoxazol-4-yl)phenyl)piperidin-4-yl)methyl)pyrrolidin-2-one (22.2 mg, yield: 19.3%). LCMS (ESI) [M+1]⁺ : m/z = 362. ¹ H NMR (500 MHz, DMSO) δ 9.31 (s, 1H), 9.10 (s, 1H), 7.47 (t, *J =* 7.9 Hz, 1H), 6.92 (t, *J =* 8.1 Hz, 1H), 3.44 (d, *J =* 11.9 Hz, 2H), 3.36 (t, *J =* 6.9 Hz, 2H), 3.11 (d, *J =* 7.2 Hz, 2H), 2.73 (t, *J* = 11.5 Hz, 2H), 2.23 (t, *J =* 8.0 Hz, 2H), 2.05 - 1.87 (m, 2H), 1.85 - 1.71 (m, 1H), 1.68 (d, *J =* 12.4 Hz, 2H), 1.37 - 1.27 (m, 2H).

### Example 48: Preparation of N-((1-(4-(1H-pyrazol-4-yl)phenyl)piperidin-4-yl)methyl)heptylamide (Compound 44) ( Prepared according to Scheme 5)

Step 48a: Preparation of N-((1-(4-(1-( tetrahydro -2H- pyran -2- yl)-1H - pyrazol -4- yl) phenyl) piperidin -4- yl) methyl) heptylamide ( Compound 0506-44) : Under nitrogen protection, a mixture of (1-(4-(1-( tetrahydro -2H- pyran -2- yl)-1H- pyrazol -4- yl) phenyl) piperidin -4- yl) methanamine (0509-45) (60 mg, 0.176 mmol, 1.0 eq.), heptanoic acid (25 mg, 0.194 mmol, 1.1 eq.), HATU (100 mg, 0.264 mmol, 1.5 eq.), N,N -diisopropylethylamine (68 mg, 0.528 mmol, 3.0 eq.) in N,N-dimethylformamide (3.5 ml) was stirred at room temperature for 2.0 hours. The reaction was diluted with water and extracted with ethyl acetate. The organic phase was dried and concentrated under reduced pressure. The residue was purified by prep-TLC (silica gel, eluent: dichloromethane/methanol = 14/1) to obtain a yellow solid product N-((1-(4-(1-(tetrahydro-2H-pyran-2-yl)-1H-pyrazol-4-yl)phenyl)piperidin-4-yl)methy l)heptylamide (56 mg, yield: 70.4%).

Step 48b: Preparation of N-((1-(4-(1H-pyrazol-4-yl)phenyl)piperidin-4-yl)methyl)heptylamide (Compound 44): A mixture of N-((1-(4-(1-(tetrahydro-2H-pyran-2-yl)-1H-pyrazol-4-yl)phenyl)piperidin-4-yl)methy l)heptylamide (0506-44) (56 mg, 0.124 mmol, 1.0 eq.) in hydrogen chloride-dioxane (4 M, 5 mL) was stirred at room temperature for 1.0 h. The solvent was removed under reduced pressure and the residue was diluted with water. The pH of the solution was adjusted to 9 with sodium carbonate solution and the solution was extracted with dichloromethane. The organic phase was dried and concentrated under reduced pressure. The residue was purified by prep-TLC (silica gel, eluent: dichloromethane/methanol = 12/1) to give a white solid product N-((1-(4-(1H-pyrazol-4-yl)phenyl)piperidin-4-yl)methyl)heptylamide (37 mg, yield: 81.1%). LCMS (ESI): m/z =369[M+1]⁺. ¹HNMR(500 MHz, DMSO) δ 12.75 (s, 1H), 7.88 (d, *J =* 106.5 Hz, 3H), 7.41 (d, *J =* 8.5 Hz, 2H), 6.91 (d, *J =* 8.6 Hz, 2H), 3.66 (d, *J =* 12.0 Hz, 2H), 2.97 (t, *J =* 6.1 Hz, 2H), 2.60 (t, *J =* 11.4 Hz, 2H), 2.07 (t, *J =* 7.4 Hz, 2H), 1.70 (d, *J =* 11.8 Hz, 2H), 1.49 (s, 3H), 1.24 (s, 8H), 0.86 (t, J = 6.6 Hz, 3H).

### Example 49 : Preparation of 1-(4-(1H-pyrazol-4-yl)phenyl)-N-cyclohexylpiperidine-4-carboxamide (Compound 57) ( Prepared according to Scheme 8)

Step 49a: Preparation of N-cyclohexyl-1-(4-(1-(tetrahydro-2H-pyran-2-yl)-1H-pyrazol-4-yl)phenyl)piperidine-4-carboxamide (Compound 0802-57): To a mixture of 1-(4-(1-(tetrahydro-2H-pyran-2-yl)-1H-pyrazol-4-yl)phenyl)piperidine-4-carboxylic acid (0801-55) (45 mg, 0.13 mmol, 1.0 eq.), HATU (63 mg, 0.17 mmol, 1.3 eq.) and N,N-diisopropylethylamine (33 mg, 0.25 mmol, 2.0 eq.) in dichloromethane (5 mL) was added cyclohexylamine (16 mg, 0.17 mmol, 1.3 eq.). The mixture was stirred at room temperature for 2 hours. The solvent was removed under reduced pressure. The residue was purified by Pre-TLC (silica gel, ethyl acetate: dichloromethane: methanol = 15:: 15: 1) to give white solid N-cyclohexyl-1-(4-(1-(tetrahydro-2H-pyran-2-yl)-1H-pyrazol-4-yl)phenyl)piperidine-4-carboxamide (36 mg, yield: 65%). LCMS (ESI): m/z = 437 [M+1]⁺; TLC: Rf 0.5 (dichloromethane: methanol = 20: 1).

Step 49b: Preparation of 1-(4-(1H-pyrazol-4-yl)phenyl)-N-cyclohexylpiperidine-4-carboxamide (Compound 57): A mixture of N-cyclohexyl-1-(4-(1-(tetrahydro-2H-pyran-2-yl)-1H-pyrazol-4-yl)phenyl)piperidine-4-carboxamide (0802-57) (36 mg, 0.083 mmol, 1.0 eq.) in hydrogen chloride-methanol solution (4M, 3 ml) was stirred at room temperature for 1.5 hours. The mixture was diluted with water (20 ml). Solid sodium carbonate was added to adjust the pH to 10. The mixture was filtered. The solid was washed with water and then dried under vacuum to give 1-(4-(1H-pyrazol-4-yl)phenyl)-N-cyclohexylpiperidine-4-carboxamide (21 mg, yield: 72%) as a white solid. LCMS (ESI): m/z = 353 [M+1]⁺; TLC: Rf 0.5 (dichloromethane: methanol = 10: 1). ¹ H NMR (500 MHz, DMSO) δ 12.75 (s, 1H), 7.89 (s, 2H), 7.60 (s, 1H), 7.42 (s, 2H), 6.92 (s, 2H), 3.69 (s, 2H), 3.51 (s, 1H), 2.64 (s, 2H), 2.23 (s, 1H), 1.62 (d, *J =* 74.0 Hz, 10H), 1.13 (s, 4H).

### Example 50 : Preparation of 1-(4-(1H-pyrazol-4-yl)phenyl)-N-(4-fluorophenyl)piperidine-4-carboxamide (Compound 61) ( Prepared according to Scheme 8)

Step 50a: Preparation of N-(4-fluorophenyl)-1-(4-(1-(tetrahydro-2H-pyran-2-yl)-1H-pyrazol-4-yl)phenyl)piper idine-4-carboxamide (Compound 0802-61): Under nitrogen protection, a mixture of 1-(4-(1-(tetrahydro-2H-pyran-2-yl)-1H-pyrazol-4-yl)phenyl)piperidine-4-carboxylic acid (0801-55) (60 mg, 0.17 mmol, 1.0 eq.), 4-fluoroaniline (23 mg, 0.20 mmol, 1.2 eq.), HATU (97 mg, 0.25 mmol, 1.5 eq.), N,N-diisopropylethylamine (66 mg, 0.51 mmol, 3.0 eq.) in N,N-dimethylformamide (3 ml) was stirred at room temperature for 1 hour. The reaction was diluted with water and extracted with ethyl acetate. The organic phase was dried and concentrated under reduced pressure. The residue was purified by column chromatography on silica gel (dichloromethane/methanol = 10/1) to give a yellow solid product N-(4-fluorophenyl)-1-(4-(1-(tetrahydro-2H-pyran-2-yl)-1H-pyrazol-4-yl)phenyl)piper idine-4-carboxamide (45 mg, yield: 59.30%). LCMS (ESI): m/z = 449 [M+1]⁺.

Step 50b: Preparation of 1-(4-(1H-pyrazol-4-yl)phenyl)-N-(4-fluorophenyl)piperidine-4-carboxamide (Compound 61): A mixture of (4-fluorophenyl)-1-(4-(1-(tetrahydro-2H-pyran-2-yl)-1H-pyrazol-4-yl)phenyl)piperidi ne-4-carboxamide (0802-61) (45 mg, 0.10 mmol, 1.0 eq.) in hydrogen chloride-methanol solution (4M, 2 ml) was stirred at room temperature for 2 hours. The solvent was removed under reduced pressure, the residue was diluted with water, adjusted to pH = 8 with sodium carbonate solution, and extracted with dichloromethane. The organic phase was dried and concentrated under reduced pressure. The residue was purified by prep-TLC (silica gel, eluent: dichloromethane/methanol = 12/1) to give a yellow solid product 1-(4-(1H-pyrazol-4-yl)phenyl)-N-(4-fluorophenyl)piperidine-4-carboxamide (25 mg, yield: 69.44%). LCMS (ESI): m/z =365[M+1]⁺. ¹ H NMR (500 MHz, DMSO) δ 12.77 (s, 1H), 9.95 (s, 1H), 8.02 (s, 1H), 7.79 (s, 1H), 7.63 (dd, *J =* 8.8, 5.1 Hz, 2H), 7.43 (d, *J =* 8.6 Hz, 2H), 7.12 (t, *J =* 8.8 Hz, 2H), 6.95 (d, *J =* 8.6 Hz, 2H), 3.76 (d, *J =* 12.3 Hz, 2H), 2.71 (t, *J =* 11.5 Hz, 2H), 2.46 (s, 1H), 1.84 (dd, *J =* 38.3, 5.1 Hz, 2H). 7.4 Hz, 2H), 1.76 (dd, *J =* 12.0, 2.8 Hz, 2H).

### Example 51: Preparation of 1-(4-(1H-pyrazol-4-yl)phenyl)-N-(benzofuran-5-yl)piperidine-4-carboxamide (Compound 62) ( Prepared according to Scheme 8)

Step 51a: Preparation of N-(benzofuran-5-yl)-1-(4-(1-(tetrahydro-2H-pyran-2-yl)-1H-pyrazol-4-yl)phenyl)pipe ridine-4-carboxamide (Compound 0802-62): To a mixture of 1-(4-(1-(tetrahydro-2H-pyran-2-yl)-1H-pyrazol-4-yl)phenyl)piperidine-4-carboxylic acid (0801-55) (55 mg, 0.16 mmol, 1.0 eq.), HATU (77 mg, 0.20 mmol, 1.3 eq.) and N,N-diisopropylethylamine (60 mg, 0.47 mmol, 3.0 eq.) in dichloromethane (3 mL) was added benzofuran-5-amine (25 mg, 0.19 mmol, 1.2 eq.). The mixture was stirred at room temperature overnight. The solvent was removed under reduced pressure. The residue was purified by Pre-TLC (dichloromethane:ethyl acetate=4:1) to give white solid product N-(benzofuran-5-yl)-1-(4-(1-(tetrahydro-2H-pyran-2-yl)-1H-pyrazol-4-yl)phenyl)pipe ridine-4-carboxamide (41 mg, yield: 56%). LCMS (ESI): m/z =471[M+1]⁺; TLC: Rf 0.5 (dichloromethane:methanol=30:1).

Step 51b: Preparation of 1-(4-(1H-pyrazol-4-yl)phenyl)-N-(benzofuran-5-yl)piperidine-4-carboxamide (Compound 62): A mixture of N-(benzofuran-5-yl)-1-(4-(1-(tetrahydro-2H-pyran-2-yl)-1H-pyrazol-4-yl)phenyl)pipe ridine-4-carboxamide (41 mg, 0.087 mmol, 1.0 eq.) in hydrogen chloride-methanol solution (4M, 2.5 ml) was stirred at room temperature for 2 hours. The mixture was diluted with water (20 ml). Solid sodium carbonate was added to adjust the pH to 10. The mixture was filtered. The solid was washed with water and then dried under vacuum to give 1-(4-(1H-pyrazol-4-yl)phenyl)-N-(benzofuran-5-yl)piperidine-4-carboxamide (27 mg, yield: 80%) as a white solid. LCMS (ESI): m/z = 387 [M+1]⁺; TLC: Rf 0.5 (dichloromethane: methanol = 10: 1). ¹ H NMR (500 MHz, DMSO) δ 12.77 (s, 1H), 9.92 (s, 1H), 8.03 (s, 2H), 7.93 (s, 1H), 7.79 (s, 1H), 7.47 (dd, *J =* 33.0, 7.8 Hz, 4H), 7.09 - 6.81 (m, 3H), 3.77 (d, *J =* 11.7 Hz, 2H), 2.72 (t, *J =* 11.6 Hz, 2H), 2.53 (s, 1H), 1.97 - 1.74 (m, 4H).

### Example 52: Preparation of 1-(4-(1H-pyrazol-4-yl)phenyl)-N-(naphthalen-2-yl)piperidine-4-carboxamide (Compound 64) ( Prepared according to Scheme 8)

Step 52a: Preparation of N-(naphthalen-2-yl)-1-(4-(1-(tetrahydro-2H-pyran-2-yl)-1H-pyrazol-4-yl)phenyl)pipe ridine-4-carboxamide (Compound 0802-64): To a mixture of 1-(4-(1-(tetrahydro-2H-pyran-2-yl)-1H-pyrazol-4-yl)phenyl)piperidine-4-carboxylic acid (0801-55) (55 mg, 0.16 mmol, 1.0 eq.), HATU (77 mg, 0.20 mmol, 1.3 eq.) and N,N-diisopropylethylamine (60 mg, 0.47 mmol, 3.0 eq.) in dichloromethane (3 mL) was added naphthalen-2-amine (27 mg, 0.19 mmol, 1.2 eq.). The mixture was stirred at room temperature overnight. The solvent was removed under reduced pressure. The residue was purified by Pre-TLC (silica gel, dichloromethane:ethyl acetate=4:1) to give white solid product N-(naphthalene-2-yl)-1-(4-(1-(tetrahydro-2H-pyran-2-yl)-1H-pyrazol- 4 -yl)phenyl)piperidine-4-carboxamide (41 mg, yield: 55%). LCMS (ESI): m/z =481[M+1]⁺; TLC: Rf 0.5 (dichloromethane:methanol=30:1).

Step 52b: Preparation of 1-(4-(1H-pyrazol-4-yl)phenyl)-N-(naphthalene-2-yl)piperidine-4-carboxamide (Compound 64): A mixture of N-(naphthalene-2-yl)-1-(4-(1-(tetrahydro-2H-pyran-2-yl)-1H-pyrazol-4-yl)phenyl)pip eridine-4-carboxamide (41 mg, 0.085 mmol, 1.0 eq.) in hydrogen chloride-methanol solution (4M, 2.5 ml) was stirred at room temperature for 2 hours. The mixture was diluted with water (20 ml). Solid sodium carbonate was added to adjust the pH to 10. The mixture was filtered. The solid was washed with water and then dried under vacuum to give 1-(4-(1H-pyrazol-4-yl)phenyl)-N-(naphthalene-2-yl)piperidine-4-carboxamide (26 mg, yield: 77%) as a white solid. LCMS (ESI): m/z = 397 [M+1]⁺; TLC: Rf 0.5 (dichloromethane:methanol=10:1). ¹ H NMR (500 MHz, DMSO) δ 12.77 (s, 1H), 10.12 (s, 1H), 8.33 (s, 1H), 8.12 - 7.70 (m, 5H), 7.61 (d, *J =* 8.7 Hz, 1H), 7.54 - 7.31 (m, 4H), 6.97 (d, *J =* 8.5 Hz, 2H), 3.78 (d, *J =* 12.3 Hz, 2H), 2.73 (t, *J =* 11.6 Hz, 2H), 2.58 (t, *J =* 11.5 Hz, 1H), 1.99 - 1.74 (m, 4H).

### Example 53: Preparation of N-(1-(4-(1H-pyrazol-4-yl)phenyl)piperidin-4-yl)cyclopentanecarboxamide (Compound 69) (Prepared according to Scheme 5)

Step 53a: Preparation of tert-butyl 4-(cyclopentanecarboxamido)piperidine-1-carboxylate (Compound 0504-69): To a mixture of cyclopentanecarboxylic acid (188 mg, 1.6 mmol, 1.1 eq.), tert-butyl 4-aminopiperidine-1-carboxylate (0601-73) (300 mg, 1.5 mmol, 1.0 eq.) and N,N-diisopropylethylamine (579.7 mg, 4.49 mmol, 3.0 eq.) in 10 mL N,N -dimethylformamide was added HATU (626 mg, 1.6 mmol, 1.1 eq.). The mixture was stirred at room temperature for two hours. The reaction was diluted with ethyl acetate and the organic phase was washed with water and saturated brine. Then, the organic phase was concentrated under reduced pressure, and the residue was purified by column chromatography on silica gel ( eluent: petroleum ether/ethyl acetate = 1/1 to 1/3) to obtain white solid product tert-butyl 4-(cyclopentanecarboxamido)piperidine-1-carboxylate (420 mg, yield: 94.6%). LCMS (ESI): m/z = 297 (M+H)⁺.

Step 53b: Preparation of N-(piperidin-4-yl)cyclopentanecarboxamide hydrochloride (Compound 0505-69): tert-Butyl 4-(cyclopentanecarboxamido)piperidine-1-carboxylate (0504-69) (420 mg, 1.4 mmol, 1.0 eq.) was added to 5 mL 4M hydrogen chloride - dioxane solution. The mixture was stirred at room temperature for 2 hours. The mixture was concentrated to dryness under reduced pressure and the residue was used directly in the next step without further purification. LCMS (ESI): m/z = 197 (M+H)⁺.

Step 53c: Preparation of N-(1-(4-(1-(tetrahydro-2H-pyran-2-yl)-1H-pyrazol-4-yl)phenyl)piperidin-4-yl)cyclop entanecarboxamide (Compound 0506-69): Under nitrogen protection, a mixture of N-(piperidin-4-yl)cyclopentanecarboxamide hydrochloride (0505-69) (200 mg, 0.67 mmol, 1.0 eq.) and sodium tert-butoxide (389.2 mg, 4.05 mmol, 6.0 eq.) in toluene (10 ml) was stirred at 120 °C for 30 minutes, and then 4-(4-bromophenyl)-1-(tetrahydro-2H-pyran-2-yl)-1H-pyrazole (0103-1) (227.4 mg, 0.74 mmol, 1.1 eq.), 4,5-bis(diphenylphosphino)-9,9-dimethylxanthene (38.8 mg, 0.068 mmol, 0.1 eq.) and tris(dibenzylideneacetone)dipalladium (30.9 mg, 0.034 mmol, 0.05 eq.) were added to the mixed solution and stirred at 120 °C overnight. After cooling to room temperature, the reaction was filtered through celite and the filter cake was washed with ethyl acetate. The filtrate was concentrated under reduced pressure and the residue was purified by prep-TLC (silica gel, eluent: dichloromethane/methanol = 15/1) to obtain a yellow solid product N-((1-(4-(1-(tetrahydro-2H-pyran-2-yl)-1H-pyrazol-4-yl)phenyl)piperidin-4-yl)methy l)cyclopentanecarboxamide (78.9 mg, yield: 27.7%). LCMS (ESI): m/z =423 (M+H)⁺.

Step 53d: Preparation of N-(1-(4-(1H-pyrazol-4-yl)phenyl)piperidin-4-yl)cyclopentanecarboxamide (Compound 69): N - ((1-(4-(1-(tetrahydro- 2H -pyran-2-yl)-1H - pyrazol-4-yl)phenyl)piperidin-4-yl)methyl)cyclopentanecarboxamide (0506-69) (78.9 mg, 0.19 mmol, 1.0 eq.) was added to 10 mL 4M hydrogen chloride - methanol solution and stirred at room temperature for 2 hours. The mixture was concentrated to dryness under reduced pressure. Water was added and the pH was adjusted to 10 with saturated aqueous sodium carbonate. Ethyl acetate was added to extract, washed with water, and the organic phase was dried over anhydrous sodium sulfate, concentrated under reduced pressure. The residue was purified by prep-TLC (silica gel, eluent: dichloromethane/methanol = 10/1) to give a yellow solid product N-(1-(4-(1H-pyrazol-4-yl)phenyl)piperidin-4-yl)cyclopentanecarboxamide (25 mg, yield: 39.8%). LCMS(ESI): m/z =339 (M+H)⁺. ¹H NMR (500 MHz, DMSO) δ 12.75 (s, 1H), 7.89 (s, 2H), 7.67 (d, *J =* 7.6 Hz, 1H), 7.41 (d, *J =* 8.6 Hz, 2H), 6.92 (d, *J =* 8.7 Hz, 2H), 3.72 - 3.69 (m, 1H), 3.62 (d, *J =* 12.6 Hz, 2H), 3.51 (s, 1H), 2.76 (t, *J =* 11.3 Hz, 2H), 1.79 (d, *J =* 10.9 Hz, 2H), 1.73 - 1.66 (m, 2H), 1.6 J = 11.6 Hz, 4H), 1.48 (q, *J =* 11.8 Hz, 4H).

### Example 54: Preparation of (4-(4-(1H-pyrazol-4-yl)phenyl)piperidin-1-yl)(cyclopentyl)methanone (Compound 70) (Prepared according to Scheme 9)

Step 54a: Preparation of tert-butyl 4-(4-(1-(tetrahydro-2H-pyran-2-yl)-1H-pyrazol-4-yl)phenyl)-3,6-dihydropyridine-1(2 H)-carboxylate (Compound 0902-70): Under nitrogen protection, tert-butyl 4-(4,4,5,5-tetramethyl-1,3,2-dioxaborolan-2-yl)-3,6-dihydropyridine-1(2H)-carboxyla te (0901-70) (500 mg, 1.62 mmol, 1. 0 eq.), 4-(4-bromophenyl)-1-(tetrahydro-2H-pyran-2-yl)-1H-pyrazole (0103-1) (544.3 mg, 1.78 mmol, 1.1 eq.), 1,1'-bis(diphenylphosphino)ferrocenepalladium dichloride (II) (118.3 mg, 0.16 mmol, 0.1 eq.), sodium carbonate (514 mg, 4.85 mmol, 3.0 eq.) were added to a mixed solvent of dioxane/water (30 ml/3 ml) and stirred at 85 °C overnight. The reaction was then cooled to room temperature and diluted with water and extracted with ethyl acetate. The organic layer was washed with brine, dried over anhydrous sodium sulfate, and concentrated under reduced pressure. The residue was purified by column chromatography on silica gel (petroleum ether/ethyl acetate = 10/1 to 5/1) to give a white solid product tert-butyl 4-(4-(1-(tetrahydro-2H-pyran-2-yl)-1H-pyrazol-4-yl)phenyl)-3,6-dihydropyridine-1(2 H)-carboxylate (416.8 mg, yield: 62.9%). LCMS (ESI): m/z = 410 [M+1]⁺.

Step 54b: Preparation of tert-butyl 4-(4-(1-(tetrahydro-2H-pyran-2-yl)-1H-pyrazol-4-yl)phenyl)piperidine-1-carboxylate (Compound 0903-70): Under a hydrogen atmosphere, a mixture of tert-butyl 4-(4-(1-(tetrahydro-2H-pyran-2-yl)-1H-pyrazol-4-yl)phenyl)-3,6-dihydropyridine-1(2 H)-carboxylate (0902-70) (416.8 mg, 1.0 mmol, 1.0 eq.) and palladium/carbon (64.2 mg, 15% mass ratio) in methanol (5 ml) was stirred at room temperature overnight. The reaction was filtered and concentrated to dryness under reduced pressure. The residue was purified by column chromatography on silica gel (petroleum ether/ethyl acetate = 10/1) to obtain white solid product tert-butyl 4-(4-(1-(tetrahydro-2H-pyran-2-yl)-1H-pyrazol-4-yl)phenyl)piperidine-1-carboxylate (284.9 mg, yield: 66.3%). LCMS (ESI): m/z = 412 (M+H)⁺.

Step 54c: Preparation of 4-(4-(1H-pyrazol-4-yl)phenyl)piperidine hydrochloride (Compound 0904-70): A mixture of tert-butyl 4-(4-(1-(tetrahydro-2H-pyran-2-yl)-1H-pyrazol-4-yl)phenyl)piperidine-1-carboxylate (284 mg, 0.69 mmol, 1.0 eq.) in hydrogen chloride -dioxane solution ( 4 M, 5 ml) was stirred at room temperature for one hour. The solvent was removed under reduced pressure, the residue was diluted with water, adjusted to pH = 9 with sodium carbonate solution, and extracted with dichloromethane, the organic phase was dried and concentrated under reduced pressure, and the residue was used directly in the next step without further purification. LCMS (ESI): m/z = 228 [M+1]⁺.

Step 54d: Preparation of (4-(4-(1H-pyrazol-4-yl)phenyl)piperidin-1-yl)(cyclopentyl)methanone (Compound 70): To a mixture of cyclopentanecarboxylic acid (25 mg, 0.2 mmol, 1.1 eq.), 4-(4-(1H-pyrazol-4-yl)phenyl)piperidine hydrochloride (0904-70) (62 mg, 0.2 mmol, 1.0 eq.), N,N-diisopropylethylamine (77.04 mg, 0.60 mmol, 3.0 eq.) in 10 mL N,N -dimethylformamide was added HATU (83.2 mg, 0.22 mmol, 1.1 eq.). The mixture was stirred at room temperature for two hours. The reaction was diluted with ethyl acetate, and the organic phase was washed with water and saturated brine. Then, the organic phase was concentrated under reduced pressure, and the residue was purified by prep-TLC (silica gel, eluent: petroleum ether/ethyl acetate = 1/3) to give a white solid (4-(4-(1H-pyrazol-4-yl)phenyl)piperidin-1-yl)(cyclopentyl)methanone (28 mg, yield: 31.9%). LCMS (ESI): m/z = 324 (M+H)⁺. ¹ H NMR (500 MHz, DMSO) δ 12.86 (s, 1H), 8.11 (s, 1H), 7.87 (s, 1H), 7.51 (d, *J =* 8.1 Hz, 2H), 7.21 (d, *J =* 8.1 Hz, 2H), 4.56 (d, *J =* 12.0 Hz, 1H), 4.09 (d, *J =* 13.2 Hz, 1H), 3.09 (t, *J =* 12.5 Hz, 1H), 3.01 (p, *J =* 7.9 Hz, 1H), 2.75 (t, *J =* 12.0 Hz, 1H), 2.59 (t, *J =* 12.3 Hz, 1H), 1.83 - 1.43 (m, 12H).

### Example 55: Preparation of (4-(2,3-difluoro-4-(1H-pyrazol-4-yl)phenyl)piperidin-1-yl)(pyrrolidin-1-yl)metha none (Compound 71) (Prepared according to Scheme 9)

Step 55a: Preparation of tert-butyl 4-(4-bromo-2,3-difluorophenyl)-3,6-dihydropyridine-1(2H)-carboxylate (Compound 0905-71): Under nitrogen protection, tert- butyl 4-(4,4, 5, 5-tetramethyl-1,3,2-dioxaborolan-2-yl)-3,6-dihydropyridine-1 (2H)-carboxyla te (0901-70) (378 mg, 1.22 mmol, 1 eq.), 1,4-dibromo-2,3-difluorobenzene (400 mg, 1.44 mmol, 1.2 eq.), sodium carbonate (256 mg, 2.4 mmol, 2 eq.), 1,1'-bis(diphenylphosphino)ferrocenepalladium dichloride (II) (44 mg, 0.12 mmol, 0.05 eq.) in 1,4-dioxane (8 ml) and water (0.8 ml) were mixed and stirred at 90 °C for 5 hours. The reaction was diluted with water and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 3/1) to give yellow solid product tert-butyl 4-(4-bromo-2,3-difluorophenyl)-3,6-dihydropyridine-1(2H)-carboxylate (240 mg, yield: 52.7%). LCMS (ESI) [M+1]⁺ : m/z =374.

Step 55b: Preparation of tert-butyl 4-(4-bromo-2,3-difluorophenyl)piperidine-1-carboxylate (Compound 0906-71): Under a hydrogen atmosphere, tert-butyl 4-(4-bromo-2,3-difluorophenyl)-3,6-dihydropyridine-1(2H)-carboxylate (0905-71) (212 mg, 0.57 mmol, 1.0 eq.), platinum dioxide (13 mg, 0.057 mmol, 0.1 eq.), ethanol (2 ml) and acetic acid (2 ml) were stirred at room temperature for 1 hour. The mixture was filtered, and the filtrate was concentrated under reduced pressure. The residue was adjusted to pH = 9 with sodium carbonate solution, and extracted with dichloromethane. The organic phase was dried and concentrated under reduced pressure to obtain a pale yellow solid crude product tert-butyl 4-(4-bromo-2,3-difluorophenyl)piperidine-1-carboxylate (212 mg, yield: 99.1%) LCMS (ESI): m/z = 376 [M+1]⁺.

Step 55c: Preparation of 4-(4-bromo-2,3-difluorophenyl)piperidine (Compound 0907-71): A mixture of tert-butyl 4-(4-bromo-2,3-difluorophenyl)piperidine-1-carboxylate (0906-71) (212 mg, 0.57 mmol, 1.0 eq.) in hydrogen chloride-methanol solution (4M, 4 ml) was stirred at room temperature for 2 hours. The solvent was removed under reduced pressure, and the residue was diluted with water. The pH was adjusted to 9 with sodium carbonate solution, and extracted with dichloromethane. The organic phase was dried and concentrated under reduced pressure to obtain a yellow solid crude product 4-(4-bromo-2,3-difluorophenyl)piperidine (150 mg, yield: 95.7%). LCMS (ESI): m/z = 276 [M+1]⁺.

Step 55d: Preparation of (4-(4-bromo-2,3-difluorophenyl)piperidin-1-yl)(pyrrolidin-1-yl)methanone (Compound 0908-71): 1-pyrrolidinecarbonyl chloride (88 mg, 0.65 mmol, 1.5 eq.) was added dropwise to a mixture of 4-(4-bromo-2,3-difluorophenyl)piperidine (0907-71) (120 mg, 0.44 mmol, 1 eq.), triethylamine (178 mg, 1.76 mmol, 4 eq.) in dichloromethane (2 ml), and the mixture was stirred at room temperature for 1 hour. The reaction was diluted with water and extracted with dichloromethane. The organic layer was dried over anhydrous sodium sulfate and concentrated under reduced pressure. The residue was purified by column chromatography on silica gel (dichloromethane/methanol = 60/1) to give a colorless oil (4-(4-bromo-2,3-difluorophenyl)piperidin-1-yl)(pyrrolidin-1-yl)methanone (137 mg, yield: 83.7%). LCMS (ESI): m/z = 373 [M+1]⁺.

Step 55e: Preparation of (4-(2,3-difluoro-4-(1H-pyrazol-4-yl)phenyl)piperidin-1-yl)(pyrrolidin-1-yl)methanone (Compound 71): Under nitrogen protection, (4-(4-bromo-2,3-difluorophenyl)piperidin-1-yl)(pyrrolidin-1-yl)methanone (0908-71) (137 mg, 0.37 mmol, 1 eq.), 1-methylpyrazole-4-boronic acid pinacol ester (162 mg, 0.55 mmol, 1.5 eq.), sodium carbonate (78 mg, 0.74 mmol, 2 eq.), 1,1'-bis(diphenylphosphino)ferrocenepalladium(II) dichloride (26 mg, 0.037 mmol, 0.1 eq.), acetonitrile (4 ml) and water (0.8 ml) were mixed and stirred at 90 °C for 6 hours. The reaction was diluted with water and extracted with ethyl acetate. The organic layer was washed with saturated brine, dried over anhydrous sodium sulfate, and concentrated under reduced pressure. The residue was purified by thick preparation plate (silica gel, dichloromethane/methanol = 30/1) to give a yellow solid product (4-(2,3-difluoro-4-(1H-pyrazol-4-yl)phenyl)piperidin-1-yl)(pyrrolidin-1-yl)methanone (69 mg, yield: 52.2%). LCMS (ESI): m/z = 361 [M+1]⁺. ¹ H NMR (500 MHz, DMSO) δ 13.13 (s, 1H), 8.17 (s, 1H), 7.94 (s, 1H), 7.48 (t, *J =* 7.1 Hz, 1H), 7.13 (t, *J =* 6.9 Hz, 1H), 3.78 (d, *J =* 12.6 Hz, 2H), 3.29 - 3.22 (m, 4H), 3.00 (t, *J =* 11.6 Hz, 1H), 2.80 (t, *J =* 12.2 Hz, 2H), 1.89 - 1.57 (m, 8H).

### Example 56: Preparation of (4-(2,3-difluoro-4-(1H-pyrazol-4-yl)phenyl)piperazin-1-yl)(pyrrolidin-1-yl)metha none (Compound 72) (Prepared according to Scheme 10)

Step 56a: Preparation of tert-butyl 4-(pyrrolidine-1-carbonyl)piperazine-1-carboxylate (Compound 1002-72): Under nitrogen protection, tert - butyl piperazine-1-carboxylate (500 mg, 2.7 mmol, 1 eq.), N,N-carbonyldiimidazole (650 mg, 4.0 mmol, 1.5 eq.), pyridine (2.1 mL, 22.7 mmol, 10 eq.) in tetrahydrofuran (8 mL) were mixed and stirred for 30 minutes at 0 °C. Pyrrolidine (960 mg, 13.5 mmol, 5 eq.) was then added, and the reaction was warmed to room temperature and stirred for 2 hours. The reaction was quenched with water, and pyridine was washed off with aqueous hydrochloric acid, and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 3/1) to give white solid product tert-butyl 4-(pyrrolidine-1-carbonyl)piperazine-1-carboxylate (167 mg, yield: 21.9%). LCMS (ESI) [M+1]⁺ : m/z = 284.

Step 56b: Preparation of piperazin-1-yl(pyrrolidin-1-yl)methanone (Compound 1003-72): A mixture of tert-butyl 4-(pyrrolidine-1-carbonyl)piperazine-1-carboxylate (1002-72) (167 mg, 0.59 mmol, 1 eq.) in hydrogen chloride -methanol solution (4 M, 4 ml) was stirred at room temperature for 2 hours. The solvent was removed under reduced pressure, and the residue was diluted with water. The pH was adjusted to 9 with sodium carbonate solution, and extracted with dichloromethane. The organic layer was dried over anhydrous sodium sulfate, and concentrated under reduced pressure to obtain a yellow solid ( 89 mg, yield: 82.4%). LCMS (ESI): m/z = 184 [M+1]⁺.

Step 56c: Preparation of (4-(2,3-difluoro-4-(1-(tetrahydro- 2H -pyran-2-yl)-1H - pyrazol-4-yl)phenyl)piperazin-1-yl)(pyrrolidin-1-yl)methanone (Compound 1004-72): Under nitrogen protection, piperazin-1-yl(pyrrolidin-1-yl)methanone (1003-72) (69 mg, 0.31 mmol, 1 eq.), 4-(4-bromo-2,3-difluorophenyl)-1-(tetrahydro-2H-pyran-2-yl)-1H-pyrazole (0103-4) (161 mg, 0.47 mmol, 1.5 eq.), sodium tert-butoxide (119 mg, 1.24 mmol, 4 eq.), 4,5-bis(diphenylphosphino)-9,9-dimethylxanthene (36 mg, 0.062 mmol, 0.2 eq.), tris(dibenzylideneacetone)dipalladium (28 mg, 0.031 mmol, 0.1 eq.), toluene (4 ml) were mixed and stirred overnight at 120°C. The reaction was filtered and the filtrate was concentrated under reduced pressure. The residue was purified by thick silica gel preparation (silica gel, dichloromethane/methanol = 30/1) to give a yellow solid product (4-(2,3-difluoro-4-(1-(tetrahydro-2H-pyran-2-yl)-1H-pyrazol-4-yl)phenyl)piperazin-1-yl)(pyrrolidin-1-yl)methanone (77 mg, yield: 55.8%). LCMS (ESI): m/z = 446 [M+1]⁺.

Step 56d: Preparation of (4-(2,3-difluoro-4-(1H - pyrazol-4-yl)phenyl)piperazin-1-yl)(pyrrolidin-1-yl)methanone (Compound 72): A mixture of (4-(2,3-difluoro-4-(1-(tetrahydro- 2H -pyran-2-yl)-1H - pyrazol-4-yl)phenyl)piperazin-1-yl)(pyrrolidin-1-yl)methanone (1004-72) ( 77 mg, 0.17 mmol, 1.0 eq.) in hydrogen chloride- methanol solution (4 M, 2 mL) was stirred at room temperature for 2 hours. The solvent was removed under reduced pressure, and the residue was diluted with water. The pH was adjusted to 9 with sodium carbonate solution, and extracted with dichloromethane. The organic phase was dried and concentrated under reduced pressure. The residue was purified by prep-TLC (silica gel, dichloromethane/methanol = 10/1) to give a white solid product (4-(2,3-difluoro-4-(1H-pyrazol-4-yl)phenyl)piperazin-1-yl)(pyrrolidin-1-yl)methanon e (45 mg, yield: 73.3%). LCMS (ESI): m/z = 362 [M+1]⁺. ¹ H NMR (500 MHz, DMSO) δ 13.06 (s, 1H), 8.10 (s, 1H), 7.89 (s, 1H), 7.41 (t, *J =* 7.8 Hz, 1H), 6.87 (t, *J* = 8.1 Hz, 1H), 3.32 (d, *J =* 4.7 Hz, 6H), 3.04 (s, 4H), 2.50 (s, 2H), 1.76 (s, 4H).

### Example 57: Preparation of 1-((1-(2-fluoro-4-(1H-pyrazol-3-yl)phenyl)piperidin-4-yl)methyl)pyrrolidin-2-one (Compound 76) (Prepared according to Scheme 1)

Step 57a: Preparation of 3-(4-bromo-3-fluorophenyl)-1-(tetrahydro-2H-pyran-2-yl)-1H-pyrazole (Compound 0111-76): To a mixture of 3-iodo-1-(tetrahydro-2H-pyran-2-yl)-1H-pyrazole (318 mg, 1.14 mmol, 1.0 eq.), 1,1'-bis(diphenylphosphino)ferrocenepalladium(II) chloride (65 mg, 0.08 mmol, 0.07 eq.), sodium carbonate (363 mg, 3.42 mmol, 3.0 eq.) in dioxane (5 mL) and water (1 mL) was added (4-bromo-3-fluorophenyl)boronic acid (250 mg, 1.14 mmol, 1.0 eq.). The mixture was heated to 85 °C under nitrogen atmosphere for 3 hours. The solvent was removed under reduced pressure. The residue was subjected to column chromatography on silica gel (petroleum ether: ethyl acetate 10: 1 to 8: 1) to give 3-(4-bromo-3-fluorophenyl)-1-(tetrahydro-2H-pyran-2-yl)-1H-pyrazole (340 mg, yield: 92%) as a pale yellow oil. LCMS (ESI): m/z = 325 [M+1]⁺; TLC: Rf 0.5 (petroleum ether: ethyl acetate = 5: 1).

Step 57b: Preparation of 1-((1-(2-fluoro-4-(1-(tetrahydro-2H-pyran-2-yl)-1H-pyrazol-3-yl)phenyl)piperidin-4-yl)methyl)pyrrolidin-2-one (Compound 0112-76): 1-(Piperidin-4-ylmethyl)pyrrolidin-2-one (101 mg, 0.55 mmol, 1.2 eq.) was added to a mixture of 3-(4-bromo-3-fluorophenyl)-1-(tetrahydro-2H-pyran-2-yl)-1H-pyrazole (0111-76) (150 mg, 0.46 mmol, 1.0 eq.), tris(dibenzylideneacetone)dipalladium (21 mg, 0.023 mmol, 0.05 eq.), 4,5-bis(diphenylphosphino)-9,9-dimethylxanthene (26.6 mg, 0.046 mmol, 012 eq.), sodium tert-butoxide (111 mg, 1.15 mmol, 2.5 eq.) in toluene (6 ml). The mixture was heated to 120°C under nitrogen atmosphere and reacted overnight. The mixture was diluted with water (20 ml). The aqueous layer was extracted with ethyl acetate (15 ml×3). The combined organic layer was washed with saturated brine (20 ml × 1), dried over anhydrous sodium sulfate and concentrated. The residue was purified by Pre-TLC (silica gel, dichloromethane: methanol = 15: 1) to give a pale yellow solid product 1-((1-(2-fluoro-4-(1-(tetrahydro-2H-pyran-2-yl)-1H-pyrazol-3-yl)phenyl)piperidin-4-yl)methyl)pyrrolidin-2-one (30 mg, yield: 15%). LCMS (ESI): m/z = 427 [M+1]⁺; TLC: Rf 0.5 (dichloromethane: methanol = 15: 1).

Step 57c: Preparation of 1-((1-(2-fluoro-4-(1H-pyrazol-3-yl)phenyl)piperidin-4-yl)methyl)pyrrolidin-2-one (Compound 76): A mixture of 1-((1-(2-fluoro-4-(1-(tetrahydro-2H-pyran-2-yl)-1H-pyrazol-3-yl)phenyl)piperidin-4-yl)methyl)pyrrolidin-2-one (0112-76) (30 mg, 0.07 mmol, 1.0 eq.) in hydrogen chloride-methanol solution (4M, 2.5 ml) was stirred at room temperature for 1.5 hours. The mixture was diluted with water (15 ml). Solid sodium carbonate was added to adjust the pH to 10, and then the aqueous layer was extracted with dichloromethane (15 ml×3). The combined organic layer was washed with saturated brine (15 ml×1), dried over anhydrous sodium sulfate and concentrated. The residue was purified by Pre-TLC (silica gel, dichloromethane: methanol = 10: 1) to give 1-((1-(2-fluoro-4-(1H-pyrazol-3-yl)phenyl)piperidin-4-yl)methyl)pyrrolidin-2-one (17 mg, yield: 71%) as a white solid. LCMS (ESI): m/z = 343 [M+1]⁺; TLC: Rf 0.5 (dichloromethane: methanol = 10: 1). ¹ H NMR (500 MHz, MeOD) δ 7.61 (s, 1H), 7.43 (d, *J =* 15.2 Hz, 2H), 7.05 (t, *J =* 8.6 Hz, 1H), 6.58 (s, 1H), 3.48 (dd, *J =* 18.1, 10.9 Hz, 4H), 3.22 (t, *J =* 8.9 Hz, 2H), 2.69 (t, *J =* 11.3 Hz, 2H), 2.39 (t, *J =* 8.1 Hz, 2H), 2.13 - 1.95 (m, 2H), 1.79 (ddd, *J* = 31.7, 17.6, 8.5 Hz, 3H), 1.50 - 1.36 (m, 2H).

### Example 58: Preparation of 1-((1-(2,3-difluoro-4-(1H-pyrazol-3-yl)phenyl)piperidin-4-yl)methyl)pyrrolidin-2-one (Compound 77)

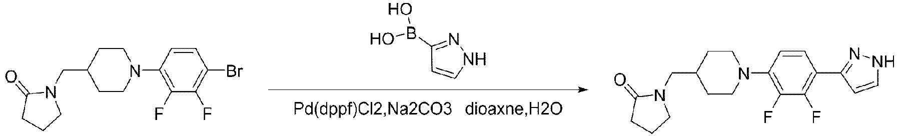

Under nitrogen protection, 1-((1-(4-bromo-2,3-difluorophenyl)piperidin-4-yl)methyl)pyrrolidin-2-one (100 mg, 0.27 mmol, 1.0 eq.), (1H-pyrazol-3-yl)boronic acid (61 mg, 0.54 mmol, 2.0 eq.), sodium carbonate (86 mg, 0.81 mmol, 2.0 eq.), 1,1'-bis(diphenylphosphino)ferrocenepalladium(II) chloride (14 mg, 0.016 mmol, 0.06 eq.) in 1,4-dioxane (5 ml) and water (1 ml) were mixed and stirred overnight at 90°C. The reaction was diluted with water and extracted with ethyl acetate. The organic layer was dried and concentrated under reduced pressure. The residue was purified by thick prep plate (silica gel, dichloromethane/methanol = 15/1) to give 1-((1-(2,3-difluoro-4-(1H-pyrazol-3-yl)phenyl)piperidin-4-yl)methyl)pyrrolidin-2-one (26 mg, yield: 26.8%) as a white solid. LCMS (ESI) [M+1]⁺: m/z = 361. ¹ H NMR (500 MHz, DMSO) δ 13.01 (s, 1H), 7.81 (s, 1H), 7.60 (s, 1H), 6.89 (s, 1H), 6.57 (s, 1H), 3.43 (d, *J =* 10.7 Hz, 2H), 3.36 (t, *J =* 6.9 Hz, 2H), 3.11 (d, *J =* 7.2 Hz, 2H), 2.72 (t, *J =* 11.2 Hz, 2H), 2.23 (t, *J =* 8.0 Hz, 2H), 2.02 - 1.89 (m, 2H), 1.82 - 1.62 (m, 3H), 1.39 - 1.27 (m, 2H).

### Example 59: Preparation of (4-(2,3-difluoro-4-(1H-pyrazol-4-yl)phenyl)piperazin-1-yl)(piperidin-1-yl)methan one (Compound 88) (Prepared according to Scheme 10)

Step 59a: Preparation of tert-butyl 4-(piperidine-1-carbonyl)piperazine-1-carboxylate (Compound 1002-88): Under nitrogen protection, a mixture of tert-butyl piperazine-1-carboxylate (200 mg, 1.36 mmol, 1.0 eq.), piperidine-1-carbonyl chloride (278 mg, 1.49 mmol, 1.1 eq.), triethylamine (206 mg, 2.03 mmol, 1.5 eq.) in dichloromethane (5 ml) was stirred at room temperature for 1 hour. The reaction was diluted with water and extracted with dichloromethane. The organic layer was dried and concentrated under reduced pressure to give tert-butyl 4-(piperidine-1-carbonyl)piperazine-1-carboxylate (380 mg, crude) as a white solid. LCMS (ESI): m/z = 298 [M+1]⁺.

Step 59b: Preparation of piperazin-1-yl(piperidin-1-yl)methanone (Compound 1003-88): A mixture of tert-butyl 4-(piperidine-1-carbonyl)piperazine-1-carboxylate (1002-88) (380 mg, 1.28 mmol, 1.0 eq.) in hydrogen chloride-methanol solution (4 M, 5 ml) was stirred at room temperature for 1.0 h. The solvent was removed under reduced pressure, and the residue was diluted with water and adjusted to pH = 8 with sodium carbonate solution. The mixture was extracted with dichloromethane. The organic layer was dried and concentrated under reduced pressure to give the product, piperazin-1-yl(piperidin-1-yl)methanone (173 mg, crude) as a yellow solid. LCMS (ESI): m/z = 198 [M+1]⁺.

Step 59c: Preparation of (4-(2,3-difluoro-4-(1-(tetrahydro-2H-pyran-2-yl)-1H-pyrazol-4-yl)phenyl)piperazin-1-yl)(piperidin-1-yl)methanone (Compound 1004-88): Under nitrogen protection, a mixture of piperazin-1-yl(piperidin-1-yl)methanone (1003-88) (80 mg, 0.404 mmol, 1.0 eq.), 4-(4-bromo-2,3-difluorophenyl)-1-(tetrahydro-2H-pyran-2-yl)-1H-pyrazole (0103-4) (139 mg, 0.404 mmol, 1.0 eq.), sodium tert-butoxide (117 mg, 1.21 mmol, 3.0 eq.), 4,5-bis(diphenylphosphino-9,9-dimethylxanthene) (24 mg, 0.04 mmol, 0.1 eq.), tris(dibenzylideneacetone)dipalladium (19 mg, 0.02 mmol, 0.05 eq.) in toluene (5 ml) was stirred at 120°C overnight. The reaction was diluted with water and extracted with ethyl acetate, and the organic phase was dried and concentrated under reduced pressure. The residue was purified by column chromatography on silica gel (petroleum ether/ethyl acetate = 1/1) to obtain a yellow solid product (4-(2,3-difluoro-4-(1-(tetrahydro-2H-pyran-2-yl)-1H-pyrazol-4-yl)phenyl)piperazin-1-yl)(piperidin-1-yl)methanone (70 mg, yield: 37.63%). LCMS (ESI): m/z = 460 [M+1]⁺.

Step 59d: Preparation of (4-(2,3-difluoro-4-(1H-pyrazol-4-yl)phenyl)piperazin-1-yl)(piperidin-1-yl)methanone (Compound 88): A mixture of ( 4-(2,3-difluoro-4-(1-(tetrahydro-2H-pyran-2-yl)-1H-pyrazol-4-yl)phenyl)piperazin-1 -yl)(piperidin-1-yl)methanone (1004-88) (70 mg, 0.15 mmol, 1.0 eq.) in hydrogen chloride - methanol solution (4 M, 3 mL) was stirred at room temperature for 1 hour. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 15/1) to give a yellow solid product (4-(2,3-difluoro-4-(1H-pyrazol-4-yl)phenyl)piperazin-1-yl)(piperidin-1-yl)methanone (30 mg, yield: 52.63%). LCMS (ESI): m/z = 376 [M+1]⁺. ¹ H NMR (500 MHz, DMSO) δ 13.06 (s, 1H), 8.11 (s, 1H), 7.88 (s, 1H), 7.41 (t, *J =* 7.8 Hz, 1H), 6.87 (t, *J* = 8.2 Hz, 1H), 3.27 (s, 4H), 3.15 (d, *J =* 5.0 Hz, 4H), 3.04 (d, *J =* 4.3 Hz, 4H), 1.51 (dd, *J =* 25.7, 3.8 Hz, 6H).

### Example 60: Preparation of N-cyclopentyl-4-(2,3-difluoro-4-(1H-pyrazol-4-yl)phenyl)piperazine-1-carboxami de (Compound 89)

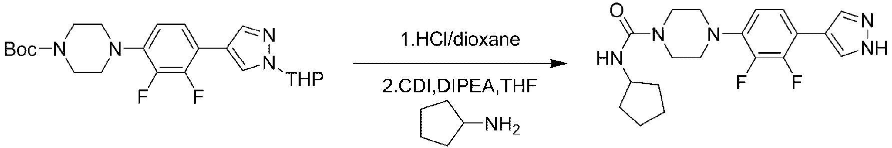

A mixture of tert-butyl 4-(2,3-difluoro-4-(1-(tetrahydro-2H-pyran-2-yl)-1H-pyrazol-4-yl)phenyl)piperazine-1 -carboxylate (156 mg, 0.35 mmol, 1.0 eq.) in hydrogen chloride-dioxane solution (4 M, 5 ml) was stirred at room temperature for 1.0 hour. The mixture was concentrated to obtain a concentrate for later use. N,N-carbonyldiimidazole (68 mg, 0.42 mmol, 1.2 eq.) was added to a mixture of cyclopentylamine (30 mg, 0.35 mmol, 1.0 eq.) and N,N-diisopropylethylamine ( 225 mg, 1.75 mmol, 5.0 eq.) in tetrahydrofuran (5 ml), and the mixture was stirred at room temperature for one hour. The previous concentrate was then added and stirred at room temperature for another hour. The reaction was quenched with water. The resulting reaction mixture was extracted with ethyl acetate and washed with saturated brine. The organic phase was concentrated, and the residue was purified by thick preparative thin layer silica gel plate (eluent: dichloromethane/methanol = 15/1) to give a white solid product N-cyclopentyl-4-(2,3-difluoro-4-(1H-pyrazol-4-yl)phenyl)piperazine-1-carboxamide (35 mg, yield: 26.51%). LCMS (ESI): m/z = 376 [M+1]⁺. ¹ H NMR (500 MHz, DMSO) δ 13.06 (s, 1H), 8.10 (s, 1H), 7.88 (s, 1H), 7.41 (t, *J =* 8.0 Hz, 1H), 6.87 (t, *J* = 8.2 Hz, 1H), 6.30 (d, *J =* 6.7 Hz, 1H), 3.92 (dd, *J =* 14.1, 7.1 Hz, 1H), 3.44 (d, *J*= 4.5 Hz, 4H), 2.99 (s, 4H), 1.84 - 1.76 (m, 2H), 1.63 (s, 2H), 1.51 - 1.33 (m, 4H).

### Example 61: Preparation of (4-(2,3-difluoro-4-(1H-pyrazol-4-yl)phenyl)-3,6-dihydropyridin-1(2H)-yl)(pyrroli din-1-yl)methanone (Compound 91) (Prepared according to Scheme 9)

Step 61a: Preparation of tert-butyl 4-(2,3-difluoro-4-(1-(tetrahydro-2H-pyran-2-yl)-1H-pyrazol-4-yl)phenyl)-3,6-dihydro pyridine-1(2H)-carboxylate (Compound 0902-91): Under nitrogen atmosphere, 4-(4-bromo-2,3-difluorophenyl)-1-(tetrahydro-2H-pyran-2-yl)-1H-pyrazole (0103-4) (340 mg, 1 mmol, 1.0 eq.), tert-butyl 4-(4,4, 5, 5-tetramethyl-1,3,2-dioxaborolan-2-yl)-3,6-dihydropyridine-1 (2H)-carboxyla te (340 mg, 1.1 mmol, 1.1 eq.), 1,1'-bis(diphenylphosphino)ferrocenepalladium(II) chloride (73 mg, 0.1 mmol, 0.1 eq.) and sodium carbonate (318 mg, 3 mmol, 3.0 eq.) were added to a mixed solvent of dioxane/water = 10/1 (11 ml), and the mixture was stirred at 90°C for 3 hours. The reaction was quenched with water and extracted with ethyl acetate. The organic layer was washed with saturated brine and concentrated under reduced pressure. The residue was purified by column chromatography on silica gel (petroleum ether/ethyl acetate = 5/1 to 2/1) to obtain a yellow oily product tert-butyl 4-(2,3-difluoro-4-(1-(tetrahydro-2H-pyran-2-yl)-1H-pyrazol-4-yl)phenyl)-3,6-dihydro pyridine-1(2H)-carboxylate (350 mg, yield: 79.18%). LCMS (ESI): m/z = 446 [M+1]⁺.

Step 61b: Preparation of (4-(2,3-difluoro-4-(1H-pyrazol-4-yl)phenyl)-3,6-dihydropyridin-1(2H)-yl)(pyrrolidin-1-yl)methanone (Compound 91): A mixture of tert-butyl 4-(2,3-difluoro-4-(1-(tetrahydro-2H-pyran-2-yl)-1H-pyrazol-4-yl)phenyl)-3,6-dihydro pyridine-1(2H)-carboxylate (0902-91) (88 mg, 0.2 mmol, 1.0 eq.) in hydrogen chloride - dioxane solution(4 M, 5 mL) was stirred at room temperature for 1.0 h. The mixture was concentrated and dissolved in tetrahydrofuran. Pyrrolidine-1-carbonyl chloride ( 20 mg, 0.15 mmol, 0.8 eq.) and N,N-diisopropylethylamine ( 98 mg, 0.76 mmol, 4.0 eq.) were added to the solution. The mixture was stirred at room temperature for one hour. The reaction was quenched with water. The mixture was extracted with ethyl acetate and washed with saturated brine. The organic phase was concentrated and the residue was purified by thick preparative thin layer silica gel plate (eluent: dichloromethane/methanol = 15/1) to obtain a white solid product (4-(2,3-difluoro-4-(1H-pyrazol-4-yl)phenyl)-3,6-dihydropyridine-1(2H)-yl)(pyrrolidin -1-yl)methanone (30 mg, yield: 42.25%). LCMS (ESI): m/z =359[M+1]⁺. ¹ H NMR (500 MHz, DMSO) δ 13.16 (s, 1H), 8.21 (s, 1H), 7.97 (s, 1H), 7.52 (t, *J =* 7.6 Hz, 1H), 7.17 (t, *J =* 7.7 Hz, 1H), 6.10 (s, 1H), 3.90 (s, 2H), 3.39 (t, *J =* 5.3 Hz, 2H), 3.30 (s, 4H), 2.49 - 2.46 (m, 2H), 1.77 (s, 4H).

### Example 62: Preparation of N-(cyclopentylmethyl)-4-(2,3-difluoro-4-(1H-pyrazol-4-yl)phenyl)piperazine-1-ca rboxamide (Compound 92)

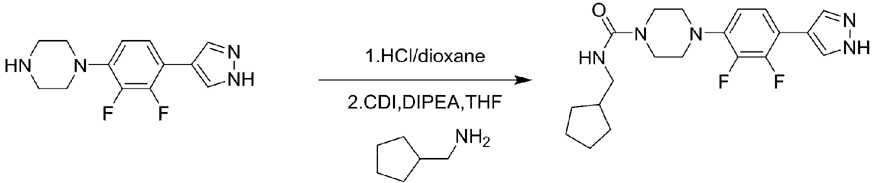

A mixture of cyclopentylmethylamine (36.8 mg, 0.273 mmol, 1.2 eq.), 1-(2,3-difluoro-4-(1H-pyrazol-4-yl)phenyl)piperazine (60 mg, 0.227 mmol, 1.0 eq.), N,N-diisopropylethylamine (146 mg, 1.135 mmol, 5.0 eq.), N,N'-carbonyldiimidazole (44 mg, 0.272 mmol, 1.2 eq.) in N,N-dimethylformamide (6 ml) was stirred at room temperature for 6.0 hours. The reaction was diluted with water and extracted with ethyl acetate. The organic layer was washed with brine, dried over anhydrous sodium sulfate, and concentrated under reduced pressure. The residue was purified by column chromatography on silica gel (eluent: dichloromethane/methanol = 50/1 to 10/1) to give a white solid product N-(cyclopentylmethyl)-4-(2,3-difluoro-4-(1H-pyrazol-4-yl)phenyl)piperazine-1-carbo xamide (11 mg, yield: 12.5%). LCMS (ESI): m/z = 390 (M+H)⁺. Melting point : 155~162 °C. ¹ H NMR (500 MHz, DMSO) δ 13.06 (s, 1H), 8.10 (s, 1H), 7.88 (s, 1H), 7.41 (t, *J =* 7.9 Hz, 1H), 6.87 (t, *J =* 8.2 Hz, 1H), 6.55 (s, 1H), 3.45 (s, 4H), 2.97 (dd, *J* = 14.4, 5.7 Hz, 6H), 2.02 (dd, *J =* 14.7, 7.4 Hz, 1H), 1.70 - 1.38 (m, 6H), 1.19 (dd, *J*= 12.1, 6.6 Hz, 2H).

### Example 63: Preparation of 2-cyclopentyl-1-(4-(2,3-difluoro-4-(1H-pyrazol-4-yl)phenyl)piperidin-1-yl)ethan-1 -one (Compound 93) (Prepared according to Scheme 9)

A mixture of 2-cyclopentylacetic acid ( 16.1 mg, 0.13 mmol, 1.05 eq.), 4-(2,3-difluoro-4-(1H-pyrazol-4-yl)phenyl)piperidine hydrochloride ( 40 mg, 0.12 mmol, 1.0 eq.), N,N-diisopropylethylamine ( 93 mg, 0.72 mmol, 6.0 eq.), HATU (51 mg, 0.13 mmol, 1.1 eq.) in N,N-dimethylformamide (3 ml) was stirred at room temperature for 1 hour. The reaction was diluted with water and extracted with ethyl acetate. The organic phase was washed with saturated brine, dried over anhydrous sodium sulfate and concentrated under reduced pressure. The residue was purified by prep-TLC (silica gel, eluent: dichloromethane/methanol = 15/1) to give a white solid product 2-cyclopentyl-1-(4-(2,3-difluoro-4-(1H-pyrazol-4-yl)phenyl)piperidin-1-yl)ethan-1-on e (31 mg, yield: 69.17%), LCMS (ESI): m/z = 374 [M+H]⁺. ¹ H NMR (500 MHz, DMSO) δ 13.13 (s, 1H), 8.17 (s, 1H), 7.93 (s, 1H), 7.48 (t, *J =* 7.3 Hz, 1H), 7.12 (t, *J* = 7.3 Hz, 1H), 4.57 (d, *J =* 12.3 Hz, 1H), 4.01 (d, *J =* 12.9 Hz, 1H), 3.19 - 3.01 (m, 2H), 2.61 (t, *J=* 12.3 Hz, 1H), 2.36 (d, *J =* 7.1 Hz, 2H), 2.20 - 2.06 (m, 1H), 1.77 (d, J = 11.1 Hz, 4H), 1.59 (s, 3H), 1.54 - 1.42 (m, 3H), 1.14 (s, 2H).

### Example 64: Preparation of N-benzyl-4-(2,3-difluoro-4-(1H-pyrazol-4-yl)phenyl)piperazine-1-carboxamide (Compound 94) (Prepared according to Scheme 10)

Step 64a: Preparation of tert-butyl 4-(benzylcarbamoyl)piperazine-1-carboxylate (Compound 1002-94): To a mixture of benzylamine (300 mg, 2.80 mmol, 1.0 eq.) in N,N-dimethylformamide (3 mL) was added carbonyl diimidazole (499 mg, 3.08 mmol, 1.1 eq.). The mixture was stirred at room temperature for 1.5 hours, then tert-butyl piperazine-1-carboxylate (626 mg, 3.36 mmol, 1.2 eq.) was added and stirring was continued at room temperature overnight. The mixture was diluted with water (30 mL). The aqueous layer was extracted with ethyl acetate (15 mL × 4). The combined organic layer was washed with saturated brine (20 mL × 1), dried over anhydrous sodium sulfate and concentrated. The residue was separated by column chromatography on silica gel (dichloromethane: methanol 50: 1) to give tert-butyl 4-(benzylcarbamoyl)piperazine-1-carboxylate (701 mg, yield: 78%) as a white solid. LCMS (ESI): m/z = 320 [M+1]⁺; TLC: Rf 0.5 (dichloromethane: methanol = 30: 1).

Step 64b: Preparation of N-benzylpiperazine-1-carboxamide (Compound 1003-94): A mixture of tert-butyl 4-(benzylcarbamoyl)piperazine-1-carboxylate (1002-94) (701 mg, 2.19 mmol, 1.0 eq.) in hydrogen chloride-methanol solution (4M, 5 mL) was stirred at room temperature for 2 hours. The mixture was diluted with water (25 mL). Solid sodium carbonate was added to adjust the pH to 10, and then the aqueous layer was extracted with dichloromethane (10 mL×6). The combined organic layer was washed with saturated brine (20 mL×1), dried over anhydrous sodium sulfate and concentrated to give white solid product N-benzylpiperazine-1-carboxamide (392 mg, yield: 82%). LCMS (ESI): m/z = 220 [M+1]⁺; TLC: Rf 0.3 (dichloromethane: methanol = 10: 1).

Step 64c: Preparation of N-benzyl-4-(2,3-difluoro-4-(1-(tetrahydro-2H-pyran-2-yl)-1H-pyrazol-4-yl)phenyl)pi perazine-1-carboxamide (Compound 1004-94): To a mixture of 4-(4-bromo-2,3-difluorophenyl)-1-(tetrahydro-2H-pyran-2-yl)-1H-pyrazole (0103-4) (150 mg, 0.44 mmol, 1.0 eq.), tris(dibenzylideneacetone)dipalladium (20 mg, 0.022 mmol, 0.05 eq.), 4,5-bis(diphenylphosphino)-9,9-dimethylxanthene (25 mg, 0.044 mmol, 0.1 eq.) and sodium tert-butoxide (105 mg, 1.09 mmol, 2.5 eq.) in toluene (6 ml) was added N-benzylpiperazine-1-carboxamide (1003-94) (15 mg, 0.53 mmol, 1.2 eq.). The mixture was heated to 120°C under nitrogen atmosphere and reacted overnight. The mixture was diluted with water (30 ml). The aqueous layer was extracted with ethyl acetate (15 ml×3). The combined organic layer was washed with saturated brine (20 ml × 1), dried over anhydrous sodium sulfate and concentrated. The residue was purified by Pre-TLC (silica gel, dichloromethane: methanol = 20: 1) to give a pale yellow oily product N-benzyl-4-(2,3-difluoro-4-(1-(tetrahydro-2H-pyran-2-yl)-1H-pyrazol-4-yl)phenyl)pi perazine-1-carboxamide (41 mg, yield: 20%). LCMS (ESI): m/z = 482 [M+1]⁺; TLC: Rf 0.5 (dichloromethane: methanol = 20: 1).

Step 64d: Preparation of N-benzyl-4-(2,3-difluoro-4-(1H-pyrazol-4-yl)phenyl)piperazine-1-carboxamide (Compound 94): A mixture of N-benzyl-4-(2,3-difluoro-4-(1-(tetrahydro-2H-pyran-2-yl)-1H-pyrazol-4-yl)phenyl)pi perazine-1-carboxamide (1004-94) (41 mg, 0.085 mmol, 1.0 eq.) in hydrogen chloride-methanol solution (4M, 2.5 mL) was stirred at room temperature for 2 hours. The mixture was diluted with water (20 mL). Solid sodium carbonate was added to adjust pH = 10, and then the aqueous layer was extracted with ethyl acetate (20 mL×3). The combined organic layer was washed with saturated brine (20 mL×1), dried over anhydrous sodium sulfate and concentrated. The residue was purified by Pre-TLC (silica gel, dichloromethane: methanol = 10: 1) to give white solid product N-benzyl-4-(2,3-difluoro-4-(1H-pyrazol-4-yl)phenyl)piperazine-1-carboxamide (23 mg, yield: 68%). LCMS (ESI): m/z = 398 [M+1]⁺; TLC: Rf 0.5 (dichloromethane: methanol = 10: 1). ¹ H NMR (500 MHz, DMSO) δ 13.07 (s, 1H), 8.11 (s, 1H), 7.88 (s, 1H), 7.41 (t, *J =* 7.8 Hz, 1H), 7.30 (dd, *J =* 15.1, 7.9 Hz, 4H), 7.19 (dt, *J =* 11.1, 6.3 Hz, 2H), 6.88 (t, *J =* 8.1 Hz, 1H), 4.27 (d, *J =* 5.6 Hz, 2H), 3.51 (s, 4H), 3.02 (d, *J =* 4.2 Hz, 4H).

### Example 65: Preparation of N-benzyl-1-(2,3-difluoro-4-(1H-pyrazol-4-yl)phenyl)piperidine-4-carboxamide (Compound 95) (Prepared according to Scheme 3)

Step 65a: Preparation of tert-butyl 4-(benzylcarbamoyl)piperidine-1-carboxylate (Compound 0302-95): A mixture of benzylamine (225 mg, 2.09 mmol, 1.2 eq.), 1-(tert-butoxycarbonyl)piperidine-4-carboxylic acid (0301) (400 mg, 1.74 mmol, 1.0 eq.), triethylamine (440 mg, 4.35 mmol, 2.5 eq.), HATU (860 mg, 2.27 mmol, 1.3 eq.) in N,N-dimethylformamide (15 ml) was stirred at room temperature for 4.0 hours. The reaction was diluted with water and extracted with ethyl acetate. The organic layer was dried and concentrated under reduced pressure. The residue was purified by column chromatography on silica gel (petroleum ether/ethyl acetate = 50/1 to 10/1) to give a yellow oily product tert-butyl 4-(benzylcarbamoyl)piperidine-1-carboxylate (450 mg, yield: 81.3%). LCMS (ESI): m/z =319 (M+H)⁺.

Step 65b: Preparation of N-benzylpiperidine-4-carboxamide (Compound 0303-95): A mixture of tert-butyl 4-(benzylcarbamoyl)piperidine-1-carboxylate (0302-95) (450 mg, 1.41 mmol, 1.0 eq.) in hydrogen chloride - methanol solution (4 M, 4 ml) was stirred at room temperature for 2.0 hours. The solvent was removed under reduced pressure. The residue was diluted with water, adjusted to pH = 9 with sodium carbonate solution, and extracted with dichloromethane. The organic phase was dried and concentrated under reduced pressure to give a yellow oil product N-benzylpiperidine-4-carboxamide (256 mg, yield: 83.1%). LCMS (ESI): m/z = 219 [M+1]⁺.

Step 65c: Preparation of N-benzyl-1-(2,3-difluoro-4-(1-(tetrahydro-2H-pyran-2-yl)-1H-pyrazol-4-yl)phenyl)pi peridine-4-carboxamide (Compound 0304-95). Under nitrogen protection, a mixture of N-benzylpiperidine-4-carboxamide (0303-95) (123 mg, 0.564 mmol, 1.6 eq.), 4-(4-bromo-2,3-difluorophenyl)-1-(tetrahydro-2H-pyran-2-yl)-1H-pyrazole (0103-4) (120 mg, 0.353 mmol, 1.0 eq.), sodium tert-butoxide (101 mg, 1.059 mmol, 3.0 eq.), 4,5-bis(diphenylphosphino)-9,9-dimethylxanthene (20.4 mg, 0.035 mmol, 0.1 eq.) and tris(dibenzylideneacetone)dipalladium (16.2 mg, 0.018 mmol, 0.05 eq.) in toluene (8 ml) was stirred at 120°C overnight. The reaction was diluted with water and extracted with ethyl acetate, and the organic phase was dried and concentrated under reduced pressure. The residue was purified by column chromatography on silica gel (dichloromethane/methanol = 150/1 to 50/1) to give a yellow solid product N-benzyl-1-(2,3-difluoro-4-(1-(tetrahydro-2H-pyran-2-yl)-1H-pyrazol-4-yl)phenyl)pi peridine-4-carboxamide (120 mg, yield: 70.8%). LCMS (ESI): m/z = 481 [M+1]⁺.

Step 65d: Preparation of N-benzyl-1-(2,3-difluoro-4-(1H-pyrazol-4-yl)phenyl)piperidine-4-carboxamide (Compound 95): A mixture of N-benzyl-1-(2,3-difluoro-4-(1-(tetrahydro-2H-pyran-2-yl)-1H-pyrazol-4-yl)phenyl)pi peridine-4-carboxamide (0304-95) (120 mg, 0.25 mmol, 1.0 eq.) in hydrogen chloride - methanol solution (4 M, 5 mL) was stirred at room temperature for 2.0 hours. The solvent was removed under reduced pressure. The residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 10/1) to give a yellow solid product N-benzyl-1-(2,3-difluoro-4-(1H-pyrazol-4-yl)phenyl)piperidine-4-carboxamide (49 mg, yield: 49.5%). LCMS (ESI): m/z = 397 [M+1]⁺. Melting point : 165-173 ° C. ¹ H NMR (500 MHz, DMSO) δ 13.05 (s, 1H), 8.34 (s, 1H), 7.98 (s, 2H), 7.43 - 7.28 (m, 3H), 7.24 (d, *J =* 7.5 Hz, 3H), 6.87 (t, *J =* 8.2 Hz, 1H), 4.29 (d, *J =* 5.5 Hz, 2H), 3.43 (d, *J* = 11.5 Hz, 2H), 2.74 (t, *J* = 11.1 Hz, 2H), 2.36 (s, 1H), 1.91 - 1.68 (m, 4H).

### Example 66: Preparation of cyclohexyl(4-(2,3-difluoro-4-(1H-pyrazol-4-yl)phenyl)piperidin-1-yl)methanone (Compound 97) (Prepared according to Scheme 9)

Step 66a: Preparation of tert-butyl 4-(2,3-difluoro-4-(1-(tetrahydro-2H-pyran-2-yl)-1H-pyrazol-4-yl)phenyl)piperidine-1-carboxylate (Compound 0903-97): Zinc powder (0.63 g, 9.65 mmol, 3.0 eq.) was baked at high temperature (500 °C) for 15 minutes under nitrogen protection and allowed to cool naturally. N,N-dimethylacetamide (8 ml) and 1,2-dibromoethane (60 mg, 0.32 mmol, 0.1 eq.) were added to the cooled zinc powder, and then the reaction was initiated at 50 °C. When its reaction temperature became 35 °C, trimethylsilyl chloride (35 mg, 0.32 mmol, 0.1 eq.) was added to the mixture. The mixture was reacted at 35 °C for 5 minutes. Then, tert-butyl 4-iodopiperidine-1-carboxylate (1.0 g, 3.22 mmol, 1.0 eq.) was slowly added to the mixture, and the temperature was maintained at 40 °C. The mixture was reacted at 40 °C for 30 minutes. The mixture was filtered and the filtrate was immediately used for the next reaction. Under nitrogen protection, a mixture of 4-(4-bromo-2,3-difluorophenyl)-1-(tetrahydro-2H-pyran-2-yl)-1H-pyrazole (0103-4) (200 mg, 0.588 mmol, 1.0 eq.), the filtrate of the previous step, cuprous iodide (23 mg, 0.118 mmol, 1.0 eq.), 1,1-bis(diphenylphosphino)ferrocenepalladium dichloride dichloromethane complex (48 mg, 0.059 mmol, 0.1 eq.) was stirred at 85 °C overnight. The reaction was diluted with water and extracted with ethyl acetate. The organic layer was dried and concentrated under reduced pressure. The residue was purified by column chromatography on silica gel (petroleum ether/ethyl acetate = 4/1) to give a yellow solid product tert-butyl 4-(2,3-difluoro-4-(1-(tetrahydro-2H-pyran-2-yl)-1H-pyrazol-4-yl)phenyl)piperidine-1-carboxylate (130 mg, yield: 49.43%). LCMS (ESI): m/z = 448 [M+1]⁺.

Step 66b: Preparation of 4-(2,3-difluoro-4-(1H-pyrazol-4-yl)phenyl)piperidine hydrochloride (Compound 0904-97): A mixture of tert-butyl 4-(2,3-difluoro-4-(1-(tetrahydro-2H-pyran-2-yl)-1H-pyrazol-4-yl)phenyl)piperidine-1-carboxylate (0903-97) (130 mg, 0.29 mmol, 1.0 eq.) in hydrogen chloride - methanol solution (4 M, 4 mL) was stirred at room temperature for 2.0 hours. The solvent was removed under reduced pressure. The residue was used directly in the next step without further purification. LCMS (ESI): m/z = 264 [M+1]⁺.

Step 66c: Preparation of cyclohexyl(4-(2,3-difluoro-4-(1H-pyrazol-4-yl)phenyl)piperidin-1-yl)methanone ( Compound 97) : Under nitrogen protection, a mixture of 4-(2,3-difluoro-4-(1H-pyrazol-4-yl)phenyl)piperidine hydrochloride (0904-97) (40 mg, 0.134 mmol, 1.0 eq.), cyclohexanecarboxylic acid (20.6 mg, 0.161 mmol, 1.2 eq.), HATU (76.4 mg, 0.201 mmol, 1.5 eq.), N,N-diisopropylethylamine (51.8 mg, 0.402 mmol, 3.0 eq.) in N,N-dimethylformamide (3 ml) was stirred at room temperature for 2.0 hours. The reaction was diluted with water and extracted with ethyl acetate. The organic phase was dried and concentrated under reduced pressure. The residue was purified by prep-TLC (silica gel, eluent: ethyl acetate) to give cyclohexyl(4-(2,3-difluoro-4-(1H-pyrazol-4-yl)phenyl)piperidin-1-yl)methanone (11 mg, yield: 22.1%) as a yellow solid product. ¹ H NMR (500 MHz, DMSO) δ 13.13 (s, 1H), 8.17 (s, 1H), 7.94 (s, 1H), 7.47 (t, *J =* 7.3 Hz, 1H), 7.13 (t, *J =* 7.4 Hz, 1H), 4.57 (d, *J =* 11.5 Hz, 1H), 4.06 (d, *J =* 12.1 Hz, 1H), 3.11 (dt, *J =* 24.3, 12.2 Hz, 2H), 2.61 (d, *J =* 8.2 Hz, 2H), 1.87 - 1.75 (m, 2H), 1.58 (dd, *J=* 91.4, 16.8 Hz, 8H), 1.39 - 1.28 (m, 4H).

### Example 67: Preparation of cyclopentyl(4-(2,3-difluoro-4-(1H-pyrazol-4-yl)phenyl)piperidin-1-yl)methanone (Compound 98) (Prepared according to Scheme 9)

Under nitrogen protection, a mixture of 4-(2,3-difluoro-4-(1H-pyrazol-4-yl)phenyl)piperidine hydrochloride (0904-97) (40 mg, 0.119 mmol, 1.0 eq.), cyclopentanecarboxylic acid (17 mg, 0.143 mmol, 1.2 eq.), HATU (69 mg, 0.179 mmol, 1.5 eq.), N,N-diisopropylethylamine (47 mg, 0.358 mmol, 3.0 eq.) in N,N-dimethylformamide (5 ml) was stirred at room temperature for 1 hour. The reaction was diluted with water and extracted with ethyl acetate. The organic layer was dried and concentrated under reduced pressure. The residue was purified by prep-TLC (silica gel, dichloromethane/methanol = 20/1) to give cyclopentyl(4-(2,3-difluoro-4-(1H-pyrazol-4-yl)phenyl)piperidin-1-yl)methanone (38 mg, yield: 88.37%) as a white solid. LCMS (ESI): m/z =360[M+1]⁺. ¹ H NMR (500 MHz, DMSO) δ 13.13 (s, 1H), 8.17 (s, 1H), 7.93 (s, 1H), 7.47 (t, *J =* 7.3 Hz, 1H), 7.13 (t, *J =* 7.3 Hz, 1H), 4.57 (d, *J =* 11.9 Hz, 1H), 4.11 (d, *J =* 12.4 Hz, 1H), 3.07 (ddd, *J =* 39.6, 20.1, 10.5 Hz, 3H), 2.63 (t, *J* = 12.2 Hz, 1H), 1.69 (ddd, *J =* 76.8, 51.7, 34.0 Hz, 12H).

### Example 68: Preparation of cyclopentyl(4-(2,6-difluoro-4-(1H-pyrazol-4-yl)phenyl)piperidin-1-yl)methanone (Compound 99) ( Prepared according to Scheme 9)

Step 68a: Preparation of tert-butyl 4-(2,6-difluoro-4-(1-(tetrahydro-2H-pyran-2-yl)-1H-pyrazol-4-yl)phenyl)-3,6-dihydro pyridine-1(2H)-carboxylate (Compound 0902-99): To a mixture of 4-(4-bromo-3,5-difluorophenyl)-1-(tetrahydro-2H-pyran-2-yl)-1H-pyrazole (0103-5) (0.2 g, 0.58 mmol, 1.0 eq.), 1,1'-bis(diphenylphosphino)ferrocenepalladium(II) chloride (33 mg, 0.041 mmol, 0.07 eq.) and sodium carbonate (185 mg, 1.75 mmol, 3.0 eq.) in dioxane (5 ml) and water (1 ml) was added tert-butyl 4-(4,4, 5, 5-tetramethyl-1,3,2-dioxaborolan-2-yl)-3,6-dihydropyridine-1 (2H)-carboxyla te (216 mg, 0.70 mmol, 1.25 eq.). The mixture was heated to 100°C under nitrogen atmosphere overnight. The solvent was removed under reduced pressure. The residue was separated by column chromatography on silica gel (petroleum ether: ethyl acetate = 3: 1) to give a pale yellow oily product tert-butyl 4-(2,6-difluoro-4-(1-(tetrahydro-2H-pyran-2-yl)-1H-pyrazol-4-yl)phenyl)-3,6-dihydro pyridine-1(2H)-carboxylate (259 mg, yield: 100%). LCMS (ESI): m/z = 446 [M+1]⁺; TLC: Rf 0.5 (petroleum ether: ethyl acetate = 1: 1).

Step 68b: Preparation of tert-butyl 4-(2,6-difluoro-4-(1-(tetrahydro-2H-pyran-2-yl)-1H-pyrazol-4-yl)phenyl)piperidine-1-carboxylate (Compound 0903-99): To a mixture of tert-butyl 4-(2,6-difluoro-4-(1-(tetrahydro-2H-pyran-2-yl)-1H-pyrazol-4-yl)phenyl)-3,6-dihydro pyridine-1(2H)-carboxylate (0902-99) (230 mg, 0.52 mmol, 1.0 eq.) in ethanol (8 mL) was added palladium hydroxide on carbon (60 mg). The mixture was heated to 65 °C under hydrogen balloon pressure and reacted overnight. The mixture was filtered. The filtrate was concentrated under reduced pressure to give tert-butyl 4-(2,6-difluoro-4-(1-(tetrahydro-2H-pyran-2-yl)-1H-pyrazol-4-yl)phenyl)piperidine-1-carboxylate (220 mg, yield: 95%) as a colorless oil. LCMS (ESI): m/z = 448 [M+1]⁺; TLC: Rf 0.5 (petroleum ether:ethyl acetate = 1:1).

Step 68c: Preparation of 4-(2,6-difluoro-4-(1H-pyrazol-4-yl)phenyl)piperidine hydrochloride (Compound 0904-99): A mixture of tert-butyl 4-(2,6-difluoro-4-(1-(tetrahydro-2H-pyran-2-yl)-1H-pyrazol-4-yl)phenyl)piperidine-1-carboxylate (0903-99) (220 mg, 0.49 mmol, 1.0 eq.) in hydrogen chloride-methanol solution (4M, 3 mL) was stirred at room temperature for 2 hours. The solvent was removed under reduced pressure. The residue was dried under vacuum to give 4-(2,6-difluoro-4-(1H-pyrazol-4-yl)phenyl)piperidine hydrochloride (161 mg, crude) as a white solid. LCMS (ESI): m/z = 264 [M+1]⁺; TLC: Rf 0.2 (dichloromethane:methanol= 1 0: 1).

Step 68d: Preparation of cyclopentyl(4-(2,6-difluoro-4-(1H-pyrazol-4-yl)phenyl)piperidin-1-yl)methanone (Compound 99): To a mixture of 4-(2,6-difluoro-4-(1H-pyrazol-4-yl)phenyl)piperidine hydrochloride (0904-99) (61 mg, 0.20 mmol, 1.0 eq.), cyclopentylcarboxylic acid (28 mg, 0.24 mmol, 1.2 eq.) and N,N-diisopropylethylamine (79 mg, 0.60 mmol, 3.0 eq.) in dichloromethane (5 mL) was added HATU (100 mg, 0.26 mmol, 1.3 eq.). The mixture was stirred at room temperature for 1 hour. The solvent was removed under reduced pressure. The residue was purified by Pre-TLC (dichloromethane:ethyl acetate = 1:1) to give cyclopentyl(4-(2,6-difluoro-4-(1H-pyrazol-4-yl)phenyl)piperidin-1-yl)methanone (36 mg, yield: 49%) as a white solid. LCMS (ESI): m/z = 360 [M+1]⁺; TLC: Rf 0.5 (dichloromethane:ethyl acetate = 1:1). ¹ H NMR (500 MHz, MeOD) δ 7.98 (d, *J =* 56.7 Hz, 2H), 7.17 (d, *J =* 10.0 Hz, 2H), 4.70 (d, *J =* 13.2 Hz, 1H), 4.23 (d, *J =* 13.2 Hz, 1H), 3.20 (t, *J* = 13.1 Hz, 1H), 3.15 - 3.02 (m, 1H), 2.70 (t, *J* = 12.6 Hz, 1H), 1.85 (dddd, *J =* 74.2, 68.0, 42.5, 7.0 Hz, 13H).

### Example 69: Preparation of (4-(2,3-difluoro-4-(1H-pyrazol-4-yl)phenyl)piperidin-1-yl)(4,4-difluorocyclohexyl) methanone (Compound 101) (Prepared according to Scheme 9)

A mixture of 4,4-difluorocyclohexane-1-carboxylic acid (21 mg, 0.13 mmol, 1.3 eq.), 4-(2,3-difluoro-4-(1H-pyrazol-4-yl)phenyl)piperidine hydrochloride (0904-97) (34 mg, 0.10 mmol, 1.0 eq.), N,N-diisopropylethylamine (75 mg, 0.604 mmol, 6.0 eq.), HATU (42 mg, 0.11 mmol, 1.1 eq.) in N,N-dimethylformamide (3 ml) was stirred at room temperature for 2 hours. The reaction was diluted with water and extracted with ethyl acetate. The organic phase was washed with saturated brine, dried over anhydrous sodium sulfate and concentrated under reduced pressure. The residue was purified by thick prep-TLC (silica gel, eluent: dichloromethane/methanol = 15/1) to give a yellow solid product (4-(2,3-difluoro-4-(1H-pyrazol-4-yl)phenyl)piperidin-1-yl)(4,4-difluorocyclohexyl)m ethanone (35 mg, yield: 85.48%). LCMS (ESI): m/z = 410 [M+H]⁺. ¹ H NMR (500 MHz, DMSO) δ 13.13 (s, 1H), 8.18 (s, 1H), 7.93 (s, 1H), 7.48 (t, *J =* 7.5 Hz, 1H), 7.14 (t, *J =* 7.5 Hz, 1H), 4.57 (d, *J =* 12.3 Hz, 1H), 4.12 (d, *J =* 12.8 Hz, 1H), 3.14 (dt, *J* = 24.3, 12.4 Hz, 2H), 2.84 (t, *J =* 11.1 Hz, 1H), 2.63 (t, *J =* 12.3 Hz, 1H), 2.14 - 1.51 (m, 12H).

### Example 70: Preparation of (S)-(4-(2,3-difluoro-4-(1H-pyrazol-4-yl)phenyl)-3-methylpiperazin-1-yl)(pyrrolidi n-1-yl)methanone (Compound 102) (Prepared according to Scheme 10)

Step 70a: Preparation of (S)-tert-butyl 4-(4-bromo-2,3-difluorophenyl)-3-methylpiperazine-1-carboxylate (Compound 1002-102): Under nitrogen protection, a mixture of 1,4-dibromo-2,3-difluorobenzene (600 mg, 2.21 mmol, 1.0 eq.), tert-butyl (S)-3-methylpiperazine-1-carboxylate (530 mg, 2.65 mmol, 1.2 eq.), sodium tert-butoxide (531 mg, 5.53 mmol, 2.5 eq.), 1,1'-binaphthyl-2,2'-bisdiphenylphosphine (138 mg, 0.221 mmol, 0.1 eq.) and palladium acetate (50 mg, 0.221 mmol, 0.1 eq.) in toluene (25 ml) was stirred at 120 ° C for 1.5 hours. The reaction was diluted with water (30 ml) and extracted with ethyl acetate (12 ml × 4). The combined organic phases was washed with saturated brine (40 ml × 1), dried over anhydrous sodium sulfate and concentrated under reduced pressure. The residue was purified by column chromatography on silica gel (eluent: petroleum ether/ethyl acetate = 20/1 to 15/1) to obtain a yellow oily product (S)-tert-butyl 4-(4-bromo-2,3-difluorophenyl)-3-methylpiperazine-1-carboxylate (93 mg, yield: 10.7%). LCMS (ESI): m/z = 391 [M+1]⁺.

Step 70b: Preparation of (S)-1-(4-bromo-2,3-difluorophenyl)-2-methylpiperazine hydrochloride (Compound 1003-102): A mixture of (S)-tert-butyl 4-(4-bromo-2,3-difluorophenyl)-3-methylpiperazine-1-carboxylate (1002-102) (93 mg, 0.238 mmol, 1.0 eq.) in hydrogen chloride-methanol (4M, 5 mL) was stirred at room temperature for 1.5 hours. The solvent was removed under reduced pressure to give a yellow solid product (S)-1-(4-bromo-2,3-difluorophenyl)-2-methylpiperazine hydrochloride (73 mg, yield: 94.2%). LCMS (ESI): m/z = 291 [M+1]⁺.

Step 70c : Preparation of (S)-(4-(4-bromo-2,3-difluorophenyl)-3-methylpiperazine-1-yl)(pyrrolidin-1-yl)methan one : Under nitrogen protection, a mixture of (S)-1-(4-bromo-2,3-difluorophenyl)-2-methylpiperazine hydrochloride (1003-102) (73 mg, 0.223 mmol, 1.0 eq.), pyrrolidine-1-carbonyl chloride (32.7 mg, 0.245 mmol, 1.1 eq.), triethylamine ( 56 mg, 0.558 mmol, 2.5 eq.) in dichloromethane (2 ml) was stirred at room temperature for 1.5 hours. Sodium carbonate solution was added to quench the reaction, and the mixture was diluted with water (10 ml) and extracted with dichloromethane (6 ml × 4). The combined organic layer was washed with saturated brine (20 ml × 1), dried over anhydrous sodium sulfate, and concentrated under reduced pressure. The residue was purified by prep-TLC (silica gel, eluent: petroleum ether/ethyl acetate = 2/1) to give a yellow oily product (S)-(4-(4-bromo-2,3-difluorophenyl)-3-methylpiperazin-1-yl)(pyrrolidin-1-yl)methan one (77 mg, yield: 89.5%). LCMS (ESI): m/z = 388 [M+1]⁺.

Step 70d: Preparation of (S)-(4-(2,3-difluoro-4-(1H-pyrazo l-4-yl)phenyl)-3-methylpiperazin-1-yl)(pyrrolidin-1-yl)methanone (Compound 102): Under nitrogen protection, (S)-(4-(4-bromo-2,3-difluorophenyl)-3-methylpiperazin-1-yl)(pyrrolidin-1-yl)methan one (77 mg, 0.199 mmol, 1.0 eq.), tert-butyl 4-(4,4,5,5-tetramethyl-1,3,2-dioxaborolan-2-yl)-1H-pyrazole-1-carboxylate (70 mg, 0.239 mmol, 1.2 eq.), 1,1'-bis(diphenylphosphino)ferrocene dichloropalladium(II) dichloromethane complex (16 mg, 0.0199 mmol, 0.1 eq.), sodium carbonate (63 mg, 0.597 mmol, 3.0 eq.) were dissolved in a mixed solvent of dioxane/water = 5/1 (3 ml/0.6 ml), and the mixture was stirred at 90°C overnight. The reaction was diluted with water (10 ml) and extracted with ethyl acetate (6 ml × 4). The combined organic layer was washed with saturated brine (20 ml × 1), dried over anhydrous sodium sulfate, and concentrated under reduced pressure. The residue was purified by prep-TLC (silica gel, dichloromethane/methanol = 12/1) to give a white solid product (S)-(4-(2,3-difluoro-4-(1H-pyrazol-4-yl)phenyl)-3-methylpiperazin-1-yl)(pyrrolidin-1 -yl)methanone (12 mg, yield: 16.2%). LCMS (ESI): m/z = 376 [M+1]⁺. ¹ H NMR (500 MHz, DMSO) δ 13.08 (s, 1H), 8.12 (s, 1H), 7.90 (s, 1H), 7.43 (t, *J =* 7.9 Hz, 1H), 6.94 (t, *J =* 8.1 Hz, 1H), 3.51 (s, 1H), 3.37 (dd, *J =* 12.7, 2.8 Hz, 2H), 3.31 (s, 4H), 3.25 - 3.07 (m, 3H), 2.89 (dd, *J =* 9.9, 5.3 Hz, 1H), 1.76 (s, 4H), 0.94 (d, *J =* 6.3 Hz, 3H).

### Example 71: Preparation of (R)-(4-(2,3-difluoro-4-(1H-pyrazol-4-yl)phenyl)-2-methylpiperazin-1-yl)(pyrrolid in-1-yl)methanone (Compound 103) (Prepared according to Scheme 10)

Step 71a: Preparation of (R)-(2-methylpiperazine-1-yl)(pyrrolidin-1-yl)methanone (Compound 1002-103): Under nitrogen atmosphere, (S)-tert-butyl 3-methylpiperazine-1-carboxylate (500 mg, 2.5 mmol, 1.0 eq.) was dissolved in 10 mL tetrahydrofuran and triethylamine (505 mg, 5.0 mmol, 2.0 eq.) was added. The reaction system was cooled in an ice-water bath. Pyrrolidine-1-carbonyl chloride (350 mg, 2.625 mmol, 1.05 eq.) was dissolved in 1 mL tetrahydrofuran and added dropwise to the above mixture, and the mixture was stirred at room temperature for 2 hours. The reaction was diluted with water and extracted with ethyl acetate, and the organic phase was dried and concentrated under reduced pressure. The residue was stirred at room temperature for 1.0 hour in a mixture of hydrogen chloride-dioxane ( 4 M, 6 mL). The mixture was concentrated and the pH of the residue was adjusted to 9 with sodium carbonate solution. The mixture was extracted with ethyl acetate, and the organic phase was dried and concentrated under reduced pressure. The residue was used directly in the next step without further purification. LCMS (ESI): m/z = 198 [M+1]⁺.

Step 71b: Preparation of (R)-(4-(2,3-difluoro-4-(1H-pyrazol-4-yl)phenyl)-2-methylpiperazin-1-yl)(pyrrolidin-1 -yl)methanone (Compound 103): Under nitrogen atmosphere, a mixture of 4-(4-bromo-2,3-difluorophenyl)-1-(tetrahydro-2H-pyran-2-yl)-1H-pyrazole (0103-4) (100 mg, 0.294 mmol, 1.0 eq.), (R)-(2-methylpiperazin-1-yl)(pyrrolidin-1-yl)methanone (1002-103) (103 mg, 0.441 mmol, 1.5 eq.), tris(dibenzylideneacetone)dipalladium (26 mg, 0.029 mmol, 0.1 eq.), 2-dicyclohexylphosphino-2',6'-diisopropoxy-1,1'-biphenyl (27 mg, 0.058 mmol, 0.2 eq.), and sodium tert-butoxide (226 mg, 0.235 mmol, 4.0 eq.) in toluene (6 ml) was stirred at 125°C overnight. The reaction was cooled to room temperature and quenched with water. The mixture was extracted with ethyl acetate. The organic layer was dried over anhydrous sodium sulfate and concentrated under reduced pressure. The residue was purified by prep-TLC (silica gel, dichloromethane/methanol = 10/1) to give a yellow solid. The yellow solid was dissolved with hydrogen chloride-dioxane (4M, 6 ml). The mixture was stirred at room temperature for 1.0 hour. The mixture was concentrated and the pH of the residue was adjusted to 9 with sodium carbonate solution. The mixture was extracted with ethyl acetate, and the organic phase was dried and concentrated under reduced pressure. The residue was purified by prep-TLC (silica gel, dichloromethane/methanol = 10/1) to give (R)-(4-(2,3-difluoro-4-(1H-pyrazol-4-yl)phenyl)-2-methylpiperazine-1-yl)(pyrrolidin-1-yl)methanone as a yellow solid (32 mg, yield: 29.09%). LCMS (ESI): m/z = 376 [M+1]⁺. ¹H NMR (500 MHz, DMSO) δ 13.06 (s, 1H), 8.10 (s, 1H), 7.88 (s, 1H), 7.41 (t, *J =* 7.9 Hz, 1H), 6.84 (t, *J =* 8.2 Hz, 1H), 4.05 - 3.84 (m, 1H), 3.56 - 3.40 (m, 1H), 3.29 - 3.08 (m, 7H), 2.94 - 2.71 (m, 2H), 1.76 (s, 4H), 1.35 - 1.23 (m, 3H).

### Example 72: Preparation of (R)-(4-(2,3-difluoro-4-(1H-pyrazol-4-yl)phenyl)-3-methylpiperazin-1-yl)(pyrrolid in-1-yl)methanone (Compound 104) (Prepared according to Scheme 10)

Step 72a: Preparation of (R)-tert-butyl 4-(4-bromo-2,3-difluorophenyl)-3-methylpiperazine-1-carboxylate (Compound 1002-104): To a mixture of 1,4-dibromo-2,3-difluorobenzene (500 mg, 1.84 mmol, 1.0 eq.), palladium acetate (41 mg, 0.18 mmol, 0.1 eq.), 1,1'-binaphthyl-2,2'-bisdiphenylphosphine (115 mg, 0.18 mmol, 0.1 eq.) and sodium tert-butoxide (442 mg, 4.60 mmol, 2.5 eq.) in toluene (25 mL) was added (R)-tert-butyl 3-methylpiperazine-1-carboxylate (368 mg, 1.84 mmol, 1.0 eq.). The mixture was heated to 120 °C under nitrogen atmosphere for 1.5 hours. The solvent was removed under reduced pressure. The residue was separated by column chromatography on silica gel (petroleum ether: ethyl acetate 30: 1) to give (R)-tert-butyl 4-(4-bromo-2,3-difluorophenyl)-3-methylpiperazine-1-carboxylate (75 mg, yield: 10%) as a pale yellow oil. LCMS (ESI): m/z = 391 [M+1]⁺; TLC: Rf 0.5 (petroleum ether: ethyl acetate = 10: 1).

Step 72b: Preparation of (R)-(4-(4-bromo-2,3-difluorophenyl)-3-methylpiperazin-1-yl)(pyrrolidin-1-yl)methan one: A mixture of (R)-tert-butyl 4-(4-bromo-2,3-difluorophenyl)-3-methylpiperazine-1-carboxylate (1002-104) (75 mg, 0.19 mmol, 1.0 eq.) in hydrogen chloride-methanol solution (4M, 2.5 mL) was stirred at room temperature for 2 hours. The solvent was removed under reduced pressure. The residue was dissolved in dichloromethane (5 mL) and N,N-diisopropylethylamine (124 mg, 0.96 mmol, 5.0 eq.) and pyrrolidine-1-carbonyl chloride (28 mg, 0.21 mmol, 1.1 eq.) were added. The mixture was stirred at room temperature overnight. The solvent was removed under reduced pressure. The residue was separated by column chromatography on silica gel (petroleum ether: ethyl acetate 1:2) to give (R)-(4-(4-bromo-2,3-difluorophenyl)-3-methylpiperazin-1-yl)(pyrrolidin-1-yl)methan one (60 mg, yield: 81%) as a pale yellow oil. LCMS (ESI): m/z = 388 [M+1]⁺; TLC: Rf 0.3 (petroleum ether: ethyl acetate = 1:1).

Step 72c: Preparation of (R)-(4-(2,3-difluoro-4-(1H-pyrazol-4-yl)phenyl)-3-methylpiperazin-1-yl)(pyrrolidin-1 -yl)methanone (Compound 104): To a mixture of (R)-(4-(4-bromo-2,3-difluorophenyl)-3-methylpiperazin-1-yl)(pyrrolidin-1-yl)methan one (60 mg, 0.16 mmol, 1.0 eq.), 1,1'-bis(diphenylphosphino)ferrocenepalladium(II) chloride (13 mg, 0.016 mmol, 0.1 eq.), sodium carbonate (49 mg, 0.48 mmol, 3.0 eq.) in dioxane (5 ml) and water (1 ml) was added tert-butyl 4-(4,4,5,5-tetramethyl-1,3,2-dioxaborolan-2-yl)-1H-pyrazole-1-carboxylate (55 mg, 0.19 mmol, 1.2 eq.). The mixture was heated to 102oC under nitrogen atmosphere and reacted overnight. The mixture was diluted with water (20 ml). The aqueous layer was extracted with ethyl acetate (15 ml × 3). The combined organic layer was washed with saturated brine (20 ml × 1), dried over anhydrous sodium sulfate and concentrated. The residue was purified by Pre-TLC (dichloromethane: methanol = 10: 1) to give (R)-(4-(2,3-difluoro-4-(1H-pyrazol-4-yl)phenyl)-3-methylpiperazin-1-yl)(pyrrolidin-1 -yl)methanone (15 mg, yield: 26%) as a white solid. LCMS (ESI): m/z = 376 [M+1]⁺; TLC: Rf 0.5 (dichloromethane: methanol = 10: 1). ¹ H NMR (500 MHz, DMSO) δ 13.09 (s, 1H), 8.13 (s, 1H), 7.90 (s, 1H), 7.43 (s, 1H), 6.94 (s, 1H), 3.51 (s, 4H), 2.90 (s, 7H), 1.76 (s, 4H), 0.94 (d, *J =* 4.8 Hz, 3H).

### Example 73: Preparation of (S)-(4-(2,3-difluoro-4-(1H-pyrazol-4-yl)phenyl)-2-methylpiperazin-1-yl)(pyrrolidi n-1-yl)methanone (Compound 105) (Prepared according to Scheme 10)

Step 73a: Preparation of (S)-tert-butyl 3-methyl-4-(pyrrolidine-1-carbonyl)piperazine-1-carboxylate (Compound 1002-105): Under nitrogen protection, pyrrolidine-1-carbonyl chloride (400 mg, 3.00 mmol, 1.2 eq.) was added to a mixed solution of tert-butyl (S)-3-methylpiperazine-1-carboxylate (500 mg, 2.50 mmol, 1.0 eq.), triethylamine (506 mg, 5.00 mmol, 2.0 eq.) in dichloromethane. The mixture was stirred at room temperature for 2 hours. Water was added to quench the reaction and extracted with dichloromethane. The organic phase was washed with saturated brine, dried over anhydrous sodium sulfate, and concentrated under reduced pressure. The residue was purified by column chromatography on silica gel (eluent: petroleum ether/ethyl acetate = 4/1 to 1/1) to obtain a white solid product (S)-tert-butyl 3-methyl-4-(pyrrolidine-1-carbonyl)piperazine-1-carboxylate (402 mg, yield: 54.14%). MS (ES⁺): m/z = 298 [M+H]⁺.

Step 73b: Preparation of (S)-(2-methylpiperazin-1-yl)(pyrrolidin-1-yl)methanone (Compound 1003-105): A mixture of (S)-tert-butyl 3-methyl-4-(pyrrolidine-1-carbonyl)piperazine-1-carboxylate (1002-105) ( 402 mg, 1.35 mmol, 1.0 eq.) in trifluoroacetic acid/dichloromethane (1/2, 6ml) was stirred at room temperature for 2 hours. The solvent was removed under reduced pressure, and the pH of the residue was adjusted to 9 with 2 mol sodium hydroxide solution and extracted with dichloromethane. The organic phase was dried over anhydrous sodium sulfate and concentrated under reduced pressure. The residue was used in the next step without further purification. MS (ES⁺): m/z = 198 [M+H][M+H]⁺.

Step 73c: Preparation of ((2S)-4-(2,3-difluoro-4-(1-(tetrahydro-2H-pyran-2-yl)-1H-pyrazol-4-yl)phenyl)-2-met hylpiperazin-1-yl)(pyrrolidin-1-yl)methanone (Compound 1004-105): Under nitrogen protection, a mixture of (S)-(2-methylpiperazin-1-yl)(pyrrolidin-1-yl)methanone (1003-105) (75 mg, 0.38 mmol, 1.3 eq.), 4-(4-bromo-2,3-difluorophenyl)-1-(tetrahydro2H-pyran-2-yl)-1H-pyrazole (0103-4) (100 mg, 0.29 mmol, 1.0 eq.), sodium tert-butoxide (78 mg, 0.81 mmol, 2.5 eq.), 2-bicyclohexylphosphino-2',6'-diisopropoxybiphenyl (13.5 mg, 0.03 mmol, 0.1 eq.), tris(dibenzylideneacetone)dipalladium(0) (14 mg, 0.015 mmol, 0.05 eq.) in toluene (6 ml) was stirred at 120°C overnight. After cooling to room temperature, the reaction was quenched with water and extracted with ethyl acetate. The organic phase was washed with saturated brine, dried over anhydrous sodium sulfate, and concentrated under reduced pressure. The residue was purified by prep-TLC (silica gel, eluent: dichloromethane/methanol = 30/1) to give a yellow oily product ((2S)-4-(2,3-difluoro-4-(1-(tetrahydro-2H-pyran-2-yl)-1H-pyrazol-4-yl)phenyl)-2-met hylpiperazine-1-yl)(pyrrolidin-1-yl)methanone (70 mg, yield: 52.58%). MS (ES⁺): m/z = 460 [M+H][M+H]⁺.

Step 73d: Preparation of (S)-(4-(2,3-difluoro-4-(1H-pyrazol-4-yl)phenyl)-2-methylpiperazin-1-yl)(pyrrolidin-1 -yl)methanone (Compound 105): A mixture of ((2S)-4-(2,3-difluoro-4-(1-(tetrahydro2H-pyran-2-yl)-1H-pyrazol-4-yl)phenyl)-2-meth ylpiperazin-1-yl)(pyrrolidin-1-yl)methanone (1004-105) (70 mg, 0.15 mmol, 1.0 eq.) in hydrogen chloride-methanol solution (4 M, 3 mL) was stirred at room temperature for 2 hours. The solvent was removed under reduced pressure, and the pH of the residue was adjusted to 9 with sodium carbonate solution and extracted with dichloromethane. The organic phase was dried and concentrated under reduced pressure. The residue was purified by prep-TLC (silica gel, eluent: dichloromethane/methanol = 15/1) to give a white solid product (S)-(4-(2,3-difluoro-4-(1H-pyrazol-4-yl)phenyl)-2-methylpiperazin-1-yl)(pyrrolidin-1 -yl)methanone (43 mg, yield: 76.36%), MS (ES⁺): m/z = 376 [M+H]⁺. ¹ H NMR (500 MHz, DMSO) δ 13.06 (s, 1H), 8.10 (s, 1H), 7.88 (s, 1H), 7.41 (t, *J =* 7.9 Hz, 1H), 6.84 (t, *J =* 8.2 Hz, 1H), 3.97 (d, *J =* 6.1 Hz, 1H), 3.49 (d, *J =* 13.1 Hz, 1H), 3.30 (s, 2H), 3.28 - 3.26 (m, 2H), 3.26 - 3.06 (m, 3H), 2.92 - 2.72 (m, 2H), 1.76 (s, 4H), 1.31 (t, *J =* 11.0 Hz, 3H).

### Example 74: Preparation of (4-(2-fluoro-4-(1H-pyrazol-4-yl)phenyl)piperidin-1-yl)(hexahydrocyclopenta[c]py rrol-2(1H)-yl)methanone (Compound 106) (Prepared according to Scheme 9)

Under nitrogen atmosphere and ice bath conditions, triphosgene (16 mg, 0.053 mmol, 0.5 eq.) was added to a mixture of octahydrocyclopentane [c] pyrrole hydrochloride (16 mg, 0.106 mmol, 1.0 eq.) and pyridine (17 mg, 0.212 mmol, 2.0 eq.) in dichloromethane (5 ml). The mixture was stirred at room temperature for 3 hours. The reaction was quenched with 2N hydrochloric acid and extracted with dichloromethane. The organic layer was washed with saturated brine and dried over anhydrous sodium sulfate. The organic phase was concentrated under reduced pressure. The residue was dissolved in tetrahydrofuran. A solution of the residue in tetrahydrofuran was added to a mixture of 4-(2-fluoro-4-(1H-pyrazol-4-yl)phenyl)piperidine hydrochloride (30 mg, 0.106 mmol, 1.0 eq.) and N,N-diisopropylethylamine (41 mg, 0.318 mmol, 3.0 eq.) in tetrahydrofuran (5 ml). The mixture was stirred at room temperature for 3 hours. The reaction was quenched with water and extracted with ethyl acetate. The organic phase was concentrated and the residue was purified by thick preparative thin layer silica gel plate (eluent: dichloromethane/methanol = 10/1) to obtain a yellow solid product (4-(2-fluoro-4-(1H-pyrazol-4-yl)phenyl)piperidin-1-yl)(hexahydrocyclopenta[c]pyrrol -2(1H)-yl)methanone (14 mg, yield: 32.78%). LCMS(ESI): m/z =383 [M+1]⁺. ¹ H NMR (500 MHz, DMSO) δ 12.93 (s, 1H), 8.20 (s, 1H), 7.93 (s, 1H), 7.39 (t, *J =* 8.5 Hz, 2H), 7.28 (t, *J =* 8.2 Hz, 1H), 3.77 (d, *J =* 12.9 Hz, 2H), 3.55 - 3.41 (m, 2H), 3.03 (dd, *J =* 10.9, 3.6 Hz, 2H), 2.94 (t, *J* = 11.9 Hz, 1H), 2.86 (t, *J* = 12.1 Hz, 2H), 2.55 (d, *J =* 3.0 Hz, 2H), 1.77 - 1.56 (m, 7H), 1.51 (dt, *J =* 12.7, 6.4 Hz, 1H), 1.42 - 1.30 (m, 2H).

### Example 75: Preparation of 4-(4-(2,3-difluoro-4-(1H-pyrazol-4-yl)phenyl)piperidine-1-carbonyl)cyclohexane-1-one (Compound 107) (Prepared according to Scheme 9)

A mixture of 4-oxocyclohexane-1-carboxylic acid (19 mg, 0.13 mmol, 1.3 eq.), 4-(2,3-difluoro-4-(1H-pyrazol-4-yl)phenyl)piperidine hydrochloride (0904-97) (34 mg, 0.10 mmol, 1.0 eq.), N,N-diisopropylethylamine (75 mg, 0.604 mmol, 6.0 eq.), HATU (42 mg, 0.11 mmol, 1.1 eq.) in N,N-dimethylformamide (3 ml) was stirred at room temperature for 2 hours. The reaction was diluted with water and extracted with ethyl acetate. The organic phase was washed with saturated brine, dried over anhydrous sodium sulfate and concentrated under reduced pressure. The residue was purified by thick prep-TLC (silica gel, eluent: dichloromethane/methanol = 15/1) to give a white solid product 4-(4-(2,3-difluoro-4-(1H-pyrazol-4-yl)phenyl)piperidine-1-carbonyl)cyclohexane-1-o ne (30 mg, yield: 77.43%). LCMS (ESI): m/z = 379 [M+H]⁺. ¹ H NMR (500 MHz, DMSO) δ 13.14 (s, 1H), 8.18 (s, 1H), 7.94 (s, 1H), 7.48 (t, *J =* 7.3 Hz, 1H), 7.15 (t, *J* = 7.4 Hz, 1H), 4.59 (d, *J =* 11.5 Hz, 1H), 4.20 (d, *J =* 12.4 Hz, 1H), 3.25 - 3.05 (m, 3H), 2.64 (d, *J =* 11.0 Hz, 1H), 2.49 - 2.42 (m, 2H), 2.27 (s, 2H), 1.98 (s, 2H), 1.88 - 1.51 (m, 6H).

### Example 76: Preparation of 2-(4-(2,3-difluoro-4-(1H-pyrazol-4-yl)phenyl)piperidine-1-carbonyl)cyclohexane-1-one (Compound 109) (Prepared according to Scheme 9)

A mixture of ethyl 2-oxocyclohexane-1-carboxylate (170 mg, 1 mmol, 1.0 eq.), sodium hydroxide (80 mg, 2 mmol, 2.0 eq.) in water (5 ml.) was stirred at room temperature for 1.0 h. The pH of the mixture was adjusted to 3 with concentrated hydrochloric acid. The mixture was extracted with ethyl acetate. The organic phase was washed with saturated brine and concentrated. The residue was dissolved in N,N-dimethylformamide. 4-(2,3-difluoro-4-(1H-pyrazol-4-yl)phenyl)piperidine (0904-97) (30 mg, 0.09 mmol, 1.0 eq.), HATU (37 mg, 0.1 mmol, 1.1 eq.) and triethylamine (45 mg, 0.45 mmol, 5.0 eq.) were added to the solution. The mixture was stirred at room temperature for 1.0 h. The reaction was quenched with water and extracted with ethyl acetate. The organic layer was washed with saturated brine, dried over anhydrous sodium sulfate, and concentrated under reduced pressure. The residue was purified by prep-TLC (silica gel, dichloromethane/methanol = 20/1) to give a yellow solid product 2-(4-(2,3-difluoro-4-(1H-pyrazol-4-yl)phenyl)piperidine-1-carbonyl)cyclohexane-1-o ne (16 mg, yield: 45.71%). LCMS (ESI): m/z =389 [M+1]⁺. ¹ H NMR (500 MHz, DMSO) δ 13.13 (s, 1H), 8.17 (s, 1H), 7.93 (s, 1H), 7.49 (dd, *J =* 16.6, 8.3 Hz, 1H), 7.09 (dt, *J =* 30.5, 7.3 Hz, 1H), 4.73 - 4.43 (m, 1H), 4.05 - 3.86 (m, 1H), 3.80 (s, 1H), 3.15 - 2.91 (m, 2H), 2.75 - 2.52 (m, 2H), 2.28 (t, *J =* 14.3 Hz, 1H), 2.05 - 1.88 (m, 3H), 1.86 - 1.55 (m, 7H).

### Example 77: Preparation of (4-(2,3-difluoro-4-(1H-pyrazol-4-yl)phenyl)piperidin-1-yl)(1-hydroxycyclohexyl) methanone (Compound 110) (Prepared according to Scheme 9)

A mixture of 1-hydroxycyclohexane-1-carboxylic acid (14 mg, 0.13 mmol, 1.05 eq.), 4-(2,3-difluoro-4-(1H-pyrazol-4-yl)phenyl)piperidine hydrochloride (0904-97) (40 mg, 0.12 mmol, 1.0 eq.), N,N-diisopropylethylamine (93 mg, 0.72 mmol, 6.0 eq.), HATU (51 mg, 0.13 mmol, 1.1 eq.) in N,N-dimethylformamide (3 ml) was stirred at room temperature for 1 hour. The reaction was diluted with water and extracted with ethyl acetate. The organic phase was washed with saturated brine, dried over anhydrous sodium sulfate and concentrated under reduced pressure. The residue was purified by thick prep-TLC (silica gel, eluent: dichloromethane/methanol = 10/1) to give a white solid product (4-(2,3-difluoro-4-(1H-pyrazol-4-yl)phenyl)piperidin-1-yl)(1-hydroxycyclohexyl)met hanone (23 mg, yield: 49.21%). LCMS (ESI): m/z = 390 [M+H]⁺. ¹ H NMR (500 MHz, DMSO) δ 13.13 (s, 1H), 8.17 (s, 1H), 7.93 (s, 1H), 7.48 (t, *J =* 7.4 Hz, 1H), 7.10 (t, *J =* 7.4 Hz, 1H), 5.19 (s, 1H), 4.82 (s, 2H), 3.11 (t, *J =* 12.0 Hz, 1H), 2.79 (d, *J* = 30.0 Hz, 2H), 1.86 - 1.39 (m, 14H).

### Example 78: Preparation of 1-(4-(2,3-difluoro-4-(1H-pyrazol-4-yl)phenyl)piperidin-1-yl)-2-phenylethan-1-one (Compound 111) (Prepared according to Scheme 9)

Step 78a: Preparation of 2-phenylacetic acid: A mixture of methyl 2-phenylacetate (500 mg, 3.3 mmol, 1.0 eq.), sodium hydroxide (340 mg, 8.3 mmol, 2.5 eq.) in tetrahydrofuran (15 ml) was stirred at room temperature for 2.0 hours. The solvent was removed under reduced pressure. The residue was diluted with water, the pH was adjusted to 4 with hydrochloric acid solution, and extracted with dichloromethane. The organic phase was dried and concentrated under reduced pressure. The residue was purified by prep-TLC (silica gel, dichloromethane/methanol=80/1) to give a yellow solid product 2-phenylacetic acid (420 mg, yield: 92.9%). LCMS (ESI): m/z =137[M+1]⁺.

Step 78b: Preparation of 1-(4-(2,3-difluoro-4-(1H-pyrazol-4-yl)phenyl)piperidin-1-yl)-2-phenylethan-1-one ( Compound 111): A mixture of 4-(2,3-difluoro-4-(1H-pyrazol-4-yl)phenyl)piperidine (0904-97) (32 mg, 0.1 mmol, 1.0 eq.), 2-phenylacetic acid (18 mg, 0.12 mmol, 1.2 eq.), triethylamine (30.3 mg, 0.3 mmol, 3.0 eq.), HATU (49.4 mg, 0.12 mmol, 1.2 eq.) in N,N-dimethylformamide (4 ml) was stirred at room temperature for 4.0 hours. The reaction was diluted with water and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 50/1 to 1/2) to give yellow oily product 1-(4-(2,3-difluoro-4-(1H-pyrazol-4-yl)phenyl)piperidin-1-yl)-2-phenylethan-1-one (15 mg, yield: 39.2%). LCMS (ESI): m/z = 382 (M+H)⁺. Melting point : 141-152 °C. ¹ H NMR (500 MHz, DMSO) δ 13.07 (s, 1H), 8.05 (s, 2H), 7.47 (t, *J =* 7.4 Hz, 1H), 7.32 - 7.11 (m, 5H), 7.03 (t, *J =* 7.3 Hz, 1H), 4.57 (d, *J =* 12.7 Hz, 1H), 4.07 (d, *J =* 13.0 Hz, 1H), 3.75 (s, 2H), 3.09 (dt, *J =* 24.1, 12.1 Hz, 2H), 2.66 (t, *J =* 12.2 Hz, 1H), 1.73 (dd, *J =* 30.3, 12.4 Hz, 2H), 1.55 - 1.39 (m, 2H).

### Example 79: Preparation of (4-(2,3-difluoro-4-(1H-pyrazol-4-yl)phenyl)piperidin-1-yl)((1s,4r)-4-pentylcycloh exyl)methanone (Compound 112) (Prepared according to Scheme 9)

Under nitrogen protection, a mixture of 4-(2,3-difluoro-4-(1H-pyrazol-4-yl)phenyl)piperidine hydrochloride (0904-97) (33 mg, 0.099 mmol, 1.0 eq.), (1s,4r)-4-pentylcyclohexane-1-carboxylic acid (24 mg, 0.119 mmol, 1.2 eq.), HATU (57 mg, 0.148 mmol, 1.5 eq.), N,N-diisopropylethylamine (39 mg, 0.296 mmol, 3.0 eq.) in N,N-dimethylformamide (3 ml) was stirred at room temperature for 1 hour. The reaction was diluted with water and extracted with ethyl acetate. The organic layer was dried and concentrated under reduced pressure. The residue was purified by prep-TLC (silica gel, dichloromethane/methanol = 20/1) to give a yellow solid product (4-(2,3- difluoro -4-(1H-pyrazol-4-yl)phenyl)piperidin-1-yl)((1s,4r)-4-pentylcyclohexyl)methanone (20 mg, yield: 46.51%). LCMS (ESI): m/z =444[M+1]⁺. ¹ H NMR (500 MHz, DMSO) δ 13.13 (s, 1H), 8.17 (s, 1H), 7.93 (s, 1H), 7.47 (t, *J =* 7.2 Hz, 1H), 7.13 (t, *J =* 7.1 Hz, 1H), 4.57 (d, *J =* 11.2 Hz, 1H), 4.06 (d, *J =* 11.3 Hz, 1H), 3.10 (dd, *J =* 24.4, 12.3 Hz, 2H), 2.77 - 2.53 (m, 2H), 1.84 - 1.12 (m, 21H), 0.86 (t, *J =* 6.8 Hz, 3H).

### Example 80: Preparation of N-((1-(4-(1H-pyrazol-4-yl)phenyl)piperidin-4-yl)methyl)-6-phenylhexanamide (Compound 113) (Prepared according to Scheme 5)

Step 80a: Preparation of 6-phenyl-N-((1-(4-(1-(tetrahydro-2H-pyran-2-yl)-1H-pyrazol-4-yl)phenyl)piperidin-4 -yl)methyl)hexanamide (Compound 0506-113): A mixture of 6-phenylhexanoic acid (38 mg, 0.19 mmol, 1.1 eq.), (1-(4-(1-(tetrahydro-2H-pyran-2-yl)-1H-pyrazol-4-yl)phenyl)piperidin-4-yl)methana mine (0509-45) (60 mg, 0.18 mmol, 1.0 eq.), N,N-diisopropylethylamine (70 mg, 0.54 mmol, 3.0 eq.), HATU (74 mg, 0.19 mmol, 1.1 eq.) in N,N-dimethylformamide (3 ml) was stirred at room temperature for 2 hours. The reaction was diluted with water and extracted with ethyl acetate. The organic phase was washed with saturated brine, dried over anhydrous sodium sulfate and concentrated under reduced pressure. The residue was purified by prep-TLC (silica gel, eluent: dichloromethane/methanol = 30/1) to give a yellow solid product 6-phenyl-N-((1-(4-(1-(tetrahydro-2H-pyran-2-yl)-1H)-pyrazol-4-yl)phenyl)piperidin-4-yl)methyl)hexanamide (68 mg, yield: 73.35%), LCMS (ESI): m/z = 515 [M+H]⁺.

Step 80b: Preparation of N-((1-(4-(1H-pyrazol-4-yl)phenyl)piperidin-4-yl)methyl)-6-phenylhexanamide (Compound 113): To a mixed solution of 6-phenyl-N-((1-(4-(1-(tetrahydro-2H-pyran-2-yl)-1H)-pyrazol-4-yl)phenyl)piperidin-4-yl)methyl)hexanamide (0506-113) (68 mg, 0.132 mmol, 1.0 eq.) in dichloromethane (5 mL) was added trifluoroacetic acid (2 mL). The mixture was stirred at room temperature for 2 hours. The solvent was removed under reduced pressure, and the pH of the residue was adjusted to 9 with sodium carbonate solution and extracted with dichloromethane. The organic phase was dried over anhydrous sodium sulfate and concentrated under reduced pressure. The residue was purified by prep-TLC (silica gel, eluent: dichloromethane/methanol = 15/1) to give white solid product N-((1-(4-(1H-pyrazol-4-yl)phenyl)piperidin-4-yl)methyl)-6-phenylhexanamide (53 mg, yield: 93.25%). LCMS (ESI): m/z = 431 [M+H]⁺. ¹ H NMR (500 MHz, DMSO) δ 12.75 (s, 1H), 7.87 (d, *J =* 99.8 Hz, 3H), 7.41 (d, *J =* 8.5 Hz, 2H), 7.26 (t, *J =* 7.5 Hz, 2H), 7.16 (dd, *J =* 14.9, 7.3 Hz, 3H), 6.91 (d, *J =* 8.5 Hz, 2H), 3.65 (d, *J =* 12.2 Hz, 2H), 2.96 (t, *J =* 6.2 Hz, 2H), 2.58 (dd, *J =* 21.2, 9.7 Hz, 4H), 2.07 (t, *J =* 7.3 Hz, 2H), 1.69 (d, *J =* 12.2 Hz, 2H), 1.55 (dt, *J =* 15.8, 7.6 Hz, 5H), 1.26 (dd, *J =* 17.4, 10.1 Hz, 4H).

### Example 81: Preparation of N-((1-(4-(1H-pyrazol-4-yl)phenyl)piperidin-4-yl)methyl)oct-7-ynamide (Compound 114) (Prepared according to Scheme 5)

Step 81a: Preparation of N-((1-(4-(1-(tetrahydro-2H-pyran-2-yl)-1H-pyrazol-4-yl)phenyl)piperidin-4-yl)methy l)oct-7-ynamide (Compound 0506-114) : A mixture of (1-(4-(1-(tetrahydro-2H-pyran-2-yl)-1H-pyrazol-4-yl)phenyl)piperidin-4-yl)methana mine (0509-45) (50 mg, 0.14 mmol, 1.0 eq.), oct-7-ynyl acid (25 mg, 0.17 mmol, 1.2 eq.), HATU (73 mg, 0.19 mmol, 1.3 eq.), and triethylamine (30 mg, 0.29 mmol, 2.0 eq.) in N,N-dimethylformamide (5 mL) was stirred at room temperature for 1.0 h. The reaction was quenched with water and extracted with ethyl acetate. The organic layer was washed with saturated brine, dried over anhydrous sodium sulfate, and concentrated under reduced pressure. The residue was purified by prep-TLC (silica gel, dichloromethane/methanol = 20/1) to give a yellow solid product N-((1-(4-(1-(tetrahydro-2H-pyran-2-yl)-1H-pyrazol-4-yl)phenyl)piperidin-4-yl)methy l)oct-7-ynamide (58 mg, yield: 85.42%). LCMS (ESI): m/z = 463 [M+1]⁺.

Step 81b: Preparation of N-((1-(4-(1H-pyrazol-4-yl)phenyl)piperidin-4-yl)methyl)oct-7-ynamide (Compound 114): A mixture of N-((1-(4-(1-(tetrahydro-2H-pyran-2-yl)-1H-pyrazol-4-yl)phenyl)piperidin-4-yl)methy l)oct-7-ynamide (0506-114) (58 mg, 0.125 mmol, 1.0 eq.) in hydrogen chloride - dioxane solution (4 M, 3 mL) was stirred at room temperature for 1.0 h. The mixture was concentrated and the pH of the residue was adjusted to 9 with sodium carbonate solution. The mixture was filtered and slurried with methanol. The mixture was filtered and dried to give the white solid product N-((1-(4-(1H-pyrazol-4-yl)phenyl)piperidin-4-yl)methyl)oct-7-ynamide (21 mg, yield: 44.21%). LCMS (ESI): m/z =380[M+1]⁺. ¹ H NMR (500 MHz, DMSO) δ 12.75 (s, 1H), 7.99 (s, 1H), 7.78 (t, *J =* 5.2 Hz, 2H), 7.41 (d, *J =* 8.6 Hz, 2H), 6.91 (d, *J =* 8.7 Hz, 2H), 3.66 (d, *J =* 12.3 Hz, 2H), 2.97 (t, *J =* 6.2 Hz, 2H), 2.72 (s, 1H), 2.60 (t, *J =* 11.4 Hz, 2H), 2.14 (td, *J =* 7.0, 2.5 Hz, 2H), 2.0 2H), 1.71 (d, *J =* 12.3 Hz, 2H), 1.57 - 1.38 (m, 6H), 1.35 - 1.28 (m, 2H), 1.24 (t, *J =* 10.5 Hz, 2H).

### Example 82: Preparation of N-((1-(4-(1H-pyrazol-4-yl)phenyl)piperidin-4-yl)methyl)oct-7-enamide (Compound 115) (Prepared according to Scheme 5)

Step 82a: Preparation of N-((1-(4-(1-(tetrahydro-2H-pyran-2-yl)-1H-pyrazol-4-yl)phenyl)piperidin-4-yl)methy 1)oct-7-enamide (Compound 0506-115): Under nitrogen protection, a mixture of (1-(4-(1-(tetrahydro-2H-pyran-2-yl)-1H-pyrazol-4-yl)phenyl)piperidin-4-yl)methana mine (50 mg, 0.147 mmol, 1.0 eq.), oct-7-enoic acid (21 mg, 0.147 mmol, 1.0 eq.), HATU (84 mg, 0.221 mmol, 1.5 eq.), N,N-diisopropylethylamine (57 mg, 0.441 mmol, 3.0 eq.) in N,N-dimethylformamide (5 ml) was stirred at room temperature for 1 hour. The reaction was diluted with water and extracted with ethyl acetate. The organic layer was dried and concentrated under reduced pressure. The residue was purified by column chromatography on silica gel (dichloromethane/methanol = 15/1) to obtain a yellow solid product N-((1-(4-(1-(tetrahydro-2H-pyran-2-yl)-1H-pyrazol-4-yl)phenyl)piperidin-4-yl)methy 1)oct-7-enamide (35 mg, yield: 51.47%). LCMS (ESI): m/z = 465[M+1]⁺.

Step 82b: Preparation of N-((1-(4-(1H-pyrazol-4-yl)phenyl)piperidin-4-yl)methyl)oct-7-enamide (Compound 115): A mixture of N-((1-(4-(1-(tetrahydro-2H-pyran-2-yl)-1H-pyrazol-4-yl)phenyl)piperidin-4-yl)methy 1)oct-7-enamide (0509-45) (35 mg, 0.075 mmol, 1.0 eq.) in hydrogen chloride - methanol solution (4 M, 3 mL) was stirred at room temperature for 1 hour. The solvent was removed under reduced pressure, and the residue was diluted with water, adjusted to pH = 8 with sodium carbonate solution, and extracted with dichloromethane. The organic phase was dried and concentrated under reduced pressure. The residue was purified by prep-TLC (silica gel, dichloromethane/methanol = 15/1) to give a yellow solid product N-((1-(4-(1H-pyrazol-4-yl)phenyl)piperidin-4-yl)methyl)oct-7-enamide (20 mg, yield: 71.43%). LCMS(ESI) : m/z =381[M+1]⁺. ¹ H NMR (500 MHz, DMSO) δ 12.75 (s, 1H), 7.77 (s, 3H), 7.41 (d, *J* = 8.2 Hz, 2H), 6.91 (d, *J* = 8.0 Hz, 2H), 5.85 - 5.61 (m, 1H), 4.97 (dd, *J* = 29.8, 13.6 Hz, 2H), 3.66 (d, *J* = 11.8 Hz, 2H), 2.97 (t, *J* = 5.9 Hz, 2H), 2.60 (t, *J* = 11.4 Hz, 2H), 2.07 (t, *J* = 7.3 Hz, 2H), 2.18 (d, *J* = 13.7 Hz, 2H), 3. 6.8 Hz, 2H), 1.70 (d, *J =* 12.0 Hz, 2H), 1.50 (dt, *J =* 14.6, 7.3 Hz, 3H), 1.40 - 1.32 (m, 2H), 1.24 (t, *J* = 10.3 Hz, 4H).

### Example 83: Preparation of 1-(cyclohexylsulfonyl)-4-(2-fluoro-4-(1H-pyrazol-4-yl)phenyl)piperidine (Compound 119) (Prepared according to Scheme 9)

Step 83a: Preparation of tert-butyl 4-(2-fluoro-4-(1-(tetrahydro-2H-pyran-2-yl)-1H-pyrazol-4-yl)phenyl)-3,6-dihydropyri dine-1(2H)-carboxylate (Compound 0902-119): To a mixture of 4-(4-bromo-3-fluorophenyl)-1-(tetrahydro-2H-pyran-2-yl)-1H-pyrazole (0103-2) (0.3 g, 0.92 mmol, 1.0 eq.), 1,1'-bis(diphenylphosphino)ferrocenepalladium(II) chloride (53 mg, 0.065 mmol, 0.07 eq.), sodium carbonate (294 mg, 2.77 mmol, 3.0 eq.) in dioxane (7.5 ml) and water (1.5 ml) was added tert-butyl 4-(4,4,5,5-tetramethyl-1,3,2-dioxaborolan-2-yl)-3,6-dihydropyridine-1(2H)-carboxyla te (343 mg, 1.11 mmol, 1.2 eq.). The mixture was heated to 100°C under nitrogen atmosphere for overnight reaction. The solvent was removed under reduced pressure. The residue was separated by column chromatography on silica gel (petroleum ether: ethyl acetate 4: 1) to give a pale yellow oily product tert-butyl 4-(2-fluoro-4-(1-(tetrahydro-2H-pyran-2-yl)-1H-pyrazol-4-yl)phenyl)-3,6-dihydropyri dine-1(2H)-carboxylate (408 mg, yield: 100%). LCMS (ESI): m/z = 428 [M+1]⁺; TLC: Rf 0.5 (petroleum ether: ethyl acetate = 2: 1).

Step 83b: Preparation of tert-butyl 4-(2-fluoro-4-(1-(tetrahydro-2H-pyran-2-yl)-1H-pyrazol-4-yl)phenyl)piperidine-1-car boxylate (Compound 0903-119): To a mixture of tert-butyl 4-(2-fluoro-4-(1-(tetrahydro-2H-pyran-2-yl)-1H-pyrazol-4-yl)phenyl)-3,6-dihydropyri dine-1(2H)-carboxylate (0902-119) (394 mg, 0.92 mmol, 1.0 eq.) in ethanol (10 mL) was added palladium hydroxide on carbon (80 mg). The mixture was heated to 65 °C under hydrogen balloon pressure and reacted overnight. The mixture was filtered. The filtrate was concentrated under reduced pressure to give tert-butyl 4-(2-fluoro-4-(1-(tetrahydro-2H-pyran-2-yl)-1H-pyrazol-4-yl)phenyl)piperidine-1-car boxylate (375 mg, yield: 95%) as a light yellow oil. LCMS (ESI): m/z = 430 [M+1]⁺; TLC: Rf 0.5 (petroleum ether:ethyl acetate = 2:1).

Step 83c: Preparation of 4-(2-fluoro-4-(1H-pyrazol-4-yl)phenyl)piperidine hydrochloride (Compound 0904-119): A mixture of tert-butyl 4-(2-fluoro-4-(1-(tetrahydro-2H-pyran-2-yl)-1H-pyrazol-4-yl)phenyl)piperidine-1-car boxylate (0903-119) (375 mg, 0.87 mmol, 1.0 eq.) in hydrogen chloride - methanol solution (4 M, 5 mL) was stirred at room temperature for 3 hours. The solvent was removed under reduced pressure. The residue was dried under vacuum to give 4-(2-fluoro-4-(1H-pyrazol-4-yl)phenyl)piperidine hydrochloride (258 mg, crude) as a white solid. LCMS (ESI): m/z = 246 [M+1]⁺; TLC: Rf 0.2 (dichloromethane:methanol = 10:1).

Step 83d: Preparation of 1-(cyclohexylsulfonyl)-4-(2-fluoro-4-(1H-pyrazol-4-yl)phenyl)piperidine (Compound 119): To a mixture of 4-(2-fluoro-4-(1H-pyrazol-4-yl)phenyl)piperidine hydrochloride (0904-119) (60 mg, 0.21 mmol, 1.0 eq.) and N,N-diisopropylethylamine (82 mg, 0.63 mmol, 3.0 eq.) in N,N-dimethylformamide (2 ml) was added cyclohexylsulfonyl chloride (39 mg, 0.21 mmol, 1.0 eq.). The mixture was stirred at room temperature overnight. The mixture was diluted with water (20 ml). The aqueous layer was extracted with ethyl acetate (15 ml × 3). The combined organic layer was washed with saturated brine (15 ml × 1), dried over anhydrous sodium sulfate and concentrated. The residue was purified by Pre-TLC (dichloromethane: methanol = 10: 1) to give 1-(cyclohexylsulfonyl)-4-(2-fluoro-4-(1H-pyrazol-4-yl)phenyl)piperidine (11 mg, yield: 13%) as a white solid. LCMS (ESI): m/z = 392 [M+1]⁺; TLC: Rf 0.5 (dichloromethane: methanol = 10: 1). ¹ H NMR (500 MHz, DMSO) δ 12.94 (s, 1H), 8.21 (s, 1H), 7.93 (s, 1H), 7.46 - 7.34 (m, 2H), 7.29 (t, *J* = 8.1 Hz, 1H), 3.76 (d, *J =* 12.4 Hz, 2H), 3.11 (t, *J* = 11.6 Hz, 1H), 2.99 (dt, *J* = 36.5, 11.9 Hz, 3H), 2.02 (d, *J* = 11.1 Hz, 2H), 1.79 (d, *J =* 11.6 Hz, 4H), 1.66 (dd, *J =* 22.6, 12.7 Hz, 3H), 1.34 (ddd, *J* = 27.7, 17.8, 10.3 Hz, 5H).

### Example 84: Preparation of 4-(2-fluoro-4-(1H-pyrazol-4-yl)phenyl)-1-((4-fluorophenyl)sulfonyl)piperidine (Compound 120) (Prepared according to Scheme 9)

To a mixture of 4-(2-fluoro-4-(1H-pyrazol-4-yl)phenyl)piperidine hydrochloride (0904-119) (50 mg, 0.18 mmol, 1.0 eq.), N,N-diisopropylethylamine (68 mg, 0.54 mmol, 3.0 eq.) in N,N-dimethylformamide (2 ml) was added 4-fluorobenzenesulfonyl chloride (35 mg, 0.18 mmol, 1.0 eq.). The mixture was stirred at room temperature for 3 hours. The mixture was diluted with water (30 ml). The aqueous layer was extracted with ethyl acetate (15 ml × 3). The combined organic layer was washed with saturated brine (20 ml × 1), dried over anhydrous sodium sulfate and concentrated. The residue was purified by Pre-TLC (dichloromethane: methanol = 10: 1) to give 4-(2-fluoro-4-(1H-pyrazol-4-yl)phenyl)-1-((4-fluorophenyl)sulfonyl)piperidine (35 mg, yield: 49%) as a white solid. LCMS (ESI): m/z = 404 [M+1]⁺; TLC: Rf 0.5 (dichloromethane: methanol = 10: 1). ¹ H NMR (500 MHz, DMSO) δ 12.93 (s, 1H), 8.20 (s, 1H), 7.92 (s, 1H), 7.85 (dd, *J* = 8.0, 5.3 Hz, 2H), 7.51 (t, *J* = 8.6 Hz, 2H), 7.38 (d, *J =* 9.9 Hz, 2H), 7.25 (t, *J =* 7.9 Hz, 1H), 3.79 (d, *J =* 11.4 Hz, 2H), 2.75 (d, *J* = 12.6 Hz, 1H), 2.39 (t, *J* = 11.3 Hz, 2H), 1.76 (dd, *J* = 33.4, 10.7 Hz, 4H).

### Example 85: Preparation of cyclopentyl(7-(2-fluoro-4-(1H-pyrazol-4-yl)phenyl)-2,7-diazaspiro [4.4] nonan-2-yl) methanone (Compound 124) (Prepared according to Scheme 11)

Step 85a: Preparation of tert-butyl 7-(cyclopentanecarbonyl)-2,7-diazaspiro[4.4]nonane-2-carboxylate (Compound 1102-124): A mixture of tert-butyl 2,7-diazaspiro[4.4]nonane-2-carboxylate (1101-124) (400 mg, 1.77 mmol, 1.0 eq.), cyclopentanecarboxylic acid (200 mg, 1.77 mmol, 1.0 eq.), triethylamine (536 mg, 5.31 mmol, 3.0 eq.), HATU (874 mg, 2.3 mmol, 1.3 eq.) in N,N-dimethylformamide (15 ml) was stirred at room temperature for 4.0 hours. The reaction was diluted with water and extracted with ethyl acetate. The organic layer was washed with saturated brine, dried over anhydrous sodium sulfate, and concentrated under reduced pressure. The residue was purified by column chromatography on silica gel (petroleum ether/ethyl acetate = 50/1 to 10/1) to give yellow oily product tert-butyl 7-(cyclopentanecarbonyl)-2,7-diazaspiro[4.4]nonane-2-carboxylate (400 mg, yield: 70.3%). LCMS (ESI): m/z = 323 (M+H)⁺.

Step 85b: Preparation of cyclopentyl(2,7-diazaspiro[4.4]nonane-2-yl)methanone (Compound 1103-124): A mixture of tert-butyl 7-(cyclopentanecarbonyl)-2,7-diazaspiro[4.4]nonane-2-carboxylate (1102-124) (400 mg, 1.24 mmol, 1.0 eq.) in hydrogen chloride - methanol solution (4 M, 5 mL) was stirred at room temperature for 2.0 hours. The solvent was removed under reduced pressure. The residue was diluted with water and the pH was adjusted to 9 with sodium carbonate solution. It was extracted with dichloromethane. The organic phase was dried and concentrated under reduced pressure to give cyclopentyl(2,7-diazaspiro[4.4]nonane-2-yl)methanone (270 mg, yield: 97.4%) as a yellow oil. LCMS (ESI): m/z = 223[M+1]⁺.

Step 85c: Preparation of cyclopentyl(7-(2-fluoro-4-(1-(tetrahydro-2H-pyran-2-yl)-1H-pyrazol-4-yl)phenyl)-2,7 -diazaspiro[4.4]nonan-2-yl)methanone (Compound 1104-124): Under nitrogen protection, a mixture of cyclopentyl (2,7-diazaspiro [4.4] nonan-2-yl)methanone (1103-124) (173 mg, 0.782 mmol, 1.5 eq.), 4- (4-bromo-3-fluorophenyl) -1-(tetrahydro-2H-pyran-2-yl) -1H-pyrazole (170 mg, 0.521 mmol, 1.0 eq.), sodium tert-butoxide (124 mg, 1.3 mmol, 2.5 eq.), 4,5-bis (diphenylphosphino) -9,9-dimethylxanthene (43.2 mg, 0.074 mmol, 0.05 eq.), tris (dibenzylideneacetone) dipalladium (67.7 mg, 0.074 mmol, 0.05 eq.) in toluene (6 ml) was stirred at 120 °C overnight. The reaction was diluted with water and extracted with ethyl acetate, and the organic phase was dried and concentrated under reduced pressure. The residue was purified by column chromatography on silica gel (dichloromethane/methanol = 150/1 to 50/1) to give a yellow solid product cyclopentyl (7-(2-fluoro-4-(1-(tetrahydro-2H-pyran-2-yl)-1H-pyrazol-4-yl)phenyl)-2,7-diazaspiro [4.4] nonan-2-yl)methanone (120 mg, yield: 49.5%). LCMS (ESI): m/z = 467 [M+1]⁺.

Step 85d: Preparation of cyclopentyl(7-(2-fluoro-4-(1H-pyrazol-4-yl)phenyl)-2,7-diazaspiro[4.4]nonan-2-yl)m ethanone (Compound 124): A mixture of cyclopentyl(7-(2-fluoro-4-(1-(tetrahydro-2H-pyran-2-yl)-1H-pyrazol-4-yl)phenyl)-2,7 -diazaspiro[4.4]nonan-2-yl)methanone (1104-124) (70 mg, 0.15 mmol, 1.0 eq.) in hydrogen chloride - methanol solution (4 M, 4 mL) was stirred at room temperature for 2.0 hours. The solvent was removed under reduced pressure. The residue was diluted with water, adjusted to pH 9 with sodium carbonate solution and extracted with dichloromethane. The organic phase was dried and concentrated under reduced pressure. The residue was purified by prep-TLC (silica gel, dichloromethane/methanol = 10/1) to give a yellow solid product cyclopentyl (7- (2-fluoro-4- (1H-pyrazol-4-yl) phenyl) -2,7-diazaspiro [4.4] nonan-2-yl)methanone (51 mg, yield: 89.4%). LCMS (ESI): m/z = 383 [M + 1]⁺. Melting point: 122 ~ 130 °C. ¹H NMR (500 MHz, DMSO) δ 12.79 (s, 1H), 8.05 (s, 1H), 7.82 (s, 1H), 7.32 (d, *J* = 15.3 Hz, 1H), 7.24 (d, *J* = 8.1 Hz, 1H), 6.70 (td, *J* = 9.3, 3.9 Hz, 1H), 3.59 (t, *J* = 6.9 Hz, 1H), 3.44 (ddd, *J* = 31.3, 16.1, 8.7 Hz, 4H), 3.28 (s, 3H), 2.88 - 2.72 (m, 1H), 2.02 - 1.85 (m, 4H), 1.77 (s, 2H), 1.62 (d, *J* = 5.9 Hz, 4H), 1.51 (d, *J* = 4.9 Hz, 2H).

### Example 86: Preparation of 1-((3-(4-(1H-pyrazol-4-yl)phenyl)-3-azabicyclo[3.1.0]hexan-6-yl)methyl)pyrrolidi n-2-one (Compound 125) (Prepared according to Scheme 12)

Step 86a: Preparation of tert-butyl 6-((2-oxopyrrolidin-1-yl)methyl)-3-azabicyclo[3.1.0]hexane-3-carboxylate (Compound 1202-125): Under nitrogen protection, a mixture of tert-butyl 6-(hydroxymethyl)-3-azabicyclo[3.1.0]hexane-3-carboxylate (1201-125) (900 mg, 4.23 mmol, 1.0 eq.), methanesulfonyl chloride (810 mg, 6.77 mmol, 1.6 eq.), triethylamine (936 mg, 8.46 mmol, 2.0 eq.) in dichloromethane (15 ml) was stirred at room temperature for 4.0 hours. The reaction was diluted with water and extracted with dichloromethane. The organic layer was dried and concentrated under reduced pressure. The residue was purified by column chromatography on silica gel (petroleum ether/ethyl acetate = 50/1 to 10/1) to give a yellow oily product tert-butyl 6-(((methylsulfonyl)oxy)methyl)-3-azabicyclo[3.1.0]hexane-3- carboxylate (1.15 g, yield: 93.2%). LCMS (ESI): m/z = 292 (M+H)⁺. Under nitrogen protection, sodium hydride (165 mg, 4.12 mmol, 2.0 eq.) was added in portions to a mixture of 2-pyrrolidone (350 mg, 4.12 mmol, 2.0 eq.) in N,N-dimethylformamide (15 ml), and the mixture was stirred at 0 °C for 1 hour. Then, a solution of tert-butyl 6-(((methylsulfonyl)oxy)methyl)-3-azabicyclo[3.1.0]hexane-3-carboxylate (600 mg, 2.06 mmol, 1.0 eq.) in N,N-dimethylformamide (1 ml) was added dropwise under an ice bath. The reactants were stirred at 65 °C for 6 hours. Water and ethyl acetate were added for extraction. The organic layer was washed with saturated brine and concentrated under reduced pressure to obtain tert-butyl 6-((2-oxopyrrolidin-1-yl)methyl)-3-azabicyclo[3.1.0]hexane-3-carboxylate (550 mg) as a yellow oil. LCMS (ESI): m/z = 281 (M+H)⁺.

Step 86b: Preparation of 1-((3-azabicyclo[3.1.0]hexan-6-yl)methyl)pyrrolidin-2-one (Compound 1203-125): A mixture of tert-butyl 6-((2-oxopyrrolidin-1-yl)methyl)-3-azabicyclo[3.1.0]hexane-3-carboxylate (1202-125) (550 mg, 1.96 mmol, 1.0 eq.) in hydrogen chloride-methanol solution (4 M, 8 ml) was stirred at room temperature for 2.0 hours. The solvent was removed under reduced pressure. The residue was diluted with water, adjusted to pH = 9 with sodium carbonate solution, and extracted with dichloromethane. The organic phase was dried and concentrated under reduced pressure to give 1-((3-azabicyclo[3.1.0]hexan-6-yl)methyl)pyrrolidin-2-one (260 mg, yield: 73.4%) as a yellow oil. LCMS (ESI): m/z =181 [M+1]⁺.

Step 86c: Preparation of 1-((3-(4-(1-(tetrahydro-2H-pyran-2-yl)-1H-pyrazol-4-yl)phenyl)-3-azabicyclo[3.1.0]h exan-6-yl)methyl)pyrrolidin-2-one (Compound 1204-125): Under nitrogen protection, 1-((3-azabicyclo[3.1.0]hexan-6-yl)methyl)pyrrolidin-2-one (1203-125) (100 mg, 0.556 mmol, 1.5 eq.), 4-(4-bromophenyl)-1-(tetrahydro-2H-pyran-2-yl)-1H-pyrazole (0103-1) (114 mg, 0.37 mmol, 1.0 eq.), sodium tert-butoxide (105 mg, 1.11 mmol, 3.0 eq.), 2-dicyclohexylphosphino-2',6'-diisopropoxy-1,1'-biphenyl (8.6 mg, 0.019 mmol, 0.05 eq.) and tris(dibenzylideneacetone)dipalladium (17.4 mg, 0.019 mmol, 0.05 eq.) in toluene (5 ml) was stirred at 120° C overnight. The reaction was diluted with water and extracted with ethyl acetate, and the organic phase was dried and concentrated under reduced pressure. The residue was purified by prep-TLC (silica gel, dichloromethane/methanol = 150/1 to 50/1) to give a yellow solid product 1-((3-(4-(1-(tetrahydro-2H-pyran-2-yl)-1H-pyrazol-4-yl)phenyl)-3-azabicyclo[3.1.0]h exan-6-yl)methyl)pyrrolidin-2-one (110 mg, yield: 73.3%). LCMS (ESI): m/z = 407 [M+1]⁺.

Step 86d: Preparation of 1-((3-(4-(1H-pyrazol-4-yl)phenyl)-3-azabicyclo[3.1.0]hexan-6-yl)methyl)pyrrolidin-2 -one (Compound 125): A mixture of 1-((3-(4-(1-(tetrahydro2H-pyran-2-yl)-1H-pyrazol-4-yl)phenyl)-3-azabicyclo[3.1.0]he xan-6-yl)methyl)pyrrolidin-2-one (1204-125) (110 mg, 0.27 mmol, 1.0 eq.) in hydrogen chloride-methanol solution (4 M, 5 ml) was stirred at room temperature for 2.0 hours. The solvent was removed under reduced pressure, and the residue was diluted with water, adjusted to pH = 9 with sodium carbonate solution, and extracted with dichloromethane. The organic phase was dried and concentrated under reduced pressure. The residue was purified by prep-TLC (silica gel, dichloromethane /methanol = 15/1) to give a yellow solid product 1-((3-(4-(1H-pyrazol-4-yl)phenyl)-3-azabicyclo[3 .1.0]hexane-6-yl)methyl)pyrrolidin-2-one (60 mg, yield: 68.9%). LCMS (ESI): m/z = 323 [M+1]⁺. Melting point : 185~194 ° C. ¹ H NMR (500 MHz, DMSO) δ 12.70 (s, 1H), 7.95 (s, 1H), 7.74 (s, 1H), 7.36 (d, *J* = 8.5 Hz, 2H), 6.52 (d, *J* = 8.6 Hz, 2H), 3.52 (d, *J* = 9.2 Hz, 2H), 3.43 (t, *J* = 7.0 Hz, 2H), 3.13 (t, *J* = 8.7 Hz, 4H), 2.22 (t, *J* = 8.1 Hz, 2H), 2.00 - 1.88 (m, 2H), 1.64 (s, 2H), 0.91 - 0.78 (m, 1H).

### Example 87: Preparation of 1-(2-(4-(1H-pyrazol-4-yl)phenyl)isoindolin-5-ylmethyl)pyrrolidin-2-one (Compound 126) (Prepared according to Scheme 12)

Step 87a: Preparation of tert-butyl 5-(hydroxymethyl)isoindoline-2-carboxylate (Compound 1205-126): Under carbon monoxide atmosphere (50 psi), a mixture of tert-butyl 5-bromoisoindoline-2-carboxylate (2.0 g, 6.71 mmol, 1.0 eq.), 1,3-bis(diphenylphosphino)propane (554 mg, 1.34 mmol, 0.2 eq.), palladium acetate (301 mg, 1.34 mmol, 0.2 eq.), triethylamine (6.78 g, 67.1 mmol, 10.0 eq.) in N,N-dimethylformamide (4 mL) and methanol (20 mL) was placed in a sealed reaction bottle and stirred at 80 °C for 12.0 hours. The reaction was extracted with ethyl acetate and water, and the organic phase was dried and concentrated under reduced pressure. The residue was purified by column chromatography on silica gel (petroleum ether: ethyl acetate = 4: 1) to give a yellow solid product 2-(tert-butyl) 5-methylisoindoline-2,5-dicarboxylate (1.55 g, yield: 83.7%). Under nitrogen protection, lithium aluminum hydride (205.8 mg, 5.41 mmol, 1.0 ^{eq}.) was added to a solution of 2-(tert-butyl) 5-methylisoindoline-2,5-dicarboxylate (1.5 g, 5.41 mmol, 1.0 eq.) in tetrahydrofuran (60 ml) at 0 °C, and the mixture was stirred at room temperature for 1.5 hours. The reaction was quenched with water and 15% sodium hydroxide solution and extracted with ethyl acetate. The organic phase was dried and concentrated under reduced pressure. The residue was purified by column chromatography on silica gel (petroleum ether: ethyl acetate = 5: 1 to 2/1) to give a white solid product tert-butyl 5-(hydroxymethyl)isoindoline-2-carboxylate (1.2 g, yield: 88.9%).

Step 87b: Preparation of tert-butyl 5-(2-oxopyrrolidin-1-yl)methyl)isoindoline-2-carboxylate (Compound 1206-126): Under nitrogen protection, methanesulfonyl chloride (1.1 g, 9.64 mmol, 2.0 eq.) was added to a solution of tert-butyl 5-(hydroxymethyl)isoindoline-2-carboxylate (1205-126) (1.2 g, 4.82 mmol, 1.0 eq.) and triethylamine (1.46 mg, 14.46 mmol, 3.0 eq.) in dichloromethane (20 ml) at 0 °C. The mixture was stirred at room temperature for 2.0 hours. The reaction was extracted with dichloromethane and water, and the organic phase was dried and concentrated under reduced pressure. The residue was purified by column chromatography on silica gel (petroleum ether: ethyl acetate = 10: 1 to 5/1) to obtain a white solid product tert-butyl 5-(chloromethyl)isoindoline-2-carboxylate (640 mg, yield: 49.7%). Under nitrogen protection, sodium hydrogen (187 mg, 4.67 mmol, 1.0 eq.) was added in batches to a solution of pyrrolidin-2-one (397 mg, 2.34 mmol, 1.0 eq.) in N,N-dimethylformamide (20 ml) at 0 °C, and the mixture was stirred at 60 °C for 1.0 hour. tert-butyl 5-(chloromethyl)isoindoline-2-carboxylate (624 mg, 2.34 mmol, 0.5 eq.) was dissolved in 3.0 mL N,N-dimethylformamide and added dropwise to the above reaction solution. The mixture was stirred at 60 °C overnight. The reaction was extracted with ethyl acetate and water, and the organic phase was dried and concentrated under reduced pressure. The obtained residue was purified by column chromatography on silica gel (eluent: petroleum ether: ethyl acetate = 3:1 to ethyl acetate) to obtain white solid tert-butyl 5-(2-oxopyrrolidin-1-yl)methyl)isoindoline-2-carboxylate (670 mg, yield: 45.4%). LCMS (ESI): m/z = 317 (M+H)⁺.

Step 87c: Preparation of 1-(isoindolin-5-ylmethyl)pyrrolidin-2-one hydrochloride (Compound 1207-126): tert-Butyl 5-(2-oxopyrrolidin-1-yl)methyl)isoindolin-2-carboxylate (1206-126) (670 mg, 2.12 mmol, 1.0 eq.) was dissolved in 1 mL methanol, and a solution of hydrogen chloride -dioxane (4 M, 5 ml) was added dropwise. The mixture was stirred at room temperature for 1.0 hour. The reaction was concentrated to dryness under reduced pressure, and the residue was used directly in the next step without further purification. LCMS (ESI): m/z = 217 (M+H)⁺.

Step 87d: Preparation of 1-(2-(4-(1-(tetrahydro-2H-pyran-2-yl)-1H-pyrazol-4-yl)phenyl)isoindolin-5-yl)methyl) pyrrolidin-2-one (Compound 1208-126): Under nitrogen protection, a mixture of 4-(4-bromophenyl)-1-tetrahydropyran-2-ylpyran-1H-pyrazole (0103-1) (213 mg, 0.694 mmol, 1.0 eq.), 1-(isoindolin-5-ylmethyl)pyrrolidin-2-one hydrochloride (1207-126) (150 mg, 0.694 mmol, 1.0 eq.), sodium tert-butoxide (166.7 mg, 1.73 mmol, 2.5 eq.), 4,5-bis(diphenylphosphino)-9,9-dimethylxanthene (65 mg, 0.139 mmol, 0.2 eq.) and tris(dibenzylideneacetone)dipalladium (63.6 mg, 0.069 mmol, 0.1 eq.) in toluene (10 ml) was stirred at 120 °C for 12 hours. The reaction was diluted with water and extracted with ethyl acetate. The organic phase was washed with saturated brine, dried over anhydrous sodium sulfate and concentrated under reduced pressure. The residue was purified by column chromatography on silica gel (petroleum ether/ethyl acetate = 4/1 to 1/1) to obtain a yellow solid product 1-(2-(4-(1-(tetrahydro-2H-pyran-2-yl)-1H-pyrazol-4-yl)phenyl)isoindolin-5-yl)methyl) pyrrolidin-2-one (86 mg, yield: 28.1%). LCMS (ESI): m/z = 443 [M+1]⁺.

Step 87e: Preparation of 1-(2-(4-(1H-pyrazol-4-yl)phenyl)isoindolin-5-ylmethyl)pyrrolidin-2-one (Compound 126): A mixture of 1-(2-(4-(1-(tetrahydro-2H-pyran-2-yl)-1H-pyrazol-4-yl)phenyl)isoindolin-5-yl)methyl) pyrrolidin-2-one (1208-126) (86 mg, 0.19 mmol, 1.0 eq.) in hydrogen chloride-dioxane (4 M, 4 mL) was stirred at room temperature for 1.0 h. The solvent was removed under reduced pressure, the residue was diluted with water, the pH was adjusted to 9 with sodium carbonate solution, and the solution was extracted with dichloromethane. The organic phase was dried and concentrated under reduced pressure. The residue was purified by methanol slurry to give a white solid product 1-(2-(4-(1H-pyrazol-4-yl)phenyl)isoindolin-5-ylmethyl)pyrrolidin-2-one (21 mg, yield: 30.2%). LCMS(ESI): m/z =359[M+1]⁺. ¹ H NMR (500 MHz, DMSO) δ 12.72 (s, 1H), 8.07 - 7.69 (m, 2H), 7.48 (d, *J =* 8.5 Hz, 2H), 7.37 (d, *J =* 7.8 Hz, 1H), 7.26 (s, 1H), 7.17 (d, *J* = 7.6 Hz, 1H), 6.67 (d, *J* = 8.5 Hz, 2H), 4.59 (s, 4H), 4.40 (s, 2H), 3.24 (s, 2H), 2.30 (t, *J =* 8.1 Hz, 2H), 1.99 - 1.88 (m, 2H).

### Example 88: Preparation of (5-(2,3-difluoro-4-(1H-pyrazol-4-yl)phenyl)hexahydropyrrolo[3,4-c]pyrrol-2(1H)-yl)(pyrrolidin-1-yl)methanone (Compound 127) (Prepared according to Scheme 11)

Step 88a: Preparation of tert-butyl 5-(pyrrolidine-1-carbonyl)hexahydropyrrolo[3,4-c]pyrrole-2(1H)-carboxylate (Compound 1106-127): Under nitrogen protection, a mixture of tert-butyl hexahydropyrrolo[3,4-c]pyrrole-2(1H)-carboxylate (500 mg, 2.36 mmol, 1.0 eq.), pyrrolidine-1-carbonyl chloride (350 mg, 2.59 mmol, 1.1 eq.), triethylamine (360 mg, 3.54 mmol, 1.5 eq.) in dichloromethane (5 ml) was stirred at room temperature for 1.5 hours. Sodium carbonate solution was added to quench the reaction, diluted with water (15 ml) and extracted with dichloromethane (8 ml × 4). The combined organic layer was washed with saturated brine (20 ml × 1), dried over anhydrous sodium sulfate, and concentrated under reduced pressure. The residue was purified by column chromatography on silica gel (dichloromethane/methanol = 30/1 to 15/1) to give yellow oily product tert-butyl 5-(pyrrolidine-1-carbonyl)hexahydropyrrolo[3,4-c]pyrrole-2(1H)-carboxylate (701 mg, yield: 96.1%). LCMS (ESI): m/z = 310 [M+1]⁺.

Step 88b: Preparation of hexahydropyrrolo[3,4-c]pyrrol-2(1H)-yl)(pyrrolidin-1-yl)methanone (Compound 1107-127): A mixture of tert-butyl 5-(pyrrolidine-1-carbonyl)hexahydropyrrolo[3,4-c]pyrrole-2(1H)-carboxylate (1106-127) (701 mg, 2.26 mmol, 1.0 eq.) in hydrogen chloride-methanol (4 M, 5 mL) was stirred at room temperature for 1.5 hours. The solvent was removed under reduced pressure, and the residue was diluted with water (10 mL), adjusted to pH = 9 with sodium carbonate solution, and extracted with dichloromethane (6 mL × 10). The organic phase was dried over anhydrous sodium sulfate and concentrated under reduced pressure to give a yellow oily product hexahydropyrrolo[3,4-c]pyrrol-2(1H)-yl)(pyrrolidin-1-yl)methanone (452 mg, yield: 95.7%). LCMS (ESI): m/z = 210 [M + 1]⁺.

Step 88c: Preparation of (5-(2,3-difluoro-4-(1-(tetrahydro-2H-pyran-2-yl)-1H-pyrazol-4-yl)phenyl)hexahydrop yrrolo[3,4-c]pyrrol-2(1H)-yl)(pyrrolidin-1-yl)methanone (Compound 1108-127): Under nitrogen protection, a mixture of (hexahydropyrrolo[3,4-c]pyrrol-2(1H)-yl)(pyrrolidin-1-yl)methanone (1107-127) (88 mg, 0.42 mmol, 1.2 eq.), 4-(4-bromo-2,3-difluorophenyl)-1-(tetrahydro-2H-pyran-2-yl)-1H-pyrazole (0103-4) (120 mg, 0.35 mmol, 1.0 eq.), sodium tert-butoxide (84 mg, 0.875 mmol, 2.5 eq.), 4,5-bis(diphenylphosphino-9,9-dimethylxanthene) (40 mg, 0.07 mmol, 0.2 eq.) and tris(dibenzylideneacetone)dipalladium (32 mg, 0.035 mmol, 0.1 eq.) in toluene (10 ml) was stirred at 120°C overnight. The reaction was diluted with water (20 ml) and extracted with ethyl acetate (10 ml × 3). The combined organic phases was washed with saturated brine (20 ml × 1), dried over anhydrous sodium sulfate and concentrated under reduced pressure. The residue was purified by prep-TLC (silica gel, dichloromethane/methanol = 15:1) to obtain a yellow oily product (5-(2,3-difluoro-4-(1-(tetrahydro-2H-pyran-2-yl)-1H-pyrazol-4-yl)phenyl)hexahydrop yrrolo[3,4-c]pyrrol-2(1H))-yl)(pyrrolidin-1-yl)methanone (65 mg, yield: 39.6%). LCMS (ESI): m/z = 472[M+1]⁺.

Step 88d: Preparation of (5-(2,3-difluoro-4-(1H-pyrazol-4-yl)phenyl)hexahydropyrrolo[3,4-c]pyrrol-2(1H)-yl)( pyrrolidin-1-yl)methanone (Compound 127): A mixture of (5-(2,3-difluoro-4-(1-(tetrahydro-2H-pyran-2-yl)-1H-pyrazol-4-yl)phenyl)hexahydrop yrrolo[3,4-c]pyrrol-2(1H))-yl)(pyrrolidin-1-yl)methanone (1108-127) (65 mg, 0.168 mmol, 1.0 eq.) in hydrogen chloride-methanol solution (4 M, 3 ml) was stirred at room temperature for 1.5 hours. The solvent was removed under reduced pressure, the residue was diluted with water (10 ml), adjusted to pH = 9 with sodium carbonate solution, extracted with dichloromethane (4 ml × 5), and the organic phase was dried over anhydrous sodium sulfate and concentrated under reduced pressure. The residue was purified by prep-TLC (silica gel, dichloromethane/methanol = 12/1) to obtain a white solid product (5-(2,3-difluoro-4-(1H-pyrazol-4-yl)phenyl)hexahydropyrrolo[3,4-c]pyrrol-2(1H)-yl)( pyrrolidin-1-yl)methanone (31 mg, yield: 47.7%). LCMS (ESI): m/z = 388 [M+1]⁺. ¹ H NMR (500 MHz, DMSO) δ 12.97 (s, 1H), 8.02 (s, 1H), 7.81 (d, *J =* 10.9 Hz, 1H), 7.30 (t, *J* = 8.3 Hz, 1H), 6.56 (t, *J* = 8.5 Hz, 1H), 3.55 (dt, *J* = 24.2, 12.3 Hz, 4H), 3.25 (d, *J* = 6.0 Hz, 8H), 2.91 (s, 2H), 1.74 (s, 4H).

### Example 89: Preparation of (8-(2,3-difluoro-4-(1H-pyrazol-4-yl)phenyl)-3,8-diazabicyclo[3.2.1] octan-3-yl)(py rrolidin-1-yl)methanone (Compound 128) (Prepared according to Scheme 10)

Step 89a: Preparation of tert-butyl 8-(2,3-difluoro-4-(1-(tetrahydro-2H-pyran-2-yl)-1H-pyrazol-4-yl)phenyl)-3,8-diazabi cyclo[3.2.1] octane-3-carboxylate (Compound 1005-128): Under nitrogen protection, a mixture of tert-butyl 3,8-diazabicyclo[3.2.1]octane-3-carboxylate (1001-128) (225 mg, 1.06 mmol, 1.2 eq.), 4-(4-bromo-2,3-difluorophenyl)-1-(tetrahydro2H-pyran-2-yl)-1H-pyrazole (0103-4) (300 mg, 0.87 mmol, 1.0 eq.), sodium tert-butoxide (210 mg, 2.19 mmol, 2.5 eq.), 4,5-bis(diphenylphosphino)-9,9-dimethylxanthene (51 mg, 0.087 mmol, 0.1 eq.) and tris(dibenzylideneacetone)dipalladium(0) (40 mg, 0.044 mmol, 0.05 eq.) in toluene (12 ml) was stirred at 120°C overnight. After cooling to room temperature, the reaction was quenched with water and extracted with ethyl acetate. The organic phase was washed with saturated brine, dried over anhydrous sodium sulfate, and concentrated under reduced pressure. The residue was purified by column chromatography on silica gel (eluent: petroleum ether/ethyl acetate = 4/1 to 2/1) to give a yellow oil product tert-butyl 8-(2,3-difluoro-4-(1-(tetrahydro-2H-pyran-2-yl)-1H-pyrazol-4-yl)phenyl)-3,8-diazabi cyclo[3.2.1] octane-3-carboxylate (140 mg, yield: 33.56%). MS (ES⁺): m/z = 475 [M+H]⁺.

Step 89b: Preparation of 8-(2,3-difluoro-4-(1H-pyrazol-4-yl)phenyl)-3,8-diazabicyclo[3.2.1]octane hydrochloride (Compound 1006-128): A mixture of tert-butyl 8-(2,3-difluoro-4-(1-(tetrahydro-2H-pyran-2-yl)-1H-pyrazol-4-yl)phenyl)-3,8-diazabi cyclo[3.2.1]octane-3-carboxylate (1005-128) (140 mg, 0.28 mmol, 1.0 eq.) in hydrogen chloride-methanol solution (4 M, 5 mL) was stirred at room temperature for 2 hours. The solvent was removed under reduced pressure and the residue was used in the next step without further purification. MS (ES⁺): m/z = 291 [M+H]⁺.

Step 89c: Preparation of (8-(2,3-difluoro-4-(1H-pyrazol-4-yl)phenyl)-3,8-diazabicyclo[3.2.1]octan-3-yl)(pyrrol idin-1-yl)methanone (Compound 128): A mixture of 8-(2,3-difluoro-4-(1H-pyrazol-4-yl)phenyl)-3,8-diazabicyclo[3.2.1]octane hydrochloride (1006-128) (104 mg, 0.28 mmol, 1.0 eq.), N,N-diisopropylethylamine (224 mg, 1.74 mmol, 6.0 eq.) and pyrrolidine-1-carbonyl chloride (58 mg, 0.43 mmol, 1.5 eq.) in dichloromethane (5 ml) was stirred at room temperature overnight. The reaction was quenched with water and extracted with dichloromethane. The organic phase was washed with saturated brine, dried over anhydrous sodium sulfate, and concentrated under reduced pressure. The residue was purified by prep-TLC (silica gel, dichloromethane/methanol = 10/1) to obtain a yellow solid product (8-(2,3-difluoro-4-(1H-pyrazol-4-yl)phenyl)-3,8-diazabicyclo[3.2.1]octan-3-yl)(pyrrol idin-1-yl)methanone. (80 mg, yield: 71.20%) MS (ES⁺): m/z = 388 [M+H]⁺. ¹ H NMR (500 MHz, DMSO) δ 13.01 (s, 1H), 8.07 (s, 1H), 7.85 (s, 1H), 7.33 (t, *J =* 7.9 Hz, 1H), 6.85 (t, *J =* 8.3 Hz, 1H), 4.18 (s, 2H), 3.51 (d, *J* = 11.8 Hz, 2H), 3.24 (t, *J =* 6.1 Hz, 4H), 3.14 (d, *J* = 12.4 Hz, 2H), 1.92 - 1.83 (m, 2H), 1.82 - 1.75 (m, 2H), 1.73 (s, 4H).

### Example 90: Preparation of (4-(2-fluoro-4-(1H-pyrazol-4-yl)phenyl)piperazin-1-yl)(pyrrolidin-1-yl)methanon e (Compound 129) (Prepared according to Scheme 10)

Step 90a: Preparation of tert-butyl 4-(2-fluoro-4-(1-(tetrahydro-2H-pyran-2-yl)-1H-pyrazol-4-yl)phenyl)piperazine-1-car boxylate (Compound 1005-129): Under nitrogen protection, a solution of tert-butyl piperazine-1-carboxylate (1001) (274 mg, 1.47 mmol, 1.2 eq.), 4-(4-bromo-3-fluorophenyl)-1-(tetrahydro-2H-pyran-2-yl)-1H-pyrazole (0103-2) (398 mg, 1.22 mmol, 1.0 eq.), sodium tert-butoxide (235 mg, 2.45 mmol, 2.0 eq.), 4,5-bis(diphenylphosphino)-9,9-dimethylxanthene (127 mg, 0.24 mmol, 0.2 eq.) and tris(dibenzylideneacetone)dipalladium (110 mg, 0.12 mmol, 0.1 eq.) in toluene (18 mL) was stirred at 120 °C overnight. The reaction was diluted with water (50 mL) and extracted with ethyl acetate (15 mL × 2). The combined organic layer was washed with brine (40 mL × 2), dried over anhydrous sodium sulfate, and concentrated in vacuo. The residue was purified by column chromatography on silica gel (eluent: petroleum ether/ethyl acetate = 50/1 to 2/1) to obtain tert-butyl 4-(2-fluoro-4-(1-(tetrahydro-2H-pyran-2-yl)-1H-pyrazol-4-yl)phenyl)piperazine-1-car boxylate (380 mg, yield: 70.38%). LCMS (ESI): m/z = 431 [M+1]⁺.

Step 90b: Preparation of 1-(2-fluoro-4-(1H-pyrazol-4-yl)phenyl)piperazine hydrochloride (Compound 1006-129): A mixture of tert-butyl 4-(2-fluoro-4-(1-(tetrahydro-2H-pyran-2-yl)-1H-pyrazol-4-yl)phenyl)piperazine-1-car boxylate (1005-129) (150 mg, 0.348 mmol, 1.0 eq.) in hydrogen chloride-methanol solution (4 M, 3 mL) was stirred at room temperature for 4 hours. The solvent was removed under reduced pressure to give 1-(2-fluoro-4-(1H-pyrazol-4-yl)phenyl)piperazine hydrochloride (105 mg, yield: 95.45%). LCMS (ESI): m/z = 247 [M+1]⁺.

Step 90c: Preparation of (4-(2-fluoro-4-(1H-pyrazol-4-yl)phenyl)piperazin-1-yl)(pyrrolidin-1-yl)methanone (Compound 129): Under nitrogen protection, a mixture of 1-(2-fluoro-4-(1H-pyrazol-4-yl)phenyl)piperazine hydrochloride (1006-129) (65 mg, 0.264 mmol, 1.0 eq.), pyrrolidine-1-carbonyl chloride (28 mg, 0.211 mmol, 0.8 eq.), N,N-diisopropylethylamine (68 mg, 0.528 mmol, 2.0 eq.) in tetrahydrofuran (3 mL) was stirred at 30 °C overnight. The reaction was diluted with water (20 mL) and extracted with ethyl acetate (8 mLx2). The combined organic phases was washed with saturated brine (20 mL×1), dried over anhydrous sodium sulfate and concentrated under vacuum. The residue was purified by Prep-TLC (silica gel, dichloromethane/methanol = 10:1) to give (4-(2-fluoro-4-(1H-pyrazol-4-yl)phenyl)piperazin-1-yl)(pyrrolidin-1-yl)methanone (74 mg, yield: 89.16%) as a white solid. LCMS (ESI): m/z = 344 [M+1]⁺. ¹ H NMR (500 MHz, DMSO) δ 12.87 (s, 1H), 8.14 (s, 1H), 7.89 (s, 1H), 7.38 (dd, *J =* 33.8, 11.2 Hz, 2H), 7.01 (t, *J* = 8.9 Hz, 1H), 3.32 (d, *J =* 5.1 Hz, 8H), 3.00 (d, *J* = 4.3 Hz, 4H), 1.76 (s, 4H).

### Example 91: Preparation of (4-(2-fluoro-4-(1H-pyrazol-4-yl)phenyl)-2,6-dimethylpiperazin-1-yl)(pyrrolidin-1 -yl)methanone (Compound 130) (Prepared according to Scheme 10)

Step 91a: Preparation of tert-butyl 3,5-dimethyl-4-(pyrrolidine-1-carbonyl)piperazine-1-carboxylate (Compound 1002-130): Under nitrogen protection, a solution of tert-butyl 3,5-dimethylpiperazine-1-carboxylate (1001) (500 mg, 2.345 mmol, 1.0 eq.), piperidine-1-carbonyl chloride (342 mg, 2.57 mmol, 1.1 eq.), and triethylamine (590 mg, 5.84 mmol, 2.5 eq.) in tetrahydrofuran (10 ml) was stirred at 70 °C for three days. The reaction was diluted with water and extracted with ethyl acetate. The organic layer was dried and concentrated under reduced pressure. The residue was purified by prep-TLC (silica gel, petroleum ether/ethyl acetate = 1/1) to obtain a yellow oily product tert-butyl 3,5-dimethyl-4-(pyrrolidine-1-carbonyl)piperazine-1-carboxylate (520 mg, yield: 71.33%). LCMS (ESI): m/z = 312 [M+1]⁺.

Step 91b: Preparation of (2,6-dimethylpiperazin-1-yl)(pyrrolidin-1-yl)methanone hydrochloride (Compound 1003-130): A mixture of tert-butyl 3,5- dimethyl -4-(pyrrolidine-1-carbonyl)piperazine-1-carboxylate (1002-130) (520 mg, 1.67 mmol, 1.0 eq.) in hydrogen chloride-methanol solution (4 M, 5 ml) was stirred at room temperature for 2.0 hours. The reaction was concentrated under reduced pressure to obtain a yellow solid product (2,6-dimethylpiperazin-1-yl)(pyrrolidin-1-yl)methanone hydrochloride (400 mg, crude product). LCMS (ESI): m/z = 212 [M+1]⁺.

Step 91c: Preparation of ((4-(2-fluoro-4-(1-(tetrahydro-2H-pyran-2-yl)-1H-pyrazol-4-yl)phenyl)-2,6-dimethylp iperazin-1-yl)(pyrrolidin-1-yl)methanone (Compound 1004-130): Under nitrogen protection, a mixture of (2,6-dimethylpiperazin-1-yl)(pyrrolidin-1-yl)methanone hydrochloride (1003-130) (200 mg, 0.94 mmol, 1.0 eq.), 4-(4-bromo-3 -fluorophenyl)-1-(tetrahydro-2H-pyran-2-yl)-1H-pyrazole (0103-2) (307 mg, 0.94 mmol, 1.0 eq.), sodium tert-butoxide (272 mg, 2.82 mmol, 3.0 eq.), 4,5-bis(diphenylphosphino)-9,9-dimethylxanthene (9 mg, 0.018 mmol, 0.02 eq.) and tris(dibenzylideneacetone)dipalladium (9 mg, 0.009 mmol, 0.01 eq.) in toluene (5 ml) was stirred at 120°C for 3 hours. The reaction was diluted with water and extracted with ethyl acetate, and the organic phase was dried and concentrated under reduced pressure. The residue was purified by prep-TLC (silica gel, petroleum ether/ethyl acetate = 1/1) to give a brown solid product ((4-(2-fluoro-4-(1-(tetrahydro-2H-pyran-2-yl)-1H-pyrazol-4-yl)phenyl)-2,6-dimethylp iperazin-1-yl)(pyrrolidin-1-yl)methanone (520 mg, crude). LCMS (ESI): m/z = 456 [M+1]⁺.

Step 91d: Preparation of (4-(2-fluoro-4-(1H-pyrazol-4-yl)phenyl)-2,6-dimethylpiperazin-1-yl)(pyrrolidin-1-yl) methanone (Compound 130): A mixture of (4-(2,3-difluoro-4-(1-(tetrahydro-2H-pyran-2-yl)-1H-pyrazol-4-yl)phenyl)piperazin-1-yl)(piperidin-1-yl)methanone (1004-130) (80 mg, 0.168 mmol, 1.0 eq.) in hydrogen chloride-methanol solution (4 M, 3 ml) was stirred at room temperature for 2 hours. The solvent was removed under reduced pressure. The residue was diluted with water, adjusted to pH = 9 with sodium carbonate solution, and extracted with dichloromethane. The organic phase was dried and concentrated under reduced pressure. The residue was purified by prep-TLC (silica gel, dichloromethane/methanol = 20/1) to give a yellow solid product (4-(2-fluoro-4-(1H-pyrazol-4-yl)phenyl)-2,6-dimethylpiperazin-1-yl)(pyrrolidin-1-yl) methanone (40 mg, yield: 62.50%). LCMS (ESI): m/z = 372 [M+1]⁺. ¹ H NMR (500 MHz, DMSO) δ 12.87 (s, 1H), 8.14 (s, 1H), 7.88 (s, 1H), 7.40 (d, *J* = 13.8 Hz, 1H), 7.34 (d, *J* = 8.0 Hz, 1H), 6.99 (t, *J* = 8.7 Hz, 1H), 3.57 (s, 2H), 3.34 (s, 4H), 3.01 (d, *J* = 9.6 Hz, 2H), 2.90 - 2.79 (m, 2H), 1.78 (s, 4H), 1.20 (d, *J =* 6.3 Hz, 6H).

### Example 92: Preparation of (4-(2-fluoro-4-(1H-pyrazol-4-yl)phenyl)piperazin-1-yl)(1-hydroxycyclohexyl)met hanone (Compound 132) (Prepared according to Scheme 10)

Under nitrogen protection, a mixture of 1-(2-fluoro-4-(1H-pyrazol-4-yl)phenyl)piperazine (1006-129) (110 mg, 0.39 mmol, 1.0 eq.), 1-hydroxycyclohexane-1-carboxylic acid (67 mg, 0.47 mmol, 1.2 eq.), HATU (178.6 mg, 0.47 mmol, 1.2 eq.), N,N-diisopropylethylamine (301 mg, 2.34 mmol, 6.0 eq.) in N,N-dimethylformamide (3 ml) was stirred at room temperature for 2.0 hours. The reaction was diluted with water and extracted with ethyl acetate. The organic phase was dried and concentrated under reduced pressure. The residue was purified by prep-TLC (silica gel, eluent: ethyl acetate) to give a yellow solid product (4-(2-fluoro-4-(1H-pyrazol-4-yl)phenyl)piperazin-1-yl)(1-hydroxycyclohexyl)methan one (40 mg, yield: 40.4%). LCMS (ESI): m/z = 373 [M+1]⁺. ¹ H NMR (500 MHz, DMSO) δ 12.91 (s, 1H), 8.17 (s, 1H), 7.90 (s, 1H), 7.43 (d, *J* = 14.1 Hz, 1H), 7.35 (d, *J* = 8.2 Hz, 1H), 7.01 (t, *J* = 8.9 Hz, 1H), 5.29 (s, 1H), 4.11 (d, *J* = 5.1 Hz, 2H), 3.63 (s, 2H), 2.98 (s, 4H), 1.85 - 1.35 (m, 9H), 1.20 (dd, *J =* 24.1, 12.3 Hz, 1H).

### Example 93: Preparation of (4-(2-fluoro-4-(1H-pyrazol-4-yl)phenyl)piperazin-1-yl)(hexahydrocyclopenta[c]p yrrol-2(1H)-yl)methanone (Compound 133) (Prepared according to Scheme 10)

Under nitrogen protection, a solution of triphosgene (50 mg, 0.170 mmol, 0.5 eq.) in dichloromethane (1 ml) was added dropwise at 0 °C to a mixture of octahydrocyclopenta[c]pyrrole hydrochloride (50 mg, 0.339 mmol, 1.0 eq.) and pyridine (40 mg, 0.508 mmol, 1.5 eq.) in dichloromethane (2 ml). The mixture was stirred at room temperature for 5 hours. A 1 mol/L hydrochloric acid solution (8 ml) was added dropwise to the reaction solution. The aqueous phase was extracted with dichloromethane (8 ml × 3). The combined organic phases was washed with saturated sodium bicarbonate solution and brine (10 ml × 2), then dried over anhydrous sodium sulfate and concentrated under reduced pressure. The residue was dissolved in dioxane (2 ml), and then 1-(2-fluoro-4-(1H-pyrazol-4-yl)phenyl)piperazine hydrochloride (1006-129) (30 mg, 0.094 mmol, 0.3 eq.) and triethylamine (28 mg, 0.282 mmol, 0.9 eq.) were added, and the mixture was stirred at room temperature overnight. The reaction was diluted with water (10 ml) and extracted with ethyl acetate (6 ml × 4). The combined organic phases was washed with saturated brine (20 ml × 1), dried over anhydrous sodium sulfate and concentrated under reduced pressure. The residue was purified by prep-TLC (silica gel, dichloromethane/methanol = 16/1) to obtain a white solid product (4-(2-fluoro-4-(1H-pyrazol-4-yl)phenyl)piperazine-1-yl)(hexahydrocyclopenta[c]pyrr ole-2(1H)-yl)methanone (27 mg, yield: 75%). LCMS(ESI): m/z =384[M+1]⁺. ¹ H NMR (500 MHz, DMSO) δ 12.87 (s, 1H), 8.14 (s, 1H), 7.88 (s, 1H), 7.41 (d, *J =* 14.2 Hz, 1H), 7.34 (d, *J* = 8.2 Hz, 1H), 7.01 (t, *J* = 8.9 Hz, 1H), 3.49 (dd, *J* = 10.9, 7.7 Hz, 2H), 3.31 (d, *J =* 4.5 Hz, 4H), 3.06 (dd, *J =* 11.0, 3.6 Hz, 2H), 2.98 (d, *J =* 4.3 Hz, 4H), 2.5 3.2 Hz, 2H), 1.81 - 1.60 (m, 3H), 1.51 (dt, *J =* 12.6, 6.4 Hz, 1H), 1.42 - 1.28 (m, 2H).

### Example 94: Preparation of 1-(2-fluoro-4-(1H-pyrazol-4-yl)phenyl)-4-((4-fluorophenyl)sulfonyl)piperazine (Compound 134) (Prepared according to Scheme 10)

Under nitrogen protection, a solution of 4-fluorobenzenesulfonyl chloride (37 mg, 0.188 mmol, 1.2 eq.) in dichloromethane (1 ml) was added dropwise at 0 °C to a mixture of 1-(2-fluoro-4-(1H-pyrazol-4-yl)phenyl)piperazine hydrochloride (1006-129) (50 mg, 0.157 mmol, 1.0 eq.), triethylamine (39.6 mg, 0.392 mmol, 2.5 eq.) in dichloromethane (2 ml). The mixture was stirred at room temperature for 1 hour. The reaction was diluted with water (10 ml) and extracted with dichloromethane (6 ml × 4). The combined organic layer was washed with saturated brine (20 ml × 1), dried over anhydrous sodium sulfate, and concentrated under reduced pressure. The residue was purified by prep-TLC (silica gel, dichloromethane/methanol = 16/1) to give 1-(2-fluoro-4-(1H-pyrazol-4-yl)phenyl)-4-((4-fluorophenyl)sulfonyl)piperazine (39 mg, yield: 61.9%) as a white solid product. LCMS (ESI): m/z = 405 [M+1]⁺. ¹ H NMR (500 MHz, DMSO) δ 12.87 (s, 1H), 8.13 (s, 1H), 7.86 (dd, *J =* 8.4, 5.2 Hz, 3H), 7.52 (t, *J* = 8.7 Hz, 2H), 7.39 (d, *J* = 14.0 Hz, 1H), 7.33 (d, *J* = 8.2 Hz, 1H), 7.00 (t, *J* = 8.8 Hz, 1H), 3.07 (s, 8H).

### Example 95: Preparation of (4-(2-fluoro-4-(1H-pyrazol-4-yl)phenyl)piperidin-1-yl)(isoindolin-2-yl)methanone (Compound 136) (Prepared according to Scheme 9)

Triphosgene (372.2 mg, 1.25 mmol, 1.5 eq.) was added dropwise to a dichloromethane solution containing isoindoline (100 mg, 0.84 mmol, 1.0 eq.) and pyridine (132.3 mg, 1.7 mmol, 2.0 eq.) under an ice-salt bath. The mixture was stirred at 5 °C for 30 minutes. The solvent was removed under reduced pressure under nitrogen protection, and the residue was used directly in the next step without further purification. The product obtained in the previous step (crude product, dissolved in tetrahydrofuran) was added to a mixture of 4-(2-fluoro-4-(1H-pyrazol-4-yl)phenyl)piperidine hydrochloride (0904-119) (30 mg, 0.12 mmol, 1.0 eq.), N,N-diisopropylethylamine (47.5 mg, 0.37 mmol, 3.0 eq.) in 10 mL tetrahydrofuran. The mixture was stirred at room temperature for one hour. The reaction was diluted with ethyl acetate, and the organic phase was washed with water and saturated brine. The organic phase was concentrated under reduced pressure, and the residue was purified by prep-TLC (silica gel, eluent: dichloromethane/methanol = 10/1) to give a white solid product (4-(2-fluoro-4-(1H-pyrazol-4-yl)phenyl)piperidin-1-yl)(isoindolin-2-yl)methanone (34.7 mg, yield: 72.7%). LCMS (ESI): m/z = 391 (M+H)⁺. ¹ H NMR (500 MHz, DMSO) δ 12.94 (s, 1H), 8.18 (s, 1H), 7.95 (s, 1H), 7.45 - 7.39 (m, 2H), 7.36 - 7.30 (m, 3H), 7.27 (dd, *J =* 5.5, 3.2 Hz, 2H), 4.74 (s, 4H), 3.88 (d, *J =* 12.9 Hz, 2H), 3.00 (t, *J =* 11.7 Hz, 1H), 2.90 (t, *J* = 11.6 Hz, 2H), 1.83 - 1.64 (m, 4H).

### Example 96: Preparation of cyclopentyl(4-(2-fluoro-4-(1H-pyrazol-4-yl)phenyl)piperidin-1-yl)methanone (Compound 138) (Prepared according to Scheme 9)

To a mixture of 4-(2-fluoro-4-(1H-pyrazol-4-yl)phenyl)piperidine hydrochloride (0904-119) (60 mg, 0.21 mmol, 1.0 eq.), cyclopentanecarboxylic acid (27 mg, 0.23 mmol, 1.1 eq.), N,N-diisopropylethylamine (82 mg, 0.63 mmol, 3.0 eq.) in N,N-dimethylformamide (1.5 ml) was added HATU (97 mg, 0.26 mmol, 1.2 eq.). The mixture was stirred at room temperature for 1 hour. The reaction mixture was diluted with water (20 ml). The aqueous layer was extracted with ethyl acetate (15 ml × 3). The combined organic layer was washed with saturated brine (20 ml × 1), dried over anhydrous sodium sulfate and concentrated. The residue was purified by Pre-TLC (dichloromethane: methanol = 15: 1) to give cyclopentyl(4-(2-fluoro-4-(1H-pyrazol-4-yl)phenyl)piperidin-1-yl)methanone (46 mg, yield: 63%) as a white solid. LCMS (ESI): m / z = 342 [M + 1]⁺; TLC: Rf 0.5 (dichloromethane: methanol = 15: 1). ¹ H NMR (500 MHz, MeOD) δ 7.86 (s, 2H), 7.26 (d, *J* = 8.0 Hz, 1H), 7.18 (dd, *J* = 18.7, 10.6 Hz, 2H), 4.63 (d, *J* = 13.2 Hz, 1H), 4.15 (d, *J* = 13.2 Hz, 1H), 3.14 (t, *J* = 12.4 Hz, 1H), 3.10 - 2.96 (m, 2H), 2.65 (t, *J*= 12.0 Hz, 1H), 1.92 - 1.49 (m, 12H).

### Example 97: Preparation of (4-(4-(1H-pyrazol-4-yl)phenyl)piperidin-1-yl)(1-hydroxycyclohexyl)methanone (Compound 139) (Prepared according to Scheme 9)

Under nitrogen protection, HATU (154 mg, 0.404 mmol, 1.2 eq.) was added to a mixture of 4-(4-(1H-pyrazol-4-yl)phenyl)piperidine hydrochloride (0904-70) (100 mg, 0.337 mmol, 1.0 eq.), 1-hydroxycyclohexane-1-carboxylic acid (49 mg, 0.337 mmol, 1.0 eq.), N,N-diisopropylethylamine (261 mg, 2.02 mmol, 6.0 eq.) in N,N-dimethylformamide (4 ml). The mixture was stirred at room temperature for 2 hours. The reaction was diluted with water (20 ml) and extracted with ethyl acetate (10 ml × 4). The combined organic layer was washed with saturated brine (20 ml × 3), dried over anhydrous sodium sulfate, and concentrated under reduced pressure. The residue was purified by prep-TLC (silica gel, dichloromethane/methanol = 16/1) to give a white solid product (4-(4-(1H-pyrazol-4-yl)phenyl)piperidin-1-yl)(1-hydroxycyclohexyl)methanone (51 mg, yield: 42.9%). LCMS (ESI): m/z =354[M+1]⁺. ¹ H NMR (500 MHz, DMSO) δ 12.86 (s, 1H), 8.11 (s, 1H), 7.86 (s, 1H), 7.51 (d, *J =* 8.1 Hz, 2H), 7.20 (d, *J =* 8.1 Hz, 2H), 5.17 (s, 1H), 4.81 (s, 2H), 3.04-2.53 (m, 3H), 1.88-1.37 (m, 14H).

### Example 98: Preparation of (4-(2-fluoro-4-(1H-pyrazol-4-yl)phenyl)piperidin-1-yl)(1-hydroxycyclohexyl)meth anone (Compound 140) (Prepared according to Scheme 9)

HATU (85 mg, 0.22 mmol, 1.1 eq.) was added to a mixture of 1-hydroxycyclohexane-1-carboxylic acid (30 mg, 0.2 mmol, 1.0 eq.), 4-(2-fluoro-4-(1H-pyrazol-4-yl)phenyl)piperidine hydrochloride (0904-119) (50 mg, 0.2 mmol, 1.0 eq.), and N,N-diisopropylethylamine (78.7 mg, 0.6 mmol, 3.0 eq.) in 5 mL N,N-dimethylformamide. The mixture was stirred at room temperature for two hours. The reaction was diluted with ethyl acetate. The organic phase was washed with water and saturated brine and concentrated under reduced pressure. The residue was purified by column chromatography on silica gel (eluent: dichloromethane /methanol = 10/1) to give a white solid product (4-(2-fluoro-4-(1H-pyrazol-4-yl)phenyl)piperidin-1-yl)(1-hydroxycyclohexyl)methan one (34.8 mg, yield: 45.2%). LCMS (ESI): m/z = 372 (M+H)⁺. ¹ H NMR (500 MHz, DMSO) δ 12.94 (s, 1H), 8.19 (s, 1H), 7.94 (s, 1H), 7.40 (dd, *J* = 8.7, 6.6 Hz, 2H), 7.25 (t, *J* = 8.1 Hz, 1H), 5.18 (s, 1H), 4.74 (d, *J =* 83.8 Hz, 2H), 2.87 (dt, *J =* 76.7, 13.9 Hz, 3H), 1.81 - 1.39 (m, 14H).

### Example 99: Preparation of (4-(2-fluoro-4-(1H-pyrazol-4-yl)phenyl)piperidin-1-yl)(1-fluorocyclohexyl)metha none (Compound 141) (Prepared according to Scheme 9)

HATU (72 mg, 0.188 mmol, 1.2 eq.) was added to a mixture of 4-(2-fluoro-4-(1H- pyrazol-4-yl)phenyl)piperidine hydrochloride (0904-119) (50 mg, 0.157 mmol, 1.0 eq.), 1-fluorocyclohexane-1-carboxylic acid (23 mg, 0.157 mmol, 1.0 eq.), and diisopropylethylamine (71 mg, 0.55 mmol, 3.5 eq.) in 2 mL N,N -dimethylformamide. The mixture was stirred at room temperature for half an hour and diluted with ethyl acetate. The organic phase was washed with water and saturated brine and concentrated under reduced pressure. The residue was purified by prep-TLC (silica gel, eluent: dichloromethane/methanol = 20/1) to give a white solid product (4-(2-fluoro-4-(1H-pyrazol-4-yl)phenyl)piperidin-1-yl)(1-fluorocyclohexyl)methanone (47 mg, yield: 79.6%). LCMS (ESI): m/z = 374 (M+H)⁺. Melting point: 163~165 ° C. ¹ H NMR (500 MHz, DMSO) δ 12.93 (s, 1H), 8.20 (s, 1H), 7.94 (s, 1H), 7.40 (t, *J* = 10.5 Hz, 2H), 7.28 (t, *J* = 8.0 Hz, 1H), 4.48 (s, 2H), 3.10 (dd, *J =* 30.6, 18.5 Hz, 2H), 2.73 (s, 1H), 1.86 (dd, *J =* 58.0, 10.9 Hz, 6H), 1.67 - 1.52 (m, 7H), 1.29 (d, *J =* 12.3 Hz, 1H).

### Example 100: Preparation of (4-(2-fluoro-4-(1H-pyrazol-4-yl)phenyl)piperidin-1-yl)(tetrahydrofuran-3-yl)met hanone (Compound 144) (Prepared according to Scheme 9)

HATU (123 mg, 0.323 mmol, 1.2 eq.) was added to a mixture of 4-(2-fluoro-4-(1H-pyrazol-4-yl)phenyl)piperidine hydrochloride (0904-119) (85 mg, 0.269 mmol, 1.0 eq.), 3-tetrahydrofurancarboxylic acid (31 mg, 0.269 mmol, 1.0 eq.), N,N-diisopropylethylamine (207 mg, 1.60 mmol, 6.0 eq.) in N,N-dimethylformamide (3 ml). The mixture was stirred at room temperature for 2 hours. The reaction was diluted with water (15 ml) and extracted with ethyl acetate (10 ml × 4). The combined organic layer was washed with saturated brine (20 ml × 3), dried over anhydrous sodium sulfate, and concentrated under reduced pressure. The residue was purified by prep-TLC (silica gel, dichloromethane/methanol = 20/1) to give a white solid product (4-(2-fluoro-4-(1H-pyrazol-4-yl)phenyl)piperidin-1-yl)(tetrahydrofuran-3-yl)methano ne (52 mg, yield: 55.9%). LCMS(ESI): m/z =344[M+1]⁺. ¹ H NMR (500 MHz, DMSO) δ 12.94 (s, 1H), 8.21 (s, 1H), 7.93 (s, 1H), 7.40 (t, *J* = 9.9 Hz, 2H), 7.28 (t, *J* = 8.0 Hz, 1H), 4.56 (d, *J =* 12.4 Hz, 1H), 4.08 (d, *J =* 12.8 Hz, 1H), 3.89 (dd, *J =* 18.3, 8.4 Hz, 1H), 3.79 - 3.60 (m, 3H), 3.37 (dt, *J =* 10.7, 5.9 Hz, 1H), 3.22 - 2.97 (m, 2H), 2.66 (t, *J* = 12.3 Hz, 1H), 2.16 - 1.84 (m, 2H), 1.78 (t, *J* = 15.5 Hz, 2H), 1.66 - 1.40 (m, 2H).

### Example 101: Preparation of (4-(2-fluoro-4-(1H-pyrazol-4-yl)phenyl)piperidin-1-yl)(3-hydroxycyclopentyl)met hanone (Compound 145) (Prepared according to Scheme 9)

To a mixture of 4-(2-fluoro-4-(1H-pyrazol-4-yl)phenyl)piperidine hydrochloride (0904-119) (80 mg, 0.254 mmol, 1.0 eq.), 3-hydroxycyclopentane-1-carboxylic acid (33 mg, 0.254 mmol, 1.0 eq.), N,N-diisopropylethylamine (197 mg, 1.52 mmol, 6.0 eq.) in N,N-dimethylformamide (3 ml) were added 1-hydroxybenzotriazole (41 mg, 0.305 mmol, 1.2 eq.) and 1-(3-dimethylaminopropyl)-3-ethylcarbodiimide hydrochloride (58 mg, 0.305 mmol, 1.2 eq.). The mixture was stirred at room temperature overnight. The reaction was diluted with water (15 ml) and extracted with ethyl acetate (10 ml × 4). The combined organic layer was washed with saturated brine (20 ml × 3), dried over anhydrous sodium sulfate, and concentrated under reduced pressure. The residue was purified by prep-TLC (silica gel, dichloromethane/methanol = 16/1) to obtain the up point product as a white solid (Compound 145a (cis or trans)) (10 mg, yield: 22.2%). LCMS (ESI): m/z = 358 [M+1]⁺. ¹ H NMR (500 MHz, DMSO) δ 12.93 (s, 1H), 8.20 (s, 1H), 7.93 (s, 1H), 7.40 (t, *J* = 10.4 Hz, 2H), 7.27 (t, *J* = 7.9 Hz, 1H), 4.58 (d, *J* = 5.1 Hz, 2H), 4.08 (d, *J* = 4.8 Hz, 2H), 3.20 - 2.86 (m, 3H), 2.64 (t, *J* = 12.2 Hz, 1H), 1.70 (dddd, *J* = 60.1, 54.7, 46.3, 39.8 Hz, 10H). The down point product as a yellow solid (compound 145b (trans or cis)) (10 mg, yield: 22.2%). LCMS (ESI): m/z =358[M+1]⁺. ¹ H NMR (500 MHz, DMSO) δ 12.93 (s, 1H), 8.20 (s, 1H), 7.93 (s, 1H), 7.39 (t, *J =* 10.1 Hz, 2H), 7.27 (t, *J* = 8.0 Hz, 1H), 4.56 (d, *J* = 12.8 Hz, 1H), 4.44 (d, *J* = 3.6 Hz, 1H), 4.18 (s, 1H), 4.07 (d, *J* = 11.4 Hz, 1H), 3.24 - 2.95 (m, 3H), 2.62 (t, *J =* 12.2 Hz, 1H), 1.95 - 1.38 (m, 10H).

### Example 102: Preparation of (4-(2-fluoro-4-(1H-pyrazol-4-yl)phenyl)piperidin-1-yl)(3-hydroxycyclohexyl)meth anone (Compound 148) (Prepared according to Scheme 9)

HATU (116 mg, 0.305 mmol, 1.2 eq.) was added to a mixture of 4-(2-fluoro-4-(1H-pyrazol-4-yl)phenyl)piperidine hydrochloride (0904-119) (80 mg, 0.254 mmol, 1.0 eq.), 3-hydroxycyclohexane-1-carboxylic acid (36 mg, 0.254 mmol, 1.0 eq.), N,N-diisopropylethylamine (197 mg, 1.52 mmol, 6.0 eq.) in N,N-dimethylformamide (2 ml). The mixture was stirred at room temperature for 2 hours. The reaction was diluted with water (10 ml) and extracted with ethyl acetate (8 ml × 4). The combined organic layer was washed with saturated brine (20 ml × 3), dried over anhydrous sodium sulfate, and concentrated under reduced pressure. The residue was purified by thick prep-TLC (silica gel, dichloromethane/methanol = 16/1) to give a white solid product (4-(2-fluoro-4-(1H-pyrazol-4-yl)phenyl)piperidin-1-yl)(3-hydroxycyclohexyl)methan one (54.4 mg, yield: 57.3%). LCMS (ESI): m/z = 372 [M+1]⁺. ¹ H NMR (500 MHz, DMSO) δ 12.94 (s, 1H), 8.20 (s, 1H), 7.93 (s, 1H), 7.40 (t, *J* = 10.3 Hz, 2H), 7.27 (t, *J* = 7.9 Hz, 1H), 4.69 - 4.37 (m, 2H), 3.74 (dd, *J =* 259.5, 37.9 Hz, 2H), 3.20 - 3.07 (m, 1H), 2.84 (ddd, *J* = 49.1, 31.0, 18.9 Hz, 3H), 1.85 - 1.18 (m, 12H).

### Example 103: Preparation of (4-(2-fluoro-4-(1H-pyrazol-4-yl)phenyl)piperidin-1-yl)(2-hydroxycyclohexyl)meth anone (Compound 149) (Prepared according to Scheme 9)

HATU (116 mg, 0.305 mmol, 1.2 eq.) was added to a mixture of 4-(2-fluoro-4-(1H-pyrazol-4-yl)phenyl)piperidine hydrochloride (Compound 0904-119) (80 mg, 0.254 mmol, 1.0 eq.), 2-hydroxycyclohexane-1-carboxylic acid (36 mg, 0.254 mmol, 1.0 eq.), N,N-diisopropylethylamine (197 mg, 1.52 mmol, 6.0 eq.) in N,N-dimethylformamide (2 ml). The mixture was stirred at room temperature for 2 hours. The reaction was diluted with water (10 ml) and extracted with ethyl acetate (8 ml × 4). The combined organic layer was washed with saturated brine (20 ml × 3), dried over anhydrous sodium sulfate, and concentrated under reduced pressure. The residue was purified by prep-TLC (silica gel, dichloromethane/methanol = 16/1) to give the up point product as a white solid (Compound 149a (cis or trans)) (43.3 mg, yield: 46.1%). LCMS (ESI): m/z =372[M+1]⁺. ¹ H NMR (500 MHz, DMSO) δ 12.94 (s, 1H), 8.21 (s, 1H), 7.93 (s, 1H), 7.40 (t, *J* = 10.0 Hz, 2H), 7.28 (t, *J* = 8.0 Hz, 1H), 5.06 (s, 1H), 4.58 (s, 1H), 4.00 (d, *J* = 37.1 Hz, 2H), 3.23 - 2.98 (m, 2H), 2.80 - 2.54 (m, 2H), 1.88 - 1.33 (m, 12H). The down point product as a yellow solid (Compound 149b (trans or cis)) (10 mg, yield: 10.6%). LCMS (ESI): m/z = 372 [M+1]⁺. ¹ H NMR (500 MHz, DMSO) δ 12.93 (s, 1H), 8.20 (s, 1H), 7.93 (s, 1H), 7.39 (t, *J* = 9.7 Hz, 2H), 7.33 - 7.12 (m, 1H), 4.57 (dd, *J* = 42.7, 32.7 Hz, 2H), 4.19 (d, *J* = 11.3 Hz, 1H), 3.54 (d, *J* = 30.1 Hz, 1H), 3.06 (dd, *J* = 26.8, 14.5 Hz, 2H), 2.59 (dd, *J* = 25.5, 13.9 Hz, 2H), 1.86 - 1.23 (m, 12H).

### Example 104: Preparation of (4-(4-(1H-pyrazol-4-yl)phenyl)piperidin-1-yl)(1-methoxycyclohexyl)methanone (Compound 150) (Prepared according to Scheme 9)

Under nitrogen protection, a mixture of 4-(2-fluoro-4-(1H-pyrazol-4-yl)phenyl)piperidine hydrochloride (0904-119) (120 mg, 0.378 mmol, 1.2 eq.), 1-methoxycyclohexane-1-carboxylic acid (50 mg, 0.315 mmol, 1.0 eq.), HATU (144 mg, 0.378 mmol, 1.2 eq.), N,N-diisopropylethylamine (244 mg, 1.89 mmol, 6.0 eq.) in N,N-dimethylformamide (3 ml) was stirred at room temperature for 2.0 hours. The reaction was diluted with water and extracted with ethyl acetate. The organic layer was dried and concentrated under reduced pressure. The residue was purified by prep-TLC (silica gel, ethyl acetate) to give a yellow solid product (4-(4-(1H-pyrazol-4-yl)phenyl)piperidin-1-yl)(1-methoxycyclohexyl)methanone (94 mg, yield: 77.4%). ¹ H NMR (500 MHz, DMSO) δ 12.94 (s, 1H), 8.20 (s, 1H), 7.92 (s, 1H), 7.39 (t, *J =* 10.3 Hz, 2H), 7.27 (t, *J =* 8.0 Hz, 1H), 4.91 (s, 1H), 4.61 (s, 1H), 3.19 - 2.96 (m, 5H), 2.81 (d, *J* = 79.4 Hz, 1H), 2.69 (s, 1H), 1.90 (d, *J* = 13.5 Hz, 2H), 1.80 (d, *J =* 12.6 Hz, 2H), 1.71 - 1.41 (m, 9H).

### Example 105: Preparation of 1-(4-(2-fluoro-4-(1H-pyrazol-4-yl)phenyl)piperidin-1-yl)-3-hydroxy-3-methylbuta n-1-one (Compound 151) (Prepared according to Scheme 9)

HATU (116 mg, 0.305 mmol, 1.2 eq.) was added to a mixture of 4-(2-fluoro-4-(1H-pyrazol-4-yl)phenyl)piperidine hydrochloride (0904-119) (80 mg, 0.254 mmol, 1.0 eq.), 3-hydroxy-3-methylbutyric acid (30 mg, 0.254 mmol, 1.0 eq.), N,N-diisopropylethylamine (197 mg, 1.52 mmol, 6.0 eq.) in N,N-dimethylformamide (2 ml). The mixture was stirred at room temperature for 2 hours. The reaction was diluted with water (10 ml) and extracted with ethyl acetate (8 ml × 4). The combined organic layer was washed with saturated brine (20 ml × 3), dried over anhydrous sodium sulfate, and concentrated under reduced pressure. The residue was purified by prep-TLC (silica gel, dichloromethane/methanol = 16/1) to give a yellow solid product 1-(4-(2-fluoro-4-(1H-pyrazol-4-yl)phenyl)piperidin-1-yl)-3-hydroxy-3-methylbutan-1 -one (56.4 mg, yield: 64.1%). LCMS (ESI): m/z =346[M+1]⁺. ¹ H NMR (500 MHz, DMSO) δ 12.95 (s, 1H), 8.20 (s, 1H), 7.93 (s, 1H), 7.40 (t, *J* = 9.6 Hz, 2H), 7.26 (t, *J* = 8.0 Hz, 1H), 4.90 (s, 1H), 4.62 (d, *J* = 12.9 Hz, 1H), 4.12 (d, *J* = 13.0 Hz, 1H), 3.08 (dt, *J* = 24.1, 12.3 Hz, 2H), 2.63 (t, *J* = 12.3 Hz, 1H), 2.71 (s, 1H), 1.89 (d, *J* = 1 12.3 Hz, 2H), 1.63 (d, *J* = 9.5 Hz, 1H), 1.51 (dd, *J* = 12.4, 3.3 Hz, 1H), 1.19 (s, 6H).

### Example 106: Preparation of 4-(2-fluoro-4-(1H-pyrazol-4-yl)phenyl)-N-(2-hydroxy-2-methylpropyl)piperidine-1-carboxamide (Compound 152) (Prepared according to Scheme 9)

Under nitrogen protection, a solution of triphosgene (142 mg, 0.477 mmol, 3.0 eq.) in dichloromethane (1 ml) was added dropwise at 0 °C to a mixture of 4-(2-fluoro-4-(1H-pyrazol-4-yl)phenyl)piperidine hydrochloride (0904-119) (50 mg, 0.159 mmol, 1.0 eq.), pyridine (63 mg, 0.795 mmol, 5.0 eq.) in dichloromethane (2 ml). The mixture was warmed to room temperature and stirred for 4 hours. 6 mL 1 mol/L hydrochloric acid aqueous solution was added dropwise to the reaction solution. The aqueous phase was extracted with dichloromethane (6 ml × 3). The combined organic phases was washed with saturated sodium bicarbonate solution and saturated brine (10 ml × 2), then dried over anhydrous sodium sulfate and concentrated under reduced pressure. The residue was dissolved in dichloromethane (2 ml), and 1-amino-2-methylpropan-2-ol (20 mg, 0.224 mmol, 1.4 eq.) and triethylamine (48 mg, 0.477 mmol, 3.0 eq.) were added thereto. The mixture was stirred at room temperature for 2 hours. A 2 mol/L sodium hydroxide solution was added to the reaction solution and stirred at room temperature for 2 hours. The reaction was diluted with water (10 ml) and extracted with dichloromethane (6 ml × 4). The organic phase was washed with saturated brine (20 ml × 1), dried over anhydrous sodium sulfate and concentrated under reduced pressure. The residue was purified by prep-TLC (silica gel, dichloromethane/methanol = 12/1) to obtain a white solid product 4-(2-fluoro-4-(1H-pyrazol-4-yl)phenyl)-N-(2-hydroxy-2-methylpropyl)piperidine-1-c arboxamide (21.3 mg, yield: 37.2%). LCMS(ESI): m/z =361[M+1]⁺. ¹ H NMR (500 MHz, DMSO) δ 12.93 (s, 1H), 8.20 (s, 1H), 7.93 (s, 1H), 7.39 (t, *J* = 8.7 Hz, 2H), 7.25 (t, *J* = 8.2 Hz, 1H), 6.38 (t, *J* = 5.4 Hz, 1H), 4.64 (s, 1H), 4.12 (d, *J* = 12.9 Hz, 2H), 3.12 - 2.87 (m, 3H), 2.80 (t, *J* = 12.3 Hz, 2H), 1.71 (d, *J* = 12.0 Hz, 2H), 1.56 (dd, *J* = 21.8, 12.3 Hz, 2H), 1.05 (s, 6H).

### Example 107: Preparation of (1-(2-fluoro-4-(1H-pyrazol-4-yl)phenyl)piperidin-4-yl)(hexahydrocyclopenta[c]py rrol-2(1H)-yl)methanone (Compound 153) (Prepared according to Scheme 8)

Step 107a: Preparation of methyl 1-(2-fluoro-4-(1-(tetrahydro-2H-pyran-2-yl)-1H-pyrazol-4-yl)phenyl)piperidine-4-car boxylate (Compound 0702-153): Under nitrogen protection, a mixture of 4-(4-bromo-3-fluorophenyl)-1-(tetrahydro-2H-pyran-2-yl)-1H-pyrazole (0103-2) (5 g, 15.38 mmol, 1.0 eq.), methyl piperidine-4-carboxylate (3.3 g, 23.08 mmol, 1.5 eq.), cesium carbonate (12 g, 38.45 mmol, 2.5 eq.), 2-dicyclohexylphosphino-2',6'-diisopropoxy-1,1'-biphenyl (718 mg, 1.54 mmol, 0.1 eq.) and tris(dibenzylideneacetone)dipalladium (704 mg, 0.769 mmol, 0.05 eq.) in dioxane (60 ml) was stirred at 115°C for two days. The reaction was diluted with water (120 ml) and extracted with ethyl acetate (50 ml × 3). The combined organic layer was washed with saturated brine (150 ml × 1), dried over anhydrous sodium sulfate, and concentrated under reduced pressure. The residue was purified by column chromatography on silica gel (petroleum ether/ethyl acetate = 10/1 to 1/1) to give a yellow oily product methyl 1-(2-fluoro-4-(1-(tetrahydro-2H-pyran-2-yl)-1H-pyrazol-4-yl)phenyl)piperidine-4-car boxylate (3.5 g, yield: 59%). LCMS (ESI): m/z = 388 [M+1]⁺.

Step 107b: Preparation of 1-(2-fluoro-4-(1-(tetrahydro-2H-pyran-2-yl)-1H-pyrazol-4-yl)phenyl)piperidine-4-car boxylic acid (Compound 0801-153): Methyl 1-(2-fluoro-4-(1-(tetrahydro-2H-pyran-2-yl)-1H-pyrazol-4-yl)phenyl)piperidine-4-car boxylate (0702-153) (1.2 g, 0.842 mmol, 1.0 eq.) was added to a mixed solution of 2 mol/L sodium hydroxide solution and tetrahydrofuran (8 ml + 4 ml), and the mixture was stirred at 55 °C overnight. The reaction was adjusted to pH 1 and extracted with dichloromethane (10 ml × 5). The combined organic phases was washed with saturated brine (50 ml × 1), dried over anhydrous sodium sulfate, and concentrated under reduced pressure to give a yellow solid product, 1-(2-fluoro-4-(1-(tetrahydro-2H-pyran-2-yl)-1H-pyrazol-4-yl)phenyl)piperidine-4-car boxylic acid (1.03 g, yield: 88.9%). LCMS (ESI): m/z = 375 [M+1]⁺.

Step 107c: Preparation of (1-(2-fluoro-4-(1-(tetrahydro-2H-pyran-2-yl)-1H-pyrazol-4-yl)phenyl)piperidin-4-yl)( hexahydrocyclopenta[c]pyrrol-2(1H)-yl)methanone (Compound 0803-153): A mixture of 1-(2-fluoro-4-(1-(tetrahydro-2H-pyran-2-yl)-1H-pyrazol-4-yl)phenyl)piperidine-4-car boxylic acid (0801-153) (80 mg, 0.214 mmol, 1.0 eq.), octahydrocyclopenta[c]pyrrole hydrochloride (38 mg, 0.257 mmol, 1.2 eq.), HATU (98 mg, 0.257 mmol, 1.2 eq.), N,N-diisopropylethylamine (83 mg, 0.643 mmol, 3.0 eq.) in N,N-dimethylformamide (5 ml) was stirred at room temperature for 1 hour. The reaction was diluted with water and extracted with ethyl acetate. The organic layer was dried and concentrated under reduced pressure to give a yellow solid product (1-(2-fluoro-4-(1-(tetrahydro-2H-pyran-2-yl)-1H-pyrazol-4-yl)phenyl)piperidin-4-yl)( hexahydrocyclopenta[c]pyrrol-2(1H)-yl)methanone (130 mg, crude).LCMS (ESI): m/z = 467[M+1]⁺.

Step 107d: Preparation of (1-(2-fluoro-4-(1H-pyrazol-4-yl)phenyl)piperidin-4-yl)(hexahydrocyclopentane[c]pyr rol-2(1H)-yl)methanone (Compound 153): A mixture of (1-(2-fluoro-4-(1-(tetrahydro-2H-pyran-2-yl)-1H-pyrazol-4-yl)phenyl)piperidin-4-yl)( hexahydrocyclopentane[c]pyrrol-2(1H)-yl)methanone (0803-153) (130 mg, 0.278 mmol, 1.0 eq.) in hydrogen chloride-methanol solution (4 M, 4 ml) was stirred at room temperature for 1 hour. The solvent was removed under reduced pressure, the residue was diluted with water, adjusted to pH = 8 with sodium carbonate solution, extracted with dichloromethane, and the organic phase was dried and concentrated under reduced pressure. The residue was purified by prep-TLC (silica gel, dichloromethane/methanol = 15/1) to give a white solid product (1-(2-fluoro-4-(1H-pyrazol-4-yl)phenyl)piperidin-4-yl)(hexahydrocyclopenta[c]pyrrol -2(1H)-yl)methanone (50 mg, yield: 47.17%). LCMS(ESI): m/z =383[M+1]⁺. ¹ H NMR (500 MHz, DMSO) δ 12.87 (s, 1H), 8.12 (s, 1H), 7.87 (s, 1H), 7.45 - 7.35 (m, 1H), 7.32 (d, *J* = 8.2 Hz, 1H), 6.99 (t, *J* = 8.9 Hz, 1H), 3.77 - 3.63 (m, 1H), 3.51 (dd, *J* = 11.9, 8.8 Hz, 1H), 3.36 (d, *J* = 11.6 Hz, 2H), 3.27 (s, 1H), 3.12 (dd, *J* = 12.2, 4.7 Hz, 1H), 2.75 - 2.63 (m, 3H), 2.56 (dd, *J =* 10.1, 6.3 Hz, 2H), 1.87 - 1.61 (m, 7H), 1.61 - 1.47 (m, 1H), 1.41 (ddt, *J =* 18.0, 11.9, 6.0 Hz, 2H).

### Example 108: Preparation of (1-(2-fluoro-4-(1H-pyrazol-4-yl)phenyl)piperidin-4-yl)(isoindolin-2-yl)methanone (Compound 156) (Prepared according to Scheme 8)

Step 108a: Preparation of (1-(2-fluoro-4-(1-(tetrahydro-2H-pyran-2-yl)-1H-pyrazol-4-yl)phenyl)piperidin-4-yl)( isoindolin-2-yl)methanone (Compound 0803-156): To a mixture of 1-(2-fluoro-4-(1-(tetrahydro-2H-pyran-2-yl)-1H-pyrazol-4-yl)phenyl)piperidine-4-car boxylic acid (0801-153) (50 mg, 0.134 mmol, 1.0 eq.), isoindoline (16 mg, 0.134 mmol, 1.0 eq.), N,N-diisopropylethylamine (35 mg, 0.268 mmol, 2.0 eq.) in dichloromethane (2 ml) was added HATU (61 mg, 0.161 mmol, 1.2 eq.) under nitrogen. The mixture was stirred at room temperature for 3 hours. The reaction was diluted with water (10 ml) and extracted with dichloromethane (6 ml × 4). The combined organic phases was washed with saturated brine (20 ml × 1), then dried over anhydrous sodium sulfate and concentrated under reduced pressure. The residue was purified by prep-TLC (silica gel, dichloromethane/methanol = 20/1) to give a white solid product (1-(2-fluoro-4-(1-(tetrahydro-2H-pyran-2-yl)-1H-pyrazol-4-yl)phenyl)piperidin-4-yl)( isoindolin-2-yl)methanone (48 mg, yield: 75.5%). LCMS (ESI): m/z = 475[M+1]⁺.

Step 108b: Preparation of (1-(2-Fluoro-4-(1H-pyrazol-4-yl)phenyl)piperidin-4-yl)(isoindolin-2-yl)methanone (Compound 156): A mixture of (1-(2-fluoro-4-(1-(tetrahydro-2H-pyran-2-yl)-1H-pyrazol-4-yl)phenyl)piperidin-4-yl)( isoindolin-2-yl)methanone (0803-156) (48 mg, 0.101 mmol, 1.0 eq.) in hydrogen chloride-methanol solution (4 M, 5 mL) was stirred at room temperature for 1.0 h. The reaction was concentrated under reduced pressure, and the residue was diluted with water (10 mL), adjusted to pH = 10 with sodium carbonate solution, and extracted with dichloromethane (4 mL × 5). The combined organic phase was dried over anhydrous sodium sulfate and concentrated under reduced pressure. The residue was purified by prep-TLC (silica gel, eluent: dichloromethane/methanol = 16/1) to give a white solid product (1-(2-fluoro-4-(1H-pyrazol-4-yl)phenyl)piperidin-4-yl)(isoindolin-2-yl)methanone (15 mg, yield: 38.1%). LCMS (ESI): m/z =391[M+1]⁺. ¹ H NMR (500 MHz, DMSO) δ 12.87 (s, 1H), 8.13 (s, 1H), 7.88 (s, 1H), 7.43 - 7.22 (m, 6H), 7.03 (t, *J* = 8.8 Hz, 1H), 4.96 (s, 2H), 4.66 (s, 2H), 3.41 (d, *J* = 11.6 Hz, 2H), 2.80 - 2.66 (m, 3H), 1.90 - 1.73 (m, 4H).

### Example 109: Preparation of (1-(4-(1H-pyrazol-4-yl)phenyl)piperidin-4-yl)(5,7-dihydro-6H-pyrrolo[3,4-b]pyri din-6-yl)methanone (Compound 157) (Prepared according to Scheme 8)

Step 109a: Preparation of (5,7-dihydro-6H-pyrrolo[3,4-b]pyridin-6-yl)(1-(4-(1-(tetrahydro-2H-pyran-2-yl)-1H-p yrazol-4-yl)phenyl)piperidin-4-yl)methanone (Compound 0803-157): Under nitrogen protection, 1-(4-(1-(tetrahydro-2H-pyran-2-yl)-1H-pyrazol-4-yl)phenyl)piperidine-4-carboxylic acid (0801-55) (100 mg, 0.282 mmol, 1.0 eq.), 6,7-dihydro-5H-pyrrolo[3,4-b]pyridine hydrochloride (16 mg, 0.282 mmol, 1.0 eq.), HATU (129 mg, 0.338 mmol, 1.2 eq.) was added to a mixture of N,N-diisopropylethylamine (73 mg, 0.564 mmol, 2.0 eq.) in dichloromethane (2 ml). The mixture was stirred at room temperature for 3 hours. The reaction was diluted with water (10 ml) and extracted with dichloromethane (6 ml × 4). The combined organic phases was washed with saturated brine (20 ml × 1), then dried over anhydrous sodium sulfate and concentrated under reduced pressure. The residue was purified by prep-TLC (silica gel, dichloromethane/methanol = 16/1) to give a white solid product (5,7-dihydro-6H-pyrrolo[3,4-b]pyridin-6-yl)(1-(4-(1-(tetrahydro-2H-pyran-2-yl)-1H-p yrazol-4-yl)phenyl)piperidin-4-yl)methanone (78 mg, yield: 60.9%). LCMS (ESI): m/z =458[M+1]⁺.

Step 109b: Preparation of (1-(4-(1H-pyrazol-4-yl)phenyl)piperidin-4-yl)(5,7-dihydro-6H-pyrrolo[3,4-b]pyridin-6-yl)methanone (Compound 157): A mixture of (5,7-dihydro-6H-pyrrolo[3,4-b]pyridin-6-yl)(1-(4-(1-(tetrahydro2H-pyran-2-yl)-1H-p yrazol-4-yl)phenyl)piperidin-4-yl)methanone (0803-157) (78 mg, 0.171 mmol, 1.0 eq.) in hydrogen chloride-methanol solution (4 M, 5 mL) was stirred at room temperature for 1.0 h. The reaction was concentrated under reduced pressure, and the residue was diluted with water (10 mL), adjusted to pH = 10 with sodium carbonate solution, and extracted with dichloromethane (4 mL × 5). The combined organic phase was dried over anhydrous sodium sulfate and concentrated under reduced pressure. The residue was purified by prep-TLC (silica gel, eluent: dichloromethane/methanol = 12/1) to give a yellow solid product (1-(4-(1H-pyrazol-4-yl)phenyl)piperidin-4-yl)(5,7-dihydro-6H-pyrrolo[3,4-b]pyridin-6-yl)methanone (36 mg, yield: 56.4%). LCMS (ESI): m/z =374[M+1]⁺. ¹ H NMR (500 MHz, DMSO) δ 12.76 (s, 1H), 8.48 (d, *J* = 4.5 Hz, 1H), 8.01 (s, 1H), 7.79 (t, *J* = 7.5 Hz, 2H), 7.43 (d, *J* = 8.5 Hz, 2H), 7.32 (dd, *J* = 7.5, 4.5 Hz, 1H), 6.94 (d, *J* = 7.8 Hz, 2H), 4.99 (d, *J* = 28.7 Hz, 2H), 4.65 (d, *J =* 32.8 Hz, 2H), 3.75 (d, *J =* 6.3 Hz, 2H), 2.74 (dd, *J =* 7.5, 4.5 Hz, 1H). 25.0, 12.5 Hz, 3H), 1.84 (d, *J* = 12.2 Hz, 2H), 1.74 (dd, *J* = 11.9, 6.8 Hz, 2H).

### Example 110: Preparation of 2-(1-(4-(1H-pyrazol-4-yl)phenyl)piperidine-4-carbonyl)isoindoline-5-carbonitrile (Compound 158) (Prepared according to Scheme 8)

Step 110a: Preparation of 2-(1-(4-(1-(tetrahydro-2H-pyran-2-yl)-1H-pyrazol-4-yl)phenyl)piperidine-4-carbonyl) isoindoline-5-carbonitrile (Compound 0803-158) : A mixture of 1-(4-(1-(tetrahydro-2H-pyran-2-yl)-1H-pyrazol-4-yl)phenyl)piperidine-4-carboxylic acid (0801-55) (250 mg, 0.704 mmol, 1.0 eq.), isoindoline-5-carbonitrile hydrochloride (153 mg, 0.845 mmol, 1.2 eq.), HATU (321 mg, 0.845 mmol, 1.2 eq.), N,N-diisopropylethylamine (454 mg, 3.52 mmol, 5.0 eq.) in N,N-dimethylformamide (6 ml) was stirred at room temperature for 2.0 hours. The reaction was diluted with water and extracted with ethyl acetate. The organic layer was dried and concentrated under reduced pressure. The residue was purified by column chromatography on silica gel (dichloromethane: methanol = 20: 1) to obtain a yellow solid product 2-(1-(4-(1-(tetrahydro-2H-pyran-2-yl)-1H-pyrazol-4-yl)phenyl)piperidine-4-carbonyl) isoindoline-5-carbonitrile (260 mg, yield: 76.9%). ¹ H NMR (500 MHz, DMSO) δ 12.74 (s, 1H), 8.06 - 7.69 (m, 4H), 7.57 (dd, *J* = 10.8, 8.2 Hz, 1H), 7.43 (d, *J* = 8.6 Hz, 2H), 6.94 (d, *J* = 8.6 Hz, 2H), 5.02 (d, *J* = 19.8 Hz, 2H), 4.70 (d, *J* = 16.9 Hz, 2H), 3.76 (d, *J* = 12.4 Hz, 2H), 2.85 - 2.59 (m, 3H), 1.83 (d, *J* = 11.9 Hz, 2H), 1.78 - 1.63 (m, 2H).

Step 110b: Preparation of 2-(1-(4-(1H-pyrazol-4-yl)phenyl)piperidine-4-carbonyl)isoindoline-5-carbonitrile (Compound 158): A mixture of 2-(1-(4-(1-(tetrahydro-2H-pyran-2-yl)-1H-pyrazol-4-yl)phenyl)piperidine-4-carbonyl) isoindoline-5-carbonitrile (0803-158) (260 mg, 0.54 mmol, 1.0 eq.) in hydrogen chloride-methanol solution (4 M, 6 mL) was stirred at room temperature for 1.0 h. The solvent was removed under reduced pressure, and the residue was diluted with water, adjusted to pH = 9 with sodium carbonate solution, and extracted with dichloromethane. The organic phase was dried and concentrated under reduced pressure. The residue was purified by methanol slurrying to give 2-(1-(4-(1H-pyrazol-4-yl)phenyl)piperidine-4-carbonyl)isoindoline-5-carbonitrile (60 mg, yield: 28.0%) as a purple solid product. LCMS(ESI): m/z =398[M+1]⁺. ¹ H NMR (500 MHz, DMSO) δ 12.74 (s, 1H), 8.06 - 7.69 (m, 4H), 7.57 (dd, *J* = 10.8, 8.2 Hz, 1H), 7.43 (d, *J* = 8.6 Hz, 2H), 6.94 (d, *J* = 8.6 Hz, 2H), 5.02 (d, *J* = 19.8 Hz, 2H), 4.70 (d, *J* = 16.9 Hz, 2H), 3.76 (d, *J* = 12.4 Hz, 2H), 2.85 - 2.59 (m, 3H), 1.83 (d, *J* = 11.9 Hz, 2H), 1.78 - 1.63 (m, 2H).

### Example 111: Preparation of 2-((1-(2-fluoro-4-(1H-pyrazol-4-yl)phenyl)piperidin-4-yl)methyl)hexahydrocyclo penta[c]pyrrol-1(2H)-one (Compound 160)

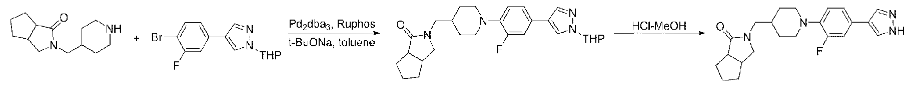

Step 111a: Preparation of 2-((1-(2-fluoro-4-(1-(tetrahydro-2H-pyran-2-yl)-1H-pyrazol-4-yl)phenyl)piperidin-4-yl)methyl)hexahydrocyclopenta[c]pyrrol-1(2H)-one:
2-(Piperidin-4-ylmethyl)hexahydrocyclopenta[c]pyrrol-1(2H)-one (150 mg, 0.68 mmol, 1.1 eq.) was added to a mixture of 4-(4-bromo-3-fluorophenyl)-1-(tetrahydro-2H-pyran-2-yl)-1H-pyrazole (0103-2) (200 mg, 0.62 mmol, 1.0 eq.), tris(dibenzylideneacetone)dipalladium (28 mg, 0.031 mmol, 0.05 eq.), 2-dicyclohexylphosphino-2',6'-diisopropoxy-1,1'-biphenyl (29 mg, 0.062 mmol, 0.1 eq.), sodium tert-butoxide (148 mg, 1.54 mmol, 2.5 eq.) in toluene (8 ml). The mixture was heated to 120°C under nitrogen atmosphere overnight. The mixture was diluted with water (15 ml). The aqueous layer was extracted with ethyl acetate (15 ml × 3). The combined organic layer was washed with saturated brine (20 ml × 1), dried over anhydrous sodium sulfate and concentrated. The residue was purified by Pre-TLC (silica gel, dichloromethane: methanol = 20: 1) to give a pale yellow oily product 2-((1-(2-fluoro-4-(1-(tetrahydro-2H-pyran-2-yl)-1H-pyrazol-4-yl)phenyl)piperidin-4-yl)methyl)hexahydrocyclopenta[c]pyrrol-1(2H)-one (267 mg, yield: 93%). LCMS (ESI): m/z = 467 [M+1]⁺; TLC: Rf 0.5 (dichloromethane: methanol = 20: 1).

Step 111b: Preparation of 2-((1-(2-fluoro-4-(1H-pyrazol-4-yl)phenyl)piperidin-4-yl)methyl)hexahydrocyclopent a[c]pyrrol-1(2H)-one (Compound 160): A mixture of 2-((1-(2-fluoro-4-(1-(tetrahydro-2H-pyran-2-yl)-1H-pyrazol-4-yl)phenyl)piperidin-4-yl)methyl)hexahydrocyclopenta[c]pyrrol-1(2H)-one (267 mg, 0.57 mmol, 1.0 eq.) in hydrogen chloride-methanol solution (4M, 5 mL) was stirred at room temperature for 4 hours. The mixture was diluted with water (20 mL), and solid sodium carbonate was added to adjust the pH to 10. The aqueous layer was extracted with ethyl acetate (15 mL×3). The combined organic layer was washed with saturated brine (20 mL×1), dried over anhydrous sodium sulfate and concentrated. The residue was diluted with methanol (3 ml) and then filtered. The solid was washed with methanol and then dried under vacuum to give 2-((1-(2-fluoro-4-(1H-pyrazol-4-yl)phenyl)piperidin-4-yl)methyl)hexahydrocyclopent a[c]pyrrol-1(2H)-one (123 mg, yield: 56%) as a white solid. LCMS (ESI): m/z = 383 [M+1]⁺; TLC: Rf 0.5 (dichloromethane: methanol = 10: 1). ¹ H NMR (500 MHz, DMSO) δ 12.86 (s, 1H), 8.12 (s, 1H), 7.86 (s, 1H), 7.34 (dd, *J* = 29.1, 11.2 Hz, 2H), 6.98 (t, *J* = 8.9 Hz, 1H), 3.56 (t, *J* = 9.2 Hz, 1H), 3.33 (s, 2H), 3.18 - 3.12 (m, 1H), 3.00 (dd, *J* = 12.8, 7.1 Hz, 2H), 2.78 (dd, *J* = 8.7, 7.1 Hz, 1H), 2.63 (dd, *J* = 21.6, 10.5 Hz, 3H), 1.83 - 1.55 (m, 7H), 1.44 - 1.09 (m, 4H).

### Example 112: Preparation of 1-((1-(2-fluoro-4-(1H-pyrazol-4-yl)phenyl)piperidin-4-yl)methyl)azepan-2-one (Compound 161) (Prepared according to Scheme 1)

Step 112a: Preparation of tert-butyl 4-((2-oxoazepan-1-yl)methyl)piperidine-1-carboxylate (Compound 0107-161): Under nitrogen protection, sodium hydride (60%, 89 mg, 2.22 mmol, 1.3 eq.) was added to a mixture of caprolactam (251 mg, 2.22 mmol, 1.3 eq.) in N,N-dimethylformamide (10 mL) at 0 °C, and the reaction mixture was stirred at room temperature for half an hour. A solution of tert-butyl 4-(((methylsulfonyl)oxy)methyl)piperidine-1-carboxylate (500 mg, 1.71 mmol, 1.0 eq.) in N,N-dimethylformamide (5 mL) was added dropwise to the reaction mixture. The reaction was stirred at 65 °C overnight. The reaction mixture was diluted with water (60 mL) and extracted with ethyl acetate (30 mL × 4). The combined organic phases was washed with saturated brine (100 ml × 3), dried over anhydrous sodium sulfate and concentrated under reduced pressure. The residue was purified by column chromatography on silica gel (petroleum ether/ethyl acetate = 5/1 to 1/2) to obtain a light yellow oily product tert-butyl 8-(2,3-difluoro-4-(1-(tetrahydro-2H-pyran-2-yl)-1H-pyrazol-4-yl)phenyl)-3,8-diazabi cyclo[3.2.1] octane-3-carboxylate (102 mg, yield: 19.2%). LCMS (ESI): m/z = 311 [M+1]⁺.

Step 112b.: Preparation of 1-(piperidin-4-ylmethyl)azepan-2-one (Compound 0108-161): A mixture of tert-butyl 4-((2-oxoazepan-1-yl)methyl)piperidine-1-carboxylate (0107-161) (102 mg, 0.329 mmol, 1.0 eq.) in hydrogen chloride-methanol solution (4 M, 6 mL) was stirred at room temperature for 2.0 hours. The reaction was concentrated under reduced pressure, the residue was diluted with water (5 mL), the pH was adjusted to 13 with sodium hydroxide solution, and extracted with dichloromethane (5 mL × 4). The combined organic phase was dried over anhydrous sodium sulfate and concentrated under reduced pressure to give a yellow oily product 1-(piperidin-4-ylmethyl)azepan-2-one (71.9 mg, yield: 100%). LCMS (ESI): m/z = 211 [M+1]⁺.

Step 112c: Preparation of 1-((1-(2-fluoro-4-(1-(tetrahydro-2H-pyran-2-yl)-1H-pyrazol-4-yl)phenyl)piperidin-4-yl)methyl)azepan-2-one (Compound 0109-161): Under nitrogen protection, a solution of 4-(4-bromo-3-fluorophenyl)-1-(tetrahydro-2H-pyran-2-yl)-1H-pyrazole (0103-2) (93 mg, 0.285 mmol, 1.0 eq.), 1-(piperidin-4-ylmethyl)azepan-2-one (0108-161) (71.9 mg, 0.342 mmol, 1.2 eq.), sodium tert-butoxide (68.5 mg, 0.713 mmol, 2.5 eq.), 2-dicyclohexylphosphino-2',6'-diisopropoxy-1,1'-biphenyl (26 mg, 0.057 mmol, 0.2 eq.) and tris(dibenzylideneacetone)dipalladium (26 mg, 0.0285 mmol, 0.1 eq.) in toluene (4 ml) was stirred at 120°C overnight. The reaction was diluted with water (15 ml) and extracted with ethyl acetate (10 ml × 3). The combined organic phases was washed with saturated brine (30 ml × 1), dried over anhydrous sodium sulfate and concentrated under reduced pressure. The residue was purified by Pre-TLC (ethyl acetate/methanol = 20/1) to give 1-((1-(2-fluoro-4-(1-(tetrahydro-2H-pyran-2-yl)-1H-pyrazol-4-yl)phenyl)piperidin-4-yl)methyl)azepan-2-one (71 mg, yield: 54.9%) as a yellow solid. LCMS (ESI): m/z = 455 [M+1]⁺.

Step 112d: 1-((1-(2-Fluoro-4-(1H-pyrazol-4-yl)phenyl)piperidin-4-yl)methyl)azepan-2-one (Compound 161): A mixture of 1-((1-(4-(1-(tetrahydro-2H-pyran-2-yl)-1H-pyrazol-4-yl)phenyl)piperidin-4-yl)methyl) pyrrolidin-2-one (0109-161) (71 mg, 0.156 mmol, 1.0 eq.) in hydrogen chloride-methanol solution (4 M, 4 mL) was stirred at room temperature for 2.0 hours. The reaction was concentrated under reduced pressure, and the residue was diluted with water (5 mL), adjusted to pH = 9 with sodium carbonate solution, and extracted with dichloromethane (5 mL × 4). The combined organic phase was dried over anhydrous sodium sulfate and concentrated under reduced pressure. The residue was purified by prep-TLC (silica gel, eluent: dichloromethane/methanol = 12/1) to give 1-((1-(2-fluoro-4-(1H-pyrazol-4-yl)phenyl)piperidin-4-yl)methyl)azepan-2-one (36 mg, yield: 62.4%) as a white solid. LCMS(ESI): m/z =371[M+1]⁺. ¹ H NMR (500 MHz, DMSO) δ 12.85 (s, 1H), 8.09 (d, *J* = 24.0 Hz, 1H), 7.87 (s, 1H), 7.34 (dd, *J =* 29.0, 11.8 Hz, 2H), 6.99 (t, *J =* 8.9 Hz, 1H), 3.38 (d, *J* = 8.6 Hz, 2H), 3.34 (s, 2H), 3.22 (d, *J* = 6.6 Hz, 2H), 2.61 (t, *J* = 11.4 Hz, 2H), 2.46 - 2.39 (m, 2H), 1.67 (d, *J* = 10.7 Hz, 5H), 1.56 (s, 4H), 1.37 - 1.28 (m, 2H).

### Example 113: Preparation of 2-((1-(2-fluoro-4-(1H-pyrazol-4-yl)phenyl)piperidin-4-yl)methyl)-1,2-thiazinane 1,1-dioxide (Compound 163) (Prepared according to Scheme 13)

Step 113a: Preparation of tert-butyl 4-((1,1-dioxido-1,2-thiazinan-2-yl)methyl)piperidine-1-carboxylate (Compound 1302-163): To a mixture of sodium hydride (60% dispersion in mineral oil, 80 mg, 2.0 mmol, 1.3 eq.) in N,N-dimethylformamide (10 mL) was added 1,2-thiazinane 1,1-dioxide (1301-163) (270 mg, 2.0 mmol, 1.3 eq.). The mixture was stirred at room temperature for 0.5 h. tert-butyl 4-(((methylsulfonyl)oxy)methyl)piperidine-1-carboxylate (0105-1) (450 mg, 1.54 mmol, 1.0 eq.) was added and the mixture was heated to 65 °C under nitrogen for 3 h. The reaction was quenched by adding saturated aqueous ammonium chloride (30 mL). The aqueous layer was extracted with ethyl acetate (20 ml × 3). The combined organic layer was washed with saturated brine (20 ml × 1), dried over anhydrous sodium sulfate and concentrated. The residue was subjected to column chromatography on silica gel (petroleum ether: ethyl acetate 2: 1) to give a pale yellow oily product tert-butyl 4-((1,1-dioxido-1,2-thiazinan-2-yl)methyl)piperidine-1-carboxylate (512 mg, yield: 100%). LCMS (ESI): m/z = 333 [M+1]⁺; TLC: Rf 0.3 (petroleum ether: ethyl acetate = 2: 1).

Step 113b: Preparation of 2-(piperidin-4-ylmethyl)-1,2-thiazinane 1,1-dioxide (Compound 1303-163): A mixture of tert-butyl 4-((1,1-dioxido-1,2-thiazinan-2-yl)methyl)piperidine-1-carboxylate (1302-163) (512 mg, 1.54 mmol, 1.0 eq.) in hydrogen chloride-methanol solution (4M, 5 mL) was stirred at room temperature for 3 hours. The solvent was removed under reduced pressure, and the residue was dissolved in methanol (5 mL) and water (1 mL). Sodium hydroxide (74 mg, 1.85 mmol, 1.2 eq.) was added. The mixture was concentrated under reduced pressure. The residue was diluted with dichloromethane (30 mL) and then filtered. The filtrate was dried over anhydrous sodium sulfate and concentrated to give 2-(piperidin-4-ylmethyl)-1,2-thiazinane 1,1-dioxide (342 mg, yield: 96%) as a white solid. LCMS (ESI): m/z = 233 [M+1]⁺; TLC: Rf 0.3 (dichloromethane:methanol= 1 0: 1).

Step 113c: Preparation of 2-((1-(2-fluoro-4-(1-(tetrahydro-2H-pyran-2-yl)-1H-pyrazol-4-yl)phenyl)piperidin-4-yl)methyl)-1,2-thiazinane 1,1-dioxide (Compound 1304-163): 2-(Piperidin-4-ylmethyl)-1,2-thiazinane 1,1-dioxide (79 mg, 0.43 mmol, 1.2 eq.) was added to a mixture of 4-(4-bromo-3-fluorophenyl)-1-(tetrahydro-2H-pyran-2-yl)-1H-pyrazole (1303-163) (130 mg, 0.40 mmol, 1.0 eq.), tris(dibenzylideneacetone)dipalladium (18 mg, 0.02 mmol, 0.05 eq.), 4,5-bis(diphenylphosphino)-9,9-dimethylxanthene (23 mg, 0.04 mmol, 0.1 eq.) and sodium tert-butoxide (96 mg, 1.0 mmol, 2.5 eq.) in toluene (8 ml). The mixture was heated to 120°C under nitrogen atmosphere and reacted overnight. The mixture was diluted with water (20 ml). The aqueous layer was extracted with ethyl acetate (15 ml×3). The combined organic layer was washed with saturated brine (20 ml × 1), dried over anhydrous sodium sulfate and concentrated. The residue was purified by Pre-TLC (dichloromethane: methanol = 40: 1) to give a pale yellow oily product 2-((1-(2-fluoro-4-(1-(tetrahydro-2H-pyran-2-yl)-1H-pyrazol-4-yl)phenyl)piperidin-4-yl)methyl)-1,2-thiazinane 1,1-dioxide (86 mg, yield: 45%). LCMS (ESI): m/z = 477 [M+1]⁺; TLC: Rf 0.5 (dichloromethane: methanol = 40: 1).

Step 113d: Preparation of 2-((1-(2-fluoro-4-(1H-pyrazol-4-yl)phenyl)piperidin-4-yl)methyl)-1,2-thiazinane 1,1-dioxide (Compound 163): A mixture of 2-((1-(2-fluoro-4-(1-(tetrahydro-2H-pyran-2-yl)-1H-pyrazol-4-yl)phenyl)piperidin-4-yl)methyl)-1,2-thiazinane 1,1-dioxide (1304-163) (86 mg, 0.18 mmol, 1.0 eq.) in hydrogen chloride-methanol solution (4M, 3 mL) was stirred at room temperature for 3 hours. The mixture was diluted with water (20 mL), solid sodium carbonate was added to adjust pH=10, and then the aqueous layer was extracted with ethyl acetate (20 mL×3). The combined organic layer was washed with saturated brine (20 mL×1), dried over anhydrous sodium sulfate and concentrated. The residue was purified by Pre-TLC (silica gel, dichloromethane: methanol = 10: 1) to give 2-((1-(2-fluoro-4-(1H-pyrazol-4-yl)phenyl)piperidin-4-yl)methyl)-1,2-thiazinane 1,1-dioxide (23 mg, yield: 32%) as a white solid. LCMS (ESI): m/z = 393 [M+1]⁺; TLC: Rf 0.5 (dichloromethane: methanol = 10: 1). ¹ H NMR (500 MHz, DMSO) δ 12.85 (s, 1H), 7.99 (s, 2H), 7.37 (d, *J* = 14.1 Hz, 1H), 7.31 (d, *J* = 8.1 Hz, 1H), 7.00 (t, *J* = 8.8 Hz, 1H), 3.33 (s, 4H), 3.10 - 3.01 (m, 2H), 2.98 (d, *J* = 6.9 Hz, 2H), 2.63 (t, *J* = 11.3 Hz, 2H), 2.02 (s, 2H), 1.77 (d, *J =* 12.1 Hz, 2H), 1.63 (s, 1H), 1.56 (s, 2H), 1.29 (dd, *J* = 21.3, 11.3 Hz, 2H).

### Example 114 Biological Activity Test

The reference compound used in this invention is compound 83 from WO 2020163689, with the following structure:

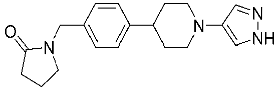

### I, 20-HETE inhibitory activity

### 1, Experimental materials

| | |
|---|---|
| Human liver microsomes | BD genest#452161 |
| Rat liver microsomes | BD genest#452591 |
| Substrate (arachidonic acid) | TM standard #70067 |
| NADPH | Millipore# 481973 |
| K⁺ buffer | Self-prepared in the laboratory (batch 20210826) |
| 96-well plate | Coring #3599 |

### 2, Experimental methods

Experimental principle: The inhibitory activity of 20-HETE production is evaluated by testing the inhibitory effects of the compound on human liver microsomes (HLM), rat liver microsomes (RLM), mouse liver microsomes (MLM), or specific substrates for CYP4 subtype enzymes (CYP4F2). This assay employs HLM, RLM, or MLM along with recombinant CYP4F2 (rCYP4F2) and recombinant CYP4A11 (rCYP4A11), which are specific substrates for CYP4 subtype enzymes, to detect the effects of the test compound at different concentrations on the metabolic rate of enzyme-specific substrates, to determine the inhibitory effects of the test compound on CYP enzymes in HLM, RLM, or MLM.

Experimental methods: Dissolve the test compound in DMSO, and subsequently dilute it with an acetonitrile and DMSO mixture (3:1). Add 2 µl of varying concentrations of the test compound and 100 µl of potassium phosphate buffer containing either liver microsomes or recombinant enzymes to a 96-well plate. After mixing well, transfer 30 µl to another well (in duplicate), add 15 µl of 400 µM substrate solution, and mix well again. Preheat at 37°C for 10 minutes, and then add 15 µl of 6 mM NADPH and incubate at 37°C for 25 minutes. After 25 minutes, add 160 µl of acetonitrile solution (containing 0.1 µg/ml internal standard) to stop the reaction. Mix well, centrifuge at 3500 g for 15 minutes, and then analyze the supernatant using LC-MS/MS.

Plot the compound concentration on the x-axis and the inhibition rate on the y-axis to draw the curve. Use GraphPad Prism 5 software to fit the curve and calculate the IC₅₀. The formula is Y = Bottom + (Top - Bottom) / (1 + 10^((LogIC₅₀ - X) × HillSlope)). X represents the logarithmic concentration of the inhibitor, and Y represents the relative enzyme activity at a given inhibitor concentration (relative to the condition without the inhibitor).

### 3, Experimental results

Calculate the inhibition percentages at concentrations of 1000 nM, 500 nM, or 100 nM as well as the half-maximal inhibitory concentration (IC₅₀) of the test compound from the above experiment. The results are shown in Tables 1 and 2.

**Table 1. Inhibitory Activity of Compounds on 20-HETE Production (HLM, RLM, or MLM**

| Compoun ds | HLM (% inh @ 500 nM) | HLM (% inh @ 100 nM) | HLM (IC50 nM) | RLM (% inh @ 500nM ) | RLM (IC50 nM) | MLM (% inh @ 1000 nM) | MLM (IC50 nM) |
|---|---|---|---|---|---|---|---|
| 1 | 79.83 | | | 54.99 | 223.5 | | 1222 |
| 2 | 79.50 | | 28.68 | 59.38 | 102.6 | | 1337 |
| 3 | 75.59 | | | | 321.6 | | |
| 4 | 85.53 | | 25.80 | 72.00 | 89.49 | | 1348 |
| 5 | 81.03 | | 28.14 | 66.71 | 44.19 | | 945.7 |
| 6 | 67.30 | | | 23.02 | | | |
| 10 | | | 46.10 | | 541.7 | | |
| 11 | 80.98 | | | | 157.2 | | |
| 12 | 76.91 | | | 17.99 | | | |
| 13 | 58.98 | | | -4.72 | | | |
| 14 | 41.24 | | | | | | |
| 15 | 64.22 | | | 12.62 | | | |
| 16 | 41.22 | | | | | | |
| 17 | 65.27 | | | 17.66 | | | |
| 18 | 47.87 | | | 8.61 | | | |
| 19 | 87.01 | | | 59.42 | 434.1 | | |
| 20 | 36.16 | | | | | | |
| 21 | 40.01 | | | | | | |
| 22 | 53.10 | | | 34.61 | | | |
| 23 | -24.84 | | | | | | |
| 25 | 80.65 | | 29.27 | 61.49 | 159.1 | | 765.9 |
| 26 | 89.67 | | | 63.62 | 123.9 | | 1671 |
| 27 | 88.87 | | 26.01 | | 23.33 | | 1154 |
| 28 | 55.98 | | | 16.98 | | | |
| 31 | 68.02 | | | 14.73 | | | |
| 33 | 61.47 | | | 8.80 | | | |
| 34 | 71.06 | | | 83.75 | | | |
| 37 | 82.89 | | | 59.01 | | | |
| 40 | 52.40 | | | 26.22 | | | |
| 41 | 57.77 | | | 15.76 | | | |
| 42 | | | 32.76 | 25.96 | 604.9 | | |
| 43 | 61.30 | | | 31.42 | | | |
| 44 | 85.05 | | 25.17 | | 33.84 | | |
| 45 | 57.83 | | | 26.43 | | | |
| 46 | | | 43.16 | 35.54 | 942.2 | | |
| 47 | | | 42.66 | 35.64 | | | |
| 48 | 73.42 | | | 39.97 | | | |
| 49 | 79.80 | | | 44.31 | 520.8 | | |
| 50 | 57.59 | | | 14.48 | | | |
| 51 | 30.17 | | | | | | |
| 54 | 81.26 | | | 32.02 | 994.7 | | |
| 55 | 62.73 | | | 21.92 | | | |
| 57 | 81.76 | | | 49.97 | | | |
| 58 | | | 16.56 | 44.45 | | | 1567 |
| 61 | 85.27 | | 17.59 | | 60.51 | | |
| 62 | 85.05 | | 19.57 | 50.28 | | | |
| 64 | 81.86 | | 19.96 | | 86.39 | | |
| 65 | 52.63 | | | 2.30 | | | |
| 66 | 69.53 | | | 64.35 | | | |
| 67 | 55.57 | | | 30.84 | | | |
| 69 | 76.97 | | | 43.82 | | | |
| 70 | 89.89 | | 19.44 | | 4.90 | | 135.3 |
| 71 | 95.89 | | 19.71 | 75.13 | | | |
| 72 | 89.73 | | 19.14 | | 5.36 | | 1329 |
| 73 | 65.78 | | | 29.56 | | | |
| 74 | 72.44 | | | 47.19 | | | |
| 76 | 80.70 | | | 40.73 | | | |
| 77 | 86.12 | | | 62.98 | | | |
| 88 | 78.05 | | | 68.94 | | | |
| 89 | 94.11 | | | 55.76 | | | |
| 91 | 96.45 | | 19.13 | | 9.09 | | 734.6 |
| 92 | 94.83 | | 26.44 | 77.48 | | | |
| 93 | 91.77 | | | 80.86 | | | 295.7 |
| 94 | 92.79 | | | 73.06 | 147.8 | | |
| 95 | 94.96 | | 19.45 | 61.73 | 340.7 | | |
| 97 | 95.80 | | 25.65 | | 25.35 | | |
| 98 | 96.23 | | 21.99 | | 9.68 | | |
| 99 | 96.13 | | | 74.05 | | | |
| 101 | 78.37 | | | 69.34 | | | |
| 102 | 84.23 | | | 76.10 | | | |
| 103 | 80.55 | | 11.97 | 74.73 | | | 743.1 |
| 104 | 78.18 | | | 73.19 | | | |
| 105 | 79.54 | | 11.07 | | 9.78 | | 1521 |
| 106 | 91.17 | | 23.23 | 84.57 | 10.73 | | 2811 |
| 107 | | | 23.64 | 69.55 | 33.36 | | 720.4 |
| 109 | 74.67 | | | 68.22 | | | |
| 110 | 92.92 | | 16.05 | 77.32 | 29.49 | | 173.8 |
| 111 | 82.17 | | | 84.99 | | | |
| 112 | 70.73 | | | 71.58 | | | |
| 113 | 87.72 | | 20.55 | 57.83 | 15.50 | | |
| 114 | 85.59 | | | 54.89 | | | |
| 115 | 85.21 | | 20.84 | | 26.19 | | |
| 119 | 87.83 | | | 75.79 | 115.8 | | |
| 120 | 93.40 | | 16.09 | 71.20 | 107.1 | | |
| 124 | 88.29 | | 32.80 | 74.61 | | | 1850 |
| 125 | 94.93 | | | 34.82 | | | |
| 126 | 64.15 | | | | | | |
| 127 | | | 18.34 | 74.10 | 17.89 | | 1905 |
| 128 | 89.99 | | | 62.11 | | | |
| 129 | 89.94 | | | 74.89 | | | |
| 130 | | | 7.62 | | 22.84 | | 413.6 |
| 132 | | | 25.61 | | | | 2424 |
| 133 | | 87.90 | | | | | |
| 134 | | | 21.87 | | | | |
| 136 | | 87.34 | 19.42 | | | | 663.5 |
| 138 | 92.84 | | | 77.16 | | | |
| 139 | | 88.13 | | | | | |
| 140 | | | 17.62 | | | | 1521 |
| 141 | | 88.15 | | | | 59.36 | |
| 144 | | 87.74 | | | | 60.66 | |
| 145a | | 86.67 | | | | 60.45 | |
| 145b | | 88.49 | | | | 48.10 | |
| 148 | | 85.60 | | | | | |
| 149a | | 84.74 | | | | | |
| 149b | | 83.42 | | | | 47.54 | |
| 150 | | 84.56 | | | | 40.18 | |
| 151 | | 82.28 | | | | | |
| 152 | | 88.49 | | | | 48.10 | |
| 153 | | 74.69 | 32.35 | | 360.6 | | |
| 156 | | | 26.17 | | | | |
| 157 | | | 17.56 | | | | |
| 158 | | | 11.71 | | | | |
| 160 | | | 23.25 | | | | |
| 161 | | 86.45 | | | | 59.36 | |
| 163 | 90.91 | | 9.53 | 64.17 | 221.8 | | 1008 |
| Referenc e 83 | 79.09 | | 51.79 | 38.16 | 598.3 | | >10000 |

As shown in Table 1, the compounds of the present invention can effectively inhibit the activity of CYP enzymes in human liver microsomes (HLM), or rat liver microsomes (RLM), or mouse liver microsomes (MLM), thereby inhibiting the production of 20-HETE.

**Table 2. Inhibitory Activity of Compounds on 20-HETE Production (rCYP4F2 and rCYP4A11)**

| Compound | rCYP4F2 (% inh @ 100 nM) | rCYP4F2 (IC50 nM) | rCYP4A11 (% inh @ 100nM) | rCYP4A11 (IC50 nM) |
|---|---|---|---|---|
| 1 | 59.47 | 24.04 | 35.41 | |
| 2 | | 15.76 | | 33.16 |
| 3 | 64.21 | | 27.80 | |
| 4 | 61.72 | 18.77 | | 53.69 |
| 5 | 71.14 | 24.93 | | 39.59 |
| 11 | 59.70 | | 46.34 | |
| 19 | 61.36 | | 58.70 | |
| 25 | 55.95 | | 30.59 | |
| 26 | 77.96 | | 53.91 | |
| 27 | 78.38 | | 52.27 | |
| 34 | 49.68 | | 1.59 | |
| 47 | 65.96 | | 41.15 | |
| 49 | 42.35 | | 23.30 | |
| 57 | 61.69 | | 8.98 | |
| 62 | 79.17 | | 25.75 | |
| 64 | 73.83 | | 40.60 | |
| 66 | 70.49 | | 9.25 | |
| 70 | 79.16 | | 65.45 | |
| 72 | 70.08 | | 50.65 | |
| 74 | 52.75 | | 17.47 | |
| Reference 83 | 54.63 | | 35.60 | |

As shown in Table 2, the compounds of the present invention effectively inhibit the activity of CYP4F2 and CYP4A11, the main enzymes that generate 20-HETE in humans, thereby inhibiting the production of 20-HETE.

### II, Pharmacokinetic (PK) experiment

### 1. Experimental methods

Male SD rats, weighing 180-250 grams, were fasted overnight before testing. The test compound was dissolved in 30% sulfobutylether-β-cyclodextrin and administered orally at a dose of 20 mg/kg. Blood was collected from the tail vein at 15 minutes, 30 minutes, and 1, 2, 4, 6, 8, and 24 hours after administration. Approximately 0.3 ml of blood was collected at each time point, placed in a centrifuge tube containing K2-EDTA, and centrifuged (2000 g for 10 minutes at 4°C) to take the plasma, which was then stored in an ultra-low temperature freezer at -70°C or below. A 50 µL plasma sample was mixed with 135 µL of acetonitrile (containing 0.5 µg/mL internal standard) and vortexed for protein precipitation. After centrifugation, the supernatant was taken for LC-MS/MS analysis.

### 2. Experimental results

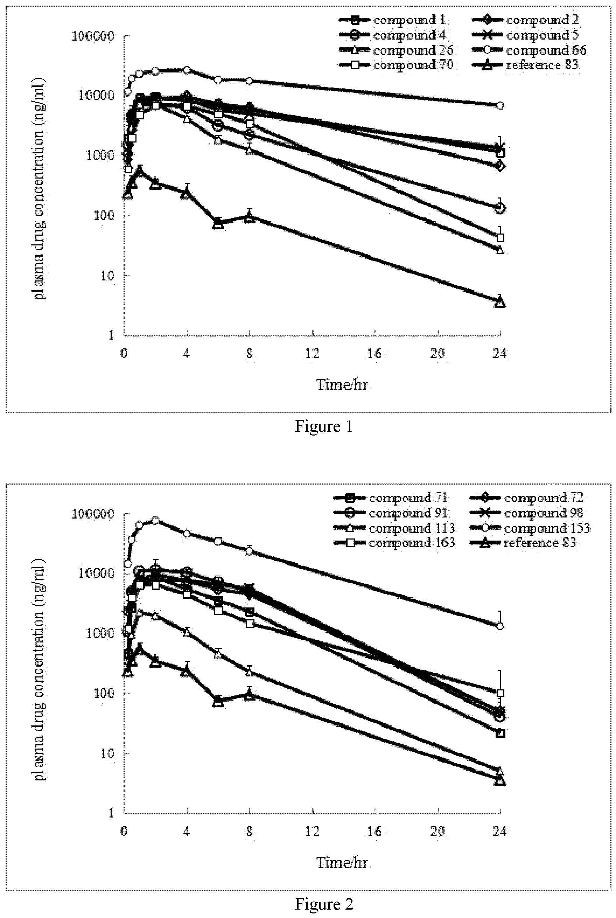

Compounds 1, 2, 4, 5, 26, 66, 70, 71, 72, 91, 98, 113, 153, and 163 provided by the present invention are well absorbed and exhibit high blood exposure after oral administration in rats. The results are shown in Figure 1 and 2 and Table 3. The 1,4-diheterocyclic substituted aromatic ring or aromatic heterocyclic compounds of the present invention have Tₘₐₓ values of 1.33-3.33 hours, Cₘₐₓ values of 2416.7-75233.3 ng/ml, and AUC₀₋₂₄ values of 10103.6-557523.7 hr*ng/mL. Compared to the reference compound 83, the compounds of the present invention exhibit improved pharmacokinetic properties. Cₘₐₓ refers to the maximum blood drug concentration, T_{1/2} refers to the half-life, AUC₀₋₂₄ refers to the area under the concentration-time curve from 0 to 24 hours, and AUC_{0-inf} refers to the area under the concentration-time curve from 0 to infinity.

**Table 3. Pharmacokinetic Parameters following Oral Administration in Rats (20 mg/kg)**

| | PK parameters | | | | |
|---|---|---|---|---|---|
| Compound | T_{1/2} (hr) | Tₘₐₓ (hr) | Cₘₐₓ (ng/ml) | AUC₀₋₂₄ (hr*ng/mL) | AUC_{0-inf} (hr*ng/mL) |
| 1 | 7.23 | 1.5 | 9985 | 113054.8 | 124909.5 |
| 2 | 5.20 | 3.33 | 10070 | 115068.2 | 120153.7 |
| 4 | 3.90 | 1.33 | 7256.7 | 58052.8 | 58969.3 |
| 5 | 8.11 | 2.67 | 9273.3 | 103272.8 | 124029.2 |
| 26 | 2.93 | 1.67 | 7150.0 | 40348.9 | 40460.3 |
| 66 | 12.46 | 3.33 | 26766.7 | 363970.8 | 485795.5 |
| 70 | 2.57 | 3 | 7325.0 | 68798.0 | 68972.0 |
| 71 | 2.41 | 2 | 8726.7 | 57913.0 | 57990.0 |
| 72 | 2.51 | 2.33 | 7877.0 | 85820.0 | 86014.7 |
| 91 | 2.22 | 2.5 | 11450.0 | 106504.0 | 106650.0 |
| 98 | 2.38 | 3 | 8700.0 | 98444.0 | 98630.0 |
| 113 | 2.93 | 1.33 | 2416.7 | 10103.6 | 10125.4 |
| 153 | 3.26 | 2 | 75233.3 | 557523.7 | 566184.6 |
| 163 | 4.03 | 1.67 | 6683.3 | 44116.5 | 44704.3 |
| Reference 83 | 3.53 | 1.50 | 562.5 | 2625.4 | 2643.8 |

### III, Pharmacological experiment

### 1. Experimental methods

Experiment 1 was to investigate the effects of compound 1 on weight loss and blood glucose lowering in a diet-induced obesity (DIO) mouse model. Male C57BL/6J mice were purchased from Zhuhai BST Biotechnology Co., Ltd. and housed in a barrier system animal facility. The animals were randomly divided into 3 groups: normal control group, DIO model control group, and DIO model compound 1 treatment group, with 6 animals per group. The treatment group received compound 1 orally at a dose of 100 mg/kg on the first day of high-fat diet (HFD) feeding, once daily for 32 consecutive days. Animal weights were measured weekly, and on day 33, they were fasted overnight but allowed access to water before an oral glucose tolerance test (OGTT).

Experiment 2 was to study the effects of compound 2 and 4 on weight loss and blood glucose lowering in a diet-induced obesity (DIO) mouse model. Male C57BL/6J mice were purchased from Zhuhai BST Biotechnology Co., Ltd. and housed in a barrier system animal facility. Animals were randomly divided into 4 groups: normal control group, DIO model control group, DIO model compound 2 treatment group, and DIO model compound 4 treatment group, with 4-5 animals per group. The model control group and treatment groups were first fed with high-fat diet (HFD) for 9 weeks (day 63), and then the treatment groups were orally administered with compound 2 or compound 4 at a dose of 50 mg/kg once daily for 4 consecutive weeks, while continuing to receive HFD. Animal weights were measured weekly, and on the last day of treatment, they were fasted overnight but allowed access to water before an oral glucose tolerance test (OGTT).

Experiment 3 was to study the effects of compound 2 on weight loss and blood glucose lowering in a diet-induced obesity (DIO) mouse model. Male C57BL/6J mice were purchased from Zhuhai BST Biotechnology Co., Ltd. and housed in a barrier system animal facility. Animals were randomly divided into 3 groups: normal control group, DIO model control group, and DIO model compound 2 treatment group, with 6-8 animals per group. The model control group and treatment group were first fed with high-fat diet (HFD) for 9 weeks (day 63), and then the treatment group was orally administered with compound 2 at a dose of 30 mg/kg once daily for 8 consecutive weeks, while continuing to receive HFD. Animal weights were measured weekly, and on the last day of treatment, they were fasted overnight but allowed access to water before an oral glucose tolerance test (OGTT).

Experiment 4 was to compare the weight loss effects of compound 2 with that of semaglutide in a diet-induced obesity (DIO) mouse model. Male C57BL/6J mice were purchased from Vital River Laboratories (Shanghai) Co., Ltd. and housed in a barrier system animal facility. Animals were randomly divided into 4 groups: normal control group, DIO model control group, compound 2 treatment group, and semaglutide treatment group, with 6 animals per group. The model control group and treatment groups were first fed with high-fat diet (HFD) for 18 weeks, after which the treatment groups were administered with compound 2 or semaglutide, respectively. Compound 2 was administered orally at a dose of 30 mg/kg once daily for 14 consecutive weeks, while semaglutide was administered subcutaneously at a dose of 30 nmol/kg once daily. After 7 weeks of semaglutide treatment, the semaglutide treatment group was switched to compound 2 treatment for another 7 weeks.

Experiment 5 was to investigate the effect of compound 2 on regulating the level of 20-HETE in a diet-induced obesity (DIO) mouse model. Male C57BL/6J mice were purchased from Guangdong Vital River Laboratory Animal Technology Co., Ltd. and housed in a barrier system animal facility. After 24 weeks of high-fat diet (HFD) induction, compound 2 was administered at a dose of 50 mg/kg once, and samples were collected after 8 hours to detect the level of 20-HETE. A solvent control group was also set up simultaneously, with 3 animals per group.

Experiment 6 was to investigate the effect of compound 2 on regulating the level of 20-HETE in a diet-induced obesity (DIO) rat model. Male SD rats were purchased from Guangdong Vital River Laboratory Animal Technology Co., Ltd. and housed in a barrier system animal facility. After 14 weeks of high-fat diet (HFD) induction, compound 2 was administered at a dose of 25 mg/kg once, and samples were collected after 8 hours to detect the level of 20-HETE. A solvent control group was also set up simultaneously, with 3 animals per group.

### 2. Experimental results

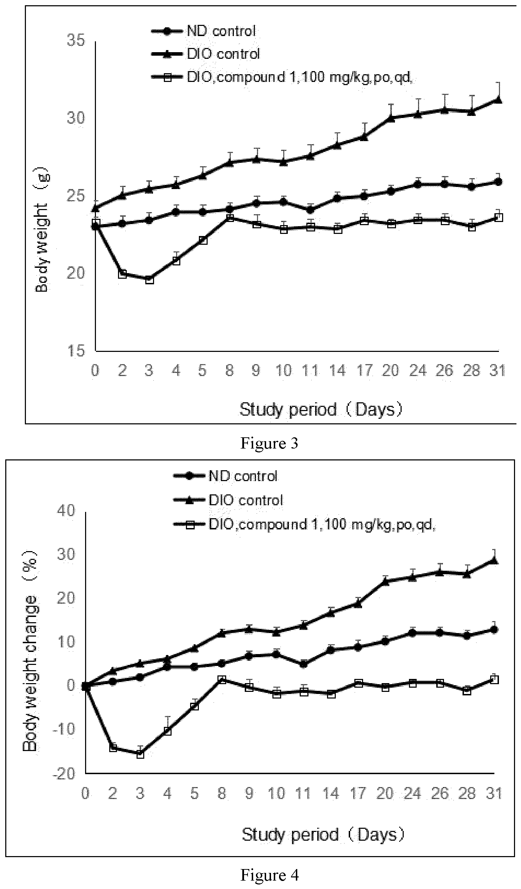

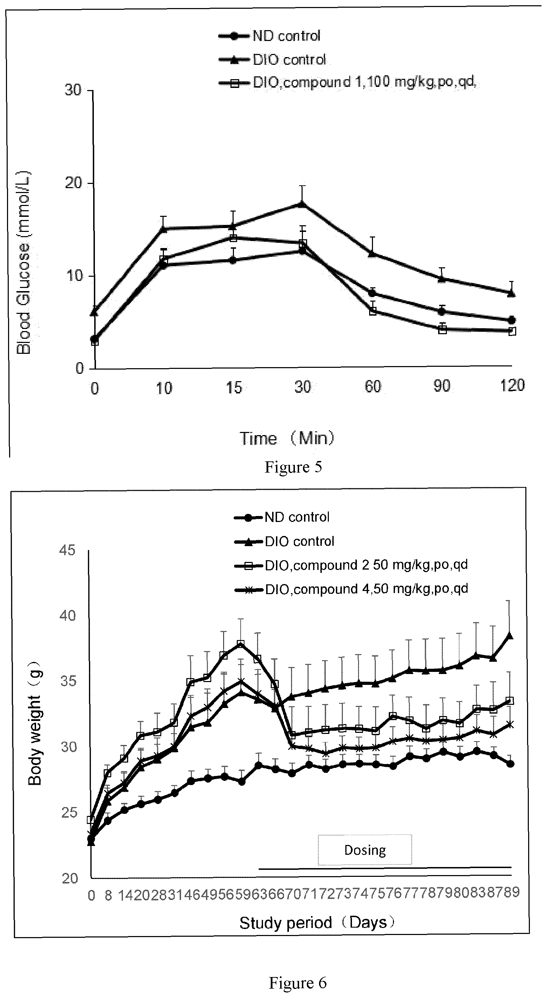

Experiment 1: There was a rapid decrease in body weight in the treatment group animals during the first 3 days of the experiment, but recovery began from the 4th day. According to the data: the body weight of the treatment group stabilized around the initial weight after 1 week and remained stable thereafter; the body weight of the normal control group showed a slight but continuous increase; the body weight of the DIO model control group continued to increase at a rate higher than that of the normal control group (Figure 3 and Figure 4); long-term administration of compound 1 was able to inhibit the increase in fasting blood glucose in DIO mice and enhance glucose metabolism (Figure 5).

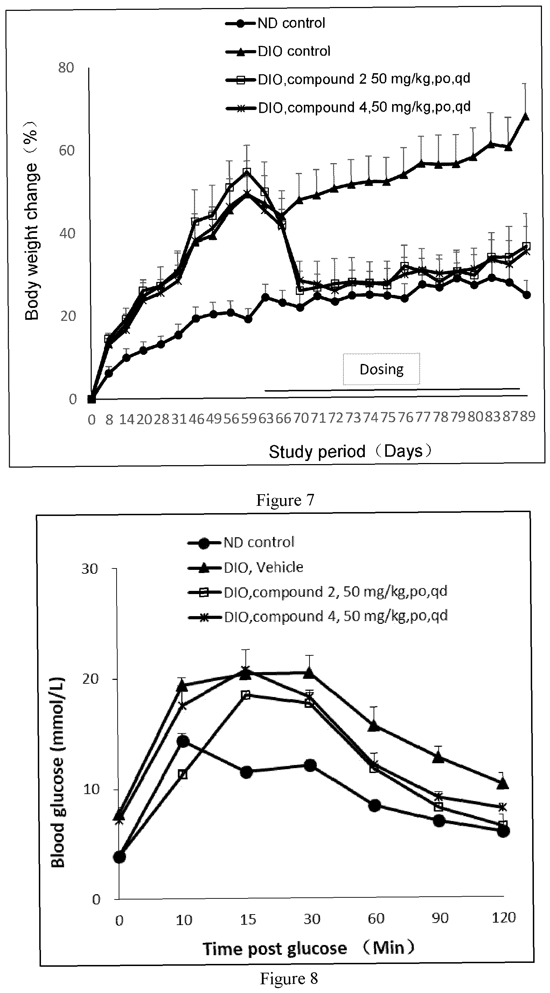

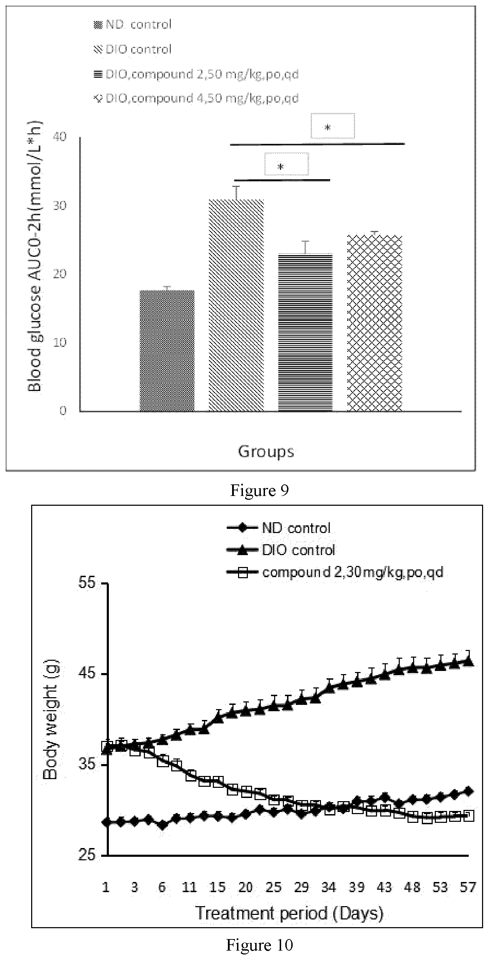

Experiment 2: The body weight of animals in each group continued to increase before dosing, with the increase in DIO mice being significantly greater than that in mice on a normal diet. Administration began on day 63, and during the first week of administration, the body weight of the animals in the two dosing groups rapidly decreased by 12%-16% of the pre-administration weight. From the second week onwards, the body weight of the animals tended to stabilize and remained stable thereafter (Figure 6 and Figure 7); long-term administration of compound 2 and compound 4 could inhibit the increase in fasting blood glucose in DIO mice, and enhance glucose metabolism (Figure 8 and Figure 9).

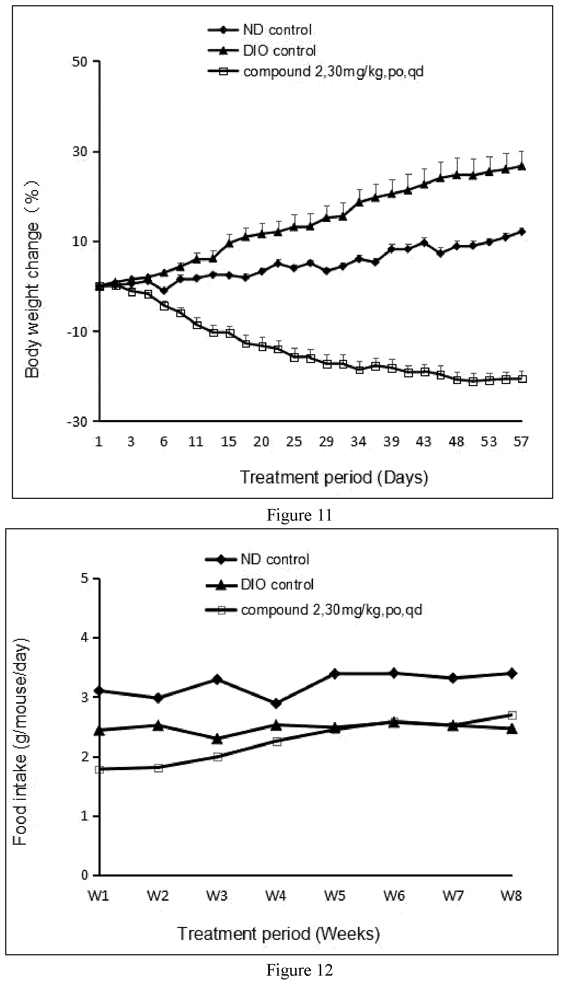

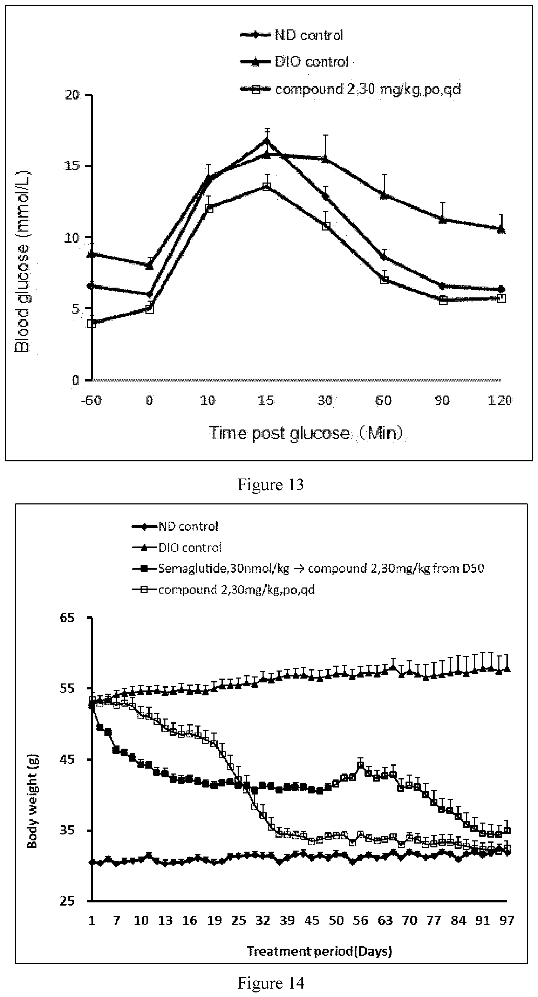

Experiment 3: The body weight of animals in each group continued to increase before dosing, with the increase in body weight of DIO mice being significantly greater than that of mice on a normal diet (a 28.9% difference in body weight between DIO mice and normal mice). Administration began on day 63 and continued for 8 weeks. The results showed that after 8 weeks of continuous administration of compound 2, the body weight of the mice decreased by 20.8% of their pre-administration weight, with a 47.1% difference in weight gain relative to the DIO model control group. The food intake of animals in the compound 2 treatment group decreased in the first 2 weeks of administration, but there was no difference in food intake compared to the DIO model control group after 2 weeks. Long-term administration of compound 2 was able to inhibit the increase in fasting blood glucose in DIO mice and enhance their glucose metabolism (Figure 10, Figure 11, Figure 12, and Figure 13).

Experiment 4: Semaglutide reached its maximum weight-loss effect after 2 weeks of administration, with a decrease of approximately 20% compared to the pre-treatment weight, and the weight was maintained for the subsequent 5 weeks. Starting from week 8, the group of animals was switched to treatment with compound 2. After 3 weeks of compound 2 administration, the body weight of the animals showed a decreasing trend, and after 7 weeks of compound 2 treatment, the body weight decreased by 33.6% of their pre-administration weight. During the first 2 weeks of semaglutide administration, the animals' food intake decreased significantly (approximately 1/3 to 1/2 of the model control group), followed by recovery, but it never fully recovered to the level of the control group. After 6 weeks of compound 2 administration, the maximum weight loss effect was achieved, with a decrease of approximately 36% compared to their pre-administration weight. The weight was maintained for the subsequent 8 weeks of administration, reaching the level of the body weight of the animals in the normal control group. Food intake of the animals slightly decreased after compound 2 administration, and returned to the level of the control group in the 6th week of administration (total 14 weeks). In this experiment, compound 2 exhibited a greater weight loss effect than semaglutide in the DIO mouse model, and switching to compound 2 after resistance to semaglutide continued to reduce the body weights of the animals.

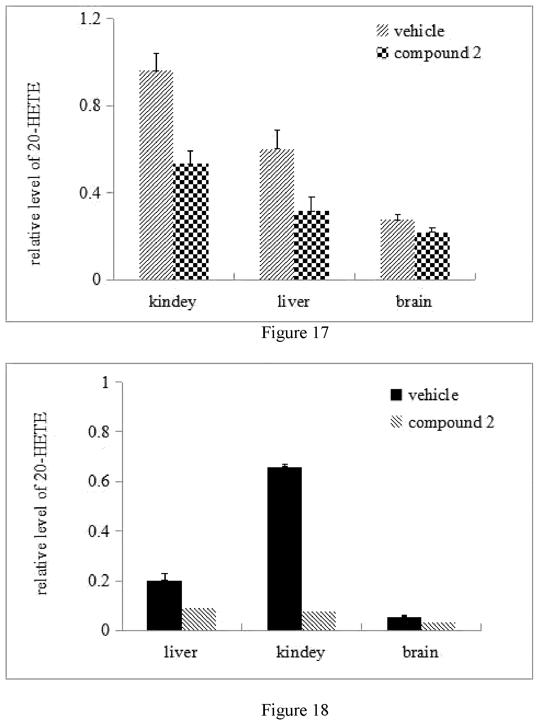

Experiment 5: In DIO mice, a single oral dose of compound 2 resulted in varying degrees of decrease in 20-HETE levels in the liver, kidneys, and brain tissues, compared to the solvent control group. The decrease was most pronounced in the liver and kidneys (Figure 17).

Experiment 6: In DIO rats, a single oral dose of compound 2 resulted in varying degrees of decrease in 20-HETE levels in the liver, kidneys, and brain tissues, compared to the solvent control group. The decrease was most pronounced in the kidneys (Figure 18).

The technical features of the embodiments above can be combined arbitrarily. To simplify description, all possible combinations of the technical features of the embodiments above are not described. However, as long as there is no contradiction in the combination of these technical features, they should be considered as falling within the scope of this specification.

The embodiments above only express several implementations of the present disclosure. The descriptions of the embodiments are relatively specific and detailed, but may not be construed as the limitation on the patent scope of the present disclosure. It should be noted that a person of ordinary skill in the art may make several variations and improvements without departing from the concept of the present disclosure. These variations and improvements all fall within the protection scope of the present disclosure. Therefore, the patent protection scope of the present disclosure shall be defined by the appended claims.

## Claims

1. A 1,4-diheterocyclic substituted aromatic ring or aromatic heterocyclic compound with a structure shown in formula (I) or its pharmaceutically acceptable salts or stereoisomers 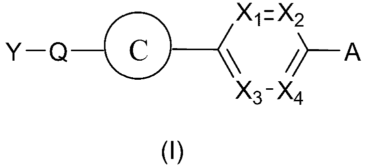
wherein, X₁, X₂, X₃, and X₄ are independently selected from: N, CH, CR₁;
each R₁ is independently selected from: halogen, C₁-C₆ alkyl, C₂-C₆ alkenyl, C₂-C₆ alkynyl, C₃-C₈ cycloalkyl, C₃-C₈ cycloalkyl methyl, halogen-substituted C₁-C₆ alkyl, hydroxyl-substituted C₁-C₆ alkyl, C₁-C₆ alkoxy-substituted C₁-C₆ alkyl, amino-substituted C₁-C₆ alkyl, C₁-C₆ alkylamine-substituted C₁-C₆ alkyl, C₆-C₁₀ aryl, 5-10 membered heteroaryl, nitro, cyano, -OR, -N(R)₂, -SR, -C(O)OR, -C(O)N(R)₂, -C(O)R, -S(O)R, -S(O)₂R, -S(O)₂N(R)₂, -N(R)C(O)R;
Q is selected from: -(CR₂R₃)ₚ-, -C(O)-, -C(S)-, -S(O)-, -S(O)₂-, -C(O)O-, -N(R₄)C(O)-, -C(O)N(R₄)-, -C(O)N(R₄)(CR₂R₃)-, -N(R₄)C(O)(CR₂R₃)-,-C(O)(CR₂R₃)-, -O-, -N(R₄)-, -S-, wherein, P is selected from: 0, 1, or 2; R₂, R₃, and R₄ are independently selected from: H, C₁-C₆ alkyl ;
A is selected from: substituted or unsubstituted 5-10 membered heterocyclic, substituted or unsubstituted 5-10 membered heteroaryl;
Y is selected from: C₁-C₁₂ alkyl, C₂-C₈ alkenyl, C₂-C₈ alkynyl, C₃-C₈ cycloalkyl methyl, halogen-substituted C₁-C₁₂ alkyl, hydroxyl-substituted C₁-C₁₂ alkyl, C₁-C₆ alkoxy-substituted C₁-C₁₂ alkyl, amino substituted C₁-C₁₂ alkyl, C₁-C₆ alkylamine-substituted C₁-C₁₂ alkyl, C₆-C₁₀ aryl-substituted C₁-C₁₂ alkyl, 5-10 membered heteroaryl-substituted C₁-C₁₂ alkyl, acetylene-substituted C₁-C₁₂ alkyl, ethylene-substituted C₁-C₁₂ alkyl, 5-10-membered heteroaryl, -C(O)R, -C (O)OR; or Y is selected from: substituted or unsubstituted 4-12-membered single or double rings, wherein the single or double ring is saturated, partially unsaturated, or aromatic single ring, fused ring, bridged ring, or spiro ring, and the atoms on the ring of the single or double ring are chemically acceptable combinations selected from one or more of C, O, N, and S;
each R is independently selected from: H, C₁-C₆ alkyl, C₂-C₆ alkenyl, C₂-C₆ alkynyl, C₃-C₈ cycloalkyl, C₃-C₈ cycloalkyl methyl, halogen-substituted C₁-C₆ alkyl, hydroxyl-substituted C₁-C₆ alkyl, C₁-C₆ alkoxy-substituted C₁-C₆ alkyl, amino-substituted C₁-C₆ alkyl, C₁-C₆ alkylamine-substituted C₁-C₆ alkyl, C₆-C₁₀ aryl;
Ring C is selected from: substituted or unsubstituted 4-10-membered heterocyclyl , substituted or unsubstituted 5-10-membered heteroaryl.

2. The 1,4-diheterocyclic substituted aromatic ring or aromatic heterocyclic compound or its pharmaceutically acceptable salts or stereoisomers according to claim 1, wherein Ring C is selected from: substituted or unsubstituted 4-10 membered heterocyclyl , and the heteroatoms in the heterocyclyl are 1-2 nitrogen atoms; the substituents in the heterocyclyl are selected from: H, halogen, C₁-C₆ alkyl, halogen-substituted C₁-C₆ alkyl, C₁-C₆ alkoxy-substituted C₁-C₆ alkyl, cyano, hydroxyl; or two substituents together with their linked C atoms form substituted or unsubstituted 4-8-membered carbon rings or 4-8-membered heterocycles.

3. The 1,4-diheterocyclic substituted aromatic ring or aromatic heterocyclic compound or its pharmaceutically acceptable salts or stereoisomers according to claim 2, wherein Ring C is selected from: 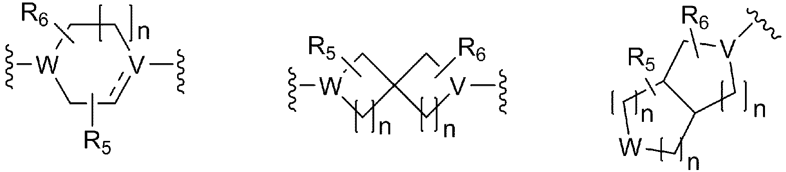 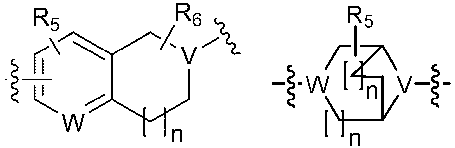 wherein,
the dashed line in the rings containing W and V indicates that it is a single bond or absent;
each R₅ and R₆ are independently selected from: H, halogen, C₁-C₆ alkyl, halogen-substituted C₁-C₆ alkyl, C₁-C₆ alkoxy-substituted C₁-C₆ alkyl, cyano, hydroxyl; Or R₅, R₆ together with their linked C atoms form substituted or unsubstituted 4-8-membered carbon rings or 4-8-membered heterocycles;
each n is independently selected from: 0, 1, 2;
V and W are independently selected from: CH, N, and V and W cannot be both CH.

4. The 1,4-diheterocyclic substituted aromatic ring or aromatic heterocyclic compound or its pharmaceutically acceptable salts or stereoisomers according to claim 3, wherein Ring C together with Q form the following groups:

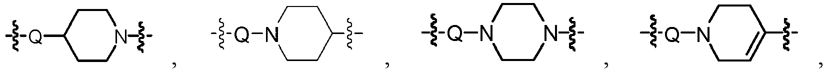

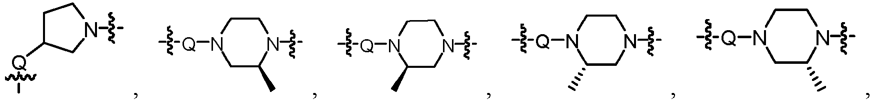

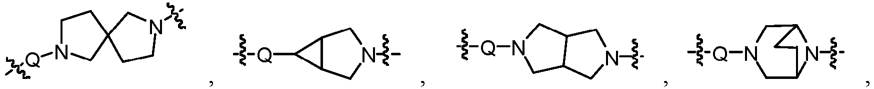

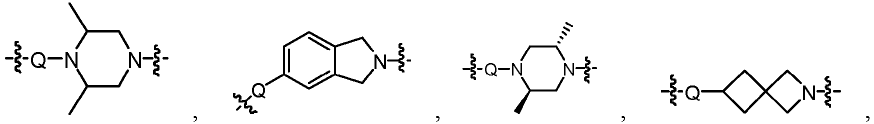

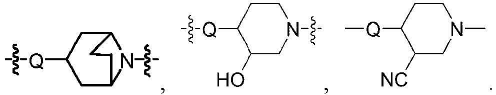

5. The 1,4-diheterocyclic substituted aromatic ring or aromatic heterocyclic compound or its pharmaceutically acceptable salts or stereoisomers according to claim 3, wherein the 1,4-diheterocyclic substituted aromatic ring or aromatic heterocyclic compound has a structure shown in formula (II)

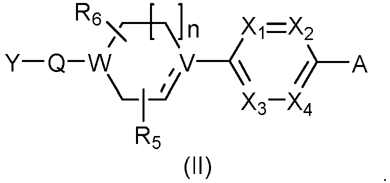

6. The 1,4-diheterocyclic substituted aromatic ring or aromatic heterocyclic compound or its pharmaceutically acceptable salts or stereoisomers according to claim 1, wherein A is selected from: 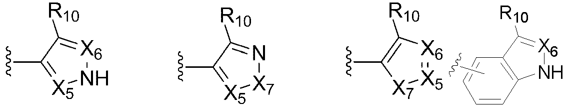 wherein,
X₅ and X₆ are independently selected from: CHN;
X₇ is selected from: O, S;
R₁₀ is selected from: H, C₁-C₆ alkyl.

7. The 1,4-diheterocyclic substituted aromatic ring or aromatic heterocyclic compound or its pharmaceutically acceptable salts or stereoisomers according to claim 6, wherein A is selected from:

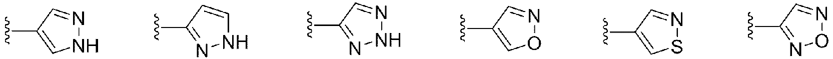

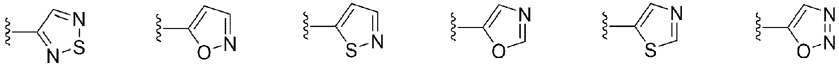

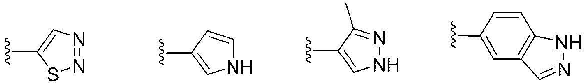

8. The 1,4-diheterocyclic substituted aromatic ring or aromatic heterocyclic compound or its pharmaceutically acceptable salts or stereoisomers according to claim 7, wherein A is selected from:

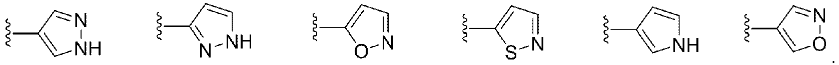

9. The 1,4-diheterocyclic substituted aromatic ring or aromatic heterocyclic compound or its pharmaceutically acceptable salts or stereoisomers according to any one of claims 1 to 8, wherein Y is selected from: C₁-C₁₀ alkyl, halogen-substituted C₁-C₆ alkyl, hydroxyl substituted C₁-C₆ alkyl, C₁-C₃ alkoxy-substituted C₁-C₆ alkyl, phenyl substituted C₁-C₆ alkyl, acetylene-substituted C₁-C₆ alkyl, ethylene-substituted C₁-C₆ alkyl, C₃-C₆ cycloalkyl methyl, 5-6-membered heteroaryl; or Y is selected from: 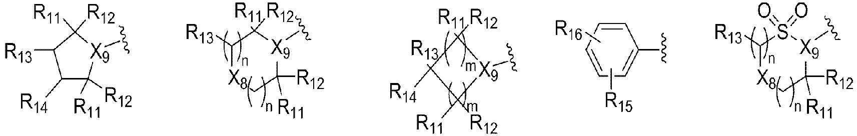
wherein, each X₈ is independently selected from: O, S, NR₁₀, CHR₁₄;
each X₉ is independently selected from: CR₁₈, N; R₁₈ is selected from: H, hydroxyl, C₁-C₆ alkyl, C₁-C₆ alkoxy, halogen;
each m is independently selected from: 1, 2, 3;
each n is independently selected from: 0, 1, 2;
each R₁₀ is independently selected from: H, C₁-C₆ alkyl, C₁-C₆ alkyl acyl;
each R₁₁ and R₁₂ are independently selected from: H, C₁-C₆ alkyl, hydroxyl, halogen; or each pair of R₁₁ and R₁₂ independently and selectively together with their linked C atoms form C=O ;
each R₁₃ and R₁₄ are independently selected from: H, C₁-C₆ alkyl, C₁-C₆ alkoxy, C₃-C₈ cycloalkyl, 3-8-membered heterocyclyl, hydroxy-substituted C₁-C₆ alkyl, hydroxyl, halogen, amino, C₁-C₆ alkyl acyl; or R₁₃, R₁₄ on the same carbon atoms together with their linked C atoms form C=O; or R₁₃, R₁₄ together with their linked C atoms form one or more R₁₇ substituted or unsubstituted C₃-C₈ cycloalkyl, C₆-C₁₀ aryl, 3-8-membered heterocyclyl, or 5-6-membered heteroaryl; or R₁₁, R₁₃ together with their linked C atoms form one or more R₁₇ substituted or unsubstituted C₃-C₈ cycloalkyl or 3-8-membered heterocyclyl;
R₁₅ and R₁₆ are independently selected from: H, halogen, C₁-C₆ alkyl, C₂-C₆ alkenyl, C₂-C₆ alkynyl, C₃-C₈ cycloalkyl, C₃-C₈ cycloalkyl methyl, halogen substituted C₁-C₆ alkyl, hydroxyl substituted C₁-C₆ alkyl, C₁-C₆ alkoxy substituted C₁-C₆ alkyl, amino substituted C₁-C₆ alkyl, C₁-C₆ alkylamine substituted C₁-C₆ alkyl, C₆-C₁₀ aryl, 5-10-membered heteroaryl, nitro, cyano, -OR, -N(R)₂, -SR, -C(O)OR, -C(O)N(R)₂, -C(O)N(R)₂, -C(O)O)R, -S(O)R, -S(O)₂R, -S(O)₂N(R)₂, -N(R)C(O)R; or adjacent R₁₅, R₁₆ together with their linked C atoms form one or more R₁₇ substituted or unsubstituted C₃-C₈ cycloalkyl, C₆-C₁₀ aryls, 3-8-membered heterocyclyl, or 5-6-membered heteroaryl;
each R₁₇ is independently selected from: halogen, hydroxyl, cyano, C₁-C₆ alkyl, C₁-C₆ alkoxy, C₁-C₆ alkyl acyl, or two R₁₇ atoms on the same carbon atom together with their linked carbon atoms form C=O.

10. The 1,4-diheterocyclic substituted aromatic ring or aromatic heterocyclic compound or its pharmaceutically acceptable salts or stereoisomers according to claim 9, wherein Y is selected from: C₁-C₈ alkyl, hydroxy-substituted C₁-C₆ alkyl, phenyl-substituted C₁-C₆ alkyl, acetylene-substituted C₁-C₆ alkyl, vinyl-substituted C₁-C₆ alkyl, C₅-C₆ cycloalkyl methyl; or, Y is selected from: 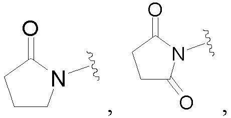 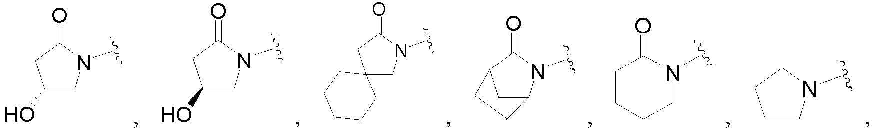 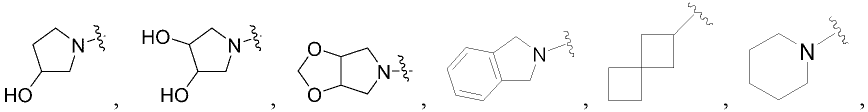 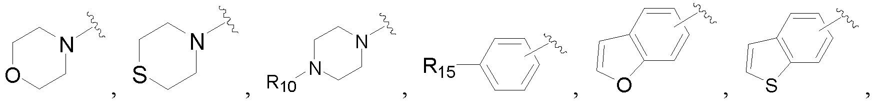 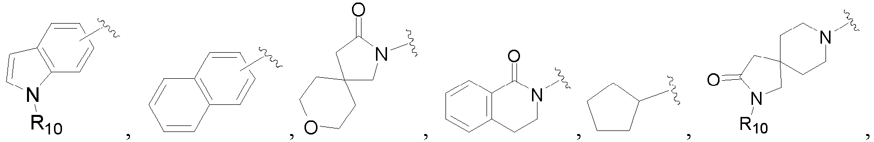 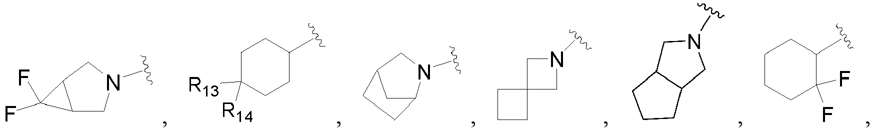 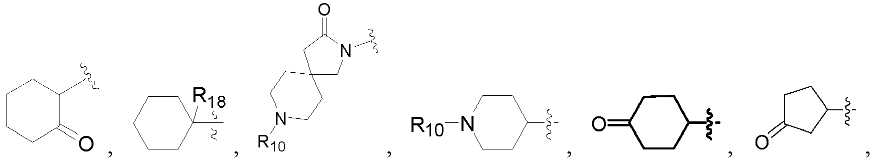 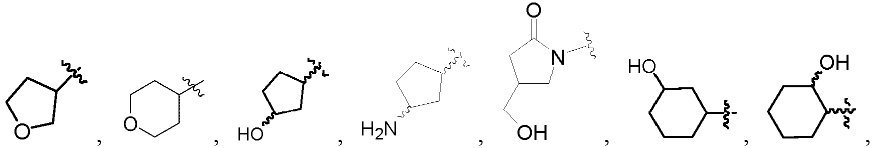 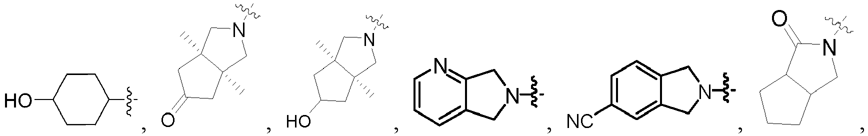 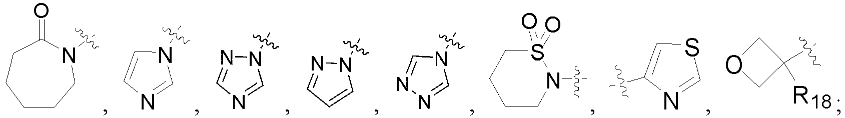 wherein
each R₁₀ is independently selected from: H, C₁-C₃ alkyl, C₁-C₃ alkyl acyl;
each R₁₃ and R₁₄ are independently selected from: H, halogen, C₁-C₆ alkyl, C₁-C₆ alkoxy, hydroxyl;
R₁₅ is selected from: H, halogen, C₁-C₃ alkyl, C₁-C₃ alkoxy;
each R₁₈ is independently selected from: H, hydroxyl, halogen, C₁-C₃ alkoxy, C₁-C₃ alkyl.

11. The 1,4-diheterocyclic substituted aromatic ring or aromatic heterocyclic compound or its pharmaceutically acceptable salts or stereoisomers according to claim 10, wherein Y is selected from: C₄-C₇ alkyl, cyclopentyl, naphthyl, hydroxyl substituted C₄-C₆ alkyl, phenyl-substituted C₁-C₆ alkyl, acetylene-substituted C₄-C₆ alkyl, ethylene-substituted C₄-C₆ alkyl, C₅-C₆ cycloalkyl methyl; or, Y is selected from: 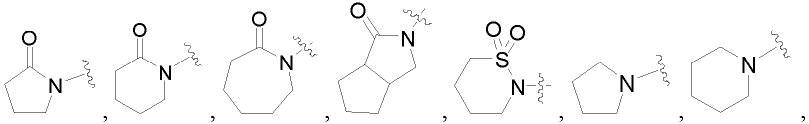 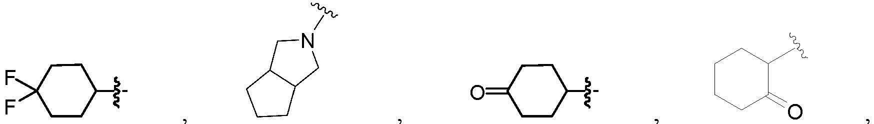 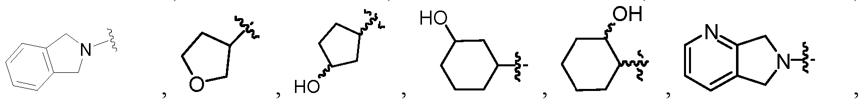 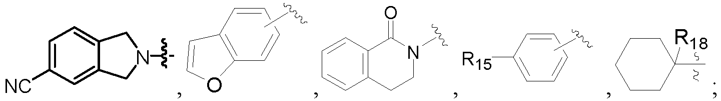 wherein R₁₅ is selected from: H or halogen; R₁₈ is selected from: H, hydroxyl, halogen, C₁-C₃ alkoxy.

12. The 1,4-diheterocyclic substituted aromatic ring or aromatic heterocyclic compound or its pharmaceutically acceptable salts or stereoisomers according to any one of claims 1 to 8, wherein Q and Y form the following groups: -Y-S(O)₂-, -Y-(CR₂R₃)ₚ-, -Y-C(O)-, -Y-N(R₄)C(O)-, -Y-C(O)N(R₄)-, -Y-C(O)N(R₄)(CR₂R₃)-, or -Y-N(R₄)C(O)(CR₂R₃)-, wherein p is selected from 0, 1 or 2; R₂, R₃, and R₄ are all H.

13. The 1,4-diheterocyclic substituted aromatic ring or aromatic heterocyclic compound or its pharmaceutically acceptable salts or stereoisomers according to claim 12, wherein Q and Y form -Y-CH₂-, wherein Y is selected from:

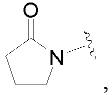

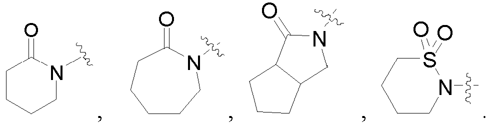

14. The 1,4-diheterocyclic substituted aromatic ring or aromatic heterocyclic compound or its pharmaceutically acceptable salts or stereoisomers according to claim 12 , wherein Q and Y form -Y-C=O, wherein Y is selected from cyclopentyl, cyclohexyl, benzyl, hydroxy substituted butyl, cyclopentyl methyl,

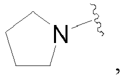

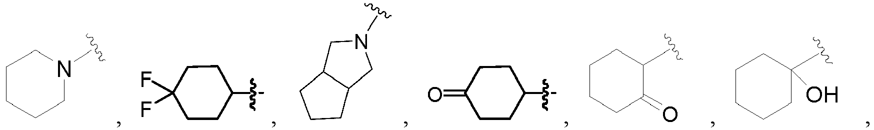

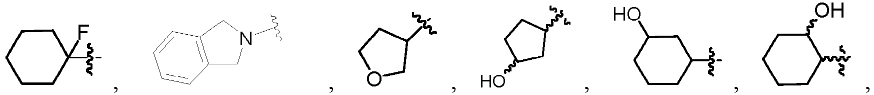

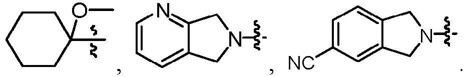

15. The 1,4-diheterocyclic substituted aromatic ring or aromatic heterocyclic compound or its pharmaceutically acceptable salts or stereoisomers according to claim 12, wherein Q and Y form Y-C(O)NHCH₂-, and Y is selected from butyl, hexyl, pentyl, cyclopentyl, phenyl, phenyl-substituted n-pentyl, acetylene-substituted n-pentyl, vinyl-substituted n-pentyl.

16. The 1,4-diheterocyclic substituted aromatic ring or aromatic heterocyclic compound or its pharmaceutically acceptable salts or stereoisomers according to claim 12, wherein Q and Y form -Y-NHC(O)-, and Y is selected from: cyclopentyl methyl, cyclohexyl, phenyl, 4-fluorophenyl, naphthyl, benzyl, hydroxy substituted butyl,

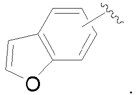

17. The 1,4-diheterocyclic substituted aromatic ring or aromatic heterocyclic compound or its pharmaceutically acceptable salts or stereoisomers according to claim 12, wherein Q and Y form -Y-NHC(O)-, and Y is selected from cyclopentane, and the Ring C together with Q form the following groups:

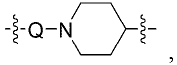

18. The 1,4-diheterocyclic substituted aromatic ring or aromatic heterocyclic 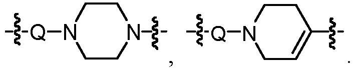 compound or its pharmaceutically acceptable salts or stereoisomers according to claim 12 , wherein Q and Y form -Y-S(O)₂-, wherein Y is selected from: cyclohexyl, 4-fluorophenyl.

19. The 1,4-diheterocyclic substituted aromatic ring or aromatic heterocyclic compound or its pharmaceutically acceptable salts or stereoisomers according to anyone of claims 1 to 8, wherein X₁, X₂, X₃, and X₄ are independently selected from: CH, CR₁.

20. The 1,4-diheterocyclic substituted aromatic ring or aromatic heterocyclic compound or its pharmaceutically acceptable salts or stereoisomers according to claim 19, wherein X₁ and X₄ cannot be both CR₁, and X₂ and X₃ cannot be both CR₁.

21. The 1,4-diheterocyclic substituted aromatic ring or aromatic heterocyclic compound or its pharmaceutically acceptable salts or stereoisomers according to any one of claims 1 to 8, wherein R₁ is selected from: halogen, C₁-C₆ alkyl, halogen-substituted C₁-C₆ alkyl, hydroxyl-substituted C₁-C₆ alkyl, C₁-C₃ alkoxy-substituted C₁-C₆ alkyl, amino-substituted C₁-C₆ alkyl, C₁-C₃ alkylamine-substituted C₁-C₆ alkyl, cyano, C₁-C₆ alkoxy, -N(C₁-C₃ alkyl)₂, -C(O)OC₁-C₃ alkyl, -C(O)NH₂, -C(O)N(C₁-C₃ alkyl)₂, -C(O)(C₁-C₃ alkyl)₂, -C(O)H.

22. The 1,4-diheterocyclic substituted aromatic ring or aromatic heterocyclic compound or its pharmaceutically acceptable salts or stereoisomers according to claim 21, wherein R₁ is selected from: halogen, C₁-C₃ alkyl, halogen-substituted C₁-C₃ alkyl, cyano, formaldehyde, hydroxyl-substituted C₁-C₃ alkyl.

23. The 1,4-diheterocyclic substituted aromatic ring or aromatic heterocyclic compound or its pharmaceutically acceptable salts or stereoisomers according to claim 21, wherein, R₁ is selected from: fluorine, chlorine, monofluoromethyl, difluoromethyl, trifluoromethyl, methyl, cyano, methoxy, formaldehyde, aminoformyl, hydroxymethyl, methoxycarbonyl, dimethylamino.

24. The 1,4-diheterocyclic substituted aromatic ring or aromatic heterocyclic compound or its pharmaceutically acceptable salts or stereoisomers according to claim 1, wherein the 1,4-diheterocyclic substituted aromatic ring or aromatic heterocyclic compound is selected from the following compounds:

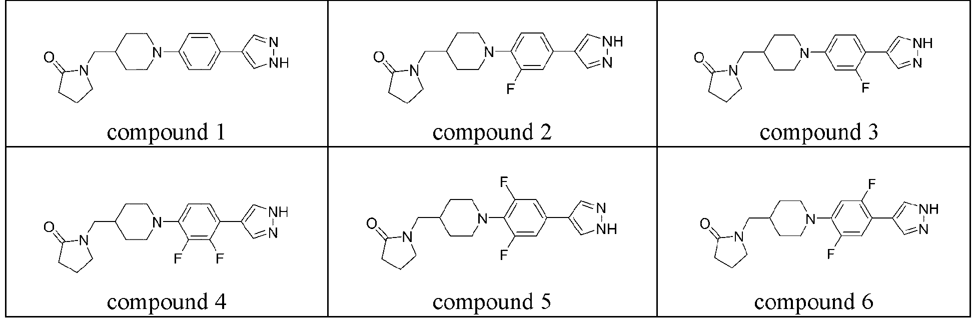

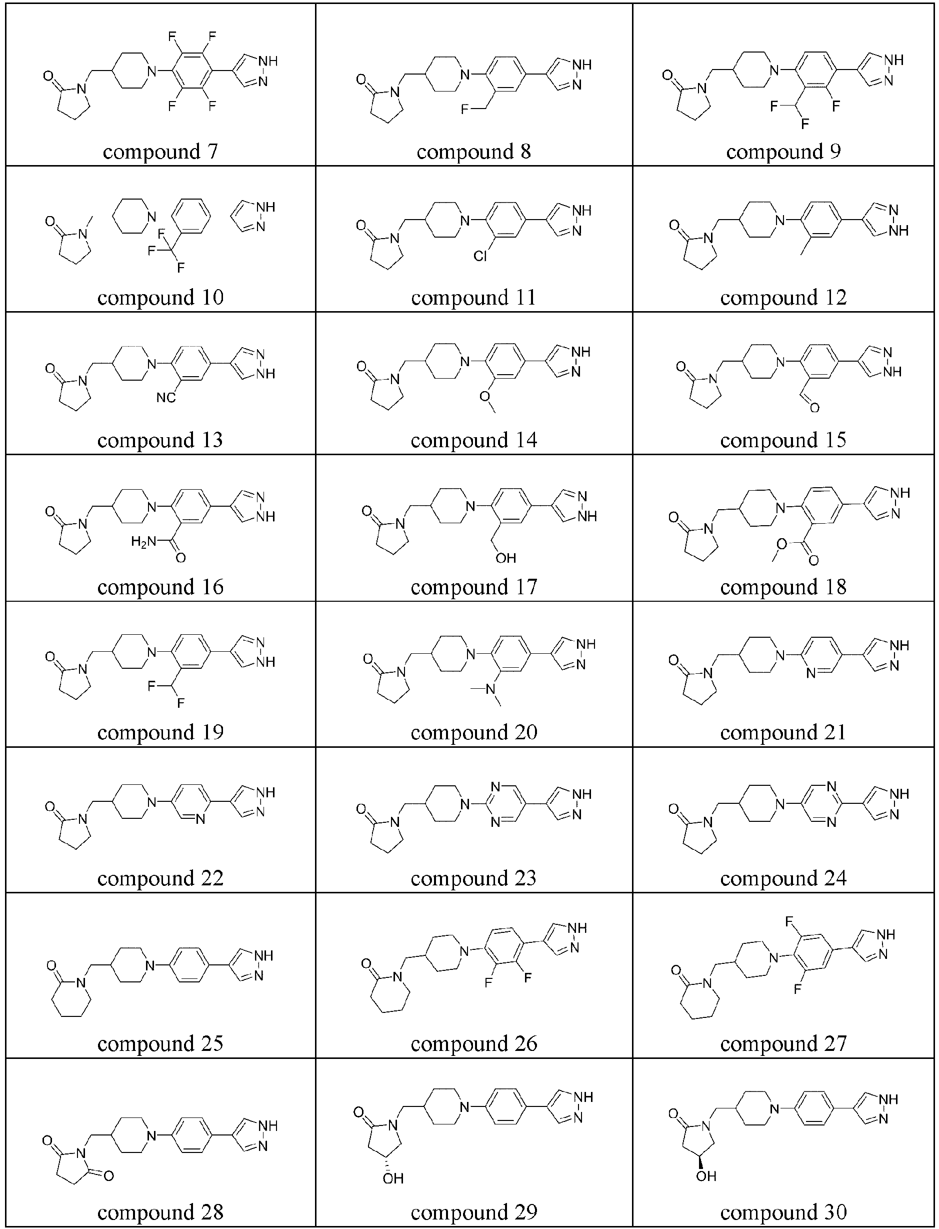

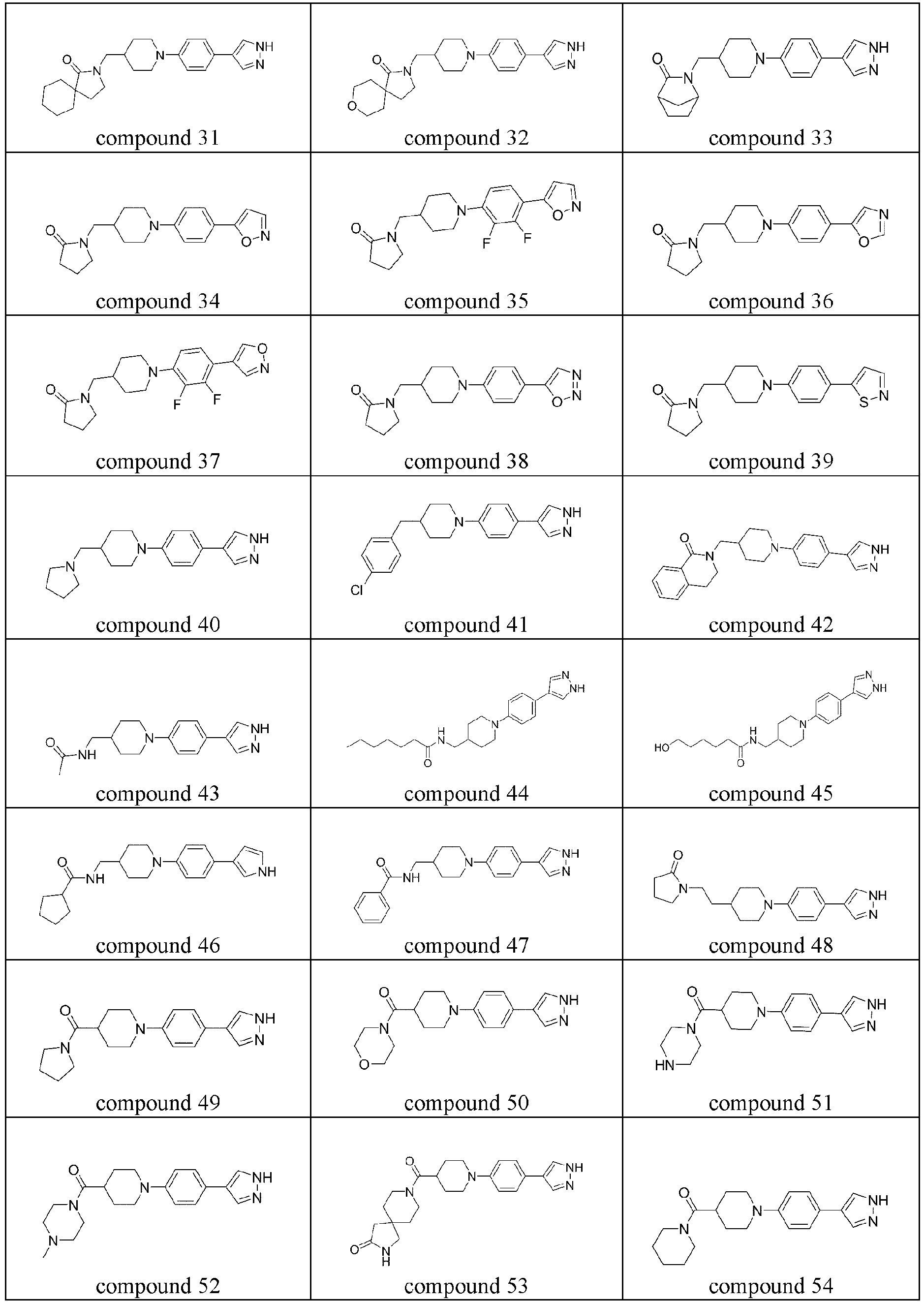

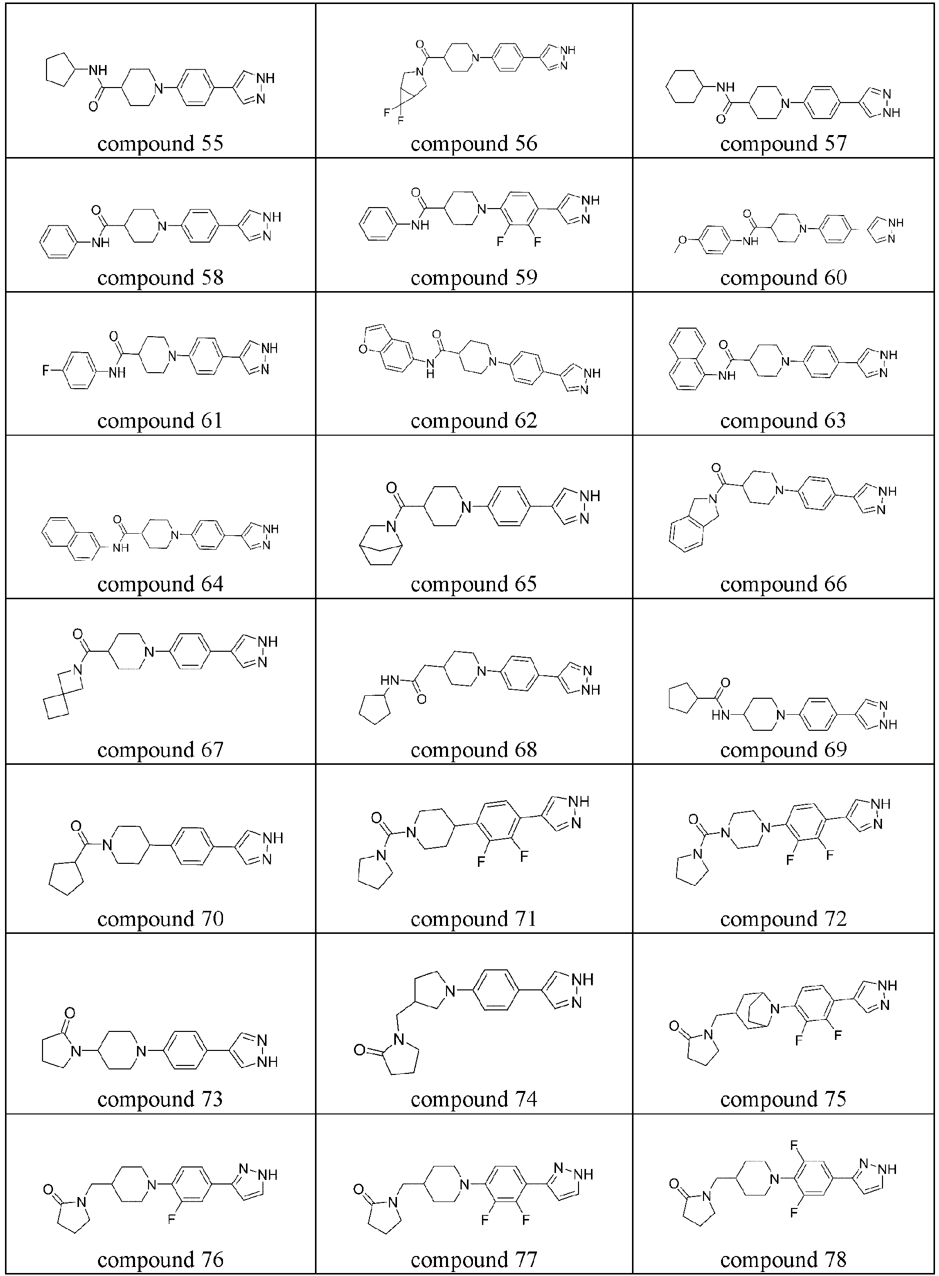

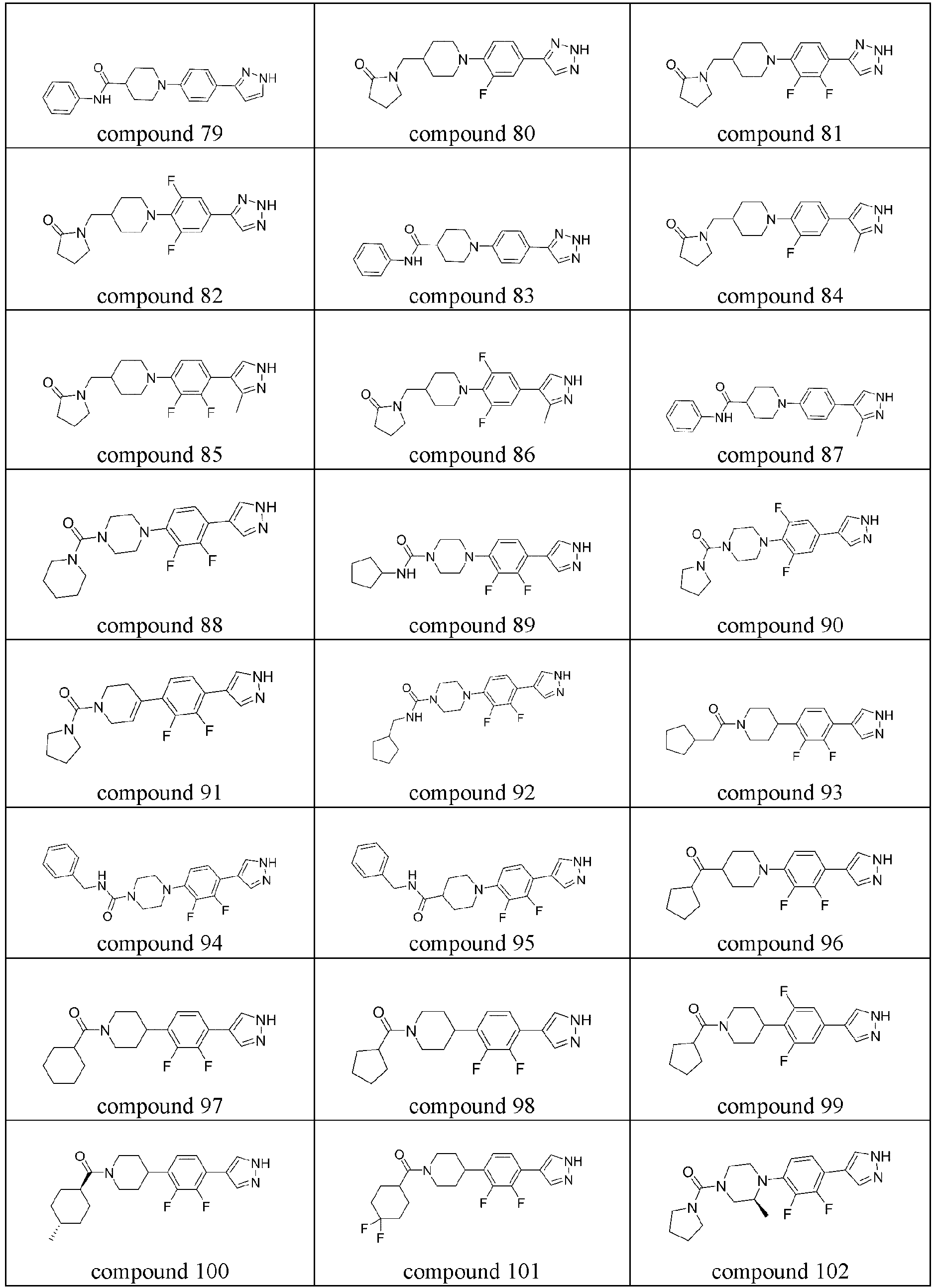

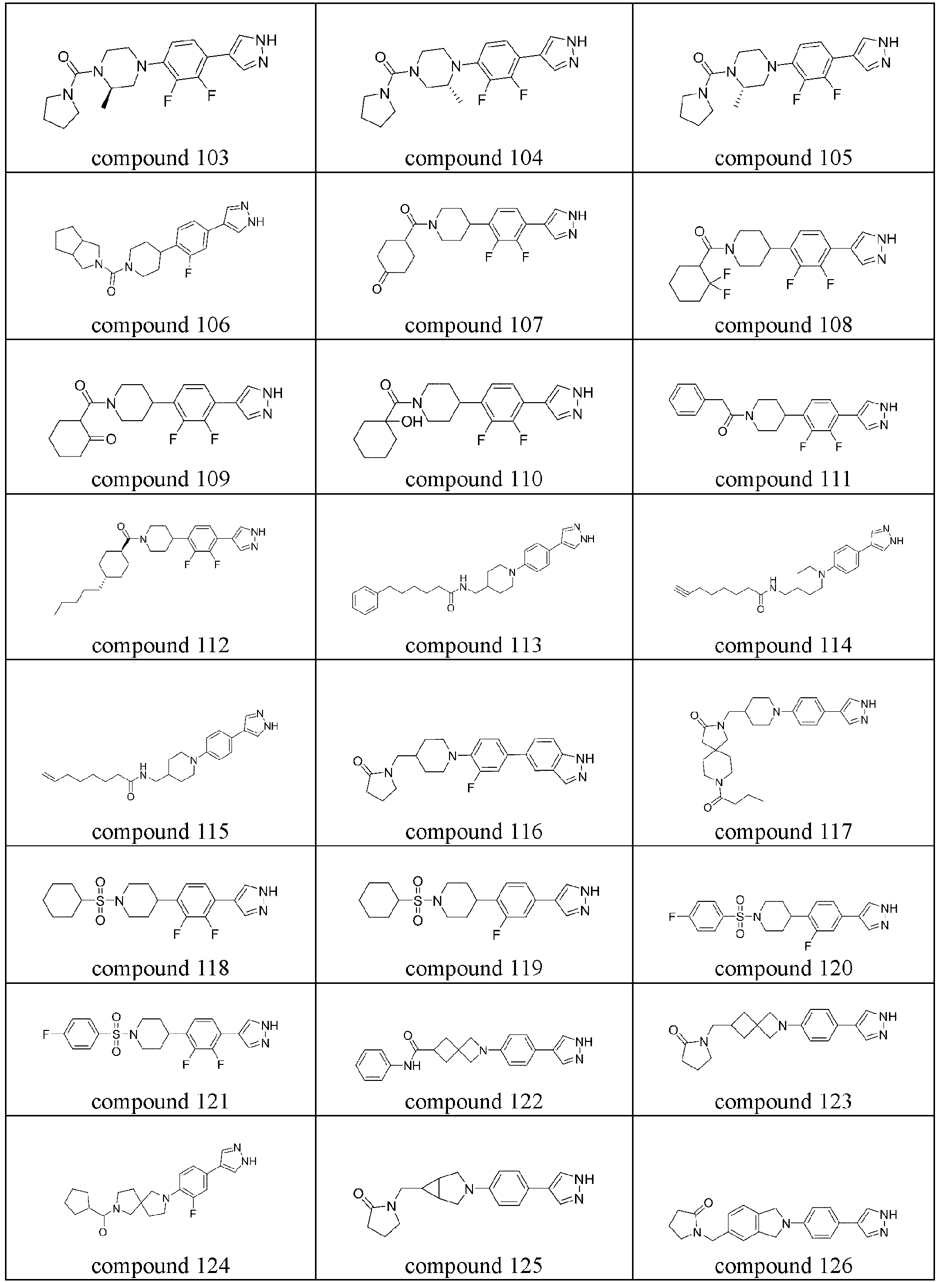

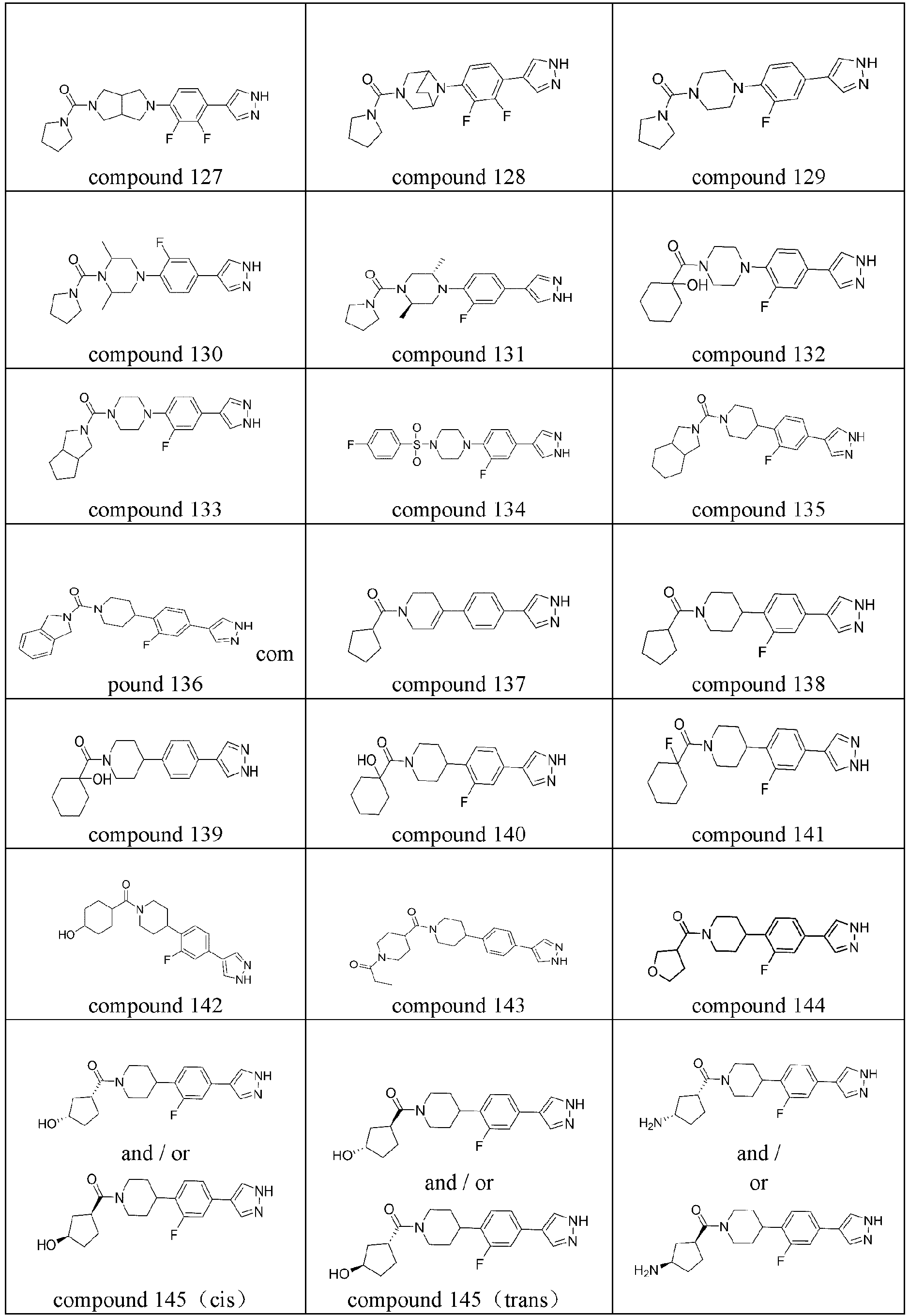

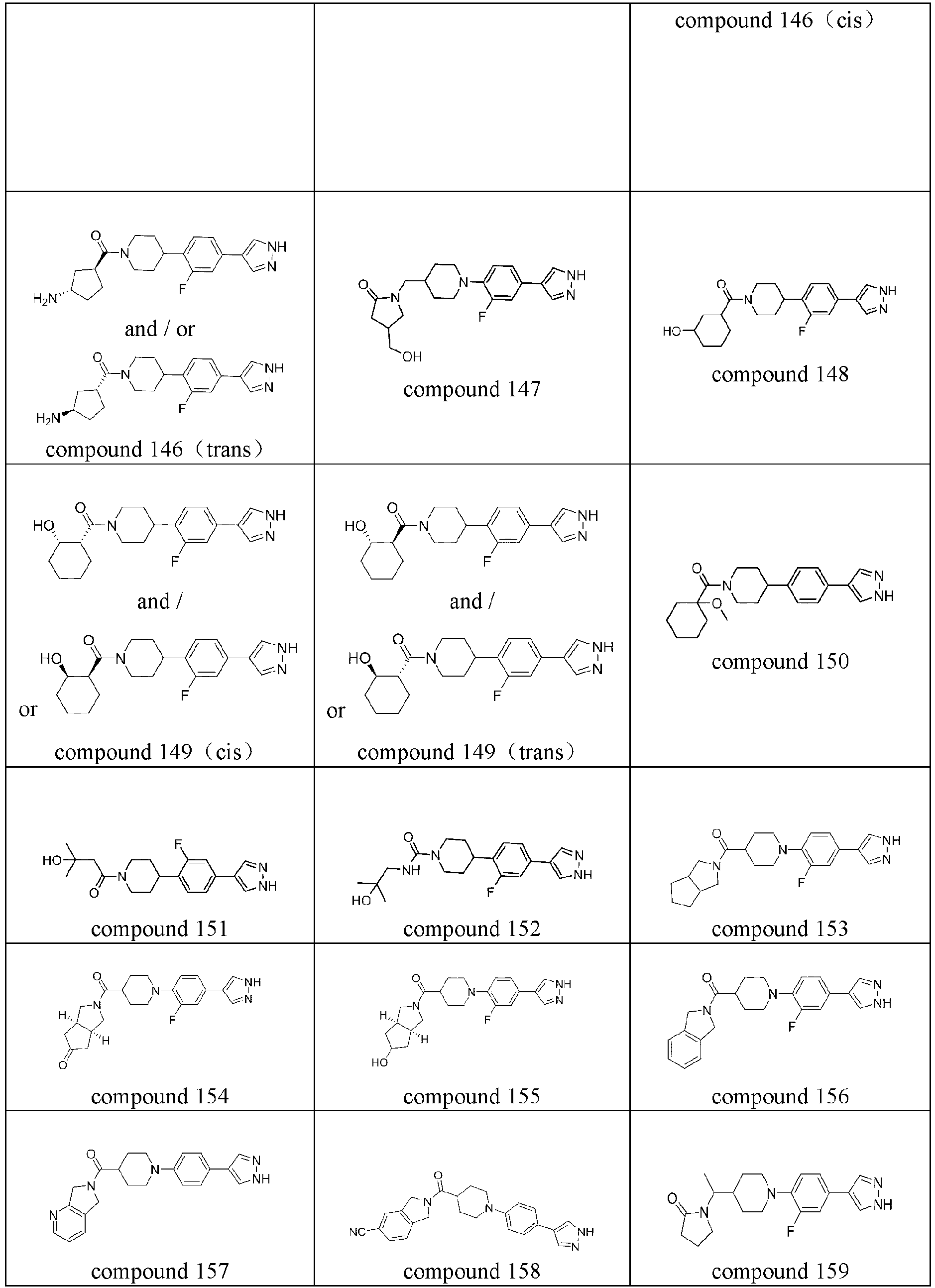

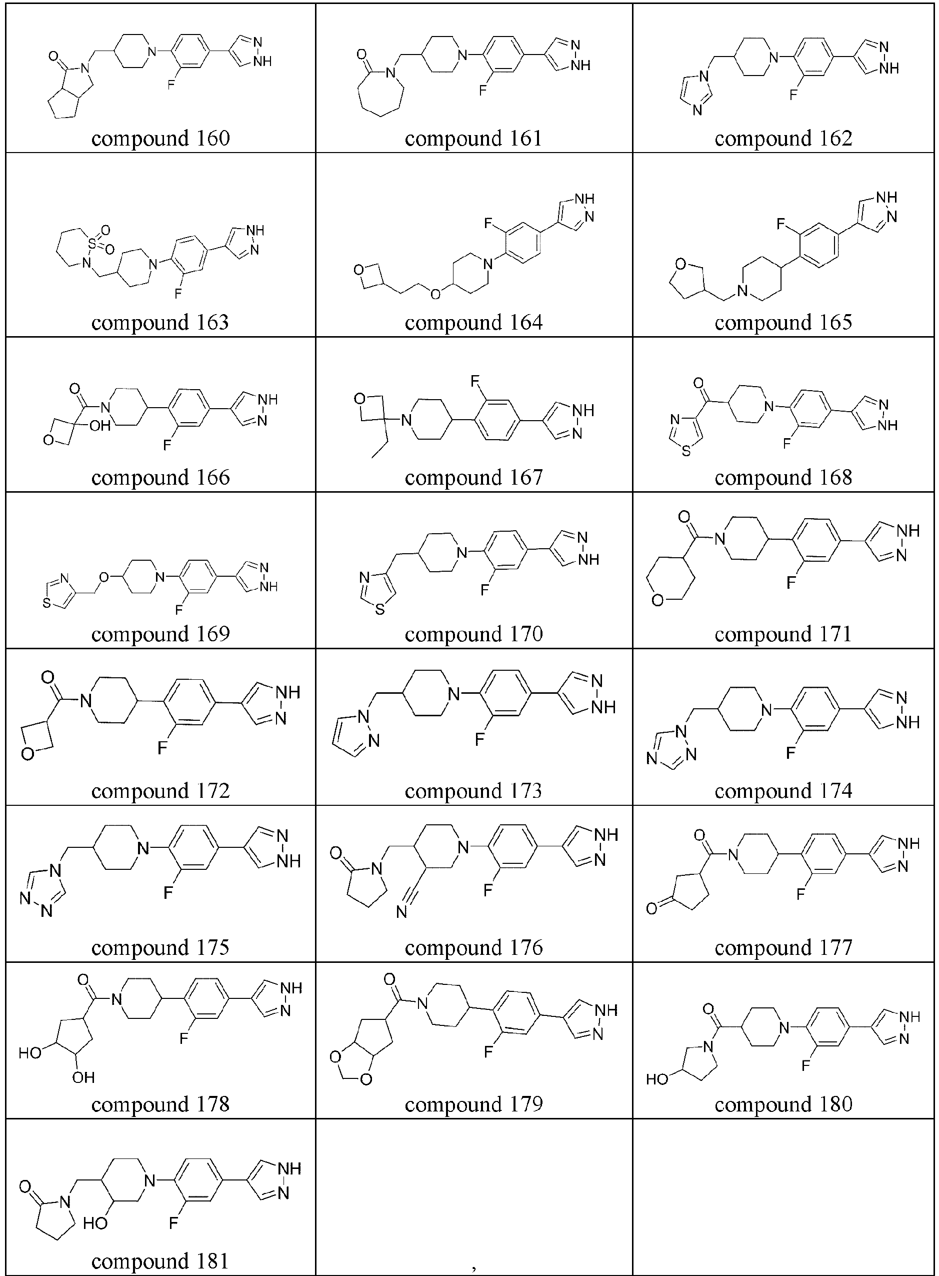

25. An application of the 1,4-diheterocyclic substituted aromatic ring or aromatic heterocyclic compound or its pharmaceutically acceptable salts or stereoisomers according to anyone of claims 1 to 24 in the preparation of 20-HETE generation inhibitors.

26. An application of the 1,4-diheterocyclic substituted aromatic ring or aromatic heterocyclic compound or its pharmaceutically acceptable salts or stereoisomers according to anyone of claims 1 to 24 in the prevention and/or treatment of diseases and/or symptoms related to the 20-HETE signaling pathway.

27. An application of GLP-1 receptor agonists in combination with the 1,4-diheterocyclic substituted aromatic ring or aromatic heterocyclic compound or its pharmaceutically acceptable salts or stereoisomers according to any one of claims 1 to 24 in the prevention and/or treatment of diseases and/or symptoms related to the 20-HETE signaling pathway.

28. The application according to claim 27, wherein the GLP-1 receptor agonist is semaglutide.

29. The application according to any one of claims 26 to 28, wherein the diseases and/or symptoms related to the 20-HETE signaling pathway are selected from: obesity, metabolic syndrome, dyslipidemia, diabetes, diabetes retinopathy, diabetes cerebrovascular disease, diabetes neuropathy, insulin resistance, hyperglycemia, hyperlipidemia, diabetes nephropathy, hypertension, cataract, osteoporosis, hyperuricemia, multiple infections caused by diabetes, non-alcoholic fatty liver disease, non-alcoholic fatty liver disease, fibrosis, heart disease, stroke, cirrhosis, metabolic acidosis, ketosis, cardiovascular discomfort, epilepsy, atherosclerosis, Parkinson's disease Disease, myocardial infarction, acute renal failure, chronic kidney disease, polycystic kidney disease, tumor, end organ damage, Alzheimer's disease.

30. The application according to claim 29, wherein the diabetes is type 1 diabetes mellitus(T1DM), type 2 diabetes mellitus(T2DM), gestational diabetes mellitus, idiopathic T1D, early-onset T2DM, maturity-onset diabetes of the young, atypical diabetes in adolescents, malnutrition related diabetes, and latent autoimmune diabetes in adults.

31. A method for preventing and/or treating diseases and/or symptoms related to the 20-HETE signaling pathway, comprising administering an effective amount of the 1,4-diheterocyclic substituted aromatic ring or aromatic heterocyclic compound or its pharmaceutically acceptable salts or stereoisomers according to anyone of claims 1 to 24.

32. The method according to claims 31, wherein the diseases and/or symptoms related to the 20-HETE signaling pathway are selected from: obesity, metabolic syndrome, dyslipidemia, diabetes, diabetes retinopathy, diabetes cerebrovascular disease, diabetes neuropathy, insulin resistance, hyperglycemia, hyperlipidemia, diabetes nephropathy, hypertension, cataract, osteoporosis, hyperuricemia, multiple infections caused by diabetes, non-alcoholic fatty liver disease, non-alcoholic fatty liver disease, fibrosis, heart disease, stroke, cirrhosis, metabolic acidosis, ketosis, cardiovascular discomfort, epilepsy, atherosclerosis, Parkinson's disease Disease, myocardial infarction, acute renal failure, chronic kidney disease, polycystic kidney disease, tumor, end organ damage, Alzheimer's disease.

33. The method according to claim 32, wherein the diabetes is type 1 diabetes mellitus, type 2 diabetes mellitus, gestational diabetes mellitus, idiopathic T1D, early-onset T2DM, maturity-onset diabetes of the young, atypical diabetes in adolescents, malnutrition related diabetes, and latent autoimmune diabetes in adults.

34. A pharmaceutical composition for the prevention and/or treatment of diseases and/or symptoms associated with the 20-HETE signaling pathway, comprising an active ingredient and pharmaceutically acceptable excipients and/or carriers, wherein the active ingredient comprises the 1,4-diheterocyclic substituted aromatic ring or aromatic heterocyclic compound or its pharmaceutically acceptable salts or stereoisomers according to anyone of claims 1 to 24.

35. A combination drug for the prevention and/or treatment of diseases and/or symptoms related to the 20-HETE signaling pathway, wherein its active ingredient includes a GLP-1 receptor agonist, and the 1,4-diheterocyclic substituted aromatic ring or aromatic heterocyclic compound or its pharmaceutically acceptable salts or stereoisomers according to anyone of claims 1 to 24; wherein, the GLP-1 receptor agonist and the 1,4-diheterocyclic substituted aromatic ring or aromatic heterocyclic compound or its pharmaceutically acceptable salts or stereoisomers according to any one of claims 1 to 24, respectively, are independent drug delivery units or jointly form a combined drug delivery unit.

36. The combination drug according to claim 35, wherein the GLP-1 receptor agonist is semaglutide.
